# EUROPEAN PATENT SPECIFICATION

(11) **EP 2 858 664 B1**
(45) Date of publication and mention of the grant of the patent: **08.05.2019**
(21) Application number: 13712656.1
(22) Date of filing: 14.03.2013
(51) Int. Cl.: A61K 38/27, C07K 14/61, A61P 5/00

(54) **HGH-XTEN FUSION PROTEIN AND ITS USE IN THE TREATMENT OF GROWTH HORMONE DEFICIENCY**
HGH-XTEN-FUSIONSPROTEIN UND DESSEN VERWENDUNG BEI DER BEHANDLUNG VON WACHSTUMSHORMONMANGEL
PROTÉINE DE FUSION HGH-XTEN ET SON UTILISATION DANS LE TRAITEMENT DU DÉFICIT EN HORMONE DE CROISSANCE

(30) Priority: 05.06.2012 US 201261689390 P; 22.06.2012 US 201261663475 P; 12.02.2013 US 201361763753 P
(43) Date of publication of application: 15.04.2015
(73) Proprietor: Amunix Pharmaceuticals, Inc., Mountain View, c (US)
(72) Inventor: CLELAND, Jeffrey L., San Carlos, CA 94070 (US); BRIGHT, George M., San Mateo, CA 94403 (US); HUMPHRISS, Eric, Menlo Park, CA 94025 (US); SCHELLENBERGER, Volker, Palo Alto, CA 94303 (US); SILVERMAN, Joshua, Sunnyvale, CA 94087 (US); STEMMER, Willem P., Los Gatos, CA 95051 (US); WANG, Chia-Wei, Santa Clara, CA 95051 (US); GEETHING, Nathan, Acton, MA 01720 (US); SPINK, Benjamin, San Carlos, CA 94070 (US)
(74) Representative: Mewburn Ellis LLP
(86) International application number: PCT/US2013/031673
(87) International publication number: WO 2013/184216

(56) References cited:
- WO-A1-2010/091122
- WO-A2-2010/144502
- JEFFREY L CLELAND ET AL: "A novel long-acting human growth hormone fusion protein (vrs-317): enhanced in vivo potency and half-life", JOURNAL OF PHARMACEUTICAL SCIENCES, AMERICAN PHARMACEUTICAL ASSOCIATION, WASHINGTON, US, [Online] vol. 101, no. 8, 1 August 2012 (2012-08-01), pages 2744-2754, XP002694804, ISSN: 0022-3549, DOI: 10.1002/JPS.23229 Retrieved from the Internet: URL:http://onlinelibrary.wiley.com/doi/10. 1002/jps.23229/pdf> [retrieved on 2012-06-07]
- Cleland et al.: "A Novel Human Growth Hormone XTEN Construct (VRS-317) for Once-a-Month Subcutaneous Administration in a Phase 1a Study of Growth Hormone Deficient Adults", Endocr Rev, Vol. 32 (03_MeetingAbstracts): P2-371 , June 2011 (2011-06), XP002697386, Retrieved from the Internet: URL:http://edrv.endojournals.org/cgi/conte nt/meeting_abstract/32/03_MeetingAbstracts /P2-371?sid=4b0f2ae6-10e2-41bf-93f5-d7e9fe e99dab [retrieved on 2013-05-17]
- VOLKER SCHELLENBERGER ET AL: "A recombinant polypeptide extends the in vivo half-life of peptides and proteins in a tunable manner", NATURE BIOTECHNOLOGY, NATURE PUBLISHING GROUP, NEW YORK, NY, US, vol. 27, no. 12, 1 December 2009 (2009-12-01), pages 1186-1190, XP002665891, ISSN: 1087-0156, DOI: 10.1038/NBT.1588 [retrieved on 2009-11-15]
- clinicaltrials.gov archive: "VRS-317 in Adult Subjects With Growth Hormone Deficiency", clinicaltrials.gov archive , 23 May 2011 (2011-05-23), XP002697387, Retrieved from the Internet: URL:http://clinicaltrials.gov/archive/NCT0 1359488/2011_05_23 [retrieved on 2013-05-17]
- CLELAND JEFFREY L ET AL: "A novel human growth hormone XTEN construct (VRS-317) for monthly administration", ENDOCRINE JOURNAL, TOKYO, JP, vol. 57, no. Suppl. 2, 1 March 2010 (2010-03-01), page S618, XP002696184, ISSN: 0918-8959

## Description

### SEQUENCE LISTING

The instant application contains a Sequence Listing which has been submitted in ASCII format via EFS-Web. Said ASCII copy, created on March 13, 2013, is named 32808-738.201_SL.txt and is 255,823 bytes in size.

### BACKGROUND OF THE INVENTION

Human growth hormone (hGH) is naturally secreted from the human anterior pituitary as intermittent pulses lasting from minutes to hours typically occurring during sleep. The rate and extent of hGH secretion decreases with aging and is maximal in puberty in normal healthy well nourished children. hGH binds to the hGH receptor initiating signaling processes involving the STAT (signal transducer and activator of transcription), the MAPK (mitogen-activated protein kinase) and the PI3K (phosphoinositide-3 kinase) pathways. Insulin-like growth factor-I (IGF-I) gene expression is activated from hGH receptor signaling resulting in secretion of IGF-I into the circulation. IGF-I forms a complex with insulin-like growth factor binding protein-3 (IGFBP-3) and the acid labile subunit (ALS). Both IGFBP-3 and ALS expression are also regulated by hGH receptor activation.

In children with growth hormone deficiency (GHD) resulting from lack of expression or secretion of hGH and not caused by a defect in the hGH receptor, replacement therapy with daily injections of rhGH is often prescribed to facilitate near normal growth and development. New bone is formed at the epiphyses in response to hGH and IGF-I resulting in linear growth until the growth plates fuse after puberty. Daily rhGH administration does not mimic the normal endogenous pulses of hGH in non-GHD children, but does result in significant increases in growth with a typical first year growth rate on treatment of 11 cm/yr. Clinical studies of continuous infusion of rhGH with a pump demonstrated comparable growth velocity and IGF-I levels to those achieved with daily rhGH injections (Jorgensen et al. J. Clin Endocrinol Metab. 70(6), 1616-23 (1990); Laursen, T. et al. J Clin Endocrinol Metab. 80(8), 2410-8 (1995); Tauber, M. et al. J Clin Endocrinol Metab. 76(5), 1135-9 (1993)). Therefore, continuous, as well as pulsatile, administration of rhGH is efficacious.

In adulthood, hGH secretion is reduced but remains important to maintaining proper hormone balance and has been shown to facilitate decreases in fat mass and cardiovascular risk factors, and increases in lean body mass, bone mineral density, and quality of life outcomes. Adult GHD may occur as the result of traumatic injury to the brain or surgical removal of a tumor at or near the pituitary. Patients presenting with GHD in childhood may also require continued hGH replacement therapy in adulthood. In some adult GHD patients, there can be abnormally low IGF-I levels. Because IGF-I levels vary by age and sex, each adult patient must be characterized by their individual age and sex-adjusted IGF-I standard deviation score (IGF-I SDS).

The objective of hGH daily therapies is usually to titrate the adult GHD patient with rhGH dose until the patient achieves an IGF-I SDS near the middle of the range (e.g. IGF-I SDS of 0 (Cook et al., 2009 Update. Endocrine Pract. 15 (Suppl 2), 1-29 (2009)). A continuous infusion of rhGH was compared to daily rhGH therapy in adult GHD patients (7 per group) for 6 months (Laursen et al., J Clin Endocrinol Metab. 86(3),1222-8 (2001)). This study indicated that the safety profile and effects on the IGF-I responses were not significantly different between patients treated with continuous infusion of rhGH or daily rhGH therapy.

The safety of daily rhGH therapy has been studied in both GHD children and adults. In some overweight or obese patients, a trend toward increasing fasting and postprandial insulin levels has been observed. Although generally well tolerated, daily rhGH therapy may cause mild to moderate headache, arthralgia, nausea, vomiting and injection reactions.

Others have reported on various sustained release GH preparations (Cook DM, et al. 2002. J Clin Endocrinol Metab 87(10):4508-4514; Biller BM, et al. 2011. J Clin Endocrinol Metab 96(6):1718-1726; Peter F. et al., 2012. J Clin Endocrinol Metab 97(2):400-407; Fares F. et al, 2010. Endocrinology 151(9):4410-4417; Sondergaard E, et al. 2011. J Clin Endocrinol Metab 96(3):681-688; de Schepper J et al. 2011. European Journal of Endocrinology 165(3):401-409; Bidlingmaier M, et al. 2006. J Clin Endocrinol Metab 91(8):2926-2930). However, there remains a need for alternative GH therapeutics, dosages, and treatment regimens.

VRS-317 is an investigational long-acting rhGH in development for long-term replacement therapy for adults (including adults who experienced a growth hormone-related disorder as children) with GHD. VRS-317 was designed to achieve once-monthly dosing with the anticipation that a reduced frequency of administration (12 versus up to 365 injections per year) would increase treatment adherence and thereby improve overall treatment outcomes. VRS-317 is a novel rhGH fusion protein that was designed to minimize receptor mediated clearance through a reduction in receptor binding achieved without mutations to rhGH by genetically fusing extended recombinant polypeptide (XTEN) amino acid sequences to the N- and C-termini of the native hGH sequence (Cleland et al. 2012, Journal of Pharmaceutical Sciences. 101(8):2744-2754, Epub 2012 Jun 7). Functionally, the XTEN domains increase the hydrodynamic radius and reduce binding affinity to the GH receptor (GHR), *in vitro.* Despite reduced binding affinity, durable pharmacodynamics response are seen, *in vivo,* possibly relating to reduced rates of receptor mediated clearance of VRS-317 (Cleland et al. 2012 *supra*). VRS-317 was evaluated for safety, tolerability and efficacy in 50 adults with GHD in a 60-day, double-blind, randomized, placebo (PBO)-controlled, single ascending dose escalation studying VRS-317/kg (ClinicalTrials.gov NCT01359488).

Cleland et al., 2011 ("A Novel Human Growth Hormone XTEN Construct (VRS-317) for Once-a-Month Subcutaneous Administration in a Phase 1a Study of Growth Hormone-Deficient Adults", poster presentated at: The Endocrine Society's 93rd Annual Meeting & Expo, June 4-7, 2011, Boston, presentation number: P2-371) describes preclinical studies carried out in monkeys involving VRS-317 and note the existence of a Phase 1a study in humans.

Cleland et al. 2010 ("A novel human growth hormone XTEN construct (VRS-317) for monthly administration", Endocrine Journal. 57 (Suppl. 2):S618) also reports preclinical studies carried out in monkeys involving VRS-317.

### SUMMARY OF THE INVENTION

The present invention concerns an improved therapeutic regimen for growth hormone deficiency ("GHD") therapy. In particular, the invention concerns a fusion protein comprising human growth hormone fused to one or more extended recombinant polypeptides (XTEN) (the fusion protein hereinafter referred to as "hGH-XTEN") for use in a method of treating human adult growth hormone deficiency (AGHD) in a human patient, as defined in the claims.

In one aspect, the present invention provides a hGH-XTEN fusion protein comprising comprising an amino acid sequence having at least 90% sequence identity to SEQ ID NO:1,
for use in a method of treating human adult growth hormone deficiency (AGHD) in a human patient,
wherein the method comprises administering to the human patient with AGHD successive therapeutically effective bodyweight adjusted bolus doses of between 0.05 mg/kg and 3.0 mg/kg of the hGH-XTEN fusion protein,
wherein the dosage period between the initial bolus dose and a successive administration of a bolus dose is 17, 16, 15, 14, or 13 days,
wherein successive bolus doses of the hGH-XTEN fusion protein are:
(i) effective to maintain the patient's serum IGF-I standard deviation score (SDS) between -2.0 and +2.0 for at least 7 days after administration of the bolus doses; or
(ii) effective to maintain a plasma concentration of said fusion protein in the patient at more than 10 ng/mL for a period of at least 10 days after administration of the bolus doses.
   In one embodiment, the method comprises administering to a human patient with GHD an hGH-XTEN fusion protein comprising the amino acid sequence of SEQ ID NO:1. In one
   other embodiment, the bolus dose is (i) between about 0.05 mg/kg and about 0.8 mg/kg; or (ii) between about 0.8 mg/kg and about 1.2 mg/kg. In another embodiment, the bolus dose is administered subcutaneously. In another embodiment, the human patient has a serum IGF-I standard deviation score (SDS) between about -2.0 and about 2.0 following administration. In one other embodiment, the IGF-I SDS is selected from the group consisting of greater than about -2.0, greater than about -1.5, greater than about -1.0, greater than about -0.5, greater than about 0, greater than about 0.5, greater than about 1.0, and greater than about 1.5. In one embodiment, the administration of the bolus dose results in a normalization of IGF-I SDS in the human patient for at least about 7 days, at least about 10 days, at least about 14 days, at least about 16 days, or at least about 21 days. In one other embodiment, the bolus dose is selected from the group consisting of about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and about 3.0 mg/kg.

Methods for treatment of the human or animal body by surgery or therapy and diagnostic methods practised on the human or animal body are not part of the invention.

Disclosed herein is a method of treating human growth hormone deficiency (GHD) in a human patient with an hGH-XTEN fusion protein as a bolus dose that is equivalent to less than an hGH/kg/day dosage. In one embodiment, the method comprises administering to a human patient with GHD an hGH-XTEN fusion protein comprising (i) an amino acid sequence having at least about 90% sequence identity to SEQ ID NO:1; or (ii) the amino acid sequence of SEQ ID NO:1. In another embodiment, the bolus dose is a therapeutically effective bodyweight adjusted bolus dose of the hGH-XTEN fusion protein. In one other embodiment, the bolus dose is equivalent to less than an hGH/kg/day dosage between about 2 µg hGH/kg/day and about 20 µg hGH/kg/day. In an additional embodiment, the bolus dose is administered once, every week, every two weeks, every three weeks, or monthly. In one embodiment, the administration of the bolus dose is monthly. In another embodiment, the bolus dose is administered subcutaneously. In other embodiments, the bolus dose is equivalent to less
than an hGH/kg/day dosage selected from the group consisting of about 2 µg hGH/kg/day, about 4 µg hGH/kg/day, about 6 µg hGH/kg/day, about 8 µg hGH/kg/day, about 10 µg hGH/kg/day, about 12 µg hGH/kg/day, about 14 µg hGH/kg/day, about 16 µg hGH/kg/day, about 18 µg hGH/kg/day, about 18.6 µg hGH/kg/day, and about 20 µg hGH/kg/day. In one other embodiment, the hGH/kg/day dosage is over about 30 days. In another embodiment, the human patient has a serum IGF-I standard deviation score (SDS) between about -2.0 and about 2.0 following administration. In one other embodiment, the IGF-I SDS is selected from the group consisting of greater than about-2.0, greater than about -1.5, greater than about -1.0, greater than about -0.5, greater than about 0, greater than about 0.5, greater than about 1.0, and greater than about 1.5. In an additional embodiment, the human patient exhibits said serum IGF-I SDS following administration of the bolus dose, wherein the administration is once, weekly, every two weeks, every three weeks, or monthly.

Also disclosed herein is a method of treating human growth hormone deficiency (GHD) in a human patient with an hGH-XTEN fusion protein as a bolus dose that is effective to maintain a IGF-I standard deviation score (SDS) in the patient. In one embodiment, the method comprises administering to a human patient with GHD an hGH-XTEN fusion protein comprising (i) an amino acid sequence having at least about 90% sequence identity to SEQ ID NO:1; or (ii) the amino acid sequence of SEQ ID NO:1. In another embodiment, the hGH-XTEN fusion protein is administered as a therapeutically effective bodyweight adjusted bolus dose. In one additional embodiment, the bolus dose is effective to maintain the patient's serum IGF-I standard deviation score (SDS) between about -2.0 and about 2.0 for at least 7 days after administration of the bolus dose. In other embodiments, the bolus dose is (i) between about 0.05 mg/kg and about 0.8 mg/kg; (ii) between about 0.8 mg/kg and about 1.2 mg/kg; or (iii) between about 0.05 mg/kg and about 3.0 mg/kg. In another embodiment, the bolus dose is effective to maintain the patient's serum IGF-I SDS between about -2.0 and about 2.0 for at least 20 days after administration of the bolus dose. In one embodiment, the bolus dose is administered subcutaneously. In another embodiment, the human patient has a clinically significant reduction in at least one parameter selected from serum cholesterol, serum triglycerides, and serum low-density lipoprotein (LDL) after administration of the bolus dose, wherein the administration is
selected from the group consisting of once, weekly, every two weeks, every three weeks, and monthly.

Also disclosed herein is a method of treating human growth hormone deficiency (GHD) in a human patient with an hGH-XTEN fusion protein as a bolus dose that is effective to maintain a plasma concentration of said fusion protein in the patient. In one embodiment, the method comprises administering to a human patient with GHD an hGH-XTEN fusion protein comprising (i) an amino acid sequence having at least about 90% sequence identity to SEQ ID NO:1; or (ii) the amino acid sequence of SEQ ID NO:1. In another embodiment, the hGH-XTEN fusion protein is administered as a therapeutically effective bodyweight adjusted bolus dose. In one other embodiment, the bolus dose is effective to maintain a plasma concentration of said fusion protein in the patient at more than about 10 ng/mL for a period of at least 10 days after administration of the bolus dose. In another embodiment, the bolus dose is (i) between about 0.05 mg/kg and about 0.8 mg/kg; (ii) between about 0.8 mg/kg and about 1.2 mg/kg; or (iii) between about 0.05 mg/kg and about 3.0 mg/kg. In one other embodiment, the bolus dose is effective to maintain a plasma concentration of said fusion protein in the patient at more than about 10 ng/mL for a period of at least about 14 days, at least 20 days, at least about 28 days, or at least about 30 days after administration of the bolus dose. In other embodiments, the bolus dose is effective to maintain a plasma concentration of said fusion protein in the patient at more than about 10 ng/mL for a period of at least 20 days or at least about 30 days after administration of the bolus dose. In another embodiment, the bolus dose is effective to maintain a plasma concentration of said fusion protein in the patient at more than about 100 ng/mL for a period of at least 10 days after administration of the bolus dose. In one embodiment, the bolus dose is administered subcutaneously. In another embodiment, the human patient has a clinically significant reduction in at least one parameter selected from serum cholesterol, serum triglycerides, and serum low-density lipoprotein (LDL) after administration of the bolus dose, wherein the administration is selected from the group consisting of once, weekly, every two weeks, every three weeks, and monthly.

Also disclosed herein is a method of treating human growth hormone deficiency (GHD) in a human patient with an hGH-XTEN fusion protein as a bolus dose that is effective in increasing the patient's IGF-I SDS in the absence of clinically significant level of side-effects. In one embodiment, the method
comprises administering to a human patient with GHD an hGH-XTEN fusion protein comprising (i) an amino acid sequence having at least about 90% sequence identity to SEQ ID NO:1; or (ii) the amino acid sequence of SEQ ID NO:1. In another embodiment, the hGH-XTEN fusion protein is administered as a therapeutically effective bodyweight adjusted bolus dose. In one other embodiment, the bolus dose is effective in increasing the patient's IGF-SDS by at least 0.5 or at least 1.0 above the subject's baseline IGF-I SDS after administration. In one additional embodiment, the increase in IGF-SDS is in the absence of a clinically significant level of side-effects. In one embodiment, the side effect is selected from the group consisting of headache, arthralgia, myalgia, edema, nausea, and muscle fatigue. In one embodiment, the bolus dose is administered subcutaneously. In another embodiment, the human patient has a clinically significant reduction in at least one parameter selected from serum cholesterol, serum triglycerides, and serum low-density lipoprotein (LDL) after administration of the bolus dose, wherein the administration is selected from the group consisting of once, weekly, every two weeks, every three weeks, and monthly.

In one other aspect, disclosed herein is a bolus dose of an hGH-XTEN fusion protein. In one embodiment, the hGH-XTEN fusion protein comprising (i) an amino acid sequence having at least about 90% sequence identity to SEQ ID NO:1; or (ii) the amino acid sequence of SEQ ID NO:1. In another embodiment, the bolus dose is a therapeutically effective bodyweight adjusted bolus dose. In another embodiment, the bolus dose comprises (i) between about 0.05 mg/kg and about 0.8 mg/kg; (ii) between about 0.8 mg/kg and about 1.2 mg/kg; or (iii) between about 0.05 mg/kg and about 3.0 mg/kg, of the hGH-XTEN fusion protein. In one additional embodiment, the bolus dose is for use in treating human GHD in a subject (e.g., a human patient) in need. In one other embodiment, the bolus dose is formulated for subcutaneous administration.

In another aspect, disclosed herein is an hGH-XTEN fusion protein for use in a method for the treatment of human GHD in a human patient, wherein the method comprises administering to the patient a bolus dose of the hGH-XTEN fusion protein. In one other aspect, disclosed herein is the use of an hGH-XTEN fusion protein in the manufacture of a medicament for the treatment of GHD in a human patient, wherein the hGH-XTEN fusion protein is administered to the patient as a bolus dose. In one embodiment, the hGH-XTEN fusion protein comprising (i) an amino acid sequence having at least about 90% sequence identity to SEQ ID NO:1; or (ii) the amino acid sequence of SEQ ID NO:1. In another embodiment, the bolus dose is a therapeutically effective bodyweight adjusted bolus dose. In one additional embodiment, the bolus dose comprises between about 0.05 mg/kg and about 3.0 mg/kg. In one other embodiment, the bolus dose is administered every week, every two weeks, every three weeks, or monthly. In one embodiment, the bolus dose is administered subcutaneously. In other embodiments, the human patient has a serum IGF-I standard deviation score (SDS) between about -2.0 and about 2.0 following administration of the bolus dose. In another embodiment, the IGF-I SDS is selected from the group consisting of greater than about-2.0, greater than about -1.5, greater than about -1.0, greater than about -0.5, greater than about 0, greater than about 0.5, greater than about 1.0, and greater than about 1.5. In a further embodiment, the administration of the bolus dose is once, weekly, every two weeks, every three weeks, or monthly. In one embodiment, the human patient has a clinically significant reduction in at least one parameter selected from serum cholesterol, serum triglycerides, and serum LDL after administration of the bolus dose, wherein the administration is once, weekly, every two weeks, every three weeks, or monthly.

Also disclosed herein is a method of increasing the efficacy of human growth hormone (hGH) therapy in a human patient, wherein the hGH therapy comprises the administration of an hGH-XTEN fusion protein. In one embodiment, hGH-XTEN fusion protein comprises (i) an amino acid sequence having at least about 90% sequence identity to SEQ ID NO:1; or (ii) the amino acid sequence of SEQ ID NO:1. In one other embodiment, the method comprises the step of measuring or monitoring the IGF-I standard deviation score (SDS) in a plasma or serum sample obtained from the patient during an initial dosage period of administration of an initial dose of human growth hormone-XTEN (hGH-XTEN) fusion protein. In another embodiment, the method further comprises the step of determining a subsequent dose of hGH-XTEN fusion protein administered over a subsequent dosage period based on the IGF-I SDS observed during the initial dosage period. In other embodiments, the determining step comprises determining a subsequent dosage period based upon the IGF-I SDS observed during the initial dosage period. In one additional embodiment, the subsequent dose and/or the subsequent dosing period improves the efficacy of the treatment during the subsequent dosage period.

Also disclosed is a kit comprising a pharmaceutical composition, which comprises an hGH-XTEN fusion protein for the
treatment of human GHD. In one embodiment, the hGH-XTEN fusion protein comprises (i) an amino acid sequence having at least about 90% sequence identity to SEQ ID NO:1; or (ii) the amino acid sequence of SEQ ID NO:1. In another embodiment, the kit comprises a container which holds a pharmaceutical composition comprising the hGH-XTEN fusion protein. In one additional embodiment, the kit further comprises a package insert associated with said container. In other embodiments, the package insert indicates that said composition is for the treatment of growth hormone deficiency by administration of an initial dose of the hGH-XTEN fusion protein and a plurality of subsequent doses of the hGH-XTEN fusion protein. In another embodiment, the initial dose and plurality of subsequent bolus doses each comprise a bolus dose. In one other embodiment, the bolus dose is a therapeutically effective bodyweight adjusted bolus dose. In one embodiment, the initial dose of the hGH-XTEN fusion protein is between about 0.05 mg/kg and about 3.0 mg/kg. In another embodiment, the plurality of subsequent doses of the hGH-XTEN fusion protein in between about 0.05 mg/kg and about 3.0 mg/kg. In one embodiment, the doses are administered once, every week, every two weeks, every three weeks, or monthly.

### BRIEF DESCRIPTION OF THE DRAWINGS

FIG. 1 provides the amino acid sequence for an hGH-XTEN fusion protein (hGH sequence is underlined and bold) (SEQ ID NO:1).
FIG. 2 summarizes the study phases of the VRS-317 Phase I study.
FIG. 3 summarizes the patient disposition.
FIG. 4 shows the human pharmacokinetic (PK) profile for various single doses of VRS-317.
FIG. 5 illustrates a dose response: change in mean IGF-I SDS for mg VRS-317/kg doses.
FIG. 6 illustrates a sustained IGF-I response to a single dose of VRS-317.
FIG. 7 summarizes adverse events reported after administration of various single doses of VRS-317.

### DESCRIPTION OF THE INVENTION

Before the embodiments of the invention are described, it is to be understood that such embodiments are provided by way of example only, and that various alternatives to the embodiments of the invention described herein may be employed in practicing the invention, within the definition of the invention in the claims. Numerous variations, changes, and substitutions will now occur to those skilled in the art without departing from the invention, as defined by the claims.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting. Numerous variations, changes, and substitutions will now occur to those skilled in the art without departing from the invention, as defined in the claims.

### DEFINITIONS

As used herein, the following terms have the meanings ascribed to them unless specified otherwise.

As used in the specification and claims, the singular forms "a", "an" and "the" include plural references unless the context clearly dictates otherwise. For example, the term "a cell" includes a plurality of cells, including mixtures thereof.

The terms "polypeptide", "peptide", and "protein" are used interchangeably herein to refer to polymers of amino acids of any length. The polymer may be linear or branched, it may comprise modified amino acids, and it may be interrupted by non amino acids. The terms also encompass an amino acid polymer that has been modified, for example, by disulfide bond formation, glycosylation, lipidation, acetylation, phosphorylation, or any other manipulation, such as conjugation with a labeling component.

As used herein the term "amino acid" refers to either natural and/or unnatural or synthetic amino acids, including but not limited to glycine and both the D or L optical isomers, and amino acid analogs and peptidomimetics. Standard single or three letter codes are used to designate amino acids.

The term "natural L-amino acid" means the L optical isomer forms of glycine (G), proline (P), alanine (A), valine (V), leucine (L), isoleucine (I), methionine (M), cysteine (C), phenylalanine (F), tyrosine (Y), tryptophan (W), histidine (H), lysine (K), arginine (R), glutamine (Q), asparagine (N), glutamic acid (E), aspartic acid (D), serine (S), and threonine (T).

The term "non-naturally occurring," as applied to sequences and as used herein, means polypeptide or polynucleotide sequences that do not have a counterpart to, are not complementary to, or do not have a high degree of homology with a wild-type or naturally-occurring sequence found in a mammal. For example, a non-naturally occurring polypeptide or fragment may share no more than 99%, 98%, 95%, 90%, 80%, 70%, 60%, 50% or even less amino acid sequence identity as compared to a natural sequence when suitably aligned.

The terms "hydrophilic" and "hydrophobic" refer to the degree of affinity that a substance has with water. A hydrophilic substance has a strong affinity for water, tending to dissolve in, mix with, or be wetted by water, while a hydrophobic substance substantially lacks affinity for water, tending to repel and not absorb water and tending not to dissolve in or mix with or be wetted by water. Amino acids can be characterized based on their hydrophobicity. A number of scales have been developed. An example is a scale developed by Levitt, M, et al., J Mol Biol (1976) 104:59, which is listed in Hopp, TP, et al., Proc Natl Acad Sci U S A (1981) 78:3824. Examples of "hydrophilic amino acids" are arginine, lysine, threonine, alanine, asparagine, and glutamine. Of particular interest are the hydrophilic amino acids aspartate, glutamate, and serine, and glycine. Examples of "hydrophobic amino acids" are tryptophan, tyrosine, phenylalanine, methionine, leucine, isoleucine, and valine.

A "fragment" is a truncated form of a native biologically active protein that retains at least a portion of the therapeutic and/or biological activity. A "variant" is a protein with sequence homology to the native biologically active protein that retains at least a portion of the therapeutic and/or biological activity of the biologically active protein. For example, a variant protein may share at least 70%, 75%, 80%, 85%, 90%, 95%, 96%, 97%, 98% or 99% amino acid sequence identity with the reference biologically active protein. As used herein, the term "biologically active protein moiety" includes proteins modified deliberately, as for example, by site directed mutagenesis, insertions, or accidentally through mutations.

A "host cell" includes an individual cell or cell culture which can be or has been a recipient for the subject vectors. Host cells include progeny of a single host cell. The progeny may not necessarily be completely identical (in morphology or in genomic of total DNA complement) to the original parent cell due to natural, accidental, or deliberate mutation. A host cell includes cells transfected in vivo with a vector of this invention.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. As is apparent to those of skill in the art, a non-naturally occurring polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration or number of molecules per volume is generally greater than that of its naturally occurring counterpart. In general, a polypeptide made by recombinant means and expressed in a host cell is considered to be "isolated."

An "isolated" polynucleotide or polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal or extra-chromosomal location different from that of natural cells.

A "chimeric" protein contains at least one fusion polypeptide comprising regions in a different position in the sequence than that which occurs in nature. The regions may normally exist in separate proteins and are brought together in the fusion polypeptide; or they may normally exist in the same protein but are placed in a new arrangement in the fusion polypeptide. A chimeric protein may be created, for example, by chemical synthesis, or by creating and translating a polynucleotide in which the peptide regions are encoded in the desired relationship.

"Conjugated", "linked," "fused," and "fusion" are used interchangeably herein. These terms refer to the joining together of two or more chemical elements or components, by whatever means including chemical conjugation or recombinant means. For example, a promoter or enhancer is operably linked to a coding sequence if it affects the transcription of the sequence. Generally, "operably linked" means that the DNA sequences being linked are contiguous, and in reading phase or in-frame. An "in-frame fusion" refers to the joining of two or more open reading frames (ORFs) to form a continuous longer ORF, in a manner that maintains the correct reading frame of the original ORFs. Thus, the resulting recombinant fusion protein is a single protein containing two or more segments that correspond to polypeptides encoded by the original ORFs (which segments are not normally so joined in nature).

In the context of polypeptides, a "linear sequence" or a "sequence" is an order of amino acids in a polypeptide in an amino to carboxyl terminus direction in which residues that neighbor each other in the sequence are contiguous in the primary structure of the polypeptide. A "partial sequence" is a linear sequence of part of a polypeptide that is known to comprise additional residues in one or both directions.

"Heterologous" means derived from a genotypically distinct entity from the rest of the entity to which it is being compared. For example, a glycine rich sequence removed from its native coding sequence and operatively linked to a coding sequence other than the native sequence is a heterologous glycine rich sequence. The term "heterologous" as applied to a polynucleotide, a polypeptide, means that the polynucleotide or polypeptide is derived from a genotypically distinct entity from that of the rest of the entity to which it is being compared.

The terms "polynucleotides", "nucleic acids", "nucleotides" and "oligonucleotides" are used interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The term "complement of a polynucleotide" denotes a polynucleotide molecule having a complementary base sequence and reverse orientation as compared to a reference sequence, such that it could hybridize with a reference sequence with complete fidelity.

"Recombinant" as applied to a polynucleotide means that the polynucleotide is the product of various combinations of in vitro cloning, restriction and/or ligation steps, and other procedures that result in a construct that can potentially be expressed in a host cell.

The terms "gene" or "gene fragment" are used interchangeably herein. They refer to a polynucleotide containing at least one open reading frame that is capable of encoding a particular protein after being transcribed and translated. A gene or gene fragment may be genomic or cDNA, as long as the polynucleotide contains at least one open reading frame, which may cover the entire coding region or a segment thereof. A "fusion gene" is a gene composed of at least two heterologous polynucleotides that are linked together.

"Homology" or "homologous" refers to sequence similarity or interchangeability between two or more polynucleotide sequences or two or more polypeptide sequences. When using a program such as BestFit to determine sequence identity, similarity or homology between two different amino acid sequences, the default settings may be used, or an appropriate scoring matrix, such as blosum45 or blosum80, may be selected to optimize identity, similarity or homology scores. Preferably, polynucleotides that are homologous are those which hybridize under stringent conditions as defined herein and have at least 70%, preferably at least 80%, more preferably at least 90%, more preferably 95%, more preferably 97%, more preferably 98%, and even more preferably 99% sequence identity to those sequences.

"Ligation" refers to the process of forming phosphodiester bonds between two nucleic acid fragments or genes, linking them together. To ligate the DNA fragments or genes together, the ends of the DNA must be compatible with each other. In some cases, the ends will be directly compatible after endonuclease digestion. However, it may be necessary to first convert the staggered ends commonly produced after endonuclease digestion to blunt ends to make them compatible for ligation.

The terms "stringent conditions" or "stringent hybridization conditions" includes reference to conditions under which a polynucleotide will hybridize to its target sequence, to a detectably greater degree than other sequences (e.g., at least 2-fold over background). Generally, stringency of hybridization is expressed, in part, with reference to the temperature and salt concentration under which the wash step is carried out. Typically, stringent conditions will be those in which the salt concentration is less than about 1.5 M Na ion, typically about 0.01 to 1.0 M Na ion concentration (or other salts) at pH 7.0 to 8.3 and the temperature is at least about 30 °C for short polynucleotides (e.g., 10 to 50 nucleotides) and at least about 60 °C for long polynucleotides (e.g., greater than 50 nucleotides) for example, "stringent conditions" can include hybridization in 50% formamide, 1 M NaCl, 1% SDS at 37 °C, and three washes for 15 min each in 0.1×SSC/1% SDS at 60 °C to 65 °C. Alternatively, temperatures of about 65 °C, 60 °C, 55 °C, or 42 °C may be used. SSC concentration may be varied from about 0.1 to 2×SSC, with SDS being present at about 0.1%. Such wash temperatures are typically selected to be about 5 °C to 20 °C lower than the thermal melting point for the specific sequence at a defined ionic strength and pH. The Tm is the temperature (under defined ionic strength and pH) at which 50% of the target sequence hybridizes to a perfectly matched probe. An equation for calculating Tm and conditions for nucleic acid hybridization are well known and can be found in Sambrook, J. et al. (1989) Molecular Cloning: A Laboratory Manual, 2nd ed., vol. 1-3, Cold Spring Harbor Press, Plainview N.Y.; specifically see volume 2 and chapter 9. Typically, blocking reagents are used to block non-specific hybridization. Such blocking reagents include, for instance, sheared and denatured salmon sperm DNA at about 100-200 µg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art.

The terms "percent identity" and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity may be measured over the length of an entire defined polynucleotide sequence, or may be measured over a shorter length, for example, over the length of a fragment taken from a larger, defined polynucleotide sequence, for instance, a fragment of at least 45, at least 60, at least 90, at least 120, at least 150, at least 210 or at least 450 contiguous residues. Such lengths are exemplary only, and it is understood that any fragment length supported by the sequences shown herein, in the tables, figures or Sequence Listing, may be used to describe a length over which percentage identity may be measured.

"Percent (%) amino acid sequence identity," with respect to the polypeptide sequences identified herein, is defined as the percentage of amino acid residues in a query sequence that are identical with the amino acid residues of a second, reference polypeptide sequence or a portion thereof, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Percent identity may be measured over the length of an entire defined polypeptide sequence, or may be measured over a shorter length, for example, over the length of a fragment taken from a larger, defined polypeptide sequence, for instance, a fragment of at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured.

The term "non-repetitiveness" as used herein in the context of a polypeptide refers to a lack or limited degree of internal homology in a peptide or polypeptide sequence. The term "substantially non-repetitive" can mean, for example, that there are few or no instances of four contiguous amino acids in the sequence that are identical amino acid types or that the polypeptide has a subsequence score (defined infra) of 10 or less or that there isn't a pattern in the order, from N- to C-terminus, of the sequence motifs that constitute the polypeptide sequence. The term "repetitiveness" as used herein in the context of a polypeptide refers to the degree of internal homology in a peptide or polypeptide sequence. In contrast, a "repetitive" sequence may contain multiple identical copies of short amino acid sequences. For instance, a polypeptide sequence of interest may be divided into n-mer sequences and the number of identical sequences can be counted. Highly repetitive sequences contain a large fraction of identical sequences while non-repetitive sequences contain few identical sequences. In the context of a polypeptide, a sequence can contain multiple copies of shorter sequences of defined or variable length, or motifs, in which the motifs themselves have non-repetitive sequences, rendering the full-length polypeptide substantially non-repetitive. The length of polypeptide within which the non-repetitiveness is measured can vary from 3 amino acids to about 200 amino acids, about from 6 to about 50 amino acids, or from about 9 to about 14 amino acids. "Repetitiveness" used in the context of polynucleotide sequences refers to the degree of internal homology in the sequence such as, for example, the frequency of identical nucleotide sequences of a given length. Repetitiveness can, for example, be measured by analyzing the frequency of identical sequences.

A "vector" is a nucleic acid molecule, preferably self-replicating in an appropriate host, which transfers an inserted nucleic acid molecule into and/or between host cells. The term includes vectors that function primarily for insertion of DNA or RNA into a cell, replication of vectors that function primarily for the replication of DNA or RNA, and expression vectors that function for transcription and/or translation of the DNA or RNA. Also included are vectors that provide more than one of the above functions. An "expression vector" is a polynucleotide which, when introduced into an appropriate host cell, can be transcribed and translated into a polypeptide(s). An "expression system" usually connotes a suitable host cell comprised of an expression vector that can function to yield a desired expression product.

"Serum degradation resistance," as applied to a polypeptide, refers to the ability of the polypeptides to withstand degradation in blood or components thereof, which typically involves proteases in the serum or plasma. The serum degradation resistance can be measured by combining the protein with human (or mouse, rat, monkey, as appropriate) serum or plasma, typically for a range of days (e.g. 0.25, 0.5, 1, 2, 4, 8, 16 days), typically at about 37 °C. The samples for these time points can be run on a Western blot assay and the protein is detected with an antibody. The antibody can be to a tag in the protein. If the protein shows a single band on the western, where the protein's size is identical to that of the injected protein, then no degradation has occurred. In this exemplary method, the time point where 50% of the protein is degraded, as judged by Western blots or equivalent techniques, is the serum degradation half-life or "serum half-life" of the protein.

The term "t1/2" as used herein means the terminal half-life calculated as ln(2)/Kₑₗ . Kₑₗ is the terminal elimination rate constant calculated by linear regression of the terminal linear portion of the log concentration vs. time curve. Half-life typically refers to the time required for half the quantity of an administered substance deposited in a living organism to be metabolized or eliminated by normal biological processes. The terms "t1/2 ", "terminal half-life", "elimination half-life" and "circulating half-life" are used interchangeably herein.

"Apparent Molecular Weight Factor" or "Apparent Molecular Weight" are related terms referring to a measure of the relative increase or decrease in apparent molecular weight exhibited by a particular amino acid sequence. The Apparent Molecular Weight is determined using size exclusion chromatography (SEC) and similar methods compared to globular protein standards and is measured in "apparent kD" units. The Apparent Molecular Weight Factor is the ratio between the Apparent Molecular Weight and the actual molecular weight; the latter predicted by adding, based on amino acid composition, the calculated molecular weight of each type of amino acid in the composition.

The "hydrodynamic radius" or "Stokes radius" is the effective radius (Rh in nm) of a molecule in a solution measured by assuming that it is a body moving through the solution and resisted by the solution's viscosity. In the embodiments of the invention, the hydrodynamic radius measurements of the XTEN fusion proteins correlate with the 'Apparent Molecular Weight Factor', which is a more intuitive measure. The "hydrodynamic radius" of a protein affects its rate of diffusion in aqueous solution as well as its ability to migrate in gels of macromolecules. The hydrodynamic radius of a protein is determined by its molecular weight as well as by its structure, including shape and compactness. Methods for determining the hydrodynamic radius are well known in the art, such as by the use of size exclusion chromatography (SEC), as described in U.S. Patent Nos. 6,406,632 and 7,294,513. Most proteins have globular structure, which is the most compact three-dimensional structure a protein can have with the smallest hydrodynamic radius. Some proteins adopt a random and open, unstructured, or 'linear' conformation and as a result have a much larger hydrodynamic radius compared to typical globular proteins of similar molecular weight.

"Physiological conditions" refer to a set of conditions in a living host as well as in vitro conditions, including temperature, salt concentration, pH, that mimic those conditions of a living subject. A host of physiologically relevant conditions for use in in vitro assays have been established. Generally, a physiological buffer contains a physiological concentration of salt and is adjusted to a neutral pH ranging from about 6.5 to about 7.8, and preferably from about 7.0 to about 7.5. A variety of physiological buffers is listed in Sambrook et al. (1989). Physiologically relevant temperature ranges from about 25 °C to about 38 °C, and preferably from about 35 °C to about 37 °C.

A "reactive group" is a chemical structure that can be coupled to a second reactive group. Examples for reactive groups are amino groups, carboxyl groups, sulfhydryl groups, hydroxyl groups, aldehyde groups, azide groups. Some reactive groups can be activated to facilitate coupling with a second reactive group. Non-limiting examples for activation are the reaction of a carboxyl group with carbodiimide, the conversion of a carboxyl group into an activated ester, or the conversion of a carboxyl group into an azide function.

"Controlled release agent", "slow release agent", "depot formulation" or "sustained release agent" are used interchangeably to refer to an agent capable of extending the duration of release of a polypeptide of the invention relative to the duration of release when the polypeptide is administered in the absence of agent. Different embodiments of the present invention may have different release rates, resulting in different therapeutic amounts.

The terms "antigen", "target antigen" or "immunogen" are used interchangeably herein to refer to the structure or binding determinant that an antibody fragment or an antibody fragment-based therapeutic binds to or has specificity against.

The term "payload" as used herein refers to a protein or peptide sequence that has biological or therapeutic activity; the counterpart to the pharmacophore of small molecules. Examples of payloads include, but are not limited to, cytokines, enzymes, hormones and blood and growth factors. Payloads can further comprise genetically fused or chemically conjugated moieties such as chemotherapeutic agents, antiviral compounds, toxins, or contrast agents. These conjugated moieties can be joined to the rest of the polypeptide via a linker that may be cleavable or non-cleavable.

The term "antagonist", as used herein, includes any molecule that partially or fully blocks, inhibits, or neutralizes a biological activity of a native polypeptide disclosed herein. Methods for identifying antagonists of a polypeptide may comprise contacting a native polypeptide with a candidate antagonist molecule and measuring a detectable change in one or more biological activities normally associated with the native polypeptide. In the context of the present invention, antagonists may include proteins, nucleic acids, carbohydrates, antibodies or any other molecules that decrease the effect of a biologically active protein.

The term "agonist" is used in the broadest sense and includes any molecule that mimics a biological activity of a native polypeptide disclosed herein. Suitable agonist molecules specifically include agonist antibodies or antibody fragments, fragments or amino acid sequence variants of native polypeptides, peptides, small organic molecules, etc. Methods for identifying agonists of a native polypeptide may comprise contacting a native polypeptide with a candidate agonist molecule and measuring a detectable change in one or more biological activities normally associated with the native polypeptide.

"Activity" for the purposes herein refers to an action or effect of a component of a fusion protein consistent with that of the corresponding native biologically active protein, wherein "biological activity" refers to an in vitro or in vivo biological function or effect, including but not limited to receptor binding, antagonist activity, agonist activity, or a cellular or physiologic response.

As used herein, "treatment" or "treating," or "palliating" or "ameliorating" is used interchangeably herein. These terms refer to an approach for obtaining beneficial or desired results including but not limited to a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. For prophylactic benefit, the compositions may be administered to a subject at risk of developing a particular disease, or to a subject reporting one or more of the physiological symptoms of a disease, even though a diagnosis of this disease may not have been made.

A "therapeutic effect", as used herein, refers to a physiologic effect, including but not limited to the cure, mitigation, amelioration, or prevention of disease in humans or other animals, or to otherwise enhance physical or mental wellbeing of humans or animals, caused by a fusion polypeptide of the invention other than the ability to induce the production of an antibody against an antigenic epitope possessed by the biologically active protein. Determination of a therapeutically effective amount is well within the capability of those skilled in the art, especially in light of the detailed disclosure provided herein.

The terms "therapeutically effective amount" and "therapeutically effective dose", as used herein, refers to an amount of a biologically active protein, either alone or as a part of a fusion protein composition, that is capable of having any detectable, beneficial effect on any symptom, aspect, measured parameter or characteristics of a disease state or condition when administered in one or repeated doses to a subject. Such effect need not be absolute to be beneficial.

A "pharmaceutically acceptable carrier" refers to an ingredient in a pharmaceutical composition, other than an active ingredient, which is nontoxic to a subject., A pharmaceutically acceptable carrier includes, but is not limited to, a buffer, excipient, stabilizer, or preservative.

The term "therapeutically effective dose regimen", as used herein, refers to a schedule for consecutively administered doses of a biologically active protein, either alone or as a part of a fusion protein composition, wherein the doses are given in therapeutically effective amounts to result in sustained beneficial effect on any symptom, aspect, measured parameter or characteristics of a disease state or condition.

### I). GENERAL TECHNIQUES

The practice of the present invention employs, unless otherwise indicated, conventional techniques of immunology, biochemistry, chemistry, molecular biology, microbiology, cell biology, genomics and recombinant DNA, which are within the skill of the art. See Sambrook, J. et al., "Molecular Cloning: A Laboratory Manual," 3rd edition, Cold Spring Harbor Laboratory Press, 2001; "Current protocols in molecular biology", F. M. Ausubel, et al. eds.,1987; the series "Methods in Enzymology," Academic Press, San Diego, CA.; "PCR 2: a practical approach", M.J. MacPherson, B.D. Hames and G.R. Taylor eds., Oxford University Press, 1995; "Antibodies, a laboratory manual" Harlow, E. and Lane, D. eds., Cold Spring Harbor Laboratory, 1988; "Goodman & Gilman's The Pharmacological Basis of Therapeutics," 11th Edition, McGraw-Hill, 2005; and Freshney, R.I., "Culture of Animal Cells: A Manual of Basic Technique," 4th edition, John Wiley & Sons, Somerset, NJ, 2000.

### II). GROWTH HORMONE

The present invention concerns an improved therapeutic regimen for GHD therapy. In particular, the invention concerns a hGH-XTEN fusion protein for use in a method of treating AGHD in a human patient, as defined in the claims. Also disclosed herein is a method of treating human growth hormone deficiency (GHD) with a hGH-XTEN fusion protein.

### (a) Growth hormone proteins

"Growth Hormone" or "GH" means a growth hormone protein and species and sequence variants thereof, and includes, but is not limited to, the 191 single-chain amino acid sequence of human GH. The GH can be the native, full-length protein or can be a truncated fragment or a sequence variant that retains at least a portion of the biological activity of the native protein. There are two known types of human GH (hereinafter "hGH") derived from the pituitary gland: one having a molecular weight of about 22,129 daltons (22kD hGH) and the other having a molecular weight of about 20,000 daltons (20kD hGH). The 20kD HGH has an amino acid sequence that corresponds to that of 22kD hGH consisting of 191 amino acids except that 15 amino acid residues from the 32nd to the 46th of 22kD hGH are missing. Some reports have shown that the 20kD hGH has been found to exhibit lower risks and higher activity than 22kD hGH. The invention contemplates use of the 22 kD, the 20kD hGH, as well as species and sequence variants and truncated fragments thereof as being appropriate for use as a fusion partner with XTEN disclosed herein for hGH-XTEN compositions. The cloned gene for hGH has been expressed in a secreted form in Escherichia coli (United States Patent No. 4,898,830; Chang, C. N., et al., Gene 55:189 [1987]) and its DNA and amino acid sequence has been reported (Goeddel, et al. Nature ,281:544 [1979]); Gray, et al., Gene 39: 247[1985]).

The invention contemplates inclusion in the hGH-XTEN compositions sequences with homology to GH sequences, sequence fragments that are natural, such as from humans and non-natural sequence variants which retain at least a portion of the biologic activity or biological function of GH and/or that are useful for preventing, treating, mediating, or ameliorating a GH-related disease, deficiency, disorder or condition. In addition, native sequences homologous to human GH may be found by standard homology searching techniques, such as NCBI BLAST.

Effects of GH on the tissues of the body can generally be described as anabolic. Like most other protein hormones, native GH acts by interacting with a specific plasma membrane receptor, referred to as growth hormone receptor. GH acts on the liver and other tissues to stimulate production of IGF-I, which is responsible for the growth promoting effects of GH and also reflects the amount produced. IGF-I, in turn, has stimulatory effects on osteoblast and chondrocyte activity to promote bone growth. In one embodiment, the disclosure provides a hGH-XTEN that exhibits at least one of the properties of native GH hereinabove described herein.

In one embodiment, the GH incorporated into the subject compositions is a recombinant polypeptide with a sequence corresponding to a protein found in nature. In another embodiment, the GH is a sequence variant, fragment, homolog, or a mimetics of a natural sequence that retains at least a portion of the biological activity of the corresponding native GH. In one other embodiment, the GH is human GH comprising the following amino acid sequence: FPTIPLSRLFDNAMLRAHRLHQLAFDTYQEFEEAYIPKEQKYSFLQNPQTSLCFSES IPTPSNREETQQKSNLELLRISLLLIQSWLEPVQFLRSVFANSLVYGASDSNVYDLL KDLEEGIQTLMGRLEDGSPRTGQIFKQTYSKFDTNSHNDDALLKNYGLLYCFRKD MDKVETFLRIVQCRSVEGSCGF (SEQ ID NO:2). Any human GH sequences or homologous derivatives constructed by shuffling individual mutations between families that retain at least a portion of the biological activity of the native GH may be useful for the fusion proteins of this disclosure. GH that can be incorporated into a hGH-XTEN fusion protein can include a protein that exhibits at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity to SEQ ID NO:2.

### III). HUMAN GROWTH HORMONE-XTEN FUSION PROTEIN COMPOSITIONS FOR TREATING GHD

The present invention concerns an improved therapeutic regimen for growth hormone deficiency (GHD) therapy. In particular, the invention concerns a hGH-XTEN fusion protein for use in a method of treating AGHD in a human patient, as defined in the claim. The hGH fusion proteins suitable for use in the present disclosure comprise a human growth hormone polypeptide and one or more XTEN sequences as described herein, and as disclosed in Schellenberger et al. WO10/144502A2 and WO10/091122.

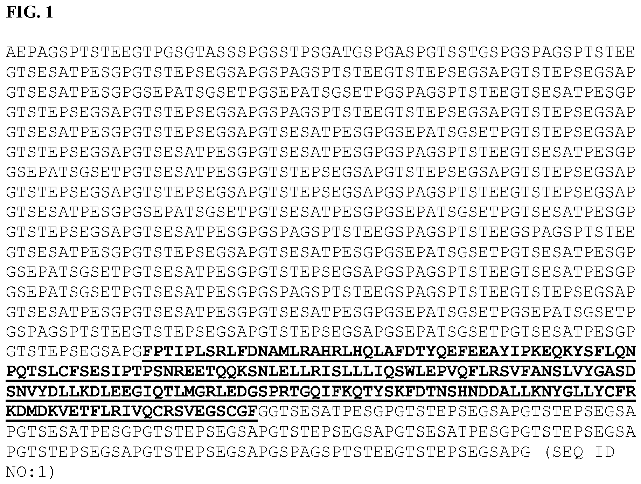

In one other aspect, the hGH-XTEN fusion proteins are isolated monomeric fusion proteins of GH comprising the full-length sequence or sequence variants of GH covalently linked to one or more extended recombinant polypeptides ("XTEN" or "XTENs"). In one embodiment, the hGH-XTEN fusion protein comprises an amino acid sequence shown in FIG. 1 (SEQ ID NO: 1), or pharmacologically active variants thereof. Also disclosed herein is a hGH-XTEN fusion protein that comprises an amino acid sequence selected from Table 1.

The fusion protein VRS-317, is composed of recombinant human growth hormone (rhGH) and two recombinant polypeptides, referred to as XTEN as described in Schellenberger et al. (2009). Nat Biotechnol 27, 1186-90, Schellenberger et al. WO10/144502A2, and WO10/091122. The XTEN domain, two unstructured hydrophilic chains of amino acids, provides half-life extension for rhGH. The molecular weight of VRS-317 is 118.9 kDa, with rhGH contributing 22.1 kDa and the remaining mass contributed by the XTEN construct. The mass ratio of rhGH to VRS-317 is therefore 1:5.37. The amino acid sequence of the VRS-317 fusion protein is provided in FIG. 1.

**Table 1 - Exemplary hGH-XTEN fusion proteins**

| **hGH- XTEN Name*** | **Amino Acid Sequence** | **SEQ ID NO:** | **DNA Nucleotide Sequence** | **SEQ ID NO:** |
|---|---|---|---|---|
| AM864-hGH | | 3 | | 4 |
| | | | | |
| | | | | |
| Y576-hGH | | 5 | | 6 |
| | | | | |
| | | | | |
| AE912-hGH | | 7 | | 8 |
| | | | | |
| | | | | |
| AE912-hGH-AE144 | | 9 | | 10 |
| | | | | |
| | | | | |
| | | | | |
| AE912-hGH-AE288 | | 11 | | 12 |
| | | | | |
| | | | | |
| | | | | |
| AM875-hGH | | 13 | | 14 |
| | | | | |
| | | | | |

Further characterization of the exemplary hGH-XTEN fusion proteins provided in Table 1 can be found in the examples (*e.g.,* Examples 27-35) of Schellenberger et al. WO10/144502A2.

Disclosed herein is the use of hGH-XTEN fusion proteins comprising one of the amino acid sequences shown in FIG. 1, Table 1, or as described in Schellenberger et al. WO10/144502A2. In addition, pharmacologically active variants of any of the hGH-XTEN fusion proteins described and referred to herein are also contemplated.

As described more fully below, the fusion proteins optionally include spacer sequences that further comprise cleavage sequences to release the GH from the fusion protein when acted on by a protease, releasing GH from the XTEN sequence(s).

In one aspect, the disclosure provides an isolated fusion protein comprising at least a first biologically active growth hormone protein covalently linked to one or more extended recombinant polypeptides ("XTEN"), resulting in a growth hormone-XTEN fusion protein composition (hereinafter "hGH-XTEN"). In one embodiment, the growth hormone is human growth hormone or a sequence variant of hGH. As described more fully below, the fusion proteins optionally include spacer sequences that further comprise cleavage sequences to release the GH from the fusion protein when acted on by a protease.

The term "hGH-XTEN", as used herein, is meant to encompass fusion polypeptides that comprise a payload region comprising a biologically active GH that mediates one or more biological or therapeutic activities associated with growth hormone and at least one other region comprising at least a first XTEN polypeptide that serves as a carrier. In one embodiment, the disclosure provides an hGH-XTEN fusion protein comprising the sequence set forth in Table 1.

The GH of the subject compositions, together with their corresponding nucleic acid and amino acid sequences, are well known in the art and descriptions and sequences are available in public databases such as Chemical Abstracts Services Databases (e.g., the CAS Registry), GenBank, The Universal Protein Resource (UniProt) and subscription provided databases such as GenSeq (e.g., Derwent). Polynucleotide sequences may be a wild type polynucleotide sequence encoding a given GH (e.g., either full length or mature), or in some instances the sequence may be a variant of the wild type polynucleotide sequence (e.g., a polynucleotide which encodes the wild type biologically active protein, wherein the DNA sequence of the polynucleotide has been optimized, for example, for expression in a particular species; or a polynucleotide encoding a variant of the wild type protein, such as a site directed mutant or an allelic variant. It is well within the ability of the skilled artisan to use a wild-type or consensus cDNA sequence or a codon-optimized variant of a GH to create fusion protein constructs contemplated by the invention using methods known in the art and/or in conjunction with the guidance and methods provided herein, and described more fully in the Examples of Schellenberger et al. WO10/144502A2.

The GH for inclusion in the hGH-XTEN of the disclosure include any growth hormone or sequence variant of biologic, therapeutic, prophylactic, or diagnostic interest or function, or that is useful for mediating or preventing or ameliorating a disease, disorder or condition associated with growth, growth hormone deficiency or defect when administered to a subject. Of particular interest are hGH-XTEN fusion protein compositions for which an increase in a pharmacokinetic parameter, increased solubility, increased stability, or some other enhanced pharmaceutical or pharmacodynamic property compared to native GH is sought, or for which increasing the terminal half-life would improve efficacy, safety, or result in reduce dosing frequency and/or improve patient compliance. Thus, the hGH-XTEN fusion protein compositions are prepared with various objectives in mind, including improving the therapeutic efficacy of the bioactive GH by, for example, increasing the in vivo exposure or the length that the hGH-XTEN remains within the therapeutic window when administered to a subject, compared to a GH not linked to XTEN.

In one embodiment, the GH incorporated into the subject compositions can be a recombinant polypeptide with a sequence corresponding to a protein found in nature, such as human growth hormone. In one embodiment, the GH is human GH comprising the following amino acid sequence:

In another embodiment, the GH is a sequence variant, fragment, homolog, or mimetic of a natural sequence that retain at least a portion of the biological activity of the native GH. In non-limiting examples, a GH is a sequence that exhibits at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity to the protein sequence of SEQ ID NO: 2. In one embodiment, the hGH-XTEN fusion protein comprises a single GH molecule linked to an XTEN (as described more fully below). In another embodiment, the hGH-XTEN fusion protein comprises a single GH molecule linked to a first and a second XTEN, with an N- to C-terminus configuration of XTEN-GH-XTEN, in which the GH is a sequence that exhibits at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity to the human growth hormone protein sequence (SEQ ID NO: 2), and the first and/or the second XTEN are sequences that exhibits at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity to a sequence selected from Table 3.

In general, the GH fusion partner component of the hGH-XTEN exhibits a binding specificity to a given target or another desired biological characteristic when used in vivo or when utilized in an in vitro assay. For example, the hGH-XTEN is an agonist, having the ability to bind to a transmembrane receptor for growth hormone. In one embodiment, the binding of hGH-XTEN to growth receptor leads to receptor dimerization and lead to at least a portion of the activation of intercellular signal transduction pathway compared to native growth hormone. In one embodiment, the hGH-XTEN bound to a transmembrane receptor for growth hormone would exhibit at least about 1%, or about 5%, or about 10%, or about 15%, or about 20%, or about 25%, or about 30%, or about 40%, or about 50%, or about 60%, or about 70%, or about 80%, or about 90%, or at least about 95% of the activation of intercellular signal transduction pathway compared to native growth hormone not linked to XTEN.

The subject hGH-XTEN of the present invention exhibits an enhancement of one or more pharmacokinetic or pharmacodynamic parameters, which optionally is enhanced by release of GH from the fusion protein by cleavage of a spacer sequence. The hGH-XTEN with enhanced pharmacokinetic parameters permits less frequent dosing or an enhanced pharmacologic effect, such as but not limited to maintaining the biologically active hGH-XTEN within the therapeutic window between the minimum effective dose or blood concentration (Cmin) and the maximum tolerated dose or blood concentration (Cmax). In addition, the hGH-XTEN with enhanced pharmacodynamic parameters permits lower and/or less frequent dosing or an enhanced pharmacodynamic effect, such as but not limited to a sustained or normalized IGF-I standard deviation score (IGF-I SDS). In such cases, the linking of the GH to a fusion protein comprising a select XTEN sequence(s) can result in an improvement in these properties, making them more useful as therapeutic or preventive agents compared to GH not linked to XTEN.

### IV). XTENDED RECOMBINANT POLYPEPTIDES

The present invention concerns an improved therapeutic regimen for GHD therapy, as defined by the claims. Disclosed herein are methods for bolus dose administration of a human growth hormone-XTEN (hGH-XTEN) fusion protein to a patient with GHD. Accordingly, in one aspect, the present disclosure concerns a method of treating human growth hormone deficiency (GHD) with a hGH-XTEN recombinant polypeptide or fusion protein.

In another aspect, the present disclosure provides XTEN polypeptide compositions that are useful as a fusion protein partner to which GH is linked, resulting in a hGH-XTEN fusion protein. XTEN are generally extended length polypeptides with non-naturally occurring, substantially non-repetitive sequences that are composed mainly of small hydrophilic amino acids, with the sequence having a low degree or no secondary or tertiary structure under physiologic conditions.

XTENs have utility as a fusion protein partners partner in that they serve as a "carrier", conferring certain desirable pharmacokinetic, physicochemical and pharmaceutical properties when linked to a GH protein to a create a fusion protein. Such desirable properties include but are not limited to enhanced pharmacokinetic parameters and solubility characteristics the compositions, amongst other properties described herein. Such fusion protein compositions have utility to treat certain growth hormone-related diseases, disorders or conditions, as described herein. As used herein, "XTEN" specifically excludes antibodies or antibody fragments such as single-chain antibodies or Fc fragments of a light chain or a heavy chain.

In some embodiments, XTEN are long polypeptides having greater than about 100 to about 3000 amino acid residues, preferably greater than 400 to about 3000 residues when used as a carrier or cumulatively when more than one XTEN unit is used in a single fusion protein. In other embodiments, when used as a linker between fusion protein components or where an increase in half-life of the fusion protein is not needed but where an increase in solubility or other physico/chemical property for the GH fusion partner component is desired, an XTEN sequence shorter than 100 amino acid residues, such as about 96, or about 84, or about 72, or about 60, or about 48, or about 36 amino acid residues are incorporated into a fusion protein composition with the GH to effect the property.

The selection criteria for the XTEN to be linked to the biologically active proteins used to create the inventive fusion proteins compositions generally relate to attributes of physical/chemical properties and conformational structure of the XTEN that is, in turn, used to confer enhanced pharmaceutical and pharmacokinetic properties to the fusion proteins. The XTEN of the present disclosure exhibit one or more of the following advantageous properties: conformational flexibility, enhanced aqueous solubility, high degree of protease resistance, low immunogenicity, low binding to mammalian receptors, and increased hydrodynamic (or Stokes) radii; properties that make them particularly useful as fusion protein partners. Non-limiting examples of the properties of the fusion proteins comprising GH that is enhanced by XTEN include increases in the overall solubility and/or metabolic stability, reduced susceptibility to proteolysis, reduced immunogenicity, reduced rate of absorption when administered subcutaneously or intramuscularly, and enhanced pharmacokinetic properties such as longer terminal half-life and increased area under the curve (AUC), slower absorption after subcutaneous or intramuscular injection (compared to GH not linked to XTEN and administered by a similar route) such that the Cmax is lower, which, in turn, results in reductions in adverse effects of the GH that, collectively, results in an increased period of time that a fusion protein of a hGH-XTEN composition administered to a subject retains therapeutic activity.

A variety of methods and assays are known in the art for determining the physical/chemical properties of proteins such as the compositions comprising the inventive XTEN; properties such as secondary or tertiary structure, solubility, protein aggregation, melting properties, contamination and water content. Such methods include analytical centrifugation, EPR, HPLC-ion exchange, HPLC-size exclusion, HPLC-reverse phase, light scattering, capillary electrophoresis, circular dichroism, differential scanning calorimetry, fluorescence, HPLC-ion exchange, HPLC-size exclusion, IR, NMR, Raman spectroscopy, refractometry, and UV/Visible spectroscopy. Additional methods are disclosed in Arnau et al, Prot Expr and Purif (2006) 48, 1-13. Application of these methods to the disclosure would be within the grasp of a person skilled in the art.

Typically, XTEN are designed to behave like denatured peptide sequences under physiological conditions, despite the extended length of the polymer. Denatured describes the state of a peptide in solution that is characterized by a large conformational freedom of the peptide backbone. Most peptides and proteins adopt a denatured conformation in the presence of high concentrations of denaturants or at elevated temperature. Peptides in denatured conformation have, for example, characteristic circular dichroism (CD) spectra and are characterized by a lack of long-range interactions as determined by NMR. "Denatured conformation" and "unstructured conformation" are used synonymously herein. In some embodiments, the disclosure provides XTEN sequences that, under physiologic conditions, resemble denatured sequences largely devoid in secondary structure. In other cases, the XTEN sequences are substantially devoid of secondary structure under physiologic conditions. "Largely devoid," as used in this context, means that less than 50% of the XTEN amino acid residues of the XTEN sequence contribute to secondary structure as measured or determined by the means described herein. "Substantially devoid," as used in this context, means that at least about 60%, or about 70%, or about 80%, or about 90%, or about 95%, or at least about 99% of the XTEN amino acid residues of the XTEN sequence do not contribute to secondary structure, as measured or determined by the methods described herein.

A variety of methods have been established in the art to discern the presence or absence of secondary and tertiary structures in a given polypeptide. In particular, secondary structure can be measured spectrophotometrically, e.g., by circular dichroism spectroscopy in the "far-UV" spectral region (190-250 nm). Secondary structure elements, such as alpha-helix and beta-sheet, each give rise to a characteristic shape and magnitude of CD spectra. Secondary structure can also be predicted for a polypeptide sequence via certain computer programs or algorithms, such as the well-known Chou-Fasman algorithm (Chou, P. Y., et al. (1974) Biochemistry, 13: 222-45) and the Garnier-Osguthorpe-Robson ("GOR") algorithm (Gamier J, Gibrat JF, Robson B. (1996), GOR method for predicting protein secondary structure from amino acid sequence. Methods Enzymol 266:540-553), as described in US Patent Application Publication No. 20030228309A1. For a given sequence, the algorithms can predict whether there exists some or no secondary structure at all, expressed as the total and/or percentage of residues of the sequence that form, for example, alpha-helices or beta-sheets or the percentage of residues of the sequence predicted to result in random coil formation (which lacks secondary structure).

In some embodiments, the XTEN sequences used in the inventive fusion protein compositions can have an alpha-helix percentage ranging from 0% to less than about 5% as determined by the Chou-Fasman algorithm. In other cases, the XTEN sequences of the fusion protein compositions have a beta-sheet percentage ranging from 0% to less than about 5% as determined by the Chou-Fasman algorithm. In some embodiments, the XTEN sequences of the fusion protein compositions have an alpha-helix percentage ranging from 0% to less than about 5% and a beta-sheet percentage ranging from 0% to less than about 5% as determined by the Chou-Fasman algorithm. In some embodiments, the XTEN sequences of the fusion protein compositions have an alpha-helix percentage less than about 2% and a beta-sheet percentage less than about 2%. In other cases, the XTEN sequences of the fusion protein compositions have a high degree of random coil percentage, as determined by the GOR algorithm. In some embodiments, an XTEN sequence have at least about 80%, more preferably at least about 90%, more preferably at least about 91%, more preferably at least about 92%, more preferably at least about 93%, more preferably at least about 94%, more preferably at least about 95%, more preferably at least about 96%, more preferably at least about 97%, more preferably at least about 98%, and most preferably at least about 99% random coil, as determined by the GOR algorithm.

### 1. Non-repetitive Sequences

In some embodiments, XTEN sequences of the compositions are substantially non-repetitive. In general, repetitive amino acid sequences have a tendency to aggregate or form higher order structures, as exemplified by natural repetitive sequences such as collagens and leucine zippers, or form contacts resulting in crystalline or pseudocrystaline structures. In contrast, the low tendency of non-repetitive sequences to aggregate enables the design of long-sequence XTENs with a relatively low frequency of charged amino acids that would be likely to aggregate if the sequences were otherwise repetitive. Typically, the hGH-XTEN fusion proteins comprise XTEN sequences of greater than about 100 to about 3000 amino acid residues, preferably greater than 400 to about 3000 cumulative residues, wherein the sequences are substantially non-repetitive. In one embodiment, the XTEN sequences have greater than about 100 to about 3000 amino acid residues, preferably greater than 400 to about 3000 amino acid residues, in which no three contiguous amino acids in the sequence are identical amino acid types unless the amino acid is serine, in which case no more than three contiguous amino acids are serine residues. In the foregoing embodiment, the XTEN sequence would be substantially non-repetitive.

The degree of repetitiveness of a polypeptide or a gene are measured by computer programs or algorithms or by other means known in the art. Repetitiveness in a polypeptide sequence can, for example, be assessed by determining the number of times shorter sequences of a given length occur within the polypeptide. For example, a polypeptide of 200 amino acid residues has 192 overlapping 9-amino acid sequences (or 9-mer "frames") and 198 3-mer frames, but the number of unique 9-mer or 3-mer sequences will depend on the amount of repetitiveness within the sequence. A score is generated (hereinafter "subsequence score") that is reflective of the degree of repetitiveness of the subsequences in the overall polypeptide sequence. In the context of the present invention, "subsequence score" means the sum of occurrences of each unique 3-mer frame across a 200 consecutive amino acid sequence of the polypeptide divided by the absolute number of unique 3-mer subsequences within the 200 amino acid sequence. Examples of such subsequence scores derived from the first 200 amino acids of repetitive and non-repetitive polypeptides are presented in Example 44 of Schellenberger et al. WO10/144502A2. In some embodiments, the present disclosure provides hGH-XTEN each comprising one or more
XTEN in which the XTEN have a subsequence score less than 12, more preferably less than 10, more preferably less than 9, more preferably less than 8, more preferably less than 7, more preferably less than 6, and most preferably less than 5. In the embodiments hereinabove described in this paragraph, an XTEN with a subsequence score less than about 10 (i.e., 9, 8, 7, etc.) is "substantially non-repetitive."

The non-repetitive characteristic of XTEN impart to fusion proteins with GH a greater degree of solubility and less tendency to aggregate compared to polypeptides having repetitive sequences. These properties facilitate the formulation of XTEN-comprising pharmaceutical preparations containing extremely high drug concentrations, in some cases exceeding 100 mg/ml.

Furthermore, the XTEN polypeptide sequences of the embodiments are designed to have a low degree of internal repetitiveness in order to reduce or substantially eliminate immunogenicity when administered to a mammal. Polypeptide sequences composed of short, repeated motifs largely limited to three amino acids, such as glycine, serine and glutamate, may result in relatively high antibody titers when administered to a mammal despite the absence of predicted T-cell epitopes in these sequences. This may be caused by the repetitive nature of polypeptides, as it has been shown that immunogens with repeated epitopes, including protein aggregates, cross-linked immunogens, and repetitive carbohydrates are highly immunogenic and can, for example, result in the cross-linking of B-cell receptors causing B-cell activation. (Johansson, J., et al. (2007) Vaccine, 25 :1676-82 ; Yankai, Z., et al. (2006) Biochem Biophys Res Commun, 345 :1365-71 ; Hsu, C. T., et al. (2000) Cancer Res, 60:3701-5); Bachmann MF, et al. Eur J Immunol. (1995) 25(12):3445-3451).

### 2. Exemplary Sequence Motifs

The present disclosure encompasses XTEN that comprise multiple units of shorter sequences, or motifs, in which the amino acid sequences of the motifs are non-repetitive. In designing XTEN sequences, it was discovered that the non-repetitive criterion may be met despite the use of a "building block" approach using a library of sequence motifs that are multimerized to create the XTEN sequences. Thus, while an XTEN sequence may consist of multiple units of as few as four different types of sequence motifs, because the motifs themselves generally consist of non-repetitive amino acid sequences, the overall XTEN sequence is rendered substantially non-repetitive.

In one embodiment, XTEN have a non-repetitive sequence of greater than about 100 to about 3000 amino acid residues, preferably greater than 400 to about 3000 residues, wherein at least about 80%, or at least about 85%, or at least about 90%, or at least about 95%, or at least about 97%, or about 100% of the XTEN sequence consists of non-overlapping sequence motifs, wherein each of the motifs has about 9 to 36 amino acid residues. In other embodiments, at least about 80%, or at least about 85%, or at least about 90%, or at least about 95%, or at least about 97%, or about 100% of the XTEN sequence consists of non-overlapping sequence motifs wherein each of the motifs has 9 to 14 amino acid residues. In still other embodiments, at least about 80%, or at least about 85%, or at least about 90%, or at least about 95%, or at least about 97%, or about 100% of the XTEN sequence component consists of non-overlapping sequence motifs wherein each of the motifs has 12 amino acid residues. In these embodiments, it is preferred that the sequence motifs be composed mainly of small hydrophilic amino acids, such that the overall sequence has an unstructured, flexible characteristic. Examples of amino acids that are included in XTEN, are, e.g., arginine, lysine, threonine, alanine, asparagine, glutamine, aspartate, glutamate, serine, and glycine. As a result of testing variables such as codon optimization, assembly polynucleotides encoding sequence motifs, expression of protein, charge distribution and solubility of expressed protein, and secondary and tertiary structure, it was discovered that XTEN compositions with enhanced characteristics mainly include glycine (G), alanine (A), serine (S), threonine (T), glutamate (E) and proline (P) residues wherein the sequences are designed to be substantially non-repetitive. In one embodiment, XTEN sequences have predominately four to six types of amino acids selected from glycine (G), alanine (A), serine (S), threonine (T), glutamate (E) or proline (P) that are arranged in a substantially non-repetitive sequence that is greater than about 100 to about 3000 amino acid residues, preferably greater than 400 to about 3000 residues in length. In some embodiments, XTEN have sequences of greater than about 100 to about 3000 amino acid residues, preferably greater than 400 to about 3000 residues, wherein at least about 80% of the sequence consists of non-overlapping sequence motifs wherein each of the motifs has 9 to 36 amino acid residues wherein each of the motifs consists of 4 to 6 types of amino acids selected from glycine (G), alanine (A), serine (S), threonine (T), glutamate (E) and proline (P), and wherein the content of any one amino acid type in the full-length XTEN does not exceed 30%. In other embodiments, at least about 90% of the XTEN sequence consists of non-overlapping sequence motifs wherein each of the motifs has 9 to 36 amino acid residues wherein the motifs consist of 4 to 6 types of amino acids selected from glycine (G), alanine (A), serine (S), threonine (T), glutamate (E) and proline (P), and wherein the content of any one amino acid type in the full-length XTEN does not exceed 30%. In other embodiments, at least about 90% of the XTEN sequence consists of non-overlapping sequence motifs wherein each of the motifs has 12 amino acid residues consisting of 4 to 6 types of amino acids selected from glycine (G), alanine (A), serine (S), threonine (T), glutamate (E) and proline (P), and wherein the content of any one amino acid type in the full-length XTEN does not exceed 30%. In yet other embodiments, at least about 90%, or about 91%, or about 92%, or about 93%, or about 94%, or about 95%, or about 96%, or about 97%, or about 98%, or about 99%, to about 100% of the XTEN sequence consists of non-overlapping sequence motifs wherein each of the motifs has 12 amino acid residues consisting of glycine (G), alanine (A), serine (S), threonine (T), glutamate (E) and proline (P), and wherein the content of any one amino acid type in the full-length XTEN does not exceed 30%.

In still other embodiments, XTENs comprise non-repetitive sequences of greater than about 100 to about 3000 amino acid residues, preferably greater than 400 to about 3000 amino acid residues wherein at least about 80%, or at least about 90%, or about 91%, or about 92%, or about 93%, or about 94%, or about 95%, or about 96%, or about 97%, or about 98%, or about 99% of the sequence consists of non-overlapping sequence motifs of 9 to 14 amino acid residues wherein the motifs consist of 4 to 6 types of amino acids selected from glycine (G), alanine (A), serine (S), threonine (T), glutamate (E) and proline (P), and wherein the sequence of any two contiguous amino acid residues in any one motif is not repeated more than twice in the sequence motif. In other embodiments, at least about 90%, or about 91%, or about 92%, or about 93%, or about 94%, or about 95%, or about 96%, or about 97%, or about 98%, or about 99% of an XTEN sequence consists of non-overlapping sequence motifs of 12 amino acid residues wherein the motifs consist of 4 to 6 types of amino acids selected from glycine (G), alanine (A), serine (S), threonine (T), glutamate (E) and proline (P), and wherein the sequence of any two contiguous amino acid residues in any one sequence motif is not repeated more than twice in the sequence motif. In other embodiments, at least about 90%, or about 91%, or about 92%, or about 93%, or about 94%, or about 95%, or about 96%, or about 97%, or about 98%, or about 99% of an XTEN sequence consists of non-overlapping sequence motifs of 12 amino acid residues wherein the motifs consist of glycine (G), alanine (A), serine (S), threonine (T), glutamate (E) and proline (P), and wherein the sequence of any two contiguous amino acid residues in any one sequence motif is not repeated more than twice in the sequence motif. In yet other embodiments, XTENs consist of 12 amino acid sequence motifs wherein the amino acids are selected from glycine (G), alanine (A), serine (S), threonine (T), glutamate (E) and proline (P), and wherein the sequence of any two contiguous amino acid residues in any one sequence motif is not repeated more than twice in the sequence motif, and wherein the content of any one amino acid type in the full-length XTEN does not exceed 30%. In the foregoing embodiments hereinabove described in this paragraph, the XTEN sequences would be substantially non-repetitive.

In some embodiments, the disclosure provides compositions comprising non-repetitive XTEN sequence(s) of greater than about 100 to about 3000 amino acid residues, of cumulatively greater than 400 to about 3000 residues, wherein at least about 80%, or at least about 90%, or about 91%, or about 92%, or about 93%, or about 94%, or about 95%, or about 96%, or about 97%, or about 98%, or about 99% to about 100% of the sequence consists of multiple units of two or more non-overlapping sequence motifs selected from the amino acid sequences of Table 2. In some embodiments, the XTEN comprises non-overlapping sequence motifs in which about 80%, or at least about 90%, or about 91%, or about 92%, or about 93%, or about 94%, or about 95%, or about 96%, or about 97%, or about 98%, or about 99% to about 100% of the sequence consists of two or more non-overlapping sequences selected from a single motif family of Table 2, resulting in a "family" sequence in which the overall sequence remains substantially non-repetitive. Accordingly, in these embodiments, an XTEN sequence comprises multiple units of non-overlapping sequence motifs of the AD motif family, or the AE motif family, or the AF motif family, or the AG motif family, or the AM motif family of sequences of Table 2. In other embodiments, the XTEN comprises motif sequences from two or more of the motif families of Table 2. In other embodiments, the XTEN comprises motif sequences from two or more of the motif families of Table 2.

**Table 2: XTEN Sequence Motifs of 12 Amino Acids and Motif Families**

| **Motif Family*** | **SEQ ID NO:** | **MOTIF SEQUENCE** |
|---|---|---|
| AD | 15 | GESPGGSSGSES |
| AD | 16 | GSEGSSGPGESS |
| AD | 17 | GSSESGSSEGGP |
| AD | 18 | GSGGEPSESGSS |
| AE, AM | 19 | GSPAGSPTSTEE |
| AE, AM, AQ | 20 | GSEPATSGSETP |
| AE, AM, AQ | 21 | GTSESATPESGP |
| AE, AM, AQ | 22 | GTSTEPSEGSAP |
| AF, AM | 23 | GSTSESPSGTAP |
| AF, AM | 24 | GTSTPESGSASP |
| AF, AM | 25 | GTSPSGESSTAP |
| AF, AM | 26 | GSTSSTAESPGP |
| AG, AM | 27 | GTPGSGTASSSP |
| AG, AM | 28 | GSSTPSGATGSP |
| AG, AM | 29 | GSSPSASTGTGP |
| AG, AM | 30 | GASPGTSSTGSP |

| | | |
|---|---|---|
| Denotes individual motif sequences that, when used together in various permutations, results in a "family sequence" | | |

In other embodiments, the hGH-XTEN composition comprises a non-repetitive XTEN sequence of greater than about 100 to about 3000 amino acid residues, preferably greater than 400 to about 3000 residues, wherein at least about 80%, or at least about 90%, or about 91%, or about 92%, or about 93%, or about 94%, or about 95%, or about 96%, or about 97%, or about 98%, or about 99% to about 100% of the sequence consists of non-overlapping 36 amino acid sequence motifs selected from one or more of the polypeptide sequences of Tables 8-11 of Schellenberger et al. WO10/144502A2.

In those embodiments wherein the XTEN component of the hGH-XTEN fusion protein has less than 100% of its amino acids consisting of four to six amino acid selected from glycine (G), alanine (A), serine (S), threonine (T), glutamate (E) and proline (P), or less than 100% of the sequence consisting of the sequence motifs of Table 2, or less than 100% sequence identity with an XTEN from Table 3, the other amino acid residues are selected from any other of the 14 natural L-amino acids, but are preferentially selected from hydrophilic amino acids such that the XTEN sequence contains at least about 90%, or at least about 91%, or at least about 92%, or at least about 93%, or at least about 94%, or at least about 95%, or at least about 96%, or at least about 97%, or at least about 98%, or at least about 99% hydrophilic amino acids. The XTEN amino acids that are not glycine (G), alanine (A), serine (S), threonine (T), glutamate (E) and proline (P) are interspersed throughout the XTEN sequence, are located within or between the sequence motifs, or are concentrated in one or more short stretches of the XTEN sequence. In such cases where the XTEN component of the hGH-XTEN comprises amino acids other than glycine (G), alanine (A), serine (S), threonine (T), glutamate (E) and proline (P), it is preferred that the amino acids not be hydrophobic residues and should not substantially confer secondary structure of the XTEN component. Hydrophobic residues that are less favored in construction of XTEN include tryptophan, phenylalanine, tyrosine, leucine, isoleucine, valine, and methionine. Additionally, one can design the XTEN sequences to contain few (e.g. less than 5%) or none of the following amino acids: cysteine (to avoid disulfide formation and oxidation), methionine (to avoid oxidation), asparagine and glutamine (to avoid desamidation). Thus, in some embodiments, the XTEN component of the hGH-XTEN fusion protein comprising other amino acids in addition to glycine (G), alanine (A), serine (S), threonine (T), glutamate (E) and proline (P) would have a sequence with less than 5% of the residues contributing to alpha-helices and beta-sheets as measured by the Chou-Fasman algorithm and have at least 90%, or at least about 95% or more random coil formation as measured by the GOR algorithm.

### 3. Length of Sequence

In another aspect of the present disclosure, the invention encompasses hGH-XTEN compositions comprising carriers of XTEN polypeptides with extended length sequences. The present invention makes use of the discovery that increasing the length of non-repetitive, unstructured polypeptides enhances the unstructured nature of the XTENs and correspondingly enhances the biological and pharmacokinetic properties of fusion proteins comprising the XTEN carrier. As described more fully in the Examples, proportional increases in the length of the XTEN, even if created by a fixed repeat order of single family sequence motifs (e.g., the four AE motifs of Table 2), result in a sequence with a higher percentage of random coil formation, as determined by GOR algorithm, compared to shorter XTEN lengths. In general, increasing the length of the unstructured polypeptide fusion partner, as described in the Examples, results in a fusion protein with a disproportional increase in terminal half-life compared to fusion proteins with unstructured polypeptide partners with shorter sequence lengths.

Non-limiting examples of XTEN contemplated for inclusion in the hGH-XTEN of the disclosure are presented in Table 3. In one embodiment, the disclosure provides hGH-XTEN compositions wherein the XTEN sequence length of the fusion protein(s) is greater than about 100 to about 3000 amino acid residues, and in some cases is greater than 400 to about 3000 amino acid residues, wherein the XTEN confers enhanced pharmacokinetic properties on the hGH-XTEN in comparison to GH not linked to XTEN. In some embodiments, the XTEN sequences of the hGH-XTEN compositions of the present disclosure can be about 100, or about 144, or about 288, or about 401, or about 500, or about 600, or about 700, or about 800, or about 900, or about 1000, or about 1500, or about 2000, or about 2500 or up to about 3000 amino acid residues in length. In other cases, the XTEN sequences can be about 100 to 150, about 150 to 250, about 250 to 400, 401 to about 500, about 500 to 900, about 900 to 1500, about 1500 to 2000, or about 2000 to about 3000 amino acid residues in length. In one embodiment, the hGH-XTEN can comprise an XTEN sequence wherein the sequence exhibits at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity to a XTEN selected from Table 3. In some embodiments, the XTEN sequence is designed for optimized expression as the N-terminal component of the hGH-XTEN by inclusion of encoding nucleotides for an optimized N-terminal leader sequence (NTS) in the XTEN portion of the gene encoding the fusion protein. In another embodiment, the N-terminal XTEN sequence of the expressed hGH-XTEN has at least 90% sequence identity to any sequence selected from Table 3. In one embodiment, the N-terminal XTEN sequence of the expressed hGH-XTEN has at least 90% sequence identity to the sequence of AE48 or AM48, AE624, AE911, AE912 or AM923.

In other embodiments, the hGH-XTEN fusion protein comprises a first and a second XTEN sequence, wherein the cumulative total of the residues in the XTEN sequences is greater than about 400 to about 3000 amino acid residues. In embodiments of the foregoing, the hGH-XTEN fusion protein comprises a first and a second XTEN sequence wherein the sequences each exhibit at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity to at least a first or additionally a second XTEN selected from Table 3. Examples where more than one XTEN is used in a hGH-XTEN composition include, but are not limited to constructs with an XTEN linked to both the N- and C-termini of at least one GH.

As described more fully below, the disclosure provides methods in which the hGH-XTEN is designed by selecting the length of the XTEN to confer a target half-life on a fusion protein administered to a subject. In general, XTEN lengths longer that about cumulative 400 residues incorporated into the hGH-XTEN compositions result in longer half-life compared to shorter cumulative lengths; e.g., shorter than about 280 residues. However, in another embodiment, hGH-XTEN fusion proteins are designed to comprise XTEN with a longer sequence length that is selected to additionally confer slower rates of systemic absorption after subcutaneous or intramuscular administration to a subject. In such embodiments, the Cmax is reduced in comparison to a comparable dose of a GH not linked to XTEN, thereby contributing to the ability to keep the hGH-XTEN within the therapeutic window for the composition. Thus, the XTEN confers the property of a depot to the administered hGH-XTEN, in addition to the other physical/chemical properties described herein.

**Table 3: XTEN Polypeptides**

| **XTEN Name** | **SEQ ID NO:** | **Amino Acid Sequence** |
|---|---|---|
| AE48 | 31 | MAEPAGSPTSTEEGTPGSGTASSSPGSSTPSGATGSPGASPGTSSTGS |
| AM48 | 32 | MAEPAGSPTSTEEGASPGTSSTGSPGSSTPSGATGSPGSSTPSGATGS |
| AE144 | 33 | |
| AF144 | 34 | |
| AE288 | 35 | |
| AF504 | 36 | |
| AF540 | 37 | |
| AD576 | 38 | |
| | | |
| AE576 | 39 | |
| AF576 | 40 | |
| AE624 | 41 | |
| AD836 | 42 | |
| AE864 | 43 | |
| AF864 | 44 | |
| AG864 | 45 | |
| AM875 | 46 | |
| | | |
| AE912 | 47 | |
| AM923 | 48 | |
| AM1318 | 49 | |
| | | |
| BC 864 | 50 | |
| BD864 | 51 | |
| AE911 | 52 | |
| | | |
| AE146 | 53 | |
| AE48.1 | 81 | AEPAGSPTSTEEGTPGSGTASSSPGSSTPSGATGSPGASPGTSSTGS |
| AM48.1 | 82 | AEPAGSPTSTEEGASPGTSSTGSPGSSTPSGATGSPGSSTPSGATGS |
| AE912.1 | 83 | |
| AE912.2 | 84 | |
| AE146.1 | 85 | |

### 4. XTEN segments

In one embodiment, the disclosure provides an isolated hGH-XTEN fusion protein wherein the cumulative length of the XTEN component is greater than about 100 to about 3000 amino acid residues containing at least one polypeptide sequence segment selected from Table 3 (and Tables 8, 9, 10, 11, and 12 of Schellenberger et al. WO10/144502A2, which is incorporated herein by reference in its entirety) and wherein at least about 90%, or at least about 91%, or at least about 92%, or at least about 93%, or at least about 94%, or at least about 95%, or at least about 96%, or at least about 97%, or at least about 98% or more of the remainder of the XTEN sequence by and large contains hydrophilic amino acids and less than about 2% of the remainder of the XTEN consists of hydrophobic or aromatic amino acids, or cysteine. In some embodiments, the XTEN contains multiple segments wherein the segments are identical or different. In another embodiment, the disclosure provides an isolated hGH-XTEN fusion protein wherein the cumulative length of the XTEN component is greater than about 100 to about 3000 amino acid residues and comprises at least one sequence segment of at least about 100 to about 923, or at least about 100 to about 875, or at least about 100 to about 576, or at least about 100 to about 288, or at least about 100 to about 144 amino acid residues wherein the sequence segment(s) consists of at least three different types of amino acids and the sum of glycine (G), alanine (A), serine (S), threonine (T), glutamate (E) and proline (P) residues in the sequence segment(s) constitutes at least about 90%, or at least about 91%, or at least about 92%, or at least about 93%, or at least about 94%, or at least about 95%, or at least about 96%, or at least about 97%, or at least about 98%, or at least about 99% of the total amino acid sequence of the sequence segment and at least about 90%, or at least about 91%, or at least about 92%, or at least about 93%, or at least about 94%, or at least about 95%, or at least about 96%, or at least about 97%, or at least about 98% of the remainder of the XTEN sequence(s) consist of hydrophilic amino acids and less than about 2% of the remainder of the XTEN sequence(s) consists of hydrophobic or aromatic amino acids, or cysteine. In another embodiment, the disclosure provides an isolated hGH-XTEN fusion protein wherein the cumulative length of the XTEN component is greater than about 100 to about 3000 amino acid residues and comprises at least one sequence segment of at least about 200 to about 923, or at least about 200 to about 875, or at least about 200 to about 576, or at least about 200 to about 288 amino acid residues wherein the sequence segment(s) the sum of glycine (G), alanine (A), serine (S), threonine (T), glutamate (E) and proline (P) residues in the sequence segment(s) constitutes at least about 90%, or at least about 91%, or at least about 92%, or at least about 93%, or at least about 94%, or at least about 95%, or at least about 96%, or at least about 97%, or at least about 98%, or at least about 99% of the total amino acid sequence of the sequence segment and wherein the subsequence score of the segment is less than 12, more preferably less than 10, more preferably less than 9, more preferably less than 8, more preferably less than 7, more preferably less than 6, and most preferably less than 5, and at least about 90%, or at least about 91%,, or at least about 92%, or at least about 93%, or at least about 94%, or at least about 95%, or at least about 96%, or at least about 97%, or at least about 98% of the remainder of the XTEN sequence(s) consist of hydrophilic amino acids and less than about 2% of the remainder of the XTEN sequence(s) consists of hydrophobic, aromatic or cysteine amino acids.

### 5. N-terminal XTEN expression-enhancing sequences

In some embodiments, the disclosure provides a short-length XTEN sequence incorporated as the N-terminal portion of the hGH-XTEN fusion protein. The expression of the fusion protein is enhanced in a host cell transformed with a suitable expression vector comprising an optimized N-terminal leader polynucleotide sequence (that encodes the N-terminal XTEN) incorporated into the polynucleotide encoding the binding fusion protein. It has been discovered, as described in Examples 14-17 of Schellenberger et al. WO10/144502A2, that a host cell transformed with such an expression vector comprising an optimized N-terminal leader sequence (NTS) in the binding fusion protein gene results in greatly-enhanced expression of the fusion protein compared to the expression of a corresponding fusion protein from a polynucleotide not comprising the NTS, and obviates the need for incorporation of a non-XTEN leader sequence used to enhance expression. In one embodiment, the disclosure provides hGH-XTEN fusion proteins comprising an NTS wherein the expression of the binding fusion protein from the encoding gene in a host cell is enhanced about 50%, or about 75%, or about 100%, or about 150%, or about 200%, or about 400% compared to expression of a hGH-XTEN fusion protein not comprising the N-terminal XTEN sequence (where the encoding gene lacks the NTS).

In one embodiment, the N-terminal XTEN polypeptide of the hGH-XTEN comprises a sequence that exhibits at least about 80%, more preferably at least about 90%, more preferably at least about 91%, more preferably at least about 92%, more preferably at least about 93%, more preferably at least about 94%, more preferably at least about 95%, more preferably at least about 96%, more preferably at least about 97%, more preferably at least about 98%, more preferably at least 99%, or exhibits 100% sequence identity to the amino acid sequence of AE48, AE48.1, AM48, or AM48.1, the respective amino acid sequences of which are as follows:
AE48: MAEPAGSPTSTEEGTPGSGTASSSPGSSTPSGATGSPGASPGTSSTGS (SEQ ID NO: 54)
AE48.1: AEPAGSPTSTEEGTPGSGTASSSPGSSTPSGATGSPGASPGTSSTGS (SEQ ID NO: 81)
AM48: MAEPAGSPTSTEEGASPGTSSTGSPGSSTPSGATGSPGSSTPSGATGS (SEQ ID NO: 55)
AM48.1: AEPAGSPTSTEEGASPGTSSTGSPGSSTPSGATGSPGSSTPSGATGS (SEQ ID NO:82).

In another embodiment, the N-terminal XTEN polypeptide of the hGH-XTEN comprises a sequence exhibiting a % identity to AE48, AM48 or AE912, as described herein, wherein the N-terminal M residue is absent (*e.g*., AE48.1 - SEQ ID NO: 81; AM48.1 - SEQ ID NO: 82; and AE912.1 - SEQ ID NO: 83). In an additional embodiment, the C-terminal XTEN poly peptide of the hGH-XTEN comprises a sequence exhibiting a % identity to AE146, as described herein, (*e.g.,* AE146 - SEQ ID NO: 53; or AE146.1 - SEQ ID NO: 85).

In another embodiment, the short-length N-terminal XTEN is linked to an XTEN of longer length to form the N-terminal region of the hGH-XTEN fusion protein, wherein the polynucleotide sequence encoding the short-length N-terminal XTEN confers the property of enhanced expression in the host cell, and wherein the long length of the expressed XTEN contributes to the enhanced properties of the XTEN carrier in the fusion protein, as described above. In the foregoing, the short-length XTEN is linked to any of the XTEN disclosed herein (e.g., an XTEN of Table 3) and the resulting XTEN, in turn, is linked to the N-terminal of any of the GH disclosed herein (e.g., a GH comprising the sequence of SEQ ID NO:2) as a component of the fusion protein. Alternatively, polynucleotides encoding the short-length XTEN (or its complement) is linked to polynucleotides encoding any of the XTEN (or its complement) disclosed herein and the resulting gene encoding the N-terminal XTEN, in turn, is linked to the 5' end of polynucleotides encoding any of the GH (or to the 3' end of its complement) disclosed herein. In some embodiments, the N-terminal XTEN polypeptide with long length exhibits at least about 80%, or at least about 90%, or at least about 91%, or at least about 92%, or at least about 93%, or at least about 94%, or at least about 95%, or at least about 96%, or at least about 97%, or at least about 98%, or at least 99%, or exhibits 100% sequence identity to an amino acid sequence selected from the group consisting of the sequences AE624, AE911, AE912, and AM923.

In any of the foregoing N-terminal XTEN embodiments described above, the N-terminal XTEN can have from about one to about six additional amino acid residues, preferably selected from GESTPA, to accommodate the restriction endonuclease restriction sites that would be employed to join the nucleotides encoding the N-terminal XTEN to the gene encoding the targeting moiety of the fusion protein. The methods for the generation of the N-terminal sequences and incorporation into the fusion proteins of the disclosure are described more fully in the Examples.

### 6. Net charge

In other embodiments, the XTEN polypeptides have an unstructured characteristic imparted by incorporation of amino acid residues with a net charge and/or reducing the proportion of hydrophobic amino acids in the XTEN sequence. The overall net charge and net charge density is controlled by modifying the content of charged amino acids in the XTEN sequences. In some embodiments, the net charge density of the XTEN of the compositions may be above +0.1 or below -0.1 charges/residue. In other embodiments, the net charge of a XTEN can be about 0%, about 1%, about 2%, about 3%, about 4%, about 5%, about 6%, about 7%, about 8%, about 9%, about 10% about 11%, about 12%, about 13%, about 14%, about 15%, about 16%, about 17%, about 18%, about 19%, or about 20% or more.

Since most tissues and surfaces in a human or animal have a net negative charge, in some embodiments, the XTEN sequences are designed to have a net negative charge to minimize non-specific interactions between the XTEN containing compositions and various surfaces such as blood vessels, healthy tissues, or various receptors. Not to be bound by a particular theory, the XTEN can adopt open conformations due to electrostatic repulsion between individual amino acids of the XTEN polypeptide that individually carry a net negative charge and that are distributed across the sequence of the XTEN polypeptide. Such a distribution of net negative charge in the extended sequence lengths of XTEN can lead to an unstructured conformation that, in turn, can result in an effective increase in hydrodynamic radius. In preferred embodiments, the negative charge is conferred by incorporation of glutamic acid residues. Accordingly, in one embodiment the disclosure provides XTEN in which the XTEN sequences contain about 8, 10, 15, 20, 25, or even about 30% glutamic acid. Generally, the glutamic residues would be spaced uniformly across the XTEN sequence. In some cases, the XTEN can contain about 10-80, or about 15-60, or about 20-50 glutamic residues per 20kD of XTEN that can result in an XTEN with charged residues that would have very similar pKa, which can increase the charge homogeneity of the product and sharpen its isoelectric point, enhancing the physicochemical properties of the resulting hGH-XTEN fusion protein for, example, simplifying purification procedures.

The XTEN of the compositions of the present disclosure generally have no or a low content of positively charged amino acids. In some embodiments the XTEN may have less than about 10% amino acid residues with a positive charge, or less than about 7%, or less than about 5%, or less than about 2%, or less than about 1% amino acid residues with a positive charge. However, the disclosure contemplates constructs where a limited number of amino acids with a positive charge, such as lysine, are incorporated into XTEN to permit conjugation between the epsilon amine of the lysine and a reactive group on a peptide, a linker bridge, or a reactive group on a drug or small molecule to be conjugated to the XTEN backbone. In one embodiment of the foregoing, the XTEN has between about 1 to about 100 lysine residues, or about 1 to about 70 lysine residues, or about 1 to about 50 lysine residues, or about 1 to about 30 lysine residues, or about 1 to about 20 lysine residues, or about 1 to about 10 lysine residues, or about 1 to about 5 lysine residues, or alternatively only a single lysine residue. Using the foregoing lysine-containing XTEN, fusion proteins are constructed that comprises XTEN, a growth hormone, plus a chemotherapeutic agent useful in the treatment of growth-related diseases or disorders, wherein the maximum number of molecules of the agent incorporated into the XTEN component is determined by the numbers of lysines or other amino acids with reactive side chains (e.g., cysteine) incorporated into the XTEN.

In some embodiments, the XTEN sequence comprises charged residues separated by other residues such as serine or glycine, which leads to better expression or purification behavior. Based on the net charge, some XTENs have an isoelectric point (pI) of 1.0, 1.5, 2.0, 2.5, 3.0, 3.5, 4.0, 4.5, 5.0, 5.5, 6.0, or even 6.5. In preferred embodiments, the XTEN will have an isoelectric point between 1.5 and 4.5. In these embodiments, the XTEN incorporated into the hGH-XTEN fusion protein compositions carry a net negative charge under physiologic conditions that contribute to the unstructured conformation and reduced binding of the XTEN component to mammalian proteins and tissues.

As hydrophobic amino acids impart structure to a polypeptide, the disclosure provides that the content of hydrophobic amino acids in the XTEN will typically be less than 5%, or less than 2%, or less than 1% hydrophobic amino acid content. In one embodiment, the amino acid content of methionine and tryptophan in the XTEN component of a hGH-XTEN fusion protein is typically less than 5%, or less than 2%, and most preferably less than 1%. In another embodiment, the XTEN will have a sequence that has less than 10% amino acid residues with a positive charge, or less than about 7%, or less that about 5%, or less than about 2% amino acid residues with a positive charge, the sum of methionine and tryptophan residues will be less than 2%, and the sum of asparagine and glutamine residues will be less than 10% of the total XTEN sequence.

### 7. Low immunogenicity

Also disclosed herein are compositions in which the XTEN sequences have a low degree of immunogenicity or are substantially non-immunogenic. Several factors can contribute to the low immunogenicity of XTEN, e.g., the non-repetitive sequence, the unstructured conformation, the high degree of solubility, the low degree or lack of self-aggregation, the low degree or lack of proteolytic sites within the sequence, and the low degree or lack of epitopes in the XTEN sequence.

Conformational epitopes are formed by regions of the protein surface that are composed of multiple discontinuous amino acid sequences of the protein antigen. The precise folding of the protein brings these sequences into a well-defined, stable spatial configurations, or epitopes, that can be recognized as "foreign" by the host humoral immune system, resulting in the production of antibodies to the protein or the activation of a cell-mediated immune response. In the latter case, the immune response to a protein in an individual is heavily influenced by T-cell epitope recognition that is a function of the peptide binding specificity of that individual's HLA-DR allotype. Engagement of a MHC Class II peptide complex by a cognate T-cell receptor on the surface of the T-cell, together with the cross-binding of certain other co-receptors such as the CD4 molecule, can induce an activated state within the T-cell. Activation leads to the release of cytokines further activating other lymphocytes such as B cells to produce antibodies or activating T killer cells as a full cellular immune response.

The ability of a peptide to bind a given MHC Class II molecule for presentation on the surface of an APC (antigen presenting cell) is dependent on a number of factors; most notably its primary sequence. In one embodiment, a lower degree of immunogenicity is achieved by designing XTEN sequences that resist antigen processing in antigen presenting cells, and/or choosing sequences that do not bind MHC receptors well. The invention provides hGH-XTEN fusion proteins with substantially non-repetitive XTEN polypeptides designed to reduce binding with MHC II receptors, as well as avoiding formation of epitopes for T-cell receptor or antibody binding, resulting in a low degree of immunogenicity. Avoidance of immunogenicity is, in part, a direct result of the conformational flexibility of XTEN sequences; i.e., the lack of secondary structure due to the selection and order of amino acid residues. For example, of particular interest are sequences having a low tendency to adapt compactly folded conformations in aqueous solution or under physiologic conditions that could result in conformational epitopes. The administration of fusion proteins comprising XTEN, using conventional therapeutic practices and dosing, would generally not result in the formation of neutralizing antibodies to the XTEN sequence, and also reduce the immunogenicity of the GH fusion partner in the hGH-XTEN compositions.

In one embodiment, the XTEN sequences utilized in the subject fusion proteins can be substantially free of epitopes recognized by human T cells. The elimination of such epitopes for the purpose of generating less immunogenic proteins has been disclosed previously; see for example WO 98/52976, WO 02/079232, and WO 00/3317. Assays for human T cell epitopes have been described (Stickler, M., et al. (2003) J Immunol Methods, 281: 95-108). Of particular interest are peptide sequences that can be oligomerized without generating T cell epitopes or non-human sequences. This is achieved by testing direct repeats of these sequences for the presence of T-cell epitopes and for the occurrence of 6 to 15-mer and, in particular, 9-mer sequences that are not human, and then altering the design of the XTEN sequence to eliminate or disrupt the epitope sequence. In some embodiments, the XTEN sequences are substantially non-immunogenic by the restriction of the numbers of epitopes of the XTEN predicted to bind MHC receptors. With a reduction in the numbers of epitopes capable of binding to MHC receptors, there is a concomitant reduction in the potential for T cell activation as well as T cell helper function, reduced B cell activation or upregulation and reduced antibody production. The low degree of predicted T-cell
epitopes can be determined by epitope prediction algorithms such as, e.g., TEPITOPE (Sturniolo, T., et al. (1999) Nat Biotechnol, 17: 555-61), as shown in Example 45. The TEPITOPE score of a given peptide frame within a protein is the log of the Kd (dissociation constant, affinity, off-rate) of the binding of that peptide frame to multiple of the most common human MHC alleles, as disclosed in Sturniolo, T. et al. (1999) Nature Biotechnology 17:555). The score ranges over at least 20 logs, from about 10 to about - 10 (corresponding to binding constraints of 10e10 Kd to 10e-10 Kd), and can be reduced by avoiding hydrophobic amino acids that serve as anchor residues during peptide display on MHC, such as M, I, L, V, F. In some embodiments, an XTEN component incorporated into a hGH-XTEN does not have a predicted T-cell epitope at a TEPITOPE score of about -5 or greater, or -6 or greater, or -7 or greater, or -8 or greater, or at a TEPITOPE score of -9 or greater. As used herein, a score of "-9 or greater" would encompass TEPITOPE scores of 10 to -9, inclusive, but would not encompass a score of - 10, as -10 is less than -9.

In another embodiment, the inventive XTEN sequences, including those incorporated into the subject hGH-XTEN fusion proteins, are rendered substantially non-immunogenic by the restriction of known proteolytic sites from the sequence of the XTEN, reducing the processing of XTEN into small peptides that can bind to MHC II receptors. In another embodiment, the XTEN sequence is rendered substantially non-immunogenic by the use a sequence that is substantially devoid of secondary structure, conferring resistance to many proteases due to the high entropy of the structure. Accordingly, the reduced TEPITOPE score and elimination of known proteolytic sites from the XTEN render the XTEN compositions, including the XTEN of the hGH-XTEN fusion protein compositions, substantially unable to be bound by mammalian receptors, including those of the immune system. In one embodiment, an XTEN of a hGH-XTEN fusion protein can have > 100 nM Kd binding to a mammalian receptor, or greater than 500 nM Kd, or greater than 1 µM Kd towards a mammalian cell surface or circulating polypeptide receptor.

Additionally, the non-repetitive sequence and corresponding lack of epitopes of XTEN limit the ability of B cells to bind to or be activated by XTEN. A repetitive sequence is recognized and can form multivalent contacts with even a few B cells and, as a consequence of the cross-linking of multiple T-cell independent receptors, can stimulate B cell proliferation and antibody production. In contrast, while a XTEN can make contacts with many different B cells over its extended sequence, each individual B cell may only make one or a small number of contacts with an individual XTEN due to the lack of repetitiveness of the sequence. Not being to be bound by any theory, XTENs typically have a much lower tendency to stimulate proliferation of B cells and thus an immune response. In one embodiment, the hGH-XTEN have reduced immunogenicity as compared to the corresponding GH that is not fused. In one embodiment, the administration of up to three parenteral doses of a hGH-XTEN to a mammal result in detectable anti-hGH-XTEN IgG at a serum dilution of 1:100 but not at a dilution of 1:1000. In another embodiment, the administration of up to three parenteral doses of a hGH-XTEN to a mammal result in detectable anti-GH IgG at a serum dilution of 1:100 but not at a dilution of 1:1000. In another embodiment, the administration of up to three parenteral doses of a hGH-XTEN to a mammal result in detectable anti-XTEN IgG at a serum dilution of 1:100 but not at a dilution of 1:1000. In the foregoing embodiments, the mammal can be a mouse, a rat, a rabbit, or a cynomolgus monkey.

An additional feature of XTENs with non-repetitive sequences relative to sequences with a high degree of repetitiveness is non-repetitive XTENs form weaker contacts with antibodies. Antibodies are multivalent molecules. For instance, IgGs have two identical binding sites and IgMs contain 10 identical binding sites. Thus antibodies against repetitive sequences can form multivalent contacts with such repetitive sequences with high avidity, which can affect the potency and/or elimination of such repetitive sequences. In contrast, antibodies against non-repetitive XTENs may yield monovalent interactions, resulting in less likelihood of immune clearance such that the hGH-XTEN compositions can remain in circulation for an increased period of time.

### 8. Increased hydrodynamic radius

In another aspect, the present disclosure provides XTEN in which the XTEN polypeptides have a high hydrodynamic radius that confers a corresponding increased Apparent Molecular Weight to the hGH-XTEN fusion protein incorporating the XTEN. As detailed in Example 37, the linking of XTEN to GH sequences results in hGH-XTEN compositions that can have increased hydrodynamic radii, increased Apparent Molecular Weight, and increased Apparent Molecular Weight Factor compared to a GH not linked to an XTEN. For example, in therapeutic applications in which prolonged half-life is desired, compositions in which a XTEN with a high hydrodynamic radius is incorporated into a fusion protein comprising one or more GH can effectively enlarge the hydrodynamic radius of the composition beyond the glomerular pore size of approximately 3-5 nm (corresponding to an apparent molecular weight of about 70 kDA) (Caliceti. 2003. Pharmacokinetic and biodistribution properties of poly(ethylene glycol)-protein conjugates. Adv Drug Deliv Rev 55:1261-1277), resulting in reduced renal clearance of circulating proteins. The hydrodynamic radius of a protein is determined by its molecular weight as well as by its structure, including shape or compactness. Not to be bound by a particular theory, the XTEN can adopt open conformations due to electrostatic repulsion between individual charges of the peptide or the inherent flexibility imparted by the particular amino acids in the sequence that lack potential to confer secondary structure. The open, extended and unstructured conformation of the XTEN polypeptide can have a greater proportional hydrodynamic radius compared to polypeptides of a comparable sequence length and/or molecular weight that have secondary and/or tertiary structure, such as typical globular proteins. Methods for determining the hydrodynamic radius are well known in the art, such as by the use of size exclusion chromatography (SEC), as described in U.S. Patent Nos. 6,406,632 and 7,294,513. As the results of Example 37 of Schellenberger et al. WO10/144502A2 demonstrate, the addition of increasing lengths of XTEN results in proportional increases in the parameters of hydrodynamic radius, Apparent Molecular Weight, and Apparent Molecular Weight Factor, permitting the tailoring of hGH-XTEN to desired characteristic cut-off Apparent Molecular Weights or hydrodynamic radii. Accordingly, in certain embodiments, the hGH-XTEN fusion protein can be configured with an XTEN such that the fusion protein can have a hydrodynamic radius of at least about 5 nm, or at least about 8 nm, or at least about 10 nm, or 12 nm, or at least about 15 nm. In the foregoing embodiments, the large hydrodynamic radius conferred by the XTEN in an hGH-XTEN fusion protein can lead to reduced renal clearance of the resulting fusion protein, leading to a corresponding increase in terminal half-life, an increase in mean residence time, and/or a decrease in renal clearance rate.

In another embodiment, an XTEN of a chosen length and sequence can be selectively incorporated into a hGH-XTEN to create a fusion protein that have, under physiologic conditions, an Apparent Molecular Weight of at least about 150 kDa, or at least about 300 kDa, or at least about 400 kDa, or at least about 500 kDA, or at least about 600 kDa, or at least about 700 kDA, or at least about 800 kDa, or at least about 900 kDa, or at least about 1000 kDa, or at least about 1200 kDa, or at least about 1500 kDa, or at least about 1800 kDa, or at least about 2000 kDa, or at least about 2300 kDa or more. In another embodiment, an XTEN of a chosen length and sequence can be selectively linked to a GH to result in a hGH-XTEN fusion protein that has, under physiologic conditions, an Apparent Molecular Weight Factor of at least three, alternatively of at least four, alternatively of at least five, alternatively of at least six, alternatively of at least eight, alternatively of at least 10, alternatively of at least 15, or an Apparent Molecular Weight Factor of at least 20 or greater. In another embodiment, the hGH-XTEN fusion protein has, under physiologic conditions, an Apparent Molecular Weight Factor that is about 4 to about 20, or is about 6 to about 15, or is about 8 to about 12, or is about 9 to about 10 relative to the actual molecular weight of the fusion protein.

### V). hGH-XTEN STRUCTURAL CONFIGURATIONS AND PROPERTIES

The human growth hormone (GH) of the subject compositions are not limited to native, full-length polypeptides, but also include recombinant versions as well as biologically and/or pharmacologically active variants or fragments thereof. For example, it will be appreciated that various amino acid deletions, insertions and substitutions can be made in the GH to create variants without departing from the spirit of the disclosure with respect to the biological activity or pharmacologic properties of the GH. Examples of conservative substitutions for amino acids in polypeptide sequences are shown in Table 4. However, in embodiments of the hGH-XTEN in which the sequence identity of the GH is less than 100% compared to a specific sequence disclosed herein, the invention contemplates substitution of any of the other 19 natural L-amino acids for a given amino acid residue of the given GH, which may be at any position within the sequence of the GH, including adjacent amino acid residues. If any one substitution results in an undesirable change in biological activity, then one of the alternative amino acids can be employed and the construct evaluated by the methods described herein, or using any of the techniques and guidelines for conservative and non-conservative mutations set forth, for instance, in U.S. Pat. No. 5,364,934, or using methods generally known in the art. In addition, variants can include, for instance, polypeptides wherein one or more amino acid residues are added or deleted at the N- or C-terminus of the full-length native amino acid sequence of a GH that retains some if not all of the biological activity of the native peptide.

**Table 4: Exemplary conservative amino acid substitutions**

| Original Residue | Exemplary Substitutions |
|---|---|
| Ala (A) | val; leu; ile |
| Arg (R) | lys; gin; asn |
| Asn (N) | gin; his; lys; arg |
| Asp (D) | glu |
| Cys (C) | ser |
| Gln (Q) | asn |
| Glu (E) | asp |
| Gly (G) | pro |
| His (H) | asn: gin: lys: arg |
| xIle (I) | leu; val; met; ala; phe: norleucine |
| Leu (L) | norleucine: ile: val; met; ala: phe |
| Lys (K) | arg: gin: asn |
| Met (M) | leu; phe; ile |
| Phe (F) | leu: val: ile; ala |
| Pro (P) | gly |
| Ser (S) | thr |
| Thr (T) | ser |
| Trp (W) | tyr |
| Tyr(Y) | trp: phe: thr: ser |
| Val (V) | ile; leu; met; phe; ala; norleucine |

### (a) Fusion Protein Configurations

The disclosure provides fusion protein compositions with the GH and XTEN components linked in specific N- to C-terminus configurations. In some embodiments, one or more GHs are linked to one or more XTENs, either at the N-terminus or at the C-terminus, with or without a spacer, to form a block copolymer, and the sequential arrangement of the GHs and the XTENs in the fusion protein are the same as the configuration known in the block copolymer chemistry. When there is more than one GH, XTEN, or spacer, each of the GH, the XTEN, or the spacer have the same or different sequences, and the GHs and/or XTENs are linked either continuously or alternately (regular or irregular). Thus, in all of the formulae provided herein, when there is more than one GH, XTEN, or spacer, each of the GH, XTEN, and spacer are the same or different. In some embodiments, the fusion protein is a monomeric fusion protein with a GH linked to one XTEN polypeptide. In other embodiments, the fusion protein is a monomeric fusion protein with a GH linked to two or more XTEN polypeptides. In still other embodiments, the fusion protein is a monomeric fusion protein with two or more GH linked to one XTEN polypeptide. In still other embodiments, the fusion protein is a monomeric fusion protein with two or more GH linked to two or more XTEN polypeptide. Table 5 provides non-limiting examples of configurations that are encompassed by the disclosure; numerous other variations will be apparent to the ordinarily skilled artisan, including the incorporation the spacer and cleavage sequences disclosed herein or known in the art.

**Table 5: hGH-XTEN configurations**

| **Components*** | **Configuration**** |
|---|---|
| Single GH; Single XTEN | GH-XTEN |
| | XTEN-GH |
| Single GH; Multiple XTEN | XTEN-GH-XTEN |
| | GH-XTEN-XTEN |
| | XTEN-XTEN-GH |
| | XTEN-GH-XTEN-XTEN |
| | XTEN-XTEN-GH-XTEN |
| | XTEN-XTEN-GH-XTEN |
| Multiple GH, Single XTEN | GH-XTEN-GH |
| | XTEN-GH-GH |
| | GH-GH-XTEN |
| | GH-XTEN-GH-GH |
| Multiple GH; Multiple XTEN | GH-XTEN-GH-XTEN |
| | XTEN-GH-XTEN-GH |
| | XTEN-XTEN-GH-XTEN-GH |
| | XTEN-XTEN-GH-GH |
| | GH-XTEN-XTEN-GH |
| | GH-GH-XTEN-XTEN |
| | GH-GH-XTEN-XTEN-GH |
| | GH-XTEN-GH-XTEN-GH |

| | |
|---|---|
| * Characterized as single for 1 component or multiple for 2 or more of that component ** Reflects N- to C-terminus configuration of the growth factor and XTEN components | |

The disclosure contemplates fusion proteins compositions that are in a configuration shown in Table 5 and that retain at least a portion of the biological activity of the corresponding GH not linked to the XTEN. In other embodiments, the GH component either becomes biologically active or has an increase in activity upon its release from the XTEN by cleavage of an optional cleavage sequence incorporated within spacer sequences into the hGH-XTEN, described more fully below.

In one embodiment of the hGH-XTEN composition, the disclosure provides a fusion protein of formula I:

(XTEN)ₓ-GH-(XTEN)_{y} I

wherein independently for each occurrence, GH is a human growth hormone; x is either 0 or 1 and y is either 0 or 1 wherein x+y ≥1; and XTEN is an extended recombinant polypeptide.

In another embodiment of the hGH-XTEN composition, the disclosure provides a fusion protein of formula II:

(XTEN)ₓ-(GH)-(S)_{y}-(XTEN)_{y} II

wherein independently for each occurrence, GH is a human growth hormone; S is a spacer sequence having between 1 to about 50 amino acid residues that can optionally include a cleavage sequence; x is either 0 or 1 and y is either 0 or 1 wherein x+y ≥1; and XTEN is an extended recombinant polypeptide.

In another embodiment, the disclosure provides an isolated fusion protein, wherein the fusion protein is of formula III:

(GH)-(S)ₓ-(XTEN)-(S)_{y}-(GH)-(S)_{z}-(XTEN)_{z} III

wherein independently for each occurrence, GH is a human growth hormone; S is a spacer sequence having between 1 to about 50 amino acid residues that can optionally include a cleavage sequence; x is either 0 or 1; y is either 0 or 1; z is either 0 or 1; and XTEN is an extended recombinant polypeptide.

In another embodiment, the disclosure provides an isolated fusion protein, wherein the fusion protein is of formula IV:

(XTEN)ₓ-(S)_{y}-(GH)-(S)_{z}-(XTEN)-(GH) IV

wherein independently for each occurrence, GH is a human growth hormone; S is a spacer sequence having between 1 to about 50 amino acid residues that can optionally include a cleavage sequence; x is either 0 or 1; y is either 0 or 1; z is either 0 or 1; and XTEN is an extended recombinant polypeptide.

In another embodiment, the disclosure provides an isolated fusion protein, wherein the fusion protein is of formula V:

(GH)ₓ-(S)ₓ-(GH)-(S)_{y}-(XTEN) V

wherein independently for each occurrence, GH is a growth hormone; S is a spacer sequence having between 1 to about 50 amino acid residues that can optionally include a cleavage sequence; x is either 0 or 1; y is either 0 or 1; and XTEN is an extended recombinant polypeptide.

In another embodiment, the disclosure provides an isolated fusion protein, wherein the fusion protein is of formula VI:

(XTEN)-(S)ₓ-(GH)-(S)_{y}-(GH) VI

wherein independently for each occurrence, GH is a growth hormone; S is a spacer sequence having between 1 to about 50 amino acid residues that can optionally include a cleavage sequence; x is either 0 or 1; y is either 0 or 1; and XTEN is an extended recombinant polypeptide.

In another embodiment, the disclosure provides an isolated fusion protein, wherein the fusion protein is of formula VII:

(XTEN)-(S)ₓ-(GH)-(S)_{y}-(GH)-(XTEN) VII

wherein independently for each occurrence, GH is a growth hormone; S is a spacer sequence having between 1 to about 50 amino acid residues that can optionally include a cleavage sequence; x is either 0 or 1; y is either 0 or 1; and XTEN is an extended recombinant polypeptide.

In another embodiment, the disclosure provides an isolated fusion protein, wherein the fusion protein is of formula VIII:

((S)ₘ-(GH)ₓ-(S)ₙ-(XTEN)_{y}-(S)ₒ)ₜ VIII

wherein t is an integer that is greater than 0 (1, 2, 3, etc.); independently each of m, n, o, x , and y is an integer (0, 1, 2, 3, etc.), GH is a growth hormone; S is an spacer, optionally comprising a cleavage site; and XTEN is an extended recombinant polypeptide, with the proviso that: (1) x+ y > 1, (2) when t = 1, x>0 and y>0, (3) when there is more than one GH, S, or XTEN, each GH, XTEN, or S are the same or are independently different; and (4) when t >1, each m, n, o, x, or y within each subunit are the same or are independently different.

In another embodiment, the disclosure provides an isolated fusion protein, wherein the fusion protein is of formula IX:

(XTEN)ₓ-(S)ₓ-(GH)-(S)_{y}-(XTEN)_{y} IX

wherein independently for each occurrence, GH is a human growth hormone; S is a spacer sequence having between 1 to about 50 amino acid residues that can optionally include a cleavage sequence; x is either 0 or 1 and y is either 0 or 1 wherein x+y ≥1; and XTEN is an extended recombinant polypeptide.

In some embodiments, administration of a therapeutically effective amount of a fusion protein of an embodiment of formulas I-VIII to a subject in need thereof results in a gain in time of at least two-fold, or at least three-fold, or at least four-fold, or at least five-fold, or at least 10-fold, or at least 20-fold, or at least 40-fold, or at least 100-fold or more spent within a therapeutic window for the fusion protein compared to the corresponding GH not linked to the XTEN and administered at a comparable amount administered to a subject. In other embodiments, administration of a therapeutically effective dose of a fusion protein of an embodiment of formulas I-VIII to a subject in need thereof can result in a gain in time between consecutive doses necessary to maintain a therapeutically effective dose regimen of at least 48 h, or at least 72 h, or at least about 96 h, or at least about 120 h, or at least about 7 days, or at least about 14 days, or at least about 21 days, or at least about 28 days, or at least about monthly between consecutive doses compared to a dose schedule for GH not linked to required to maintain a therapeutically effective dose regimen.

Any spacer sequence group is optional in the fusion proteins encompassed by the invention. The spacer is provided to enhance expression of the fusion protein from a host cell or to decrease steric hindrance such that the GH component may assume its desired tertiary structure and/or interact appropriately with its target receptor. For spacers and methods of identifying desirable spacers, see, for example, George, et al. (2003) Protein Engineering 15:871-879. In one embodiment, the spacer comprises one or more peptide sequences that are between 1-50 amino acid residues in length, or about 1-25 residues, or about 1-10 residues in length. Spacer sequences, exclusive of cleavage sites, can comprise any of the 20 natural L amino acids, and will preferably comprise hydrophilic amino acids that are sterically unhindered that can include, but not be limited to, glycine (G), alanine (A), serine (S), threonine (T), glutamate (E) and proline (P). In some cases, the spacer can be polyglycines or polyalanines, or is predominately a mixture of combinations of glycine and alanine residues. The spacer polypeptide exclusive of a cleavage sequence is largely to substantially devoid of secondary structure; e.g., less than about 10%, or less than about 5% as determined by the Chou-Fasman and/or GOR algorithms. In one embodiment, one or both spacer sequences in a hGH-XTEN fusion protein composition each further
contains a cleavage sequence, which are identical or different, wherein the cleavage sequence may be acted on by a protease to release the GH from the fusion protein.

In some embodiments, the incorporation of the cleavage sequence into the hGH-XTEN is designed to permit release of a GH that becomes active or more active upon its release from the XTEN. The cleavage sequences are located sufficiently close to the GH sequences, generally within 18, or within 12, or within 6, or within 2 amino acids of the GH sequence terminus, such that any remaining residues attached to the GH after cleavage do not appreciably interfere with the activity (e.g., such as binding to a receptor) of the GH, yet provide sufficient access to the protease to be able to effect cleavage of the cleavage sequence. In some embodiments, the cleavage site is a sequence that can be cleaved by a protease endogenous to the mammalian subject such that the hGH-XTEN can be cleaved after administration to a subject. In such cases, the hGH-XTEN can serve as a prodrug or a circulating depot for the GH. Examples of cleavage sites contemplated by the herein include, but are not limited to, a polypeptide sequence cleavable by a mammalian endogenous protease selected from FXIa, FXIIa, kallikrein, FVIIa, FIXa, FXa, FIIa (thrombin), Elastase-2, granzyme B, MMP-12, MMP-13, MMP-17 or MMP-20, or by non-mammalian proteases such as TEV, enterokinase, PreScission™ protease (rhinovirus 3C protease), and sortase A. Sequences known to be cleaved by the foregoing proteases and others are known in the art. Exemplary cleavage sequences and cut sites within the sequences are presented in Table 6, as well as sequence variants thereof. For example, thrombin (activated clotting factor II) acts on the sequence LTPRSLLV (SEQ ID NO: 56) [Rawlings N.D., et al. (2008) Nucleic Acids Res., 36: D320], which would be cut after the arginine at position 4 in the sequence. Active FIIa is produced by cleavage of FII by FXa in the presence of phospholipids and calcium and is down stream from factor IX in the coagulation pathway. Once activated its natural role in coagulation is to cleave fibrinogen, which then in turn, begins clot formation. FIIa activity is tightly controlled and only occurs when coagulation is necessary for proper hemostasis. However, as coagulation is an on-going process in mammals, by incorporation of the LTPRSLLV sequence (SEQ ID NO: 57) into the hGH-XTEN between the GH and the XTEN, the XTEN domain would be removed from the adjoining GH concurrent with activation of either the extrinsic or intrinsic coagulation pathways when coagulation is required physiologically, thereby releasing GH over time. Similarly, incorporation of other sequences into hGH-XTEN that are acted upon by endogenous proteases would provide for sustained release of GH that, in certain embodiments, provide a higher degree of activity for the GH from the "prodrug" form of the hGH-XTEN.

In some embodiments, only the two or three amino acids flanking both sides of the cut site (four to six amino acids total) are incorporated into the cleavage sequence. In other embodiments, the known cleavage sequence have one or more deletions or insertions or one or two or three amino acid substitutions for any one or two or three amino acids in the known sequence, wherein the deletions, insertions or substitutions result in reduced or enhanced susceptibility but not an absence of susceptibility to the protease, resulting in an ability to tailor the rate of release of the GH from the XTEN. Exemplary substitutions are shown in Table 6.

**Table 6: Protease Cleavage Sequences**

| Protease Acting Upon Sequence | Exemplary Cleavage Sequence | SEQ ID NO: | Minimal Cut Site^{∗} | SEQ ID NO: |
|---|---|---|---|---|
| FXIa | KLTR↓VVGG | 58 | KD/FL/T/R↓VA/VE/GT/GV | |
| FXIIa | TMTR↓IVGG | 59 | NA | |
| Kallikrein | SPFR↓STGG | 60 | -/-/FL/RY↓SR/RT/-/- | |
| FVIIa | LQVR↓IVGG | 61 | NA | |
| FIXa | PLGR↓IVGG | 62 | -/-/G/R↓-/-/-/- | |
| FXa | IEGR↓TVGG | 63 | IA/E/GFP/R↓STINFS/-/G | |
| FIIa (thrombin) | LTPR↓SLLV | 64 | -/-/PLA/R↓SAG/-/-/- | |
| Elastase-2 | LGPV↓SGVP | 65 | -/-/-/VIAT↓-/-/-/- | |
| Granzyme-B | VAGD↓SLEE | 66 | V/-/-/D↓-/-/-/- | |
| MMP-12 | GPAG↓LGGA | 67 | G/PA/-/G↓L/-/G/- | 68 |
| MMP-13 | GPAG↓LRGA | 69 | G/P/-/G↓L/-/GA/- | 70 |
| MMP-17 | APLG↓LRLR | 71 | -/PS/-/-↓LQ/-/LT/- | |
| MMP-20 | PALP↓LVAQ | 72 | NA | |
| TEV | ENLYFQ↓G | 73 | ENLYFQ↓G/S | 74 |
| Enterokinase | DDDK↓IVGG | 75 | DDDK↓IVGG | 76 |
| Protease 3C (PreScission™ ) | LEVLFQ↓GP | 77 | LEVLFQ↓LGP | 78 |
| Sortase A | LPKT↓GSES | 79 | L/P/KEAD/T↓G/-/EKS/S | 80 |

| | | | | |
|---|---|---|---|---|
| ↓indicates cleavage site NA: not applicable ^{∗} the listing of multiple amino acids before, between, or after a slash indicate alternative amino acids that can be substituted at the position; "-" indicates that any amino acid may be substituted for the corresponding amino acid indicated in the middle column | | | | |

In one embodiment, a GH incorporated into a hGH-XTEN fusion protein has a sequence that exhibits at least about 80% sequence identity to a sequence shown as SEQ ID NO: 2, alternatively at least about 81%, or about 82%, or about 83%, or about 84%, or about 85%, or about 86%, or about 87%, or about 88%, or about 89%, or about 90%, or about 91%, or about 92%, or about 93%, or about 94%, or about 95%, or about 96%, or about 97%, or about 98%, or about 99%, or about 100% sequence identity as compared with the sequence of SEQ ID NO: 2. The GH of the foregoing embodiment can be evaluated for activity using assays or measured or determined parameters as described herein, and those sequences that retain at least about 40%, or about 50%, or about 55%, or about 60%, or about 70%, or about 80%, or about 90%, or about 95% or more activity compared to the corresponding native GH sequence would be considered suitable for inclusion in the subject hGH-XTEN. The GH found to retain a suitable level of activity can be linked to one or more XTEN polypeptides described hereinabove. In one embodiment, a GH found to retain a suitable level of activity can be linked to one or more XTEN polypeptides having at least about 80% sequence identity to a sequence from Table 3, alternatively at least about 81%, 82%, 83%, 84%, 85%, 86%, 87%, 88%, 89%, 90%, 91%, 92%, 93%, 94%, 95%, 96%, 97%, 98%, 99%, or about 100% sequence identity as compared with a sequence of Table 3, resulting in a chimeric fusion protein.

Non-limiting examples of sequences of fusion proteins containing a single GH linked to a single XTEN are presented in Table 35 of Schellenberger et al. WO10/144502A2. In one embodiment, a hGH-XTEN composition would comprise a fusion protein having at least about 80% sequence identity to a hGH-XTEN from Table 35 of Schellenberger et al. WO10/144502A2, alternatively at least about 81%, 82%, 83%, 84%, 85%, 86%, 87%, 88%, 89%, 90%, 91%, 92%, 93%, 94%, 95%, 96%, 97%, 98%, 99%, or about 100% sequence identity as compared with a hGH-XTEN from Table 35 of Schellenberger et al. WO10/144502A2. Non-limiting examples of sequences of fusion proteins containing two molecules of XTEN linked to one or more GH are presented in Table 36 of Schellenberger et al. WO10/144502A2, but also described herein is the substitution of other GH with sequences exhibiting at least about 90% sequence identity to the sequence of SEQ ID NO: 2 linked to one or two XTEN, which may be the same or different,
exhibiting at least about 90% sequence identity selected from Table 3. In the foregoing fusion proteins hereinabove described in this paragraph, the hGH-XTEN fusion protein can further comprise a cleavage sequence from Table 6; the cleavage sequence being located between the GH and the XTEN or between adjacent GH (if more than one GH is included in the hGH-XTEN). In some cases, the hGH-XTEN comprising the cleavage sequences will also have one or more spacer sequence amino acids between the GH and the cleavage sequence or the XTEN and the cleavage sequence to facilitate access of the protease; the spacer amino acids comprising any natural amino acid, including glycine and alanine as preferred amino acids. Non-limiting examples of hGH-XTEN comprising GH, XTEN, cleavage sequence(s) and spacer amino acids are presented in Table 37 of Schellenberger et al. WO10/144502A2. However, the disclosure also contemplates substitution of any GH sequence exhibiting at least about 90% sequence identity to the sequence of SEQ ID NO: 2 for a GH sequence of Table 37, substitution of any XTEN sequence of Table 3 for an XTEN sequence of Table 37, and substitution of any cleavage sequence of Table 6 for a cleavage sequence of Table 37 of Schellenberger et al. WO10/144502A2.

### VI). USES OF THE COMPOSITIONS

The present invention provides a hGH-XTEN fusion protein for use in a method of treating human adult growth hormone deficiency (AGHD), as defined in the claims. Additionally disclosed herein is a method for achieving a beneficial effect in a disease, disorder or condition mediated by GH including, but not limited to growth hormone deficiency in a human patient. Also disclosed herein is a method for achieving a beneficial effect in a disease, disorder or condition mediated by GH including, but not limited to growth hormone deficiency in adults (including adults who experienced a growth hormone-related disorder as children). The beneficial effect includes, without limitation, treating, mediating, or ameliorating a GH-related disease, deficiency, disorder or condition. The present invention addresses disadvantages and/or limitations of GH that have a relatively short terminal half-life and/or a narrow therapeutic window.

"Growth hormone deficiency" or "GHD" as used herein refers to a disease, deficiency, disorder or condition in a human patient that would benefit from treatment with growth hormone. GHD includes disorders that are classified based on the source of the GH deficiency (e.g., pituitary GHD, hypothalamic GHD, functional GHD, and idiopathic GHD). Pituitary or "classic" GHD is the incapacity of the pituitary to produce growth hormone. "Hypothalamic GHD" is the failure of the hypothalamus to produce and/or transmit the neuroendocrine messaging hormone, growth hormone releasing hormone (GHRH), which directs a properly functioning pituitary to produce GH; "functional GHD" is the failure of other hormone and of metabolic functions related to the failure of the pituitary to produce, uptake, and/or utilize GH.

In one embodiment, the human patient having a GHD is an adult. GHD includes "adult growth hormone deficiency" or "AGHD", which may be classified based on the stage of life the GH deficiency became manifest. For example, an adult may have AGHD that is a continuation of childhood onset GHD (including child-onset GHD and child-onset idiopathic GHD), which began in infancy or childhood. The causes of childhood-onset AGHD include, without limitation, developmental defects in or near the pituitary gland; genetic problems with the production of GH; Prader-Willi syndrome; Turner's syndrome; midline facial defects; and damage to the pituitary gland or the surrounding area due to tumors, infection, radiation treatment, or severe head injury. Adults who survived brain tumors as children may be at risk of developing GHD from the effects of surgery, cranial radiation or chemotherapy.

AGHD can develop in an adult, *i.e.,* adult-onset GHD, (including adult-onset GHD and idiopathic adult onset-GHD) who was not diagnosed as being GH-deficient as a child. Adult-onset AGHD may be caused by damage or trauma to the pituitary gland. The damage is typically caused by a tumor (*e.g.,* a tumor in and/or around the pituitary gland; or a tumor in the hypothalamus). Pituitary tumors can compress the gland or damage can occur when the tumor is removed via neurosurgery. The pituitary can also be damaged by infection, blood vessel disease, severe head injury, or cranial radiation or chemotherapy for treating tumors of the head and neck. AGHD may be caused by: trauma that occurred in an adult at their birth or soon after their birth; central nervous system infection; tumors of the hypothalamus or pituitary glands; infiltrative or granulomatous disease; cranial irradiation; surgery; or idiopathic causes. GHD in the elderly becomes manifest in decreased quality of life, fatigue, and alteration of body composition. Abnormalities in body composition, bone metabolism, and lipid profile in GH-deficient and hypopituitary adults are distinct from those that occur as the result of normal aging. AGHD includes congenital or acquired GH deficiency in adults, as well as any other adult indication for which GH can be utilized (including where endogenous growth hormone levels in a subject are not necessarily deficient).

Most processes involved in growth of the body are regulated by multiple peptides and hormones, and such peptides and hormones, as well as analogues thereof, have found utility in the treatment of growth hormone-related diseases, disorders and conditions. However, the use of commercially-available growth hormones, has met with less than optimal success in the management of subjects afflicted with such diseases, disorders and conditions. In particular, dose optimization and frequency of dosing is important for peptide and hormone biologics used in the treatment of growth hormone-related diseases and disorders. The fact that growth hormone has a short half-life (e.g., usually less than 4 hours when administered subcutaneously), necessitates frequent (e.g., daily) dosing in order to achieve clinical benefit, which results in difficulties in the management of such patients. Non-compliance with daily growth hormone (GH) injections can lead to loss of treatment effects.

The present invention relates to the enhancement of the safety and tolerability, and the ability to achieve IGF-I levels within a target range in adults with GH deficiency (GHD) after administration of a single dose of the long-acting rhGH analogue, VRS-317, the sequence of which is shown in Figure 1 (SEQ ID NO: 1). As detailed in the Examples, in a randomized, double-blind, placebo-controlled, single ascending dose study, 50 GHD adults (mean age 45 yr.) were studied in 5 treatment groups of 10 subjects each (8 active, 2 placebo per group). The main outcome measures included adverse events, safety laboratories, VRS-317 pharmacokinetics and pharmacodynamics (including, but not limited to determination of IGF-I and IGFBP-3 concentrations). The results indicate that using a single-dose administration of 0.80 mg/kg of VRS-317, a mean terminal elimination half-life of 131 hours is achieved in subjects. Single VRS-317 doses of 0.05, 0.10, 0.20, 0.40 and 0.80 mg/kg (approximately equivalent to daily rhGH doses of 0.3 to 5.0 µg/kg over 30 days) safely increased the amplitude and duration of IGF-I responses in a dose-dependent manner. After a single 0.80 mg/kg dose, serum IGF-I was maintained in the normal range between -1.5 to 1.5 standard deviations (SD) for a mean of three weeks. No unexpected or serious adverse events were observed in subjects receiving VRS-317. The elimination half-life for VRS-317 is 30-60-fold longer and stimulates more durable IGF-I responses compared to previously studied rhGH products. Prolonged IGF-I responses do not come at the expense of over-exposure to high IGF-I levels. The pharmacokinetic and pharmacodynamics combined with the observed safety profile indicate the potential for safe and effective monthly dosing using VRS-317. The protocols, results, and analysis of this study are discussed further in Examples 1 and 2.

The present invention provides a hGH-XTEN fusion protein for use in method of treating adult growth hormone deficiency (AGHD) in a human patient by administering a human growth hormone-XTEN (hGH-XTEN) fusion protein to the patient. The method comprises administering the hGH-XTEN fusion protein to the patient as a therapeutically effective bodyweight adjusted bolus dosebetween 0.05 mg/kg and 3.0 mg/kg. The fusion protein comprises an amino acid sequence having at least about 90% sequence identity to SEQ ID NO:1. In one additional embodiment, the human patient is an adult.

In one aspect, the bolus dose may be administered over a range of doses. It should be noted that where reference is made to the administration of a bolus dose between about a first mg/kg and about a second mg/kg, the "first mg/kg" term may include the first mg/kg value and the "second mg/kg" term may include the second mg/kg value.

In one embodiment, the hGH-XTEN fusion protein comprises (i) an amino acid sequence having at least about 90% sequence identity to SEQ ID NO.1; (ii) the amino acid sequence of SEQ ID NO:1. Also disclosed herein are hGH-XTEN fusion proteins that comprise an amino acid sequence having at least about 90% sequence identity to SEQ ID NO: 7; the amino acid sequence of SEQ ID NO: 7; an amino acid sequence having at least about 90% sequence identity to SEQ ID NO:83; or the amino acid sequence of SEQ ID NO: 83.

Disclosed herein is a method of treating GHD in the human patient comprises administering a single dose of an hGH-XTEN fusion protein comprising an amino acid sequence having at least about 90% sequence identity to SEQ ID NO: 1. Also disclosed herein, the single dose comprises a therapeutically effective bodyweight adjusted bolus dose of the hGH-XTEN fusion protein. The bolus dose is between about 0.05 mg/kg and about 3.0 mg/kg, or between about 0.05 mg/kg and about 0.8 mg/kg. In another embodiment, the bolus dose is about 0.05 mg/kg, about 0.1 mg/kg, about 0.2 mg/kg, about 0.4 mg/kg, or about 0.8 mg/kg. Disclosed herein the human patient is an adult.

In one other aspect of the disclosure, the bolus dose of the hGH-XTEN fusion protein is administered to a human patient on a regular basis over a suitable time period, which can be finite or indefinite. Disclosed herein, the bolus dose is administered every week, every two weeks, every three weeks, or monthly. Also disclosed herein, the bolus dose is administered once a month, twice a month, three times a month, or four times a month. Also disclosed herein the bolus dose is administered about every 7 days, about every 10 days, about every 14 days, about every 21 days, about every 28 days, or about every 30 days. Disclosed herein, the bolus dose is administered on a non-daily basis, or is a non-daily bolus dose. Also disclosed herein, the human patient is an adult.

Also disclosed herein, additional bolus doses and ranges of bolus doses of the hGH-XTEN fusion protein for a human patient are suitable. In one embodiment, the bolus dose is between about 0.05 mg/kg and about 0.8 mg/kg, between about 0.8 mg/kg and about 1.2 mg/kg, or between about 0.05 mg/kg and about 3.0 mg/kg. In another embodiment, the bolus dose is selected from the group consisting of about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and about 3.0 mg/kg. In one additional embodiment, the human patient is an adult.

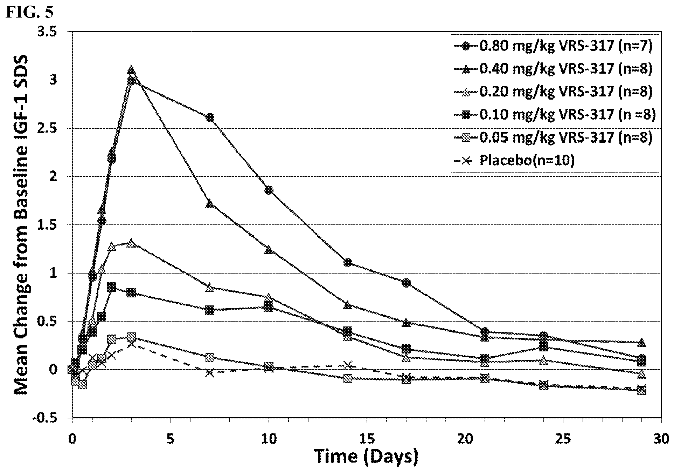

The hGH-XTEN fusion proteins for use in methods of treatment of the present invention are advantageous with respect to IGF-I levels in the human patient following treatment with hGH-XTEN fusion protein. A high level of blood IGF-I is undesirable since high IGF-I is believed to be a risk factor for cancer (Svensson et al. J Clin Endocrin Metab. epub September 26, 2012 as doi:10.1210/jc.2012-2329). IGF-I generation in humans is largely the result of GH signaling and IGF-I is an important mediator for anabolic actions observed during GH therapy (Le Roith et al. (2001). Endocr Rev 22, 53-74). Accordingly, IGF-I is an important pharmacodynamic marker for hGH-XTEN fusion protein bioactivity. In practice, IGF-I responses to GH (*e.g.,* daily rhGH therapy) are interpreted in terms of age- and gender-specific normative data (Vance et al. (1999). N Engl J Med 341, 1206-16; Molitch et al. (2011). J Clin Endocrinol Metab 96, 1587-609). The interpretation is most readily done with the use of IGF-I standard deviation scores (IGF-I SDS). Further, adults with GH deficiency, as with healthy adults, have a range of baseline IGF-I values. Accordingly, IGF-I SDS, corrected for baseline at time 0, can be used to examine potential hGH-XTEN fusion protein dose effects on IGF-I responses. For example, the time course of change in baseline corrected IGF-I SDS by dose group for VRS-317 is shown in Figure 5.

In one aspect, the present invention provides a hGH-XTEN fusion protein for use in methods of treatment of AGHD in which the human patient maintains an IGF-I response (e.g., as measured by mean IGF-I SDS) in a normal range after administration of the hGH-XTEN fusion protein, as defined in the claims. For an IGF-I SDS, a normal range is generally between about -1.5 and about 1.5 but can also be between about -2.0 and about 2.0. In one additional embodiment, the human patient is an adult.

It should be noted that where reference is made to an IGF-I SDS between about a first value (e.g., -2.0) and about a second value (e.g., 2.0), the "first value" may include the first value and the "second value" may include the second value.

In one embodiment, the present invention provides a hGH-XTEN fusion protein for use in a method of treating growth hormone deficiency (GHD) in a human patient by administering an hGH-XTEN fusion protein to the patient, wherein the human patient has a serum IGF-I standard deviation score (SDS) between about -2.0 and about 2.0 following administration. The method comprises administering the hGH-XTEN fusion protein to the patient as a bolus dose which is a therapeutically effective bodyweight adjusted bolus dose. In another embodiment, the bolus dose is effective to maintain the patient's serum IGF-I standard deviation score (SDS) between about -2.0 and about 2.0 for (i) at least 7 days; (ii) at least about 10 days; or (iii) at least about 20 days after administration of the bolus dose. In one additional embodiment, the human patient is an adult.

Also disclosed herein is a method of treating human growth hormone deficiency (GHD) in a human subject, comprising administering to the subject with GHD a pharmaceutical composition comprising an effective amount of hGH-XTEN fusion protein having the amino acid sequence set forth in FIG. 1 (SEQ ID NO:1) wherein said amount is at least about 0.05 mg/kg in a single bolus dose, and further wherein said amount is effective to maintain the subject's serum IGF-I SDS between about -1.5 and about 1.5 for (i) at least 7 days; (ii) at least about 10 days; or (iii) at least about 20 days after administration of the single bolus dose of the fusion protein. In one additional embodiment, the human patient is an adult.

In a further embodiment of the method, the effective amount of the hGH-XTEN fusion protein administered to a human patient is at least about 0.1 mg/kg, at least about 0.2 mg/kg, at least about 0.4 mg/kg, at least about 0.8 mg/kg, at least about 1.0 mg/kg, at least about 1.2 mg/kg, at least about 1.4 mg/kg, at least about 1.6 mg/kg, at least about 1.8 mg/kg, at least about 2.0 mg/kg, at least about 2.2 mg/kg, at least about 2.4 mg/kg, at least about 2.6 mg/kg, at least about 2.7 mg/kg, at least about 2.8 mg/kg, or at least 3.0 mg/kg. In one additional embodiment, the human patient is an adult.

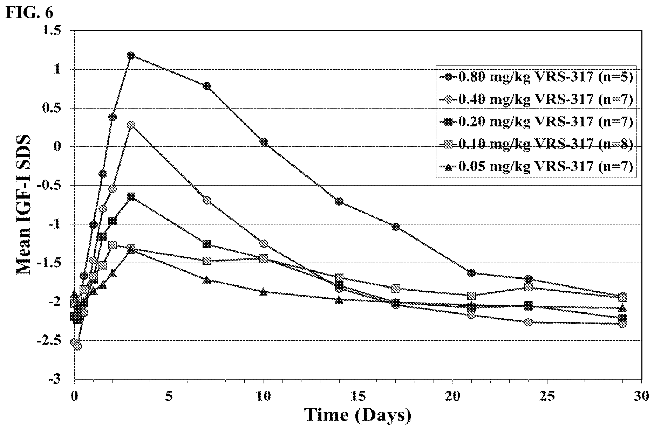

In another embodiment of the method, the effective amount administered to the human patient is between about 0.05 mg/kg and about 3.0 mg/kg. In another embodiment of the method, the effective amount administered is between about 0.2 mg/kg and about 0.8 mg/kg. In another embodiment of the method, the amount of hGH-XTEN fusion protein administered is effective to maintain the subject's serum IGF-I SDS at between about -1.5 and about 1.5 for at least about 15 or at least about 20 days after administration of a single dose of the fusion protein. For example, the mean IGF-I SDS by dose group after administration of VRS-317 is shown in Figure 6. In one additional embodiment, the human patient is an adult.

The medical uses of the present invention provides a particular advantage in that that the administration of hGH-XTEN fusion protein provides an observable and prolonged IGF-I response in the human patient (*e.g.,* as measured by IGF-I SDS) that is not accompanied by, or at the expense of, over-exposure to high levels of IGF-I, which is undesirable. In other words, the IGF-I response is maintained at an elevated level that is still considered acceptable by current standards, *e.g.,* as indicated by an IGF-I SDS of 1.5 or less, or an IGF-I SDS of 2.0 or less.

In one embodiment, the invention provides a method for achieving a beneficial effect in a human patient with growth hormone deficiency, comprising the step of administering to the subject a therapeutically-effective amount of a hGH-XTEN fusion protein wherein said administration results in the improvement of one or more biochemical or physiological parameters or clinical endpoints associated with a growth hormone-related disease, disorder or condition. The effective amount produces a beneficial effect in helping to treat (e.g., cure or reduce the severity) the deleterious effects of a growth hormone-related disease, disorder or condition. In some cases, the method for achieving a beneficial effect includes administering a therapeutically effective amount of a hGH-XTEN fusion protein composition to treat a subject with a growth hormone-related disease, disorder, or condition, including, but not limited to, congenital or acquired GH deficiency in adults (including adults who experienced a growth hormone-related disorder as children, such as Turner's Syndrome, Prader-Willi Syndrome, idiopathic short stature, or intrauterine growth retardation); and adults experiencing chronic renal failure, AIDS wasting, obesity, multiple sclerosis, aging, fibromyalgia, Crohn's disease, ulcerative colitis, muscular dystrophy, low muscle mass (e.g. bodybuilding), low bone density, or any other indication for which GH can be utilized (but for which endogenous growth hormone levels in a subject are not necessarily deficient). In one additional embodiment, the human patient is an adult.

The methods of the invention include the administration to a human patient of successive or consecutive doses of a therapeutically effective amount of the hGH-XTEN for a period of time sufficient to achieve and/or maintain the desired parameter or clinical effect, and such consecutive doses of a therapeutically effective amount establishes the therapeutically effective dose regimen for the hGH-XTEN; i.e., the schedule for consecutively administered doses of the fusion protein composition, wherein the doses are given in therapeutically effective amounts to result in a sustained beneficial effect on any clinical sign or symptom, aspect, measured parameter or characteristic of a metabolic disease state or condition, including, but not limited to, those described herein. In one embodiment of the method, the parameters include but are not limited to IGF-I concentration, ratio of IGF-I/IGFBP-3, IGFBP3 concentration, change in weight, lean body mass, change in body mass index, total body fat (adipose fat/tissue), trunk fat, response to insulin challenge, rate of division of chondrocytes, chondrocyte numbers, bone density, bone age, bone growth, bone turnover, increase in epiphyseal plate width, reduction in cholesterol, reduction in triglycerides, and reduction in LDL. In one additional embodiment, the human patient is an adult.

In one embodiment, the pharmaceutical composition is administered at a therapeutically effective dose. In another embodiment, the pharmaceutical composition is administered using multiple consecutive doses using a therapeutically effective dose regimen (as defined herein) for the length of the dosing period.

A therapeutically effective amount of the hGH-XTEN varies according to factors such as the disease state, age, sex, and weight of the individual, and the ability of the antibody or antibody portion to elicit a desired response in the individual. A therapeutically effective amount is also one in which any toxic or detrimental effects of the hGH-XTEN are outweighed by the therapeutically beneficial effects.

In one embodiment, the method comprises administering to a human patient with GHD at least two therapeutically effective bodyweight adjusted bolus doses of a human growth hormone hGH-XTEN fusion protein having at least about 90%, or at least about 95%, or at least about 96%, or at least about 97%, or at least about 98%, or at least about 99% sequence identity to the sequence as set forth in FIG. 1 (SEQ ID NO:1), wherein said administration of said bolus doses is separated by at least about 7 days, at least about 10 days, at least about 14 days, at least about 21 days, at least about 28 days, or at least about monthly and wherein the therapeutically effective bodyweight adjusted bolus dose of hGH-XTEN fusion protein is selected from the group consisting of: about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and 3.0 mg/kg. In one additional embodiment, the human patient is an adult.

In another embodiment, the therapeutically effective bodyweight adjusted bolus doses of hGH fusion protein are administered subcutaneously to the human patient. In some embodiments, the human patient has a serum IGF-I standard deviation (SD) score of greater than about -2.0, greater than about -1.5, greater than about -1.0, greater than about -0.5, or greater than about 0, greater than about 0.5, greater than about 1.0, greater than about 1.5, greater than about 1.6, greater than about 1.7, greater than about 1.8, or greater than about 1.9 following administration of the hGH-XTEN. In one embodiment, the hGH-XTEN fusion protein has the amino acid sequence shown as set forth in FIG. 1 (SEQ ID NO:1). In one additional embodiment, the human patient is an adult.

In one embodiment, the disclosure provides a method of treating human growth hormone deficiency (GHD) in a human subject, comprising administering to the subject with GHD a pharmaceutical composition comprising an effective amount of hGH-XTEN fusion protein having the amino acid sequence set forth in FIG. 1 (SEQ ID NO:1) wherein said amount is at least about 0.05 mg/kg in a single bolus dose, and further wherein said amount is effective to maintain the subject's serum IGF-I SD score between about -1.5 and about 1.5 for at least 10 days after administration of the single bolus dose of the fusion protein. In a further embodiment of the method, the amount administered is at least 0.2 mg/kg. In another embodiment of the method, the amount administered is between about 0.05 mg/kg and about 3.0 mg/kg. In another embodiment of the method, the amount administered is between about 0.2 mg/kg and about 0.8 mg/kg. In another embodiment of the method, the amount administered is effective to maintain the subject's serum IGF-I SD score between about -1.5 and about 1.5 for at least 20 days after administration of a single dose of the fusion protein. In one additional embodiment, the human patient is an adult.

In another embodiment, the disclosure provides a method of treating human growth hormone deficiency (GHD) in a human subject, comprising administering to the subject with GHD a pharmaceutical composition comprising an effective amount of hGH-XTEN fusion protein having the amino acid sequence set forth in FIG. 1 (SEQ ID NO:1) wherein said amount is at least 0.05 mg/kg in a single bolus dose and is effective to maintain a plasma concentration of said fusion protein at more than about 10 ng/mL for a period of at least 10 days after administration of the single bolus dose of the fusion protein. In a further embodiment of the method, the amount administered is at least 0.2 mg/kg. In another embodiment of the method, the amount administered is between about 0.05 mg/kg and about 3.0 mg/kg. In another embodiment of the method, the amount administered is between about 0.2 mg/kg and about 0.8 mg/kg. In another embodiment of the method, the amount administered is effective to maintain a plasma concentration of said fusion protein at more than about 10 ng/mL for a period of at least 20 days after administration of the single bolus dose of the fusion protein. In another embodiment of the method, the amount administered is effective to maintain a plasma concentration of said fusion protein at more than about 10 ng/mL for a period of at least 30 days after administration of the single bolus dose of the fusion protein. In another embodiment of the method, the amount administered is effective to maintain a plasma concentration of said fusion protein at more than about 100 ng/mL for a period of at least 10 days after administration of the single bolus dose of the fusion protein. In one additional embodiment, the human patient is an adult.

In one embodiment, the disclosure provides a method of treating human growth hormone deficiency (GHD) in a human subject comprising administering to the subject with GHD a pharmaceutical composition comprising an effective amount of hGH-XTEN fusion protein having the amino acid sequence set forth in FIG. 1 (SEQ ID NO:1) wherein said amount is at least 0.05 mg/kg in a single bolus dose and is effective in increasing the subject's plasma IGF-I SD score by at least 0.5 above the subject's baseline IGF-I SD score without causing a clinically significant level of side-effects selected from the group consisting of headache, arthralgia, myalgia, edema, nausea, and muscle fatigue after administration of the single bolus dose of the fusion protein. As used herein, "clinically significant level of side-effects" means that the side-effects are not unexpected or are not serious adverse events. Side-effects that are mild and transient, even if one of headache, arthralgia, myalgia, edema, nausea, and muscle fatigue or those otherwise known to be associated with the administration of growth hormone, would not be considered a clinically significant level. In another embodiment of the method of treating GHD, the amount administered is at least about 0.2 mg/kg. In another embodiment of the method of treating GHD, the amount administered is between about 0.05 mg/kg and about 3.0 mg/kg. In another embodiment of the method of treating GHD, the amount administered is between about 0.2 mg/kg and about 0.8 mg/kg. In another embodiment of the method of treating GHD, the amount administered is between about 0.2 mg/kg and about 3.0 mg/kg. In another embodiment of the method of treating GHD, the single bolus dose is administered subcutaneously. In another embodiment of the method of treating GHD, the pharmaceutical composition comprising the hGH-XTEN fusion protein is administered using two or more consecutive doses. In one additional embodiment, the human patient is an adult.

In one other aspect, the methods of the present disclosure related to improved therapeutic regimens for GHD therapy comprise improving lipid metabolism parameters in a subject in need, e.g., a human patient with GHD. In one embodiment, the method of improving lipid parameters in a subject in need comprises administering an at least two therapeutically effective bodyweight adjusted bolus doses of a hGH-XTEN fusion protein, wherein the administration of said bolus doses is separated by at least about 7 days, or at least about 10 days, at least about 14 days, at least about 21 days, at least about 28 days, or at least about monthly and wherein the bolus doses provide an improvement in lipid parameters in said subject. In one embodiment, the improvement in lipid parameters is an improvement selected from the group consisting of lower triglyceride levels, lower cholesterol, and lower LDL levels. In one additional embodiment, the human patient is an adult.

The disclosure provides methods to establish a dose regimen for the hGH-XTEN pharmaceutical compositions of the disclosure for human patients. The methods include administration of consecutive doses of a therapeutically effective amount of the hGH-XTEN composition using variable periods of time between doses to determine that interval of dosing sufficient to achieve and/or maintain the desired parameter, blood level or clinical effect; such consecutive doses of a therapeutically effective amount at the effective interval establishes the therapeutically effective dose regimen for the hGH-XTEN for a GHD condition. Thus, in one aspect, the disclosure provides an hGH-XTEN composition for use in a treatment regimen that is therapeutically effective for human growth hormone deficiency (GHD). In one additional embodiment, the human patient is an adult.

In another aspect, the invention provides an hGH-XTEN fusion protein for use in a treatment regimen for human growth hormone deficiency (GHD), which regimen comprises administering a hGH-XTEN fusion protein to a human patient, as defined in the claims.

In one embodiment, the treatment regimen comprises administering a bolus dose of the hGH-XTEN fusion protein to the human patient. In another embodiment, the bolus dose is (i) a therapeutically effective bodyweight adjusted bolus dose; and/or (i) between about 0.05 mg/kg and about 3.0 mg/kg. In one other embodiment, the treatment regimen comprises administering the bolus dose every week, every two weeks, every three weeks, or monthly. In one additional embodiment, the treatment regimen comprises subcutaneous administration of the bolus dose. In one additional embodiment, the human patient is an adult.

In one embodiment, the regimen comprises administering at least two bolus doses of the hGH-XTEN fusion protein to a human patient wherein the dosage is about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and 3.0 mg/kg. In one additional embodiment, the human patient is an adult. In another embodiment, the dosage is administered as at least two bolus doses wherein the administration of said bolus doses is separated by at least about 7 days, or at least about 10 days, at least about 14 days, at least about 21 days, at least about 28 days, or at least about monthly.

Also disclosued herein is a treatment regimen, where the administration of said bolus doses is separated by at least about one month, at least about 31 days, at least about 30 days, at least about 29 days, at least about 28 days, at least about 27 days, at least about 26 days, at least about 25 days, at least about 24 days, at least about 23 days, at least about 22 days, at least about 21 days, at least about 20 days, at least about 19 days, at least about 18 days, at least about 12 days, at least about 11 days, at least about 10 days, at least about 9 days, at least about 8 days, at least about 7 days, at least about 6 days, at least about 5 days, at least about 4 days, at least about 3 days, or at least about 2 days. In another embodiment, the present disclosure provides a consecutive dose regimen wherein each bolus dose is administered every week (or weekly), every two weeks, every three weeks, every four weeks, or monthly.

In one embodiment of the hGH-XTEN composition for use in a treatment regimen, the hGH-XTEN fusion protein comprises the amino acid sequence shown as set forth in FIG. 1 (SEQ ID NO:1). In one embodiment, the therapeutically effective dose treatment regimen comprises the administration of at least two therapeutically effective bodyweight adjusted bolus doses to a subject, wherein the doses are administered subcutaneously.

In general, a "bolus dose" is a dose administered within a short period of time. In another embodiment, the bolus dose is administered within about 1 to about 30 minutes, about 1 to about 20 minutes, about 1 to about 15 minutes, about 1 to about 10 minutes, or about 1 to about 5 minutes. In one embodiment, the bolus dose is administered within about 1 to about 5 minutes. In one other embodiment, the bolus does is a subcutaneous bolus dose.

In another aspect, the treatment regimen results in the human patient exhibiting an improvement in the serum IGF-I standard deviation score (SDS) following administration of a bolus dose. In one embodiment, the IGF-I SDS is between about -2.0 and about 2.0 in the patient following administration of the bolus dose. In another embodiment, the IGF-I SDS is selected from the group consisting of greater than about -2.0, greater than about -1.5, greater than about -1.0, greater than about -0.5, greater than about 0, greater than about 0.5, greater than about 1.0, and greater than about 1.5. In one additional embodiment, the human patient exhibits said IGF-I SDS following administration of the bolus dose, wherein the administration is selected from the group consisting of weekly, every two weeks, every three weeks, and monthly.

In another aspect, the treatment regimen results in normalization of IGF-I concentration in the human patient following administration. In one embodiment, the regimen results in an IGF-I concentration that is normalized for at least about 7 days, or at least about 10 days, or at least about 14 days, at least about 16 days, at least about 17 days, or at least about 21 days following the administration of the first or second dose.

In one embodiment, the regimen results in a serum IGF-I concentration that is normalized for at least about 7 days, or at least about 10 days, or at least about 14 days, at least about 17 days, or at least about 21 days following the administration of the first or second dose. As would be appreciated by one of ordinary skill in the art, "normalized" would vary according to factors such as the disease state, age, sex, and weight of the individual. In another embodiment, the regimen results in a serum IGF-I standard deviation (SD) score of greater than about -2.0, greater than about -1.5, greater than about -1.0, greater than about -0.5, or greater than about 0 following administration of the first or second dose.

In one other aspect, the treatment regimen results in a clinically significant reduction in the patient in at least one parameter related to the GHD evaluation after administration of a bolus dose. In one embodiment, the treatment regimen results in a reduction in the patient of at least one parameter selected from serum cholesterol, serum triglycerides, and serum low density lipoprotein (LDL) after administration of the bolus dose. In another embodiment, the treatment regimen comprises administration of a bolus dose weekly, every two weeks, every three weeks, or monthly.

In another embodiment, the regimen results in a clinically significant reduction in the patient in at least one parameter selected from serum cholesterol, serum triglycerides, and serum LDL after administration of the first or second bolus dose. In another embodiment, the regimen results in an AUC of at least about 11,861 ng-hr/mL, or at least about 33,375 ng-hr/mL, or at least about 91,006 ng-hr/mL, or at least about 241,288 ng-hr/mL, or at least about 402,543 ng-hr/mL after administration of the first or second bolus dose. In another embodiment of the regimen, the human patient achieves an improvement after two or more bolus doses in at least one parameter selected from bone density, bone growth, and increase in epiphyseal plate width. In one other embodiment, the foregoing improvement(s) is at least about 10%, or at least about 20%, or at least about 30%, or at least about 40%, or at least about 50%, or at least about 60%, or at least about 70%, or at least about 80%, or at least about 90%. In another embodiment, the foregoing % improvement(s) is similar to, or not inferior to, an improvement achieved by an hGH not linked to XTEN and administered daily using daily dosage equivalent amounts of hGH.

In another aspect, the present disclosure provides methods of treating human growth hormone deficiency (GHD) with a therapeutically effective amount of an hGH-XTEN fusion protein at a dosage that is equivalent to, or equivalent to less than, an effective amount of a corresponding hGH (not linked to XTEN) administered daily. In one embodiment, the present disclosure provides methods of treating human growth hormone deficiency (GHD), comprising administering to a human patient a therapeutically effective amount of a human growth hormone (hGH) -XTEN fusion protein, wherein the dosage of the hGH fusion protein is equivalent to an amount that is less than about 2 µg hGH/kg/day to about 12 µg hGH/kg/day. In one embodiment, the human patient is an adult.

In one additional aspect, the present disclosure provides methods of treating human growth hormone deficiency (GHD), comprising administering to a human patient with GHD an hGH-XTEN fusion protein at a dosage that is below or less than an equivalent daily dose of recombinant hGH (*e.g.,* a recommended daily dose of rhGH).

In one embodiment, the method comprises administering an hGH-XTEN fusion protein as a bolus dose that is equivalent to less than an hGH/kg/day dosage that is (i) between about 2 µg hGH/kg/day and about 20 µg hGH/kg/day; or (ii) between about 2 µg hGH/kg/day and about 12 µg hGH/kg/day.

In one aspect, the bolus dose may be administered over a range of doses that are equivalent to less than an hGH/kg/day dosage. It should be noted that where reference is made to a bolus dose that is equivalent to less than an hGH/kg/day dosage that is between about a first µg hGH/kg/day and about a second µg hGH/kg/day, the "first µg hGH/kg/day" term may include the first µg hGH/kg/day value and the "second µg hGH/kg/day" term may include the second µg hGH/kg/day value.

In another embodiment, the bolus dose is a therapeutically effective bodyweight adjusted bolus dose of the hGH-XTEN fusion protein. In one other embodiment, the bolus dose is equivalent to less than an hGH/kg/day dosage administered over about 7 days, about 14 days, about 21 days, about 28 days, or about 30 days. In one embodiment, the present disclosure provides methods of treating human growth hormone deficiency (GHD), comprising administering to a human patient with GHD at least two therapeutically effective bodyweight adjusted bolus doses of a human growth hormone hGH-XTEN fusion protein, wherein the administration of said bolus doses is separated by at least about one week, and wherein the dosage of the hGH-XTEN fusion protein is equivalent to (i) less than about 0.3 µg hGH/kg/day to about 18.0 µg hGH/kg/day; or (ii) less than about 0.3 µg hGH/kg/day to about 18.6 µg hGH/kg/day. In another embodiment, the dosage of the hGH-XTEN fusion protein is equivalent to (i) less than about 2 µg hGH/kg/day to about 12 µg hGH/kg/day; or (ii) less than about 2 µg hGH/kg/day to about 20 µg hGH/kg/day. In another embodiment, the administration of said bolus doses is separated by at least about 7 days, at least about 10 days, at least about 14 days, at least about 21 days, at least about 28 days, or at least about monthly. In one embodiment, the dosage equivalent amount of hGH is less than about 4.8 µg/kg/day. In one additional embodiment, the human patient is an adult.

In another embodiment, the bolus dose is equivalent to an hGH/kg/day dosage that is less than about 2 µg hGH/kg/day. In another embodiment, the dosage is equivalent to less than about 0.3 µg hGH/kg/day, about 0.6 µg hGH/kg/day, about 1.2 µg hGH/kg/day, about 2.0 µg hGH/kg/day, about 2.4 µg hGH/kg/day, about 4.0 µg hGH/kg/day, about 4.8 µg hGH/kg/day, about 6.0 µg hGH/kg/day, about 6.2 µg hGH/kg/day, about 7.4 µg hGH/kg/day, about 8.0 µg hGH/kg/day, about 8.6 µg hGH/kg/day, about 9.8 µg hGH/kg/day, about 10 µg hGH/kg/day, about 11.1 µg hGH/kg/day, about 12 µg hGH/kg/day, about 12.4 µg hGH/kg/day, about 13.6 µg hGH/kg/day, about 14 µg hGH/kg/day, about 14.8 µg hGH/kg/day, about 16.0 µg hGH/kg/day, about 16.8 µg hGH/kg/day, about 17.4 µg hGH/kg/day, about 18 µg hGH/kg/day, about 18.6 µg hGH/kg/day, or about 20 µg hGH/kg/day. In one additional embodiment, the human patient is an adult.

In one other embodiment, the bolus dose is equivalent to less than an hGH/kg/day dosage administered over about 7 days, about 14 days, about 21 days, about 28 days, or about 30 days.

In one other embodiment, method comprises administering to the patient a therapeutically effective bodyweight adjusted bolus dose of a human growth hormone-XTEN (hGH-XTEN) fusion protein comprising an amino acid sequence having at least about 90% sequence identity to the sequence set forth in FIG. 1 (SEQ ID NO.1), wherein the mass of human growth hormone administered to the patient is equivalent to less than 0.006 mg/kg/day. In another embodiment, the mass of human growth hormone administered to the patient is equivalent to between about 0.0003 mg/kg/day and about 0.005 mg/kg/day. In one other embodiment, the method comprises monthly dosing of the patient with the hGH-XTEN. In one additional embodiment, the human patient is an adult.

In yet another embodiment, the hGH-XTEN fusion protein comprises an amino acid sequence shown as set forth in FIG. 1 (SEQ ID NO:1). In other embodiments, the administration is subcutaneous administration.

In another aspect, the present disclosure provides methods of normalizing serum IGF-I levels in a subject in need thereof. In one embodiment, the method comprises administering the hGH-XTEN fusion protein to a human patient as a bolus dose that is effective in increasing the patient's IGF-I SDS by at least 0.5 or at least 1.0 above the subject's baseline IGF-I SDS. In another embodiment, the increase in IGF-I SDS is achieved in the absence of a clinically significant level of side-effects selected from the group consisting of headache, arthralgia, myalgia, edema, nausea, and muscle fatigue after administration of the bolus dose. In one additional embodiment, the bolus dose is (i) a therapeutically effective bodyweight adjusted bolus dose; and/or (ii) is administered subcutaneously.

In one other embodiment, the method comprises administering to the subject with GHD at least two therapeutically effective bodyweight adjusted bolus doses of a human growth hormone hGH-XTEN fusion protein, wherein the bolus dose provides a normal serum IGF-I level in said subject. In another embodiment, the administration of said bolus doses is separated by at least about 7 days, or at least about 10 days, at least about 14 days, at least about 21 days, at least about 28 days, or at least about monthly. In one other embodiment of the method, the administration of said bolus doses results in a normalization of serum IGF-I levels in the subject for at least about 5 days, or at least about 10 days, or at least about 14 days, or at least about 17 days, or at least about 21 days. FIG. 6 provides an illustration of normalization of IGF-I in various patients. In one other embodiment, a normal serum IGF-I level is characterized by a serum IGF-I standard deviation (SD) that is above about -2.0; above about -1.5; above about -1.0; above about 0; above about 0.5; above about 1.0; or above about 1.5. In another embodiment, a normal serum IGF-I level is characterized by a serum IGF-I standard deviation (SD) that is between about -1.5 and about 1.5; between about -1.5 and about 1.0; between about - 1.5 and about 0.5; between about -1.5 and about 0; between about -1.5 and about -0.5; and between about -1.5 and about -1.0. In one additional embodiment, the human patient is an adult.

In another embodiment, the subject is a human subject having GHD. In an additional embodiment, the administration is subcutaneous administration. In one other embodiment, the therapeutically effective bodyweight adjusted bolus dose of hGH-XTEN fusion protein is selected from the group consisting of: about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and 3.0 mg/kg. In one additional embodiment, the human patient is an adult. In an additional embodiment, the extent of normalization of IGF-I serum levels is dependent on the dose of the therapeutically effective bodyweight adjusted bolus dose of hGH fusion protein. In one other embodiment, the duration of the IGF-I normalization increases with the therapeutically effective bodyweight adjusted bolus dose of hGH fusion protein.

In another embodiment, the present disclosure provides an hGH-XTEN fusion protein for use as a medicament, or for the treatment of GHD. In another embodiment, the present disclosure provides the use of an hGH-XTEN fusion protein for the manufacture of a medicament for treating GHD in a human patient with GHD. In one other embodiment, the present disclosure provides the use of the fusion protein having the sequence set forth in FIG. 1 (SEQ ID NO:1) in the manufacture of a medicament for the treatment of GHD. In other embodiments, the hGH-XTEN fusion protein is provided in a therapeutically effective bodyweight adjusted dose suitable for bolus administration. In some embodiments, the therapeutically effective bodyweight adjusted bolus dose of hGH-XTEN fusion protein is selected from the group consisting of: about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and 3.0 mg/kg. In one additional embodiment, the human patient is an adult. In another embodiment, the therapeutically effective bodyweight adjusted bolus of hGH-XTEN fusion protein is administered subcutaneously. In some embodiments, the human patient has a serum IGF-I standard deviation (SD) score of greater than about -2.0, greater than about -1.5, greater than about -1.0, greater than about -0.5, greater than about 0, greater than about 0.5, greater than about 1.0, greater than about 1.5, greater than about 1.6, greater than about 1.7, greater than about 1.8, or greater than about 1.9 following administration of the hGH-XTEN fusion protein. In one embodiment, the hGH-XTEN fusion protein comprises an amino acid sequence shown as set forth in FIG. 1 (SEQ ID NO:1).

In another aspect, the present disclosure provides hGH-XTEN fusion protein-based therapeutic agents for treating diseases or conditions related to growth hormone deficiency (GHD). For the prevention, treatment or reduction in the severity of a given disease or condition, the appropriate dosage of a therapeutic agent of the disclosure will depend on the type of disease or condition to be treated, as defined above, the severity and course of the disease or condition, whether the agent is administered for therapeutic purposes, previous therapy, the patient's clinical history and response to the agent, and the discretion of the attending physician.

In another aspect, the present disclosure provides a method for the delaying or slowing down of the progression of a disease or condition related to GHD. In one embodiment, the method comprises administering to subject diagnosed with the disease, condition, or disorder, an effective amount of an hGH-XTEN fusion protein. In another aspect, the disclosure provides a method for treating or ameliorating indicia of a disease or condition related to GHD. In one embodiment, the method comprises administering an effective amount of an hGH-XTEN fusion protein to a subject at risk of the disease or condition, wherein the hGH-XTEN fusion protein is effective against the development of indicia of the disease or condition.

In one additional aspect, the hGH-XTEN fusion proteins provide an ameliorative effect against the development of, or the progression of, clinical and/or histological and/or biochemical and/or pathological indicia (including both symptoms and signs) of diseases or conditions related to GHD in a human subject. In one embodiment, the disease or condition is GHD. In one embodiment, the indicia include an increased level of body fat (especially central or trunk adiposity, *i.e,* the waist), anxiety and depression, lethargy, changes in mood, feelings of isolation from others, a lack of motivation, elevated levels of cholesterol in the blood (e.g., abnormally high levels of low-density lipoproteins when compared to high density lipoproteins), elevated levels of triglycerides in the blood, decreased sexual function and interest, fatigue, decreased lean muscle mass, decreased extracellular fluid volume, decreased muscle strength, decreased physical energy and stamina, and reduced bone density. In another embodiment, the subject is at risk for a disease of condition related to GHD. In general, a subject at risk will previously have incurred some damage to the pituitary gland and/or the hypothalamus. In one embodiment, the subject at risk was previously diagnosed as having a tumor associated with the pituitary gland, and/or underwent surgery, chemotherapy, or radiation therapy to treat the tumor. In another embodiment, the subject at risk previously had or presently has a reduced blood supply to the pituitary gland. In one other embodiment, the subject at risk previously suffered cranial ablation or has a history of head trauma. In some embodiments, the subject at risk previously or presently suffers from a hypothalamic-pituitary disease or disorder.

The efficacy of the treatment of diseases and conditions described herein (including GHD) can be measured by various assessments commonly used in evaluating GHD. For example, the health of hormone-secreting glands can be evaluated by, but not limited to, *e.g.,* IGF-I standard deviation score (SDS), growth hormone stimulation test (GHST), growth hormone releasing hormone (GHRH), stimulation tests, monitoring or measurement of endogenous hGH pulses, IGF-I levels, IGF-I binding protein levels, other blood or biochemical tests (e.g., total cholesterol, low-density lipoprotein (LDL) cholesterol, high-density lipoprotein (HDL) cholesterol, triglyceride, and lipids).

In one additional aspect, the present disclosure provides methods of increasing the efficacy of human growth hormone (hGH) therapy in a human patient. In another aspect, the present disclosure provides methods of determining a subsequent dose of an hGH-XTEN fusion protein administered over a subsequent dosage period when treating a human patient with GHD with the hGH-XTEN fusion protein. The "dosage period" means the time between the administration of a bolus dose (e.g., initial dose) and the next successive administration of a bolus dose (e.g., subsequent dose). The dosage period may change with one or more further successive dose or doses, or may remain constant.

In one embodiment, the foregoing methods of increasing efficacy comprise the step of monitoring the IGF-I standard deviation score (SDS) in a plasma or serum sample obtained from the patient during an initial dosage period of administration of an initial dose of human growth hormone-XTEN (hGH-XTEN) fusion protein. In one embodiment, the hGH-XTEN fusion protein comprising an amino acid sequence having at least about 90% sequence identity to SEQ ID NO:1. In another embodiment, the method further comprises the step of determining a subsequent dose of hGH-XTEN fusion protein administered over a subsequent dosage period based on the IGF-I SDS observed during the initial dosage period. In one additional embodiment, the method further comprises administering the subsequent dose over a subsequent dosage period. In one other embodiment, the subsequent dose improves the efficacy of the treatment during the subsequent dosage period. In another embodiment, the subsequent dose is higher, lower, or equivalent to the initial dose. The initial dose or subsequent dose may be any of the bolus doses described herein. In one additional embodiment, the subsequent dosage period is longer, shorter, or equivalent to the initial dosage period. The initial dosage period or subsequent dosage period may be any of the periods of time described herein (e.g., weekly, every two weeks, etc. or every 7 days, every 10 days, every 14 days, etc.).

### VII). DOSAGE FORMS AND PHARMACEUTICAL COMPOSITIONS

In another aspect, the present disclosure provides bolus doses or dosage forms comprising an hGH-XTEN fusion protein described herein.

In one embodiment, the bolus dose or dosage of an hGH-XTEN fusion protein comprises a therapeutically effective bodyweight adjusted bolus dose for a human patient. In one other embodiment, the bolus dose or dosage comprises between about 0.05 mg/kg and about 3.0 mg/kg of hGH-XTEN fusion protein. In one additional embodiment, the human patient is an adult.

In one other embodiment, the bolus dose or dosage of hGH-XTEN fusion protein is selected from the group consisting of about 0.05 mg/kg, about 0.1 mg/kg, about 0.2 mg/kg, about 0.4 mg/kg, about 0.8 mg/kg, about 1.0 mg/kg, about 1.2 mg/kg, about 1.4 mg/kg, about 1.6 mg/kg, about 1.8 mg/kg, about 2.0 mg/kg, about 2.2 mg/kg, about 2.4 mg/kg, about 2.6 mg/kg, about 2.7 mg/kg, about 2.8 mg/kg, and 3.0 mg/kg. In one additional embodiment, the human patient is an adult.

In other embodiments, the bolus dose or dosage is (i) for use in treating human GHD in a subject in need, e.g., a human patient; and/or (ii) formulated for subcutaneous administration. In one other embodiment, the hGH-XTEN fusion protein comprises the amino acid sequence shown as set forth in FIG. 1 (SEQ ID NO:1). In one embodiment, the bolus dose or dosage form is a pharmaceutical composition comprising the fusion protein having the sequence as set forth in FIG. 1 (SEQ ID NO:1) and a pharmaceutically acceptable carrier.

In another embodiment, the disclosure provides kits, comprising packaging material and at least a first container comprising the pharmaceutical composition of the foregoing embodiment and a label identifying the pharmaceutical composition and storage and handling conditions, and a sheet of instructions for the preparation and/or administration of the pharmaceutical compositions to a subject.

In one additional aspect, the present disclosure provides compositions, pharmaceutical compositions, and dose amounts of an hGH-XTEN fusion protein. In one other embodiment, the pharmaceutical composition or dose amount comprises a fusion protein having the sequence as set forth in FIG. 1 (SEQ ID NO:1), or a sequence having at least about 90% sequence identity to the sequence of SEQ ID NO.1. In another embodiment, the dose amount is for a human patient based upon the weight of the patient. In one other embodiment, the human patient is an adult. The weight of the adult human patient can range from about 45 kg to about 120 kg. In one additional embodiment, the hGH-XTEN fusion protein is provided in the pharmaceutical composition, composition, or dose amount as a certain quantity. In another embodiment, the hGH-XTEN fusion protein is provided in an amount (i) between about 2.25 mg to about 6 mg; (ii) between about 4.5 mg and about 12 mg; (iii) between about 9 mg and about 24 mg; (iv) between about 18 mg and about 48 mg; (v) between about 36 mg and about 96 mg; (vi) between about 45 mg and about 120 mg; (vii) between about 54 mg and about 144 mg; (viii) between about 63 mg and about 168 mg; (ix) between about 72 mg and about 192 mg; (x) between about 81 mg and about 216 mg; (xi) between about 90 mg and about 240 mg; (xii) between about 99 mg and about 264 mg; (xiii) between about 108 mg and about 288 mg; (xiv) between about 117 mg and about 312 mg; (xv) between about 121.5 mg and about 324 mg; (xvi) between about 126 mg and about 336 mg; or (xvii) between about 135 mg and about 360 mg. In one other embodiment, the pharmaceutical composition or dose amount further comprises a pharmaceutically acceptable carrier.

It should be noted that where reference is made to a composition, pharmaceutical composition or dose amount comprising an amount of hGH-XTEN fusion protein between about a first mg and about a second mg, the "first mg" term may include the first mg value and the "second mg" term may include the second mg value.

### VIII). ARTICLES OF MANUFACTURE

In one aspect, the present disclosure also provides kits and articles of manufacture containing materials useful for the treatment, prevention and/or diagnosis of disease (*e.g.*, GHD). In another embodiment, the disclosure provides kits, comprising packaging material and at least a first container comprising a dosage form or pharmaceutical composition of the foregoing embodiment and a label identifying the dosage form or pharmaceutical composition and storage and handling conditions, and a sheet of instructions for the reconstitution and/or administration of the dosage form or pharmaceutical compositions to a subject. In one other embodiment, the kit includes a container and a label, which can be located on the container or associated with the container. The container may be a bottle, vial, syringe, cartridge (including autoinjector cartridges), or any other suitable container, and may be formed from various materials, such as glass or plastic. The container holds a composition having an hGH-XTEN fusion protein as described herein, and may have a sterile access port. Examples of containers include a vial with a stopper that can be pierced by a hypodermic injection needle. The kits may have additional containers that hold various reagents, e.g., diluents, preservatives, and buffers. The label may provide a description of the composition as well as instructions for the intended use.

In one other aspect, the container is a pre-filled syringe. In one embodiment, the syringe is pre-filled with a composition having an hGH-XTEN fusion protein as described herein. In one additional aspect, the present disclosure provides containers of the composition having a hGH-XTEN fusion protein as described herein, wherein the container is suitable for autoinjection of the composition. In one embodiment, the container is a cartridge. In another embodiment, the container is a cartridge in an autoinjection pen. Those of ordinary skill in the art will appreciate that other suitable autoinjection devices may be used for the present invention. In some embodiments, the autoinjection device comprises a spring-loaded syringe within a cylindrical housing that shields the needle tip prior to injection. In one embodiment, the patient depresses a button on the device and the syringe needle is automatically inserted to deliver the contents.

In another embodiment, the device is a gas jet autoinjection device. In other embodiments, the gas jet device comprises a cylinder of pressurized gas but the needle is absent. Upon activation, the device propels a fine jet of liquid through the skin without the use of a needle. In one other embodiment, the device is an iontophoresis device or electromotive drug administration (EMDA) device (e.g., use of a small electric charge to deliver an agent through the skin without the use of a needle).

The kit has at least one container that includes a molecule comprising an hGH-XTEN fusion protein described herein as the active agent. The container may comprise an hGH-XTEN fusion protein dosage form or pharmaceutical composition. A label may be provided indicating that the dosage form or composition may be used to treat a disease. The label may also provide instructions for administration to a subject in need of treatment. The kit may further contain an additional container having a pharmaceutically-acceptable buffer, such as bacteriostatic water for injection (BWFI), phosphate-buffered saline, Ringer's solution and dextrose solution. Finally, the kit may also contain any other suitable materials, including other buffers, diluents, filters, needles, and syringes.

Also disclosed herein is a kit comprising a container which holds a pharmaceutical composition for administration to a human patient comprising a human growth hormone-XTEN (hGH-XTEN) fusion protein. In one embodiment, the hGH-XTEN fusion protein comprises an amino acid sequence having at least about 90% sequence identity to the sequence set forth in FIG. 1 (SEQ ID NO.1). In another embodiment, the kit further comprises a package insert associated with said container. In one other embodiment, the package insert indicates that said composition is for the treatment of growth hormone deficiency by administration of more than one dose of the composition. In one embodiment, the administration is an administration of an initial dose of between about 0.05 mg/kg and about 3.0 mg/kg of the hGH-XTEN and a plurality of subsequent doses of the hGH-XTEN in an amount of between about 0.05 mg/kg and about 3.0 mg/kg. In another embodiment, the doses are separated in time from each other by at least about 7 days. The package insert may further indicate different doses, dose ranges, and times between doses as described herein. In one additional embodiment, the human patient is an adult.

The following are examples of methods, treatment regimens, and compositions of the disclosure. It is understood that various other embodiments may be practiced, given the general description provided above.

### EXAMPLES

### EXAMPLE 1 - Phase I Preliminary Results

A Phase 1 trial of safety, pharmacokinetics (PK) and pharmacodynamics (PD) of a single dose of a human growth hormone analogue (VRS-317) for subcutaneous administration in human adults with growth hormone deficiency has been completed and is detailed herein. VRS-317, a long acting rhGH fusion protein, the sequence of which is presented in FIG. 1, was evaluated in 50 adults with GHD in a 60-day, double-blind, randomized, placebo (PBO)-controlled, single ascending dose study of 0.05, 0.10, 0.20, 0.40 and 0.80 mg VRS-317/kg (ClinicalTrials.gov NCT01359488). VRS-317 is ∼5 times the mass of rhGH due to the addition of N- and C-terminal XTEN amino acids to extend the rhGH half-life. In monkeys, VRS-317 has complete bioavailability, rapid absorption, a half-life of ∼110 hr, and produces a sustained IGF-I response for one month after a single dose.

Initially, subjects were administered daily rhGH (min. of 28 days; dose range of 0.2-1.2 mg/day) until their serum IGF-I standard deviation (SD) score was stable in the range of -1.5 and +1.5. Subjects were then withdrawn from rhGH until the IGF-I SDS was < -1 and had fallen by ≥ 0.75 before treatment with VRS-317. The subjects were observed for 48 hrs after receiving VRS-317 or PBO. PK, PD (IGF-I) and paired fasting/post-prandial glucose were measured pre-dose and at various times over 30 days after a single SC dose of VRS-317 or PBO. Preliminary results from the trial were evaluated, including safety data for 28 subjects and PK/PD for 24 VRS-317 or PBO-treated subjects. PK/PD subjects (15M, 9F) had a mean (SD) age of 46 (12) yrs and BMI of 32 (7) kg/m2.

VRS-317 achieves a Tmax 2-3 days after a SC dose and has a long circulating half-life, potentially sufficient for monthly dosing. The mean maximal increases in IGF-I SDS were 0.33, 0.32, 0.96* and 1.32** in the PBO, 0.05, 0.10 and 0.20 mg/kg/month dosing groups, respectively (*p = 0.012, **p = 0.0005 (vs. PBO)). The percentages of subjects with IGF-I SDS above pre-VRS-317 levels for the initial two weeks were 16, 66 and 100% for the 0.05, 0.10 and 0.20 mg/kg/month groups, respectively. These single VRS-317 doses are equivalent to 0.31, 0.62, and 1.24 µg hGH/kg/day (typical AGHD dosing range for daily rhGH is 2-12 µg/kg/d).

There were no drug-related serious adverse events, withdrawals after dosing or unexpected, related adverse events or injection site lipoatrophy in the subjects. Mean fasting glucose, post-prandial glucose and change from fasting to postprandial showed no significant post-dosing changes in the subjects. No safety laboratory signals were observed in the subjects. In summary, in this trial of a single SC dose of VRS-317 in adults with GHD, graded responses of IGF-I generation were safely achieved at doses lower than those typically used with daily administration of rhGH over the course of one month.

### EXAMPLE 2 - Completion of the Phase I Trial

Example 1 describes preliminary results of a Phase 1 Trial of Safety, Pharmacokinetics (PK) and Pharmacodynamics (PD) of a Single Dose of a New Human Growth Hormone Analogue (VRS-317) for Monthly Subcutaneous Administration in Adults with Growth Hormone Deficiency. The trial has concluded and the final results are reported herein.

VRS-317 was studied in 50 adults (10 placebo/40 active treated) with GHD in a 60-day, double-blinded randomized, placebo(PBO)-controlled, single ascending dose study of 0.05, 0.10, 0.20, 0.40 and 0.80 mg VRS-317/kg (ClinicalTrials.gov NCT01359488). The trial design is summarized as shown below.

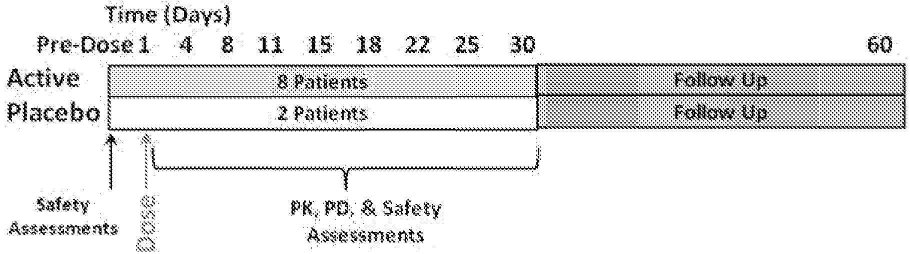

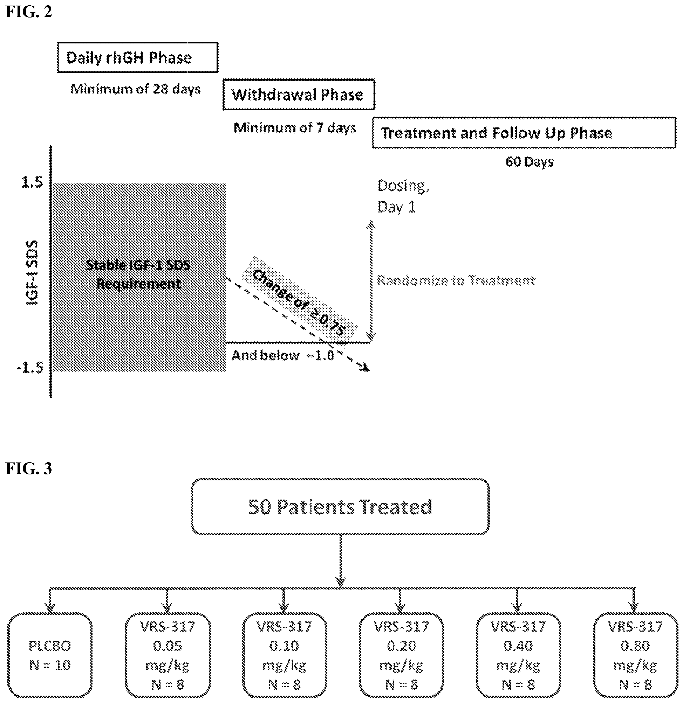

Patients were kept in the clinical unit for the first 48 hours after dosing. Immunogenicity (antibody samples) was evaluated at the following time points: pre-dose, 30 days, and 60 days following dosing. FIG. 2 depicts the study phases for the Phase 1 trial.

**Objectives:** The objectives of the study included the following: to evaluate the safety and tolerability of a single subcutaneous (SC) dose in GHD patients; to determine single dose pharmacokinetics of VRS-317 administered SC; to evaluate evidence of VRS-317 bioactivity by changes from baseline in insulin-like growth factor-1 (IGF-I) and binding protein (IGFBP-3), and bone turnover (bone alkaline phosphatase); and to determine the dose to maintain a normal range (for appropriate age/gender) for IGF-I levels in adult patients for one month after administration of a single dose.

**Dosing:** Because of a demonstrated enhancement of the *in vivo* potency of GH in monkeys receiving VRS-317 (Cleland et al. 2012 *supra*), the VRS-317 dose range for the first dose in humans was selected to approximate the daily rhGH doses in the lower half of the typical dosing range for each 30 day interval (i.e., 0.03 to 0.5 mg rhGH/day or approximately 0.3 to 5.0 µg/kg/day). The selected VRS-317 doses were 0.05, 0.10, 0.20, 0.40 and 0.80 mg/kg administered as a single subcutaneous injection.

As shown in Table 2.1 below, VRS-317 single SC dose levels were at or below the equivalent mean adult GHD daily rhGH dose of 5 µg/kg/day.

**Table 2.1**

| **Dose Level** | **VRS-317 Dose (mg/kg - one dose)** | **rhGH equivalent (µg/kg/day x 30 days)** |
|---|---|---|
| 1 | 0.05 | 0.31 |
| 2 | 0.10 | 0.62 |
| 3 | 0.20 | 1.24 |
| 4 | 0.40 | 2.48 |
| 5 | 0.80 | 4.97 |

**Patient Disposition:** Enrolled subjects had growth hormone deficiency (GHD), as confirmed by a negative response to insulin (peak GH < 5.0 ng/mL), arginine-GHRH (peak GH based on BMI) (Molitch ME, et al. 2011. J Clin Endocrinol Metab 96(6):1587-1609; Cook DM, et al. 2009. Endocrine practice : official journal of the American College of Endocrinology and the American Association of Clinical Endocrinologists 15 Suppl 2:1-29), glucagon (GH peak < 3.0 ng/mL) (Yuen KC, et al. 2009. J Clin Endocrinol Metab 94(8):2702-2707), or at least 3 other pituitary hormone deficiencies and a low IGF-I for age and gender (Molitch ME, et al. 2011 *supra*). When GHD was due to a sellar region lesion, scans showed at least 6 months of stability. Treatments for other pituitary hormone deficiencies were stable for 2 months prior to study drug administration. Free T4 was in the normal range for all subjects when VRS-317 was administered. Each subject not taking daily glucocorticoid treatment had normal responses to a standard dose (250 µg) ACTH test to rule out secondary adrenal insufficiency. For female patients receiving estrogen, transdermal treatment was used and maintained throughout the study. IGF-I responses to daily rhGH were characterized in all subjects prior to study drug administration. Key exclusion criteria included the presence of significant concurrent disease (e.g. diabetes), active malignancy, anti-hGH antibodies at screening, pregnancy, lactation or the use of oral estrogens.

FIG. 3 summarizes the patient disposition in the study. No patients dropped out of the study after treatment with VRS-317 (or placebo).

**Study procedures and method of study:** Initially, all subjects were maintained on daily rhGH for a minimum of 28 days and until two successive IGF-I standard deviation scores (SDS), drawn at least one week apart, were within the range of -1.5 to 1.5 (+ 2.0 for males). Subjects were then withdrawn from daily rhGH until their IGF-I SDS decreased by at least 0.75 and had dropped to ≤ -1.0. Subjects were then randomized to the treatment cohort enrolling at that time. On Day 1, all subjects received a single subcutaneous (SC) dose of VRS-317 or placebo administered with an insulin syringe with a 29 gauge needle. Pharmacokinetic and pharmacodynamic (PK/PD) samples were collected pre-dose and at 0.5, 1.0, 2, 4, 8, 12, 24, 36 and 48 hours after dosing. Additional PK/PD sampling was conducted on Days 4, 8, 11, 15, 18, 22, 25 and 30 after dosing. Glucose and lipid metabolism was assessed pre-dose and on Day 8, 15, 22, 30, 44 and 60 after dosing. Testing for anti-VRS-317 antibodies was conducted pre-dose and on Days 30 and 60 after dosing. Before proceeding to the next dosing level, safety data was reviewed. Laboratory safety assessments were performed prior to and at selected times after dosing. Tests included standard blood counts, biochemistries, postprandial glucose, hemoglobin A1c (HbA1c) and fasting levels of blood glucose, cholesterol, LDL, HDL and triglycerides.

**Definition of patient populations:** The safety population consisted of all 50 randomized subjects. The PK/PD population consisted of 48 subjects receiving either VRS-317 or placebo and excluded two subjects who received inappropriate doses for their weight (one subject in the 0.80 mg/kg dose group and one subject in placebo group).

**Assays:** VRS-317 concentrations in collected plasma were measured using an ELISA. The assay uses capture and detection antibodies to the XTEN and rhGH domains, respectively, to ensure detection of the intact molecule. Anti-VRS-317 antibodies were measured in samples taken pre-dose and at Day 30 and Day 60. Due to the potential for interference from high VRS-317 concentrations, samples were taken at the end of the dosing interval and assays were performed using solid-phase extraction with acid dissociation followed by a direct electrochemiluminescense assay. Anti-rhGH antibodies were measured in a direct ELISA. IGF-I was measured to bioanalytical standards using the acid extraction, IGF-II blocking radioimmunoassay (RIA), performed by Esoterix, Inc. (Calabasas Hills, CA). The lower limit of quantitation for the IGF-I assay is 15 ng/ml. IGFBP-3 was also measured by RIA at Esoterix. The lower limit of quantitation for the IGFBP-3 assay is 0.3 mg/L. Assay-specific standard deviation scores (SDS) for IGF-I and IGFBP-3 were developed using power transformed normative data (Esoterix, Calabasas Hills, CA) for the assays in use.

**PK/PD Analysis:** VRS-317 PK parameters were estimated with non-compartmental techniques using WinNonLin™ professional v5.3 (Pharsight Corporation, Mountain View, CA). The IGF- I area under the curve after a single SC dose of VRS-317 was calculated using the linear trapezoid rule and average IGF-I was calculated by dividing IGF-I AUC by the time of the dosing interval.

**Statistical Analysis:** Descriptive statistics and multivariate analyses were conducted according to a statistical analysis plan finalized prior to database lock. Laboratory parameters were analyzed for change from pre-dose baseline by ANCOVA with change as the dependent variable, treatment as cofactor and baseline value as covariate. P-values < 0.05 defined statistical significance.

### RESULTS

**Patient Disposition and Characteristics:** Sixty-nine subjects were screened for enrollment; there were 19 screen failures and 50 subjects were randomized to five groups each consisting of 8 active- and 2 placebo-treated subjects. There were no withdrawals by subjects after randomization; all 50 randomized subjects completed the 60 day dose-evaluation period. There were 21 females and 29 males with a mean age of 44.7 years (Table 2.2). Age distributions were similar in each of the five dosing cohorts; however, some gender imbalance occurred between dosing arms (placebo and 0.10 mg/kg cohorts included 6 males, 2 females; 0.80 mg/kg cohort had 3 males, 5 females). Daily rhGH doses in the stability phase were in the range of 0.4 to 0.6 mg/day (4.1-5.8 µg/kg/day) on average across all the dose groups. For subjects randomized to VRS-317, the mean change in IGF-I SDS after rhGH withdrawal ranged from -1.7 to -2.4.

Table 2.2 below provides the characteristics of randomized subjects. Values are means (minimum, maximum) except as noted. Baseline is defined as the last measurement before study drug administration.

**Table 2.2**

| **Treatment Group** | **0.05 mg/kg n = 8** | **0.10 mg/kg n = 8** | **0.20 mg/kg n = 8** | **0.40 mg/kg n = 8** | **0.80 mg/kg n = 8** | **Placebo n = 10** |
|---|---|---|---|---|---|---|
| **Age in years (range)** | 41.41 (29, 57) | 55.5 (48, 64) | 37.1 (27, 59) | 44.6 (29, 58) | 43.1 (26, 59) | 46.3 (26, 66) |
| **Male, n (%)** | 4 (50) | 6 (75) | 5 (62.5) | 5 (62.5) | 3 (37.5) | 6 (60) |
| **BMI, kg/m² (range)** | 34.2 (23, 45) | 29.5 (20, 43) | 33.7 (27, 44) | 30.8 (23, 38) | 27.5 (19, 34) | 30.0 (23, 39) |
| **Height, cm (range)** | 173.1 (160,180) | 175.8 (160,185) | 173.1 (151,183) | 169.1 (153,178) | 170.0 (159, 188) | 172.9 (159,191) |
| **Weight, kg (range)** | 103.2 (58,138) | 90.9 (61, 124) | 101.3 (70, 130) | 88.1 (66,115) | 80.7 (49,119 ) | 90.9 (61,144) |
| **rhGH dose, µg/kg/day (range)** | 5.1 (1.5, 9.6) | 4.1 (2.5, 7.3) | 5.0 (1.9, 7.9) | 5.2 (2.5, 10.5) | 5.8 (2.5, 10.2) | 5.2 (2.3, 10.3) |
| **IGF-ISDS in Daily rhGH Phase (range)** | 0.11 (-0.54,1.4) | 0.17 (-1.1,1.5) | -0.21 (-1.5,0.9) | -0.63 (-1.4, -0.1) | 0.02 (-1.3, 1.6) | -0.12 (-1.2, 0.9) |
| **IGF-I SDS in Withdraw al Phase (range)** | -1.65 (-2.2, -1.3) | -2.00 (-3.0, -1.4) | -1.92 (-2.7, - 1.1) | -2.19 (-2.8, -1.0) | -1.64 (-2.4, - 1.0) | -1.63 (-2.8, -1.2) |
| **Change in IGF-I SDS (Daily to Withdraw al** | 1.76 | 2.17 | 1.71 | 1.56 | 1.62 | 1.51 |
| **IGF-I SDS at baseline (range)** | -1.74 (-2.3, -0.8) | -2.27 (-2.9, -1.8) | -2.09 (-3.0, -1.4) | -2.30 (-2,9, -0.7) | -1.75 (-2.9,-1.1) | -1.52 (-3.1, -0.67) |

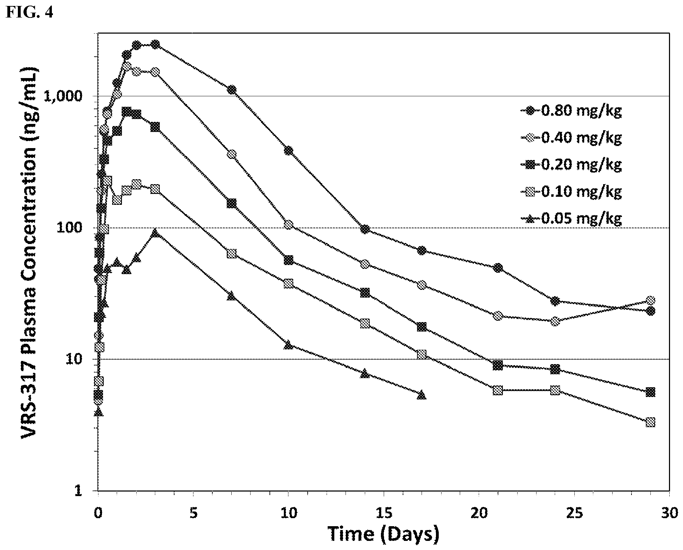

**Pharmacokinetics:** FIG. 4 shows the human pharmacokinetic (PK) profile for various single doses of VRS-317. Figure 4 shows the time course of mean VRS-317 concentrations in adult GHD subjects receiving a single subcutaneous dose on Day 1. The variance bars are omitted for clarity; the mean coefficient of variation (SD/Mean) at Cmax for VRS-317 was 57% (all doses). Table 2.3 below provides the pharmacokinetic parameters (Mean ± Standard Deviation) of VRS-317 in growth hormone-deficient adults following a single subcutaneous injection. A single SC dose resulted in rapid absorption and prolonged serum exposure to VRS-317 (Figure 4). Mean maximal VRS-317 plasma concentrations (Cmax) were reached at 44 to 82 hours (Table 2.3). VRS-317 exposure was directly proportional to dose. There was a general trend for VRS-317 elimination half-life (t1/2) to increase with increasing dose. The mean t1/2 was 131 hours at the highest dose tested (0.80 mg/kg) (Table 2.3). In multivariate analyses, the AUC₀₋ₜ for VRS-317 was highly correlated to dose (p < 0.0001) but no significant age or gender effect was observed in this population.

**Table 2.3: Pharmacokinetic Parameters Resulting from Administration of VRS-317**

| **Dose (mg/kg)** | **Cₘₐₓ (ng/mL*)** | **Tₘₐₓ (hr*)** | **AUC₀₋ₜ (ng·hr/mL*)** | **AUC_{0-∞} (ng·hr/mL*)** | **t_{1/2} (hr*)** |
|---|---|---|---|---|---|
| 0.05 | 92 ± 29 | 46 ± 27 | 11,161 ± 3,395 | 11,706 ± 3,499 | 68 ± 18 |
| 0.10 | 354 ± 368 | 44 ± 21 | 33,365 ± 16,410 | 33,822 ± 16,343 | 85 ± 34 |
| 0.20 | 889 ± 606 | 50 ± 19 | 86,429 ± 67,201 | 87,291 ± 67,068 | 90 ± 50 |
| 0.40 | 1,968 ± 676 | 48 ± 17 | 241,280 ± 121,549 | 244,601 ± 125,167 | 109 ± 57 |
| 0.80 | 2,887 ± 1,345 | 82 ± 39 | 402,541 ± 124,653 | 407,421 ± 124,915 | 131 ± 62 |

| | | | | | |
|---|---|---|---|---|---|
| * units ± S.D. Cₘₐₓ = maximum concentration; Tₘₐₓ = time to maximum concentration; AUC₀₋ₜ = area under the curve from time zero to the last measurable time point; AUC_{0-∞} = area under the curve from time zero to infinity; t_{1/2} = terminal half-life. The dose proportionality correlation coefficients (log:log) were 0.87 for Cmax and 0.93 for AUC₀₋ₜ. | | | | | |

No gender-based PK effect was observed. A significant (p=0.016) linear increase in t ½ was observed with increased dose. A dose proportional increase in Cmax and AUC was observed.

**Pharmacodynamics:** IGF-I concentration was the primary pharmacodynamic marker employed for this study. The amplitude and duration of IGF-I exposure was directly proportional to VRS-317 dose (Figure 5, Table 2.4). FIG. 5 illustrates a dose-response change in mean IGF-I SDS for 0.05, 0.10, 0.20, 0.40 and 0.80 mg VRS-317/kg. Figure 5 shows the mean change in IGF-I SDS for placebo and 5 active dosing groups (note: one subject in the 0.80 mg/kg dose group was omitted from this figure because of an error in dose administration). The variance bars are omitted for clarity; the standard deviation at Cmax for IGF-I SDS for the five active dose groups ranged from 0.7 to 1.3.

**Table 2.4**

| **Dose (mg/kg)** | **N** | **IGF-I at Stability (ng/mL*)** | **IGF-I at Baseline (ng/mL*)** | **IGF-I Cmax (ng/mL*)** | **IGF-I Cmax (SDS*)** | **IGF-I Tmax (days*)** | **IGF-I AUC₀₋ₜ (ng·hr/mL*)** | **Average IGF-I (ng/mL*)** |
|---|---|---|---|---|---|---|---|---|
| Placebo | 9 | 188 ± 49 | 106 ± 47 | ND | ND | ND | ND | 102 ± 49 |
| 0.05 | 8 | 212 ± 41 | 97 ± 47 | 137 ± 58 | -1.1 ± 0.7 | 6.4 ± 6.5 | 2837 ± 1330 | 95 ± 44 |
| 0.10 | 8 | 170 ± 30 | 57± 18 | 105 ± 43 | -1.2 ± 0.9 | 5.0 ± 2.9 | 2214 ± 855 | 74 ± 29 |
| 0.20 | 8 | 214 ± 68 | 86 ± 30 | 196 ± 58 | -0.5 ± 0.9 | 4.1 ± 1.8 | 3541 ± 1260 | 118 ± 42 |
| 0.40 | 8 | 165 ± 44 | 70 ± 40 | 248 ± 87 | 0.9 ± 1.4 | 4.5 ± 1.4 | 3771 ± 1524 | 126 ± 51 |
| 0.80 | 7 | 197 ± 76 | 89 ± 31 | 280 ± 103 | 1.4 ± 1.3 | 5.7 ± 2.1 | 4884 ± 915 | 163 ± 31 |

| | | | | | | | | |
|---|---|---|---|---|---|---|---|---|
| * units ± S.D. Stability refers to the time during daily rhGH treatment was given. Baseline refers to Day 1, prior to the dose of VRS_317 or placebo. Cₘₐₓ = maximum concentration; Tₘₐₓ = time to maximum concentration; AUC₀₋ₜ = area under the curve from time zero to the last measurable time point. The IGF-I AUC was calculated using the linear trapezoid rule. Average IGF-I was calculated by dividing AUC by the observation interval of 29 days. ND = not determined. The dose proportionality correlation coefficients (log:log) were 0.76 for baseline corrected Cmax and 0.76 for baseline corrected AUC₀₋ₜ. | | | | | | | | |

FIG. 6 illustrates a sustained IGF-I response to a single dose of VRS-317 (Patients with baseline IGF-I SDS below -1.5). Figure 6 shows the extent of normalization of IGF-I SDS after single SC dose administration of VRS-317 (note: data for 5 of the 39 subjects in Figure 4 were excluded from Figures 5-6 because their baseline IGF-I SDS was ≥ -1.5 and their inclusion would have exaggerated duration of normalization of IGF-I SDS).

An important observation was that the maxima for mean changes in IGF-I concentrations and IGF-I SDS appeared similar for the 0.40 mg/kg and 0.80 mg/kg groups. The similarity may have been caused by uneven distribution of subject characteristics affecting IGF-I responses to VRS-317. Therefore, an ANCOVA was used to examine the set of all post-dose values of IGF-I concentration for dependencies upon age, gender, treatment day, VRS-317 dose, treatment by day interaction (as factors) and baseline (pre-dose) IGF-I concentration (as covariate). Dose, day and dose and treatment by day interaction were all significant (p < 0.0001) as were age (p = 0.0034) and gender (p = 0.0224). Higher doses, male gender and younger age were all associated with greater IGF-I responses.

The extent and duration to which IGF-I SDS were normalized were also VRS-317 dose-dependent. An analysis of subjects having an IGF-I SDS below - 1.5 at the time of dosing indicated that VRS-317 increased the IGF-I SDS into the normal range of -1.5 to 1.5 in a dose-dependent manner (Figure 6). IGF-I SDS was normalized for a mean of approximately 3 weeks for the 0.80 mg/kg group. This prolonged duration of normalization did not come at the expense of overexposure to IGF-I. The forty VRS-317 treated patients had a total of 513 post-dose IGF-I SDS determinations and only 8 values (1.6%) in 6 patients were above the normal range (SDS > + 2). The individual IGF-I SDS values above + 2 ranged from 2.01 to 3.59, occurred only in the 0.40 and 0.80 mg/kg groups, were usually were observed within 72 hours after dosing and had normalized by the subsequent sampling time.

IGFBP-3 SDS were low at baseline (Mean -1.28, SD 1.82) but increased with VRS-317 dosing. The time course of change in IGFBP-3 was similar to that of IGF-I. Maximal IGFBP-3 responses were generally observed at Day 4 or Day 8. The changes in IGFBP-3 were dose-dependent. At Day 8, the least square mean changes in IGFBP-3 were 0.05, 0.17, 0.55, 0.80, and 1.41 mg/L (IGFBP-3 SDS Cmax of -0.6 to 2.6) for the 0.05, 0.10, 0.20, 0.40 and 0.80 mg/kg dosing groups, respectively. In ANCOVA, IGFBP-3 responses were dependent on VRS-317 dose, day and baseline value (all p< 0.0001) but no effects of age or gender were observed. At baseline the IGF-I/IGFBP-3 molar ratio was 0.22 ±0.05 and not statistically different between dose groups (p = 0.49). Mean maximal molar ratio values were observed on Day 4 and increased with increasing VRS-317 dose (p < 0.0001). The maximal mean molar ratio for the 0.80 mg/kg group was 0.47 ± 0.11. The maximal molar ratio value for any subject was 0.65.

### Safety Results:

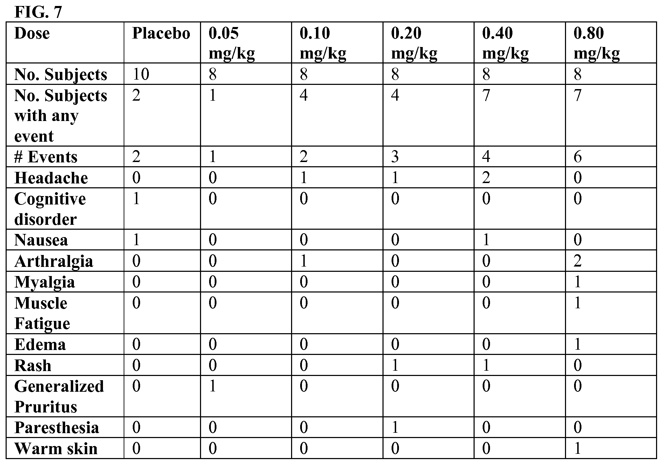

After review of safety data from a minimum of 8 patients exposed for a minimum of 7 days, patients were enrolled in all five planned dosing levels and there were no unexpected adverse events (AEs) related to the study drug. Non-laboratory AEs considered related to study drug by investigators were transient and mild (CTCAE Grade 1 except 2 cases of Grade 2) and occurred in a minority of subjects (Figure 7).

Figure 7 provides treatment-emergent adverse events possibly, probably or definitely related to study drug administration in the safety population (n = 50) of GHD Adults. Injection site reactions and laboratory events are discussed herein. Many related events (headache (4), arthralgia (3), myalgia (1) and edema (1)) were of the type typically observed when rhGH is started in adult GHD patients. The 0.40 and 0.80 mg/kg dosing groups had the greatest number of any related AEs (7 in each group) but no specific event had a clear dose-relationship.

Injection site reactions were the most commonly reported drug-related adverse event. Injection site erythema was noted in 30% of VRS-317 treated and 10% of placebo treated subjects. Injection site edema was noted in 10% of VRS-317 treated subjects and 10% of placebo treated subjects. Injection site pain or tenderness was observed in 15% of VRS-317 treated subjects. In general, for placebo and study drug-treated patients, injection site reactions appeared within 24 hours and were mild (Draize I, barely perceptible) and transient. There were no instances of injection site lipoatrophy or hypersensitivity reported through 60 days of posttreatment observation.

**Glucose & Lipid Metabolism:** The safety of rhGH has been extensively characterized in animals and humans, and glucose intolerance has been observed and reported at certain doses of rhGH. Following administration of VRS-317, glucose and lipid metabolism was regularly assessed, including during the follow-up period. No significant changes were observed by day or dose (fasting glucose, post-prandial glucose, fasting insulin, and HbA1c). A clinically-significant reduction in cholesterol, triglycerides, and LDL was observed at the 0.8 mg/kg VRS-317 dose (data not shown). There were no reported safety events or clinically meaningful changes related to any glucose metabolism parameter. No patient had a glucose result in the diabetic range (fasting ≥ 126 mg/dL, post-prandial ≥ 200 mg/dL). All mean and individual values for HbA1c remained within the normal range. No clinically meaningful changes (≥ 0.2%) were noted in change from baseline HbA1c versus placebo in any treatment group. One patient each from the 0.10 and 0.20 mg/kg dosing group had worsening of previously elevated levels of serum cholesterol, LDL and triglycerides as possibly related AEs. However, at the highest VRS-317 dose (0.80 mg/kg), there was a temporal pattern of reduction in cholesterol, LDL and triglycerides, maximal at Day 8 and persisting through Day 22. The maximal percent decreases from baseline were 11.3 (p = 0.0026), 14.6 (p = 0.014) and 14.5 % (p = 0.19) for cholesterol, LDL and triglycerides, respectively. In summary, no observed data related to glucose and lipid metabolism resulted in safety concerns.

**Antibody assessments:** Non-specific binding was noted in the anti-hGH antibody assay. No subject had a significant titer (≥1:10) of specific anti-rhGH antibodies at screening and no subject tested positive at 7 days post-daily rhGH withdrawal. A single subcutaneous administration of VRS-317 to adult GHD patients previously treated with daily rhGH resulted in a minority of subjects (4 of 40) generating an anti-VRS-317 antibody response at low titer (3 of 4 subjects at 1:5, one subject at 1:25). Three of these 4 had non-specific binding in the anti-hGH antibody assay. Analysis of potential antibody effects on clinical or pharmacological endpoints was precluded by the low number of subjects testing positive for anti-VRS-317; there were no notable differences in IGF-I responses of these four subjects.

In summary, this study in adult GHD patients provides certain safety, pharmacokinetic, and pharmacodynamic (PD) information about VRS-317. Single doses of VRS-317 were found to be safe and well tolerated (see FIG. 7). Regarding the PK profile, AUC, Cmax, and half-life of VRS-317 was found to be proportional to dose. In addition, the duration of exposure to VRS-317 was found to increase with increased dose. Regarding the PD profile, the serum IGF-I normalized in a dose dependent manner and the duration of IGF-I normalization increases with increased dose. In addition, the dosing up to the midpoint of the daily rhGH dose range resulted in normalization of IG-1 for up to 3 weeks.

VRS-317 contains XTEN domains that increase the hydrodynamic radius and reduce binding affinity to the GH receptor (GHR), *in vitro.* Despite reduced binding affinity, durable pharmacodynamic responses are seen, *in vivo,* possibly relating to reduced rates of receptor mediated clearance of VRS-317 (Cleland et al. 2012 *supra*). The reduced rate of clearance prolongs serum residence times of VRS-317, resulting in enhanced ligand time on target. The terminal elimination half-life of VRS-317 at the highest dose was 131 hours; this represents a 30- to 60-fold increase over those reported in package inserts for daily rhGH products.

The current study was the first in humans for VRS-317 and extends prior knowledge about long-acting rhGH because it represents the most prolonged duration of action of any rhGH analogue in the treatment of adults with GHD. All subjects were adults with GHD diagnosed in accordance with current consensus guidelines of The Endocrine Society, the American Association of Clinical Endocrinologists and the Growth Hormone Research Society. There was a slight preponderance of male subjects (29M, 21F) but the numbers of each gender were adequate to test for gender effects on drug distribution and pharmacodynamic effects. Each subject was initially stabilized on daily rhGH injections and, to achieve stable IGF-I SD scores within the normal range, had been taking 0.2 to 1.0 mg hGH/day (mean 0.6 mg/day) or 1.5 to 10.5 µg/kg/day. Following discontinuation of daily rhGH, IGF-I SDS decreased in all subjects with group mean decrements of 1.7 to 2.5 SD. Subjects requiring daily medication that could alter sensitivity to rhGH (e.g., insulin, oral estrogens, anti-inflammatory doses of glucocorticoids) were excluded from this first dosing study of VRS-317.

Over the VRS-317 dosing range, drug exposure parameters (Cmax and AUC) were directly and highly proportional to dose. In general, both the amplitude and duration of exposure increases with increased VRS-317 dose. No gender or age effects were detected in the VRS-317 dose-exposure relationship. VRS-317 was safe and well-tolerated at all dose levels suggesting that greater dose exposures can be explored in future human studies. The pharmacodynamic (IGF-I and IGFBP-3) responses to VRS-317 were also directly proportional to dose, with amplitude and duration increasing with increased dose. At the highest dose, the mean IGF-I SDS was maintained above -1.5 for approximately 3 weeks. Given the demonstrated proportionality between dose and duration, the duration of IGF-I normalization could be extended by increased VRS-317 doses. Over the dose range assessed in this study, the results support that the duration of IGF-I normalization does not come at the expense of over-exposure to IGF-I: only 1.6% of observed IGF-I SDS were ≥ 2 and these elevations were transient. There were age and gender effects on IGF-I responses to VRS-317 such that females and older subjects had lower responsiveness than males. Based on these analyses, females and older subjects are anticipated to have lower IGF-I responses to VRS-317. Gender differences for IGF-I induction are well known for daily rhGH and are likely due to estrogen effects on IGF-I producing cells. Similar to the effects of daily rhGH, IGF-I induction by VRS-317 in adults may be lower in females than in males.

VRS-317 was administered at doses ranging from 0.05 to 0.80 mg/kg; approximating daily rhGH doses of 0.3 to 5 µg/kg/d over 30 days. Over this range, a single dose of VRS-317 was safe and well-tolerated. There were no treatment emergent serious adverse events or suspected unexpected serious adverse reactions. No subject withdrew from the study after dosing; all subjects completed the protocol-specified 60 day safety observation period. Minimal, transient erythema at the injection site(s) was the most commonly reported adverse event. Other events considered as possibly, probably or definitely related to study drug were typical of those seen when adult GHD patients receive replacement therapy. These events were transient and were categorized as mild-moderate. No injection site lipoatrophy was observed. Surveillance for VRS-317 alterations in carbohydrate metabolism included serial measurements of fasting glucose and insulin, post-prandial glucose and HbA1c. No clinically-meaningful temporal or dose-related changes were observed in any of these parameters, indicating that the prolonged action and delayed clearance of VRS-317 did not confer any additional risk to overall glycemic safety in these patients. These findings are in accordance with previous studies with low dose daily rhGH (Yuen KC et al. 2009, *supra;* Spina LDC, et al. 2004. Growth Hormone & IGF Research 14(1):45-51; Hana V, et al. 2004, Clinical Endocrinology 60(4):442-450; Bulow B et al. 2004. Clinical Endocrinology 61(6):683-691; Yuen KC et al. 2007, Diabetes, Obesity & Metabolism 9(1):11-22) but in contrast to other studies showing elevated glucose and insulin with decreased insulin sensitivity indices during long-term daily rhGH treatment (Boguszewski CL et al. 2005 European Journal of Endocrinology 152(1):67-75; Moller N et al. 2009. Endocrine Reviews 30(2):152-177; Christopher M et al. 1998. J Clin Endocrinol Metab 83(5):1668-1681). Although two subjects in a lower dose group had increases in previously elevated levels of LDL, total cholesterol and triglycerides, there was a temporal pattern of decrease in these parameters at the highest VRS-317 dose level (0.80 mg/kg). It is considered as likely that rhGH dose and duration effects as well as individual susceptibility will influence glucose, lipid and insulin responses. Continued surveillance for alterations in lipid and glucose parameters is warranted during subsequent chronic dosing trials.

Four of the 40 VRS-317 treated subjects had detectable anti-VRS-317 antibodies appearing at Day 30 and/or 60 after VRS-317 dosing. These subjects had received VRS-317 doses of 0.2 mg/kg (1 subject), 0.40 mg/kg (2 subjects) or 0.80 mg/kg (1 subject). Three of these four had had non-specific binding in the anti-rhGH antibody screening assay.

In conclusion, single dose administration of VRS-317 is safe and well tolerated over the range of doses studied and provides prolonged normalization of IGF-I responses in adults with GHD. The safety and PK/PD profiles suggest VRS-317 doses may be further increased to prolong IGF-I responses in this population. Given its delayed clearance, VRS-317 has the potential for monthly dosing in adults with GHD.

## Claims

1. A human growth hormone-XTEN (hGH-XTEN) fusion protein comprising an amino acid sequence having at least 90% sequence identity to SEQ ID NO:1,
for use in a method of treating human adult growth hormone deficiency (AGHD) in a human patient,
wherein the method comprises administering to the human patient with AGHD successive therapeutically effective bodyweight adjusted bolus doses of between 0.05 mg/kg and 3.0 mg/kg of the hGH-XTEN fusion protein,
wherein the dosage period between the initial bolus dose and a successive administration of a bolus dose is 17, 16, 15, 14, or 13 days,
wherein successive bolus doses of the hGH-XTEN fusion protein are:
(i) effective to maintain the patient's serum IGF-I standard deviation score (SDS) between -2.0 and +2.0 for at least 7 days after administration of the bolus doses; or
(ii) effective to maintain a plasma concentration of said fusion protein in the patient at more than 10 ng/mL for a period of at least 10 days after administration of the bolus doses.

2. The hGH-XTEN fusion protein for use according to claim 1, wherein the bolus doses of hGH-XTEN fusion protein are between 0.05 mg/kg and 0.8 mg/kg or between 0.8 mg/kg and 1.2 mg/kg , or between 0.05 mg/kg and 3.0 mg/kg.

3. The hGH-XTEN fusion protein for use according to claim 1, wherein the bolus doses of hGH-XTEN fusion protein are selected from the group consisting of 0.05 mg/kg, 0.1 mg/kg, 0.2 mg/kg, 0.4 mg/kg, 0.8 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.7 mg/kg, 2.8 mg/kg, and 3.0 mg/kg.

4. The hGH-XTEN fusion protein for use according to claim 1, wherein the initial dose and the at least one subsequent dose are each in an amount (i) between 2.25 mg to 6 mg; (ii) between 4.5 mg and 12 mg; (iii) between 9 mg and 24 mg; (iv) between 18 mg and 48 mg; (v) between 36 mg and 96 mg; (vi) between 45 mg and 120 mg; (vii) between 54 mg and 144 mg; (viii) between 63 mg and 168 mg; (ix) between 72 mg and 192 mg; (x) between 81 mg and 216 mg; (xi) between 90 mg and 240 mg; (xii) between 99 mg and 264 mg; (xiii) between 108 mg and 288 mg; (xiv) between 117 mg and 312 mg; (xv) between 121.5 mg and 324 mg; (xvi) between 126 mg and 336 mg; or (xvii) between 135 mg and 360 mg.

5. The hGH-XTEN fusion protein for use according to claim 1 or claim 2, wherein the bolus doses are administered subcutaneously.

6. The hGH-XTEN fusion protein for use according to claim 1, wherein the human patient has a serum IGF-I standard deviation score (SDS) between -2.0 and 2.0 following administration.

7. The hGH-XTEN fusion protein for use according to claim 1, wherein the human patient has an IGF-I SDS selected from the group consisting of greater than -2.0, greater than -1.5, greater than -1.0, greater than -0.5, greater than 0, greater than 0.5, greater than 1.0, and greater than 1.5.

8. The hGH-XTEN fusion protein for use according to claim 1, wherein administration of the bolus dose results in a normalization of IGF-I SDS in the human patient for at least 7 days, at least 10 days, at least 14 days, at least 16 days, or at least 21 days.

9. The hGH-XTEN fusion protein for use according to claim 1, wherein the successive bolus doses of the hGH-XTEN fusion protein are effective in increasing the patient's IGF-I SDS by at least 0.5 or at least 1.0 above the subject's baseline IGF-I SDS in the absence of a clinically significant level of side-effects selected from the group consisting of headache, arthralgia, myalgia, edema, nausea, and muscle fatigue after administration of the bolus dose.

10. The hGH-XTEN fusion protein for use according to claim 1, wherein the hGH-XTEN fusion protein comprises the amino acid sequence of SEQ ID NO:1.

11. The hGH-XTEN fusion protein for use according to claim 1(i) or claim 2 as dependent on claim 1(i), wherein said bolus doses are effective to maintain the patient's serum IGF-I SDS between -2.0 and 2.0 for at least 20 days after administration of the bolus doses.

12. The hGH-XTEN fusion protein for use according to claim 1(ii) or claim 2 as dependent on claim 1(ii), wherein said bolus doses are effective to maintain a plasma concentration of said fusion protein in the patient at more than 100 ng/mL for a period of at least 10 days after administration of the bolus dose.

13. The hGH-XTEN fusion protein for use according to claim 1, wherein the human patient has a clinically significant reduction in at least one parameter selected from serum cholesterol, serum triglycerides, and serum LDL after administration of the bolus doses.

## Patentansprüche

1. Menschliches Wachstumshormon-XTEN- (hGH-XTEN-) Fusionsprotein, das eine Aminosäuresequenz mit zumindest 90 % Sequenzidentität mit Seq.-ID Nr. 1 umfasst,
zur Verwendung in einem Verfahren zur Behandlung von menschlichem adultem Wachstumshormonmangel (AGHD) bei einem menschlichen Patienten,
wobei das Verfahren das Verabreichen von aufeinanderfolgenden therapeutisch wirksamen, an das Körpergewicht angepassten Bolusdosen von 0,05 mg/kg bis 3,0 mg/kg des hGH-XTEN-Fusionsproteins an den menschlichen Patienten mit AGHD umfasst,
wobei die Dosierungsdauer zwischen der anfänglichen Bolusdosis und einer darauffolgenden Verabreichung einer Bolusdosis 17, 16, 15, 14 oder 13 Tage beträgt,
wobei aufeinanderfolgende Bolusdosen des hGH-XTEN-Fusionsproteins:
(i) wirksam sind, um den Serum-IGF-I-Standardabweichungswert (SDS) des Patienten für zumindest 7 Tage nach Verabreichung der Bolusdosen zwischen -2,0 und +2,0 zu halten; oder
(ii) wirksam sind, um eine Plasmakonzentration des Fusionsproteins in dem Patienten für zumindest 10 Tage nach Verabreichung der Bolusdosen bei mehr als 10 ng/ml zu halten.

2. hGH-XTEN-Fusionsprotein zur Verwendung nach Anspruch 1, wobei die Bolusdosen des hGH-XTEN-Fusionsproteins zwischen 0,05 mg/kg und 0,8 mg/kg oder zwischen 0,8 mg/kg und 1,2 mg/kg oder zwischen 0,05 mg/kg und 3,0 mg/kg liegen.

3. hGH-XTEN-Fusionsprotein zur Verwendung nach Anspruch 1, wobei die Bolusdosen des hGH-XTEN-Fusionsproteins aus der aus 0,05 mg/kg, 0,1 mg/kg, 0,2 mg/kg, 0,4 mg/kg, 0,8 mg/kg, 1,0 mg/kg, 1,2 mg/kg, 1,4 mg/kg, 1,6 mg/kg, 1,8 mg/kg, 2,0 mg/kg, 2,2 mg/kg, 2,4 mg/kg, 2,6 mg/kg, 2,7 mg/kg, 2,8 mg/kg und 3,0 mg/kg bestehenden Gruppe ausgewählt sind.

4. hGH-XTEN-Fusionsprotein zur Verwendung nach Anspruch 1, wobei die anfängliche Dosis und die zumindest eine darauffolgende Dosis jeweils eine Menge (i) von 2,25 mg bis 6 mg; (ii) von 4,5 mg bis 12 mg; (iii) von 9 mg bis 24 mg; (iv) von 18 mg bis 48 mg; (v) von 36 mg bis 96 mg; (vi) von 45 mg bis 120 mg; (vii) von 54 mg bis 144 mg; (viii) von 63 mg bis 168 mg; (ix) von 72 mg bis 192 mg; (x) von 81 mg bis 216 mg; (xi) von 90 mg bis 240 mg; (xii) von 99 mg bis 264 mg; (xiii) von 108 mg bis 288 mg; (xiv) von 117 mg bis 312 mg; (xv) von 121,5 mg bis 324 mg; (xvi) von 126 mg bis 336 mg; oder (xvii) von 135 mg bis 360 mg aufweisen.

5. hGH-XTEN-Fusionsprotein zur Verwendung nach Anspruch 1 oder 2, wobei die Bolusdosen subkutan verabreicht werden.

6. hGH-XTEN-Fusionsprotein zur Verwendung nach Anspruch 1, wobei der menschliche Patient nach der Verabreichung einen Serum-IGF-I-Standardabweichungswert (SDS) zwischen -2,0 und 2,0 aufweist.

7. hGH-XTEN-Fusionsprotein zur Verwendung nach Anspruch 1, wobei der menschliche Patient einen IGF-I-SDS aufweist, der aus der aus höher als -2,0, höher als -1,5, höher als -1,0, höher als -0,5, höher als 0, höher als 0,5, höher als 1,0 und höher als 1,5 bestehenden Gruppe ausgewählt ist.

8. hGH-XTEN-Fusionsprotein zur Verwendung nach Anspruch 1, wobei eine Verabreichung der Bolusdosis bei dem menschlichen Patienten für zumindest 7 Tage, zumindest 10 Tage, zumindest 14 Tage, zumindest 16 Tage oder zumindest 21 Tage zu einer Normalisierung des IGF-I-SDS führt.

9. hGH-XTEN-Fusionsprotein zur Verwendung nach Anspruch 1, wobei die aufeinanderfolgenden Bolusdosen des hGH-XTEN-Fusionsproteins wirksam sind, um den IGF-I-SDS des Patienten bei Abwesenheit eines klinisch signifikanten Ausmaßes an Nebenwirkungen, die aus der aus Kopfschmerzen, Arthralgie, Myalgie, Ödem, Übelkeit und Muskelermüdung nach Verabreichung der Bolusdosis bestehenden Gruppe ausgewählt sind, um zumindest 0,5 oder zumindest 1,0 über den Basislinien-IGF-I-SDS des Individuums zu erhöhen.

10. hGH-XTEN-Fusionsprotein zur Verwendung nach Anspruch 1, wobei das hGH-XTEN-Fusionsprotein die Aminosäuresequenz von Seq.-ID Nr. 1 umfasst.

11. hGH-XTEN-Fusionsprotein zur Verwendung nach Anspruch 1(i) oder Anspruch 2, abhängig von Anspruch 1(i), wobei die Bolusdosen wirksam sind, um den Serum-IGF-I-SDS des Patienten für zumindest 20 Tage nach Verabreichung der Bolusdosen zwischen -2,0 und 2,0 zu halten.

12. hGH-XTEN-Fusionsprotein zur Verwendung nach Anspruch 1(ii) oder Anspruch 2, abhängig von Anspruch 1(ii), wobei die Bolusdosen wirksam sind, um eine Plasmakonzentration des Fusionsproteins in dem Patienten für eine Dauer von zumindest 10 Tagen nach Verabreichung der Bolusdosis bei mehr als 100 ng/ml zu halten.

13. hGH-XTEN-Fusionsprotein zur Verwendung nach Anspruch 1, wobei der menschliche Patient nach Verabreichung der Bolusdosen eine klinisch signifikante Verringerung zumindest eines Parameters aufweist, der aus Serumcholesterin, Serumtriglyceriden und Serum-LDL ausgewählt ist.

## Revendications

1. Protéine de fusion hormone de croissance humaine-XTEN (hGH-XTEN) comprenant une séquence d'acides aminés ayant au moins 90 % d'identité de séquence avec la SEQ ID NO : 1,
pour une utilisation dans une méthode de traitement d'un déficit en hormone de croissance humaine chez l'adulte (AGHD) chez un patient humain,
dans laquelle la méthode comprend l'administration au patient humain de doses en bolus ajustées au poids corporel thérapeutiquement efficaces successives d'AGHD comprises entre 0,05 mg/kg et 3,0 mg/kg de la protéine de fusion hGH-XTEN,
dans laquelle la période de dosage entre la dose en bolus initiale et une administration successive d'une dose en bolus est de 17, 16, 15, 14 ou 13 jours,
dans laquelle les doses en bolus successives de la protéine de fusion hGH-XTEN sont :
(i) efficaces pour maintenir le score en écart type (SDS) de l'IGF-I sérique du patient entre -2,0 et +2,0 pendant au moins 7 jours après l'administration des doses en bolus ; ou
(ii) efficaces pour maintenir une concentration plasmique de ladite protéine de fusion chez le patient à plus de 10 ng/ml pendant une période d'au moins 10 jours après l'administration des doses en bolus.

2. Protéine de fusion hGH-XTEN pour une utilisation selon la revendication 1, dans laquelle les doses en bolus de la protéine de fusion hGH-XTEN sont comprises entre 0,05 mg/kg et 0,8 mg/kg ou entre 0,8 mg/kg et 1,2 mg/kg ou entre 0,05 mg/kg et 3,0 mg/kg.

3. Protéine de fusion hGH-XTEN pour une utilisation selon la revendication 1, dans laquelle les doses en bolus de la protéine de fusion hGH-XTEN sont choisies dans le groupe constitué par 0,05 mg/kg, 0,1 mg/kg, 0,2 mg/kg, 0,4 mg/kg, 0,8 mg/kg, 1,0 mg/kg, 1,2 mg/kg, 1,4 mg/kg, 1,6 mg/kg, 1,8 mg/kg, 2,0 mg/kg, 2,2 mg/kg, 2,4 mg/kg, 2,6 mg/kg, 2,7 mg/kg, 2,8 mg/kg et 3,0 mg/kg.

4. Protéine de fusion hGH-XTEN pour une utilisation selon la revendication 1, dans laquelle la dose initiale et l'au moins une dose suivante sont chacune comprises en une quantité (i) entre 2,25 mg et 6 mg ; (ii) entre 4,5 mg et 12 mg ; (iii) entre 9 mg et 24 mg ; (iv) entre 18 mg et 48 mg ; (v) entre 36 mg et 96 mg ; (vi) entre 45 mg et 120 mg ; (vii) entre 54 mg et 144 mg ; (viii) entre 63 mg et 168 mg ; (ix) entre 72 mg et 192 mg ; (x) entre 81 mg et 216 mg ; (xi) entre 90 mg et 240 mg ; (xii) entre 99 mg et 264 mg ; (xiii) entre 108 mg et 288 mg ; (xiv) entre 117 mg et 312 mg ; (xv) entre 121,5 mg et 324 mg ; (xvi) entre 126 mg et 336 mg ; ou (xvii) entre 135 mg et 360 mg.

5. Protéine de fusion hGH-XTEN pour une utilisation selon la revendication 1 ou la revendication 2, dans laquelle les doses en bolus sont administrées en sous-cutanée.

6. Protéine de fusion hGH-XTEN pour une utilisation selon la revendication 1, dans laquelle le patient humain a un score en écart type (SDS) d' IGF-I sérique entre -2,0 et 2,0 après l'administration.

7. Protéine de fusion hGH-XTEN pour une utilisation selon la revendication 1, dans laquelle le patient humain a un SDS d'IGF-I choisi dans le groupe constitué par plus de -2,0, plus de -1,5, plus de -1,0, plus de -0,5, plus de 0, plus de 0,5, plus de 1,0 et plus de 1,5.

8. Protéine de fusion hGH-XTEN pour une utilisation selon la revendication 1, dans laquelle l'administration de la dose en bolus entraîne la normalisation du SDS d'IGF-I chez le patient humain pendant au moins 7 jours, au moins 10 jours, au moins 14 jours, au moins 16 jours ou au moins 21 jours.

9. Protéine de fusion hGH-XTEN pour une utilisation selon la revendication 1, dans laquelle les doses en bolus successives de la protéine de fusion hGH-XTEN sont efficaces pour augmenter le SED d'IGF-I du patient par au moins 0,5 ou au moins 1,0 au-dessus du SDS d'IGF-I de base du sujet en l'absence d'un niveau cliniquement significatif d'effets secondaires choisis dans le groupe constitué par les céphalées, l'arthralgie, la myalgie, les oedèmes, les nausées et la fatigue musculaire après l'administration de la dose en bolus.

10. Protéine de fusion hGH-XTEN pour une utilisation selon la revendication 1, dans laquelle la protéine de fusion hGH-XTEN comprend la séquence d'acides aminés de SEQ ID NO : 1.

11. Protéine de fusion hGH-XTEN pour une utilisation selon la revendication 1(i) ou la revendication 2 dépendant de la revendication 1(i), dans laquelle lesdites doses en bolus sont efficaces pour maintenir le SDS d'IGF-I sérique du patient entre -2,0 et 2,0 pendant au moins 20 jours après l'administration des doses en bolus.

12. Protéine de fusion hGH-XTEN pour une utilisation selon la revendication 1(i) ou la revendication 2 dépendant de la revendication 1(i), dans laquelle lesdites doses en bolus sont efficaces pour maintenir une concentration plasmique de ladite protéine de fusion chez le patient à plus de 100 ng/ml pendant une période d'au moins 10 jours après l'administration de la dose en bolus.

13. Protéine de fusion hGH-XTEN pour une utilisation selon la revendication 1, dans laquelle le patient humain présente une réduction cliniquement significative d'au moins un paramètre choisi parmi le cholestérol sérique, les triglycérides sériques et le LDL sérique après l'administration des doses en bolus.


### SEQUENCE LISTING

<110> AMUNIX OPERATING INC.
<120> TREATMENT WITH HUMAN GROWTH HORMONE ANALOGUES
<130> 32808-738.601
<140>
   <141>
<150> 61/763,753
   <151> 2013-02-12
<150> 61/663,475
   <151> 2012-06-22
<150> 61/689,390
   <151> 2012-06-05
<160> 85
<170> PatentIn version 3.5
<210> 1
   <211> 1250
   <212> PRT
   <213> Artificial Sequence
<220>
   <221> source
   <223> /note="Description of Artificial Sequence: Synthetic polypeptide"
<400> 1
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
                   165                 170                 175
   Glu Ser Gly Pro Gly Thr Ser Thr Glu Pro Ser Glu Gly Ser Ala Pro
               180                 185                 190
   Gly Thr Ser Thr Glu Pro Ser Glu Gly Ser Ala Pro Gly Ser Pro Ala
           195                 200                 205
   Gly Ser Pro Thr Ser Thr Glu Glu Gly Thr Ser Thr Glu Pro Ser Glu
       210                 215                 220
   Gly Ser Ala Pro Gly Thr Ser Thr Glu Pro Ser Glu Gly Ser Ala Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Ser Glu Pro Ala Thr Ser Gly Ser Glu Thr Pro
           275                 280                 285
   Gly Thr Ser Thr Glu Pro Ser Glu Gly Ser Ala Pro Gly Thr Ser Thr
       290                 295                 300
   Glu Pro Ser Glu Gly Ser Ala Pro Gly Thr Ser Glu Ser Ala Thr Pro
   305                 310                 315                 320
   Glu Ser Gly Pro Gly Thr Ser Glu Ser Ala Thr Pro Glu Ser Gly Pro
                   325                 330                 335
   Gly Ser Pro Ala Gly Ser Pro Thr Ser Thr Glu Glu Gly Thr Ser Glu
               340                 345                 350
   Ser Ala Thr Pro Glu Ser Gly Pro Gly Ser Glu Pro Ala Thr Ser Gly
           355                 360                 365
   Ser Glu Thr Pro Gly Thr Ser Glu Ser Ala Thr Pro Glu Ser Gly Pro
       370                 375                 380
   Gly Thr Ser Thr Glu Pro Ser Glu Gly Ser Ala Pro Gly Thr Ser Thr
   385                 390                 395                 400
   Glu Pro Ser Glu Gly Ser Ala Pro Gly Thr Ser Thr Glu Pro Ser Glu
                   405                 410                 415
   Gly Ser Ala Pro Gly Thr Ser Thr Glu Pro Ser Glu Gly Ser Ala Pro
               420                 425                 430
   Gly Thr Ser Thr Glu Pro Ser Glu Gly Ser Ala Pro Gly Thr Ser Thr
           435                 440                 445
   Glu Pro Ser Glu Gly Ser Ala Pro Gly Ser Pro Ala Gly Ser Pro Thr
       450                 455                 460
   Ser Thr Glu Glu Gly Thr Ser Thr Glu Pro Ser Glu Gly Ser Ala Pro
   465                 470                 475                 480
   Gly Thr Ser Glu Ser Ala Thr Pro Glu Ser Gly Pro Gly Ser Glu Pro
                   485                 490                 495
   Ala Thr Ser Gly Ser Glu Thr Pro Gly Thr Ser Glu Ser Ala Thr Pro
               500                 505                 510
   Glu Ser Gly Pro Gly Ser Glu Pro Ala Thr Ser Gly Ser Glu Thr Pro
           515                 520                 525
   Gly Thr Ser Glu Ser Ala Thr Pro Glu Ser Gly Pro Gly Thr Ser Thr
       530                 535                 540
   Glu Pro Ser Glu Gly Ser Ala Pro Gly Thr Ser Glu Ser Ala Thr Pro
   545                 550                 555                 560
   Glu Ser Gly Pro Gly Ser Pro Ala Gly Ser Pro Thr Ser Thr Glu Glu
                   565                 570                 575
   Gly Ser Pro Ala Gly Ser Pro Thr Ser Thr Glu Glu Gly Ser Pro Ala
               580                 585                 590
   Gly Ser Pro Thr Ser Thr Glu Glu Gly Thr Ser Glu Ser Ala Thr Pro
           595                 600                 605
   Glu Ser Gly Pro Gly Thr Ser Thr Glu Pro Ser Glu Gly Ser Ala Pro
       610                 615                 620
   Gly Thr Ser Glu Ser Ala Thr Pro Glu Ser Gly Pro Gly Ser Glu Pro
   625                 630                 635                 640
   Ala Thr Ser Gly Ser Glu Thr Pro Gly Thr Ser Glu Ser Ala Thr Pro
                   645                 650                 655
   Glu Ser Gly Pro Gly Ser Glu Pro Ala Thr Ser Gly Ser Glu Thr Pro
               660                 665                 670
   Gly Thr Ser Glu Ser Ala Thr Pro Glu Ser Gly Pro Gly Thr Ser Thr
           675                 680                 685
   Glu Pro Ser Glu Gly Ser Ala Pro Gly Ser Pro Ala Gly Ser Pro Thr
       690                 695                 700
   Ser Thr Glu Glu Gly Thr Ser Glu Ser Ala Thr Pro Glu Ser Gly Pro
   705                 710                 715                 720
   Gly Ser Glu Pro Ala Thr Ser Gly Ser Glu Thr Pro Gly Thr Ser Glu
                   725                 730                 735
   Ser Ala Thr Pro Glu Ser Gly Pro Gly Ser Pro Ala Gly Ser Pro Thr
               740                 745                 750
   Ser Thr Glu Glu Gly Ser Pro Ala Gly Ser Pro Thr Ser Thr Glu Glu
           755                 760                 765
   Gly Thr Ser Thr Glu Pro Ser Glu Gly Ser Ala Pro Gly Thr Ser Glu
       770                 775                 780
   Ser Ala Thr Pro Glu Ser Gly Pro Gly Thr Ser Glu Ser Ala Thr Pro
   785                 790                 795                 800
   Glu Ser Gly Pro Gly Thr Ser Glu Ser Ala Thr Pro Glu Ser Gly Pro
                   805                 810                 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865 870 875 880
   Ala Thr Ser Gly Ser Glu Thr Pro Gly Thr Ser Glu Ser Ala Thr Pro
   885 890 895
   Glu Ser Gly Pro Gly Thr Ser Thr Glu Pro Ser Glu Gly Ser Ala Pro
   900 905 910
   Gly Phe Pro Thr Ile Pro Leu Ser Arg Leu Phe Asp Asn Ala Met Leu
   915 920 925
   Arg Ala His Arg Leu His Gln Leu Ala Phe Asp Thr Tyr Gln Glu Phe
   930 935 940
   Glu Glu Ala Tyr Ile Pro Lys Glu Gln Lys Tyr Ser Phe Leu Gln Asn
   945 950 955 960
   Pro Gln Thr Ser Leu Cys Phe Ser Glu Ser Ile Pro Thr Pro Ser Asn
   965 970 975
   Arg Glu Glu Thr Gln Gln Lys Ser Asn Leu Glu Leu Leu Arg Ile Ser
   980 985 990
   Leu Leu Leu Ile Gln Ser Trp Leu Glu Pro Val Gln Phe Leu Arg Ser
   995 1000 1005
   Val Phe Ala Asn Ser Leu Val Tyr Gly Ala Ser Asp Ser Asn Val
   1010 1015 1020
   Tyr Asp Leu Leu Lys Asp Leu Glu Glu Gly Ile Gln Thr Leu Met
   1025 1030 1035
   Gly Arg Leu Glu Asp Gly Ser Pro Arg Thr Gly Gln Ile Phe Lys
   1040 1045 1050
   Gln Thr Tyr Ser Lys Phe Asp Thr Asn Ser His Asn Asp Asp Ala
   1055 1060 1065
   Leu Leu Lys Asn Tyr Gly Leu Leu Tyr Cys Phe Arg Lys Asp Met
   1070 1075 1080
   Asp Lys Val Glu Thr Phe Leu Arg Ile Val Gln Cys Arg Ser Val
   1085 1090 1095
   Glu Gly Ser Cys Gly Phe Gly Gly Thr Ser Glu Ser Ala Thr Pro
   1100 1105 1110
   Glu Ser  Gly Pro Gly Thr Ser  Thr Glu Pro Ser Glu  Gly Ser Ala
       1115                 1120                 1125
   Pro Gly  Thr Ser Thr Glu Pro  Ser Glu Gly Ser Ala  Pro Gly Thr
       1130                 1135                 1140
   Ser Glu  Ser Ala Thr Pro Glu  Ser Gly Pro Gly Thr  Ser Thr Glu
       1145                 1150                 1155
   Pro Ser  Glu Gly Ser Ala Pro  Gly Thr Ser Thr Glu  Pro Ser Glu
       1160                 1165                 1170
   Gly Ser  Ala Pro Gly Thr Ser  Glu Ser Ala Thr Pro  Glu Ser Gly
       1175                 1180                 1185
   Pro Gly  Thr Ser Thr Glu Pro  Ser Glu Gly Ser Ala  Pro Gly Thr
       1190                 1195                 1200
   Ser Thr  Glu Pro Ser Glu Gly  Ser Ala Pro Gly Thr  Ser Thr Glu
       1205                 1210                 1215
   Pro Ser  Glu Gly Ser Ala Pro  Gly Ser Pro Ala Gly  Ser Pro Thr
       1220                 1225                 1230
   Ser Thr  Glu Glu Gly Thr Ser  Thr Glu Pro Ser Glu  Gly Ser Ala
       1235                 1240                 1245
   Pro Gly
       1250
<210> 2
   <211> 191
   <212> PRT
   <213> Homo sapiens
<400> 2
<210> 3
   <211> 1071
   <212> PRT
   <213> Artificial Sequence
<220>
   <221> source
   <223> /note="Description of Artificial Sequence: Synthetic polypeptide"
<400> 3
   Gly Gly Ser Pro Gly Thr Ser Thr Glu Pro Ser Glu Gly Ser Ala Pro
   1               5                   10                  15
   Gly Ser Glu Pro Ala Thr Ser Gly Ser Glu Thr Pro Gly Ser Pro Ala
               20                  25                  30
   Gly Ser Pro Thr Ser Thr Glu Glu Gly Ser Thr Ser Ser Thr Ala Glu
           35                  40                  45
   Ser Pro Gly Pro Gly Thr Ser Thr Pro Glu Ser Gly Ser Ala Ser Pro
       50                  55                  60
   Gly Ser Thr Ser Glu Ser Pro Ser Gly Thr Ala Pro Gly Ser Thr Ser
   65                  70                  75                  80
   Glu Ser Pro Ser Gly Thr Ala Pro Gly Thr Ser Thr Pro Glu Ser Gly
                   85                  90                  95
   Ser Ala Ser Pro Gly Thr Ser Thr Pro Glu Ser Gly Ser Ala Ser Pro
               100                 105                 110
   Gly Ser Glu Pro Ala Thr Ser Gly Ser Glu Thr Pro Gly Thr Ser Glu
           115                 120                 125
   Ser Ala Thr Pro Glu Ser Gly Pro Gly Ser Pro Ala Gly Ser Pro Thr
       130                 135                 140
   Ser Thr Glu Glu Gly Thr Ser Thr Glu Pro Ser Glu Gly Ser Ala Pro
   145                 150                 155                 160
   Gly Thr Ser Glu Ser Ala Thr Pro Glu Ser Gly Pro Gly Thr Ser Thr
                   165                 170                 175
   Glu Pro Ser Glu Gly Ser Ala Pro Gly Thr Ser Thr Glu Pro Ser Glu
               180                 185                 190
   Gly Ser Ala Pro Gly Ser Pro Ala Gly Ser Pro Thr Ser Thr Glu Glu
           195                 200                 205
   Gly Thr Ser Thr Glu Pro Ser Glu Gly Ser Ala Pro Gly Thr Ser Thr
       210                 215                 220
   Glu Pro Ser Glu Gly Ser Ala Pro Gly Thr Ser Glu Ser Ala Thr Pro
   225                 230                 235                 240
   Glu Ser Gly Pro Gly Thr Ser Glu Ser Ala Thr Pro Glu Ser Gly Pro
                   245                 250                 255
   Gly Thr Ser Thr Glu Pro Ser Glu Gly Ser Ala Pro Gly Thr Ser Thr
               260                 265                 270
   Glu Pro Ser Glu Gly Ser Ala Pro Gly Thr Ser Glu Ser Ala Thr Pro
           275                 280                 285
   Glu Ser Gly Pro Gly Thr Ser Thr Glu Pro Ser Glu Gly Ser Ala Pro
       290                 295                 300
   Gly Ser Glu Pro Ala Thr Ser Gly Ser Glu Thr Pro Gly Ser Pro Ala
   305                 310                 315                 320
   Gly Ser Pro Thr Ser Thr Glu Glu Gly Ser Ser Thr Pro Ser Gly Ala
                   325                 330                 335
   Thr Gly Ser Pro Gly Thr Pro Gly Ser Gly Thr Ala Ser Ser Ser Pro
               340                 345                 350
   Gly Ser Ser Thr Pro Ser Gly Ala Thr Gly Ser Pro Gly Thr Ser Thr
           355                 360                 365
   Glu Pro Ser Glu Gly Ser Ala Pro Gly Thr Ser Thr Glu Pro Ser Glu
       370                 375                 380
   Gly Ser Ala Pro Gly Ser Glu Pro Ala Thr Ser Gly Ser Glu Thr Pro
   385                 390                 395                 400
   Gly Ser Pro Ala Gly Ser Pro Thr Ser Thr Glu Glu Gly Ser Pro Ala
                   405                 410                 415
   Gly Ser Pro Thr Ser Thr Glu Glu Gly Thr Ser Thr Glu Pro Ser Glu
               420                 425                 430
   Gly Ser Ala Pro Gly Ala Ser Ala Ser Gly Ala Pro Ser Thr Gly Gly
           435                 440                 445
   Thr Ser Glu Ser Ala Thr Pro Glu Ser Gly Pro Gly Ser Pro Ala Gly
       450                 455                 460
   Ser Pro Thr Ser Thr Glu Glu Gly Ser Pro Ala Gly Ser Pro Thr Ser
   465                 470                 475                 480
   Thr Glu Glu Gly Ser Thr Ser Ser Thr Ala Glu Ser Pro Gly Pro Gly
                   485                 490                 495
   Ser Thr Ser Glu Ser Pro Ser Gly Thr Ala Pro Gly Thr Ser Pro Ser
               500                 505                 510
   Gly Glu Ser Ser Thr Ala Pro Gly Thr Pro Gly Ser Gly Thr Ala Ser
           515                 520                 525
   Ser Ser Pro Gly Ser Ser Thr Pro Ser Gly Ala Thr Gly Ser Pro Gly
       530                 535                 540
   Ser Ser Pro Ser Ala Ser Thr Gly Thr Gly Pro Gly Ser Glu Pro Ala
   545                 550                 555                 560
   Thr Ser Gly Ser Glu Thr Pro Gly Thr Ser Glu Ser Ala Thr Pro Glu
                   565                 570                 575
   Ser Gly Pro Gly Ser Glu Pro Ala Thr Ser Gly Ser Glu Thr Pro Gly
               580                 585                 590
   Ser Thr Ser Ser Thr Ala Glu Ser Pro Gly Pro Gly Ser Thr Ser Ser
           595                 600                 605
   Thr Ala Glu Ser Pro Gly Pro Gly Thr Ser Pro Ser Gly Glu Ser Ser
       610                 615                 620
   Thr Ala Pro Gly Ser Glu Pro Ala Thr Ser Gly Ser Glu Thr Pro Gly
   625                 630                 635                 640
   Ser Glu Pro Ala Thr Ser Gly Ser Glu Thr Pro Gly Thr Ser Thr Glu
                   645                 650                 655
   Pro Ser Glu Gly Ser Ala Pro Gly Ser Thr Ser Ser Thr Ala Glu Ser
               660                 665                 670
   Pro Gly Pro Gly Thr Ser Thr Pro Glu Ser Gly Ser Ala Ser Pro Gly
           675                 680                 685
   Ser Thr Ser Glu Ser Pro Ser Gly Thr Ala Pro Gly Thr Ser Thr Glu
       690                 695                 700
   Pro Ser Glu Gly Ser Ala Pro Gly Thr Ser Thr Glu Pro Ser Glu Gly
   705                 710                 715                 720
   Ser Ala Pro Gly Thr Ser Thr Glu Pro Ser Glu Gly Ser Ala Pro Gly
                   725                 730                 735
   Ser Ser Thr Pro Ser Gly Ala Thr Gly Ser Pro Gly Ser Ser Pro Ser
               740                 745                 750
   Ala Ser Thr Gly Thr Gly Pro Gly Ala Ser Pro Gly Thr Ser Ser Thr
           755                 760                 765
   Gly Ser Pro Gly Ser Glu Pro Ala Thr Ser Gly Ser Glu Thr Pro Gly
       770                 775                 780
   Thr Ser Glu Ser Ala Thr Pro Glu Ser Gly Pro Gly Ser Pro Ala Gly
   785                 790                 795                 800
   Ser Pro Thr Ser Thr Glu Glu Gly Ser Ser Thr Pro Ser Gly Ala Thr
                   805                 810                 815
   Gly Ser Pro Gly Ser Ser Pro Ser Ala Ser Thr Gly Thr Gly Pro Gly
               820                 825                 830
   Ala Ser Pro Gly Thr Ser Ser Thr Gly Ser Pro Gly Thr Ser Glu Ser
           835                 840                 845
   Ala Thr Pro Glu Ser Gly Pro Gly Thr Ser Thr Glu Pro Ser Glu Gly
       850                 855                 860
   Ser Ala Pro Gly Thr Ser Thr Glu Pro Ser Glu Gly Ser Ala Pro Gly
   865                 870                 875                 880
   Phe Pro Thr Ile Pro Leu Ser Arg Leu Phe Asp Asn Ala Met Leu Arg
                   885                 890                 895
   Ala His Arg Leu His Gln Leu Ala Phe Asp Thr Tyr Gln Glu Phe Glu
               900                 905                 910
   Glu Ala Tyr Ile Pro Lys Glu Gln Lys Tyr Ser Phe Leu Gln Asn Pro
           915                 920                 925
   Gln Thr Ser Leu Cys Phe Ser Glu Ser Ile Pro Thr Pro Ser Asn Arg
       930                 935                 940
   Glu Glu Thr Gln Gln Lys Ser Asn Leu Glu Leu Leu Arg Ile Ser Leu
   945                 950                 955                 960
   Leu Leu Ile Gln Ser Trp Leu Glu Pro Val Gln Phe Leu Arg Ser Val
                   965                 970                 975
   Phe Ala Asn Ser Leu Val Tyr Gly Ala Ser Asp Ser Asn Val Tyr Asp
               980                 985                 990
   Leu Leu Lys Asp Leu Glu Glu Gly  Ile Gln Thr Leu Met  Gly Arg Leu
           995                 1000                1005
   Glu Asp  Gly Ser Pro Arg Thr  Gly Gln Ile Phe Lys  Gln Thr Tyr
       1010                1015                1020
   Ser Lys  Phe Asp Thr Asn Ser  His Asn Asp Asp Ala  Leu Leu Lys
       1025                1030                1035
   Asn Tyr  Gly Leu Leu Tyr Cys  Phe Arg Lys Asp Met  Asp Lys Val
       1040                1045                1050
   Glu Thr  Phe Leu Arg Ile Val  Gln Cys Arg Ser Val  Glu Gly Ser
       1055                1060                1065
   Cys Gly  Phe
       1070
<210> 4
   <211> 3213
   <212> DNA
   <213> Artificial Sequence
<220>
   <221> source
   <223> /note="Description of Artificial Sequence: Synthetic polynucleotide"
<400> 4
   ggtgggtctc caggtacttc tactgaaccg tctgaaggca gcgcaccagg tagcgaaccg      60
   gctacttccg gttctgaaac cccaggtagc ccagcaggtt ctccaacttc tactgaagaa     120
   ggttctacca gctctaccgc agaatctcct ggtccaggta cctctactcc ggaaagcggc     180
   tctgcatctc caggttctac tagcgaatct ccttctggca ctgcaccagg ttctactagc     240
   gaatccccgt ctggtactgc tccaggtact tctactcctg aaagcggttc cgcttctcca     300
   ggtacctcta ctccggaaag cggttctgca tctccaggta gcgaaccggc aacctccggc     360
   tctgaaaccc caggtacctc tgaaagcgct actcctgaat ccggcccagg tagcccggca     420
   ggttctccga cttccactga ggaaggtacc tctactgaac cttctgaggg cagcgctcca     480
   ggtacttctg aaagcgctac cccggagtcc ggtccaggta cttctactga accgtccgaa     540
   ggtagcgcac caggtacttc taccgaaccg tccgagggta gcgcaccagg tagcccagca     600
   ggttctccta cctccaccga ggaaggtact tctaccgaac cgtccgaggg tagcgcacca     660
   ggtacttcta ccgaaccttc cgagggcagc gcaccaggta cttctgaaag cgctacccct     720
   gagtccggcc caggtacttc tgaaagcgct actcctgaat ccggtccagg tacctctact     780
   gaaccttccg aaggcagcgc tccaggtacc tctaccgaac cgtccgaggg cagcgcacca     840
   ggtacttctg aaagcgcaac ccctgaatcc ggtccaggta cttctactga accttccgaa     900
   ggtagcgctc caggtagcga acctgctact tctggttctg aaaccccagg tagcccggct     960
   ggctctccga cctccaccga ggaaggtagc tctaccccgt ctggtgctac tggttctcca    1020
   ggtactccgg gcagcggtac tgcttcttcc tctccaggta gctctacccc ttctggtgct    1080
   actggctctc caggtacctc taccgaaccg tccgagggta gcgcaccagg tacctctact    1140
   gaaccgtctg agggtagcgc tccaggtagc gaaccggcaa cctccggttc tgaaactcca    1200
   ggtagccctg ctggctctcc gacttctact gaggaaggta gcccggctgg ttctccgact    1260
   tctactgagg aaggtacttc taccgaacct tccgaaggta gcgctccagg tgcaagcgca    1320
   agcggcgcgc caagcacggg aggtacttct gaaagcgcta ctcctgagtc cggcccaggt    1380
   agcccggctg gctctccgac ttccaccgag gaaggtagcc cggctggctc tccaacttct    1440
   actgaagaag gttctaccag ctctaccgct gaatctcctg gcccaggttc tactagcgaa    1500
   tctccgtctg gcaccgcacc aggtacttcc cctagcggtg aatcttctac tgcaccaggt    1560
   acccctggca gcggtaccgc ttcttcctct ccaggtagct ctaccccgtc tggtgctact    1620
   ggctctccag gttctagccc gtctgcatct accggtaccg gcccaggtag cgaaccggca 1680
   acctccggct ctgaaactcc aggtacttct gaaagcgcta ctccggaatc cggcccaggt 1740
   agcgaaccgg ctacttccgg ctctgaaacc ccaggttcca ccagctctac tgcagaatct 1800
   ccgggcccag gttctactag ctctactgca gaatctccgg gtccaggtac ttctcctagc 1860
   ggcgaatctt ctaccgctcc aggtagcgaa ccggcaacct ctggctctga aactccaggt 1920
   agcgaacctg caacctccgg ctctgaaacc ccaggtactt ctactgaacc ttctgagggc 1980
   agcgcaccag gttctaccag ctctaccgca gaatctcctg gtccaggtac ctctactccg 2040
   gaaagcggct ctgcatctcc aggttctact agcgaatctc cttctggcac tgcaccaggt 2100
   acttctaccg aaccgtccga aggcagcgct ccaggtacct ctactgaacc ttccgagggc 2160
   agcgctccag gtacctctac cgaaccttct gaaggtagcg caccaggtag ctctactccg 2220
   tctggtgcaa ccggctcccc aggttctagc ccgtctgctt ccactggtac tggcccaggt 2280
   gcttccccgg gcaccagctc tactggttct ccaggtagcg aacctgctac ctccggttct 2340
   gaaaccccag gtacctctga aagcgcaact ccggagtctg gtccaggtag ccctgcaggt 2400
   tctcctacct ccactgagga aggtagctct actccgtctg gtgcaaccgg ctccccaggt 2460
   tctagcccgt ctgcttccac tggtactggc ccaggtgctt ccccgggcac cagctctact 2520
   ggttctccag gtacctctga aagcgctact ccggagtctg gcccaggtac ctctactgaa 2580
   ccgtctgagg gtagcgctcc aggtacttct actgaaccgt ccgaaggtag cgcaccaggt 2640
   tttccgacta ttccgctgtc tcgtctgttt gataatgcta tgctgcgtgc gcaccgtctg 2700
   caccagctgg cctttgatac ttaccaggaa tttgaagaag cctacattcc taaagagcag 2760
   aagtactctt tcctgcaaaa cccacagact tctctctgct tcagcgaatc tattccgacg 2820
   ccttccaatc gcgaggaaac tcagcaaaag tccaatctgg aactactccg catttctctg 2880
   cttctgattc agagctggct agaaccagtg caatttctgc gttccgtctt cgccaatagc 2940
   ctagtttatg gcgcatccga cagcaacgta tacgatctcc tgaaagatct cgaggaaggc 3000
   attcagaccc tgatgggtcg tctcgaggat ggctctccgc gtactggtca gatcttcaag 3060
   cagacttact ctaaatttga tactaacagc cacaatgacg atgcgcttct aaaaaactat 3120
   ggtctgctgt attgttttcg taaagatatg gacaaagttg aaaccttcct gcgtattgtt 3180
   cagtgtcgtt ccgttgaggg cagctgtggt ttc 3213
<210> 5
   <211> 768
   <212> PRT
   <213> Artificial Sequence
<220>
   <221> source
   <223> /note="Description of Artificial Sequence: Synthetic polypeptide"
<400> 5
   Gly Glu Gly Ser Gly Glu Gly Ser Glu Gly Glu Gly Ser Glu Gly Ser
   1               5                   10                  15
   Gly Glu Gly Glu Gly Ser Glu Gly Ser Gly Glu Gly Glu Gly Gly Ser
               20                  25                  30
   Glu Gly Ser Glu Gly Glu Gly Ser Glu Gly Ser Gly Glu Gly Glu Gly
           35                  40                  45
   Gly Glu Gly Ser Gly Glu Gly Glu Gly Ser Gly Glu Gly Ser Glu Gly
       50                  55                  60
   Glu Gly Gly Gly Glu Gly Ser Glu Gly Glu Gly Ser Gly Glu Gly Gly
   65                  70                  75                  80
   Glu Gly Glu Gly Ser Glu Gly Gly Ser Glu Gly Glu Gly Gly Ser Glu
                   85                  90                  95
   Gly Gly Glu Gly Glu Gly Ser Glu Gly Ser Gly Glu Gly Glu Gly Ser
               100                 105                 110
   Glu Gly Gly Ser Glu Gly Glu Gly Ser Glu Gly Gly Ser Glu Gly Glu
           115                 120                 125
   Gly Ser Glu Gly Ser Gly Glu Gly Glu Gly Ser Glu Gly Ser Gly Glu
       130                 135                 140
   Gly Glu Gly Ser Glu Gly Ser Gly Glu Gly Glu Gly Ser Glu Gly Ser
   145                 150                 155                 160
   Gly Glu Gly Glu Gly Ser Glu Gly Gly Ser Glu Gly Glu Gly Gly Ser
                   165                 170                 175
   Glu Gly Ser Glu Gly Glu Gly Ser Gly Glu Gly Ser Glu Gly Glu Gly
               180                 185                 190
   Gly Ser Glu Gly Ser Glu Gly Glu Gly Gly Gly Glu Gly Ser Glu Gly
           195                 200                 205
   Glu Gly Ser Gly Glu Gly Ser Glu Gly Glu Gly Gly Ser Glu Gly Ser
       210                 215                 220
   Glu Gly Glu Gly Gly Ser Glu Gly Ser Glu Gly Glu Gly Gly Glu Gly
   225                 230                 235                 240
   Ser Gly Glu Gly Glu Gly Ser Glu Gly Ser Gly Glu Gly Glu Gly Ser
                   245                 250                 255
   Gly Glu Gly Ser Glu Gly Glu Gly Ser Glu Gly Ser Gly Glu Gly Glu
               260                 265                 270
   Gly Ser Glu Gly Ser Gly Glu Gly Glu Gly Gly Ser Glu Gly Ser Glu
           275                 280                 285
   Gly Glu Gly Ser Gly Glu Gly Ser Glu Gly Glu Gly Ser Glu Gly Ser
       290                 295                 300
   Gly Glu Gly Glu Gly Ser Glu Gly Ser Gly Glu Gly Glu Gly Gly Ser
   305                 310                 315                 320
   Glu Gly Ser Glu Gly Glu Gly Gly Ser Glu Gly Ser Glu Gly Glu Gly
                   325                 330                 335
   Gly Ser Glu Gly Ser Glu Gly Glu Gly Gly Glu Gly Ser Gly Glu Gly
               340                 345                 350
   Glu Gly Ser Glu Gly Ser Gly Glu Gly Glu Gly Ser Gly Glu Gly Ser
           355                 360                 365
   Glu Gly Glu Gly Ser Glu Gly Ser Gly Glu Gly Glu Gly Ser Glu Gly
       370                 375                 380
   Ser Gly Glu Gly Glu Gly Gly Ser Glu Gly Ser Glu Gly Glu Gly Ser
   385                 390                 395                 400
   Glu Gly Ser Gly Glu Gly Glu Gly Gly Glu Gly Ser Gly Glu Gly Glu
                   405                 410                 415
   Gly Ser Gly Glu Gly Ser Glu Gly Glu Gly Gly Gly Glu Gly Ser Glu
               420                 425                 430
   Gly Glu Gly Ser Glu Gly Ser Gly Glu Gly Glu Gly Ser Glu Gly Ser
           435                 440                 445
   Gly Glu Gly Glu Gly Ser Glu Gly Gly Ser Glu Gly Glu Gly Gly Ser
       450                 455                 460
   Glu Gly Ser Glu Gly Glu Gly Ser Glu Gly Gly Ser Glu Gly Glu Gly
   465                 470                 475                 480
   Ser Glu Gly Gly Ser Glu Gly Glu Gly Ser Glu Gly Ser Gly Glu Gly
                   485                 490                 495
   Glu Gly Ser Glu Gly Ser Gly Glu Gly Glu Gly Ser Gly Glu Gly Ser
               500                 505                 510
   Glu Gly Glu Gly Gly Ser Glu Gly Gly Glu Gly Glu Gly Ser Glu Gly
           515                 520                 525
   Gly Ser Glu Gly Glu Gly Ser Glu Gly Gly Ser Glu Gly Glu Gly Gly
       530                 535                 540
   Glu Gly Ser Gly Glu Gly Glu Gly Gly Gly Glu Gly Ser Glu Gly Glu
   545                 550                 555                 560
   Gly Ser Glu Gly Ser Gly Glu Gly Glu Gly Ser Gly Glu Gly Ser Glu
                   565                 570                 575
   Gly Phe Pro Thr Ile Pro Leu Ser Arg Leu Phe Asp Asn Ala Met Leu
               580                 585                 590
   Arg Ala His Arg Leu His Gln Leu Ala Phe Asp Thr Tyr Gln Glu Phe
           595                 600                 605
   Glu Glu Ala Tyr Ile Pro Lys Glu Gln Lys Tyr Ser Phe Leu Gln Asn
       610                 615                 620
   Pro Gln Thr Ser Leu Cys Phe Ser Glu Ser Ile Pro Thr Pro Ser Asn
   625                 630                 635                 640
   Arg Glu Glu Thr Gln Gln Lys Ser Asn Leu Glu Leu Leu Arg Ile Ser
                   645                 650                 655
   Leu Leu Leu Ile Gln Ser Trp Leu Glu Pro Val Gln Phe Leu Arg Ser
               660                 665                 670
   Val Phe Ala Asn Ser Leu Val Tyr Gly Ala Ser Asp Ser Asn Val Tyr
           675                 680                 685
   Asp Leu Leu Lys Asp Leu Glu Glu Gly Ile Gln Thr Leu Met Gly Arg
       690                 695                 700
   Leu Glu Asp Gly Ser Pro Arg Thr Gly Gln Ile Phe Lys Gln Thr Tyr
   705                 710                 715                 720
   Ser Lys Phe Asp Thr Asn Ser His Asn Asp Asp Ala Leu Leu Lys Asn
                   725                 730                 735
   Tyr Gly Leu Leu Tyr Cys Phe Arg Lys Asp Met Asp Lys Val Glu Thr
   740 745 750
   Phe Leu Arg Ile Val Gln Cys Arg Ser Val Glu Gly Ser Cys Gly Phe
   755 760 765
<210> 6
   <211> 2304
   <212> DNA
   <213> Artificial Sequence
<220>
   <221> source
   <223> /note="Description of Artificial Sequence: Synthetic polynucleotide"
<400> 6
   ggtgagggtt ctggcgaagg ttccgaaggt gagggctccg aaggatctgg cgaaggtgag 60
   ggttccgaag gttctggcga aggtgaaggc ggttctgagg gatccgaagg tgaaggctcc 120
   gaaggatctg gcgaaggtga aggtggtgaa ggttctggcg aaggtgaggg atctggcgaa 180
   ggctctgaag gtgaaggtgg tggtgaaggc tctgaaggtg aaggatctgg tgaaggtggc 240
   gaaggtgagg gatctgaagg cggctccgaa ggtgaaggcg gatctgaagg cggcgaaggt 300
   gaaggttccg aaggttctgg tgaaggtgaa ggatctgaag gtggctccga aggtgaagga 360
   tctgaaggcg gttccgaagg tgagggctct gaaggttctg gcgaaggtga aggctctgaa 420
   ggatctggtg aaggtgaagg ttccgaaggt tctggtgaag gtgaaggttc cgaaggttct 480
   ggcgaaggtg aaggttctga aggtggctct gaaggtgaag gcggctctga aggatccgaa 540
   ggtgaaggtt ctggtgaagg ctctgaaggt gaaggcggct ctgagggttc cgaaggtgaa 600
   ggcggaggcg aaggttctga aggtgaggga tctggtgaag gttctgaagg tgaaggcggt 660
   tctgaaggtt ccgaaggtga aggtggctct gagggatccg aaggtgaagg tggcgaagga 720
   tctggtgaag gtgaaggttc tgaaggttct ggcgaaggtg agggttctgg cgaaggttcc 780
   gaaggtgagg gctccgaagg atctggcgaa ggtgagggtt ccgaaggttc tggcgaaggt 840
   gaaggcggtt ctgagggatc cgaaggtgag ggttctggcg aaggttccga aggtgagggc 900
   tccgaaggat ctggcgaagg tgagggttcc gaaggttctg gcgaaggtga aggcggttct 960
   gagggatccg aaggtgaagg cggttctgaa ggttccgaag gtgaaggtgg ctctgaggga 1020
   tccgaaggtg aaggtggcga aggatctggt gaaggtgaag gttctgaagg ttctggcgaa 1080
   ggtgagggtt ctggcgaagg ttccgaaggt gagggctccg aaggatctgg cgaaggtgag 1140
   ggttccgaag gttctggcga aggtgaaggc ggttctgagg gatccgaagg tgaaggctcc 1200
   gaaggatctg gcgaaggtga aggtggtgaa ggttctggcg aaggtgaggg atctggcgaa 1260
   ggctctgaag gtgaaggtgg tggtgaaggc tctgaaggtg aaggttccga aggttctggt 1320
   gaaggtgaag gttccgaagg ttctggcgaa ggtgaaggtt ctgaaggtgg ctctgaaggt 1380
   gaaggcggct ctgaaggatc cgaaggtgaa ggatctgaag gtggctccga aggtgaagga    1440
   tctgaaggcg gttccgaagg tgagggctct gaaggttctg gcgaaggtga aggctctgaa    1500
   ggatctggtg aaggtgaagg atctggcgaa ggctccgaag gtgaaggcgg ttctgaaggt    1560
   ggcgaaggtg aaggatctga aggtggttcc gaaggtgagg gatctgaagg tggctctgaa    1620
   ggtgaaggtg gcgaaggttc tggcgaaggt gaaggtggag gcgaaggttc tgaaggtgaa    1680
   ggttccgaag gttctggtga aggtgaggga tctggcgaag gttctgaagg ttttccgact    1740
   attccgctgt ctcgtctgtt tgataacgct atgctgcgtg cgcaccgtct gcaccagctg    1800
   gcgttcgaca cttaccagga atttgaagaa gcgtacattc cgaaggaaca gaagtactct    1860
   ttcctgcaaa acccgcagac ctccctgtgc ttcagcgaat ctattccgac tccgtccaat    1920
   cgtgaagaaa ctcagcaaaa gtccaatctg gagctgctgc gcatctctct gctgctgatt    1980
   cagagctggc tggagcctgt tcagtttctg cgttccgtct tcgccaacag cctggtttat    2040
   ggtgcttccg acagcaacgt atacgatctg ctgaaagatc tggaagaagg cattcagacc    2100
   ctgatgggtc gtctggaaga tggttctccg cgtactggtc agatcttcaa acaaacttac    2160
   tccaaatttg atactaacag ccataacgac gatgctctgc tgaaaaacta tggtctgctg    2220
   tattgcttcc gcaaggatat ggacaaagtt gaaaccttcc tgcgtattgt gcagtgtcgt    2280
   tccgttgagg gcagctgtgg tttc                                           2304
<210> 7
   <211> 1104
   <212> PRT
   <213> Artificial Sequence
<220>
   <221> source
   <223> /note="Description of Artificial Sequence: Synthetic polypeptide"
<400> 7
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
   85 90 95
   Gly Thr Ser Thr Glu Pro Ser Glu Gly Ser Ala Pro Gly Thr Ser Thr
   100 105 110
   Glu Pro Ser Glu Gly Ser Ala Pro Gly Thr Ser Glu Ser Ala Thr Pro
   115 120 125
   Glu Ser Gly Pro Gly Ser Glu Pro Ala Thr Ser Gly Ser Glu Thr Pro
   130 135 140
   Gly Ser Glu Pro Ala Thr Ser Gly Ser Glu Thr Pro Gly Ser Pro Ala
   145 150 155 160
   Gly Ser Pro Thr Ser Thr Glu Glu Gly Thr Ser Glu Ser Ala Thr Pro
   165 170 175
   Glu Ser Gly Pro Gly Thr Ser Thr Glu Pro Ser Glu Gly Ser Ala Pro
   180 185 190
   Gly Thr Ser Thr Glu Pro Ser Glu Gly Ser Ala Pro Gly Ser Pro Ala
   195 200 205
   Gly Ser Pro Thr Ser Thr Glu Glu Gly Thr Ser Thr Glu Pro Ser Glu
   210 215 220
   Gly Ser Ala Pro Gly Thr Ser Thr Glu Pro Ser Glu Gly Ser Ala Pro
   225 230 235 240
   Gly Thr Ser Glu Ser Ala Thr Pro Glu Ser Gly Pro Gly Thr Ser Thr
   245 250 255
   Glu Pro Ser Glu Gly Ser Ala Pro Gly Thr Ser Glu Ser Ala Thr Pro
   260 265 270
   Glu Ser Gly Pro Gly Ser Glu Pro Ala Thr Ser Gly Ser Glu Thr Pro
   275 280 285
   Gly Thr Ser Thr Glu Pro Ser Glu Gly Ser Ala Pro Gly Thr Ser Thr
   290 295 300
   Glu Pro Ser Glu Gly Ser Ala Pro Gly Thr Ser Glu Ser Ala Thr Pro
   305 310 315 320
   Glu Ser Gly Pro Gly Thr Ser Glu Ser Ala Thr Pro Glu Ser Gly Pro
   325 330 335
   Gly Ser Pro Ala Gly Ser Pro Thr Ser Thr Glu Glu Gly Thr Ser Glu
   340 345 350
   Ser Ala Thr Pro Glu Ser Gly Pro Gly Ser Glu Pro Ala Thr Ser Gly
   355 360 365
   Ser Glu Thr Pro Gly Thr Ser Glu Ser Ala Thr Pro Glu Ser Gly Pro
   370 375 380
   Gly Thr Ser Thr Glu Pro Ser Glu Gly Ser Ala Pro Gly Thr Ser Thr
   385 390 395 400
   Glu Pro Ser Glu Gly Ser Ala Pro Gly Thr Ser Thr Glu Pro Ser Glu
   405 410 415
   Gly Ser Ala Pro Gly Thr Ser Thr Glu Pro Ser Glu Gly Ser Ala Pro
   420 425 430
   Gly Thr Ser Thr Glu Pro Ser Glu Gly Ser Ala Pro Gly Thr Ser Thr
   435 440 445
   Glu Pro Ser Glu Gly Ser Ala Pro Gly Ser Pro Ala Gly Ser Pro Thr
   450 455 460
   Ser Thr Glu Glu Gly Thr Ser Thr Glu Pro Ser Glu Gly Ser Ala Pro
   465 470 475 480
   Gly Thr Ser Glu Ser Ala Thr Pro Glu Ser Gly Pro Gly Ser Glu Pro
   485 490 495
   Ala Thr Ser Gly Ser Glu Thr Pro Gly Thr Ser Glu Ser Ala Thr Pro
   500 505 510
   Glu Ser Gly Pro Gly Ser Glu Pro Ala Thr Ser Gly Ser Glu Thr Pro
   515 520 525
   Gly Thr Ser Glu Ser Ala Thr Pro Glu Ser Gly Pro Gly Thr Ser Thr
   530 535 540
   Glu Pro Ser Glu Gly Ser Ala Pro Gly Thr Ser Glu Ser Ala Thr Pro
   545 550 555 560
   Glu Ser Gly Pro Gly Ser Pro Ala Gly Ser Pro Thr Ser Thr Glu Glu
   565 570 575
   Gly Ser Pro Ala Gly Ser Pro Thr Ser Thr Glu Glu Gly Ser Pro Ala
               580                 585                 590
   Gly Ser Pro Thr Ser Thr Glu Glu Gly Thr Ser Glu Ser Ala Thr Pro
           595                 600                 605
   Glu Ser Gly Pro Gly Thr Ser Thr Glu Pro Ser Glu Gly Ser Ala Pro
       610                 615                 620
   Gly Thr Ser Glu Ser Ala Thr Pro Glu Ser Gly Pro Gly Ser Glu Pro
   625                 630                 635                 640
   Ala Thr Ser Gly Ser Glu Thr Pro Gly Thr Ser Glu Ser Ala Thr Pro
                   645                 650                 655
   Glu Ser Gly Pro Gly Ser Glu Pro Ala Thr Ser Gly Ser Glu Thr Pro
               660                 665                 670
   Gly Thr Ser Glu Ser Ala Thr Pro Glu Ser Gly Pro Gly Thr Ser Thr
           675                 680                 685
   Glu Pro Ser Glu Gly Ser Ala Pro Gly Ser Pro Ala Gly Ser Pro Thr
       690                 695                 700
   Ser Thr Glu Glu Gly Thr Ser Glu Ser Ala Thr Pro Glu Ser Gly Pro
   705                 710                 715                 720
   Gly Ser Glu Pro Ala Thr Ser Gly Ser Glu Thr Pro Gly Thr Ser Glu
                   725                 730                 735
   Ser Ala Thr Pro Glu Ser Gly Pro Gly Ser Pro Ala Gly Ser Pro Thr
               740                 745                 750
   Ser Thr Glu Glu Gly Ser Pro Ala Gly Ser Pro Thr Ser Thr Glu Glu
           755                 760                 765
   Gly Thr Ser Thr Glu Pro Ser Glu Gly Ser Ala Pro Gly Thr Ser Glu
       770                 775                 780
   Ser Ala Thr Pro Glu Ser Gly Pro Gly Thr Ser Glu Ser Ala Thr Pro
   785                 790                 795                 800
   Glu Ser Gly Pro Gly Thr Ser Glu Ser Ala Thr Pro Glu Ser Gly Pro
                   805                 810                 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865                 870                 875                 880
   Ala Thr Ser Gly Ser Glu Thr Pro Gly Thr Ser Glu Ser Ala Thr Pro
                   885                 890                 895
   Glu Ser Gly Pro Gly Thr Ser Thr Glu Pro Ser Glu Gly Ser Ala Pro
               900                 905                 910
   Gly Phe Pro Thr Ile Pro Leu Ser Arg Leu Phe Asp Asn Ala Met Leu
           915                 920                 925
   Arg Ala His Arg Leu His Gln Leu Ala Phe Asp Thr Tyr Gln Glu Phe
       930                 935                 940
   Glu Glu Ala Tyr Ile Pro Lys Glu Gln Lys Tyr Ser Phe Leu Gln Asn
   945                 950                 955                 960
   Pro Gln Thr Ser Leu Cys Phe Ser Glu Ser Ile Pro Thr Pro Ser Asn
                   965                 970                 975
   Arg Glu Glu Thr Gln Gln Lys Ser Asn Leu Glu Leu Leu Arg Ile Ser
               980                 985                 990
   Leu Leu Leu Ile Gln Ser Trp Leu  Glu Pro Val Gln Phe  Leu Arg Ser
           995                 1000                 1005
   Val Phe  Ala Asn Ser Leu Val  Tyr Gly Ala Ser Asp  Ser Asn Val
       1010                 1015                 1020
   Tyr Asp  Leu Leu Lys Asp Leu  Glu Glu Gly Ile Gln  Thr Leu Met
       1025                 1030                 1035
   Gly Arg  Leu Glu Asp Gly Ser  Pro Arg Thr Gly Gln  Ile Phe Lys
       1040                 1045                 1050
   Gln Thr  Tyr Ser Lys Phe Asp  Thr Asn Ser His Asn  Asp Asp Ala
       1055                 1060                 1065
   Leu Leu  Lys Asn Tyr Gly Leu  Leu Tyr Cys Phe Arg  Lys Asp Met
       1070                 1075                 1080
   Asp Lys Val Glu Thr Phe Leu Arg Ile Val Gln Cys Arg Ser Val
       1085                1090                1095
   Glu Gly Ser Cys Gly Phe
       1100
<210> 8
   <211> 3318
   <212> DNA
   <213> Artificial Sequence
<220>
   <221> source
   <223> /note="Description of Artificial Sequence: Synthetic polynucleotide"
<400> 8
   atggctgaac ctgctggctc tccaacctcc actgaggaag gtaccccggg tagcggtact      60
   gcttcttcct ctccaggtag ctctacccct tctggtgcaa ccggctctcc aggtgcttct     120
   ccgggcacca gctctaccgg ttctccaggt agcccggctg gctctcctac ctctactgag     180
   gaaggtactt ctgaaagcgc tactcctgag tctggtccag gtacctctac tgaaccgtcc     240
   gaaggtagcg ctccaggtag cccagcaggc tctccgactt ccactgagga aggtacttct     300
   actgaacctt ccgaaggcag cgcaccaggt acctctactg aaccttctga gggcagcgct     360
   ccaggtactt ctgaaagcgc taccccggaa tctggcccag gtagcgaacc ggctacttct     420
   ggttctgaaa ccccaggtag cgaaccggct acctccggtt ctgaaactcc aggtagcccg     480
   gcaggctctc cgacctctac tgaggaaggt acttctgaaa gcgcaacccc ggagtccggc     540
   ccaggtacct ctaccgaacc gtctgagggc agcgcaccag gtacttctac cgaaccgtcc     600
   gagggtagcg caccaggtag cccagcaggt tctcctacct ccaccgagga aggtacttct     660
   accgaaccgt ccgagggtag cgcaccaggt acctctactg aaccttctga gggcagcgct     720
   ccaggtactt ctgaaagcgc taccccggag tccggtccag gtacttctac tgaaccgtcc     780
   gaaggtagcg caccaggtac ttctgaaagc gcaacccctg aatccggtcc aggtagcgaa     840
   ccggctactt ctggctctga gactccaggt acttctaccg aaccgtccga aggtagcgca     900
   ccaggtactt ctactgaacc gtctgaaggt agcgcaccag gtacttctga aagcgcaacc     960
   ccggaatccg gcccaggtac ctctgaaagc gcaaccccgg agtccggccc aggtagccct    1020
   gctggctctc caacctccac cgaagaaggt acctctgaaa gcgcaacccc tgaatccggc    1080
   ccaggtagcg aaccggcaac ctccggttct gaaaccccag gtacctctga aagcgctact    1140
   ccggagtctg gcccaggtac ctctactgaa ccgtctgagg gtagcgctcc aggtacttct    1200
   actgaaccgt ccgaaggtag cgcaccaggt acttctaccg aaccgtccga aggcagcgct    1260
   ccaggtacct ctactgaacc ttccgagggc agcgctccag gtacctctac cgaaccttct 1320
   gaaggtagcg caccaggtac ttctaccgaa ccgtccgagg gtagcgcacc aggtagccca 1380
   gcaggttctc ctacctccac cgaggaaggt acttctaccg aaccgtccga gggtagcgca 1440
   ccaggtacct ctgaaagcgc aactcctgag tctggcccag gtagcgaacc tgctacctcc 1500
   ggctctgaga ctccaggtac ctctgaaagc gcaaccccgg aatctggtcc aggtagcgaa 1560
   cctgcaacct ctggctctga aaccccaggt acctctgaaa gcgctactcc tgaatctggc 1620
   ccaggtactt ctactgaacc gtccgagggc agcgcaccag gtacttctga aagcgctact 1680
   cctgagtccg gcccaggtag cccggctggc tctccgactt ccaccgagga aggtagcccg 1740
   gctggctctc caacttctac tgaagaaggt agcccggcag gctctccgac ctctactgag 1800
   gaaggtactt ctgaaagcgc aaccccggag tccggcccag gtacctctac cgaaccgtct 1860
   gagggcagcg caccaggtac ctctgaaagc gcaactcctg agtctggccc aggtagcgaa 1920
   cctgctacct ccggctctga gactccaggt acctctgaaa gcgcaacccc ggaatctggt 1980
   ccaggtagcg aacctgcaac ctctggctct gaaaccccag gtacctctga aagcgctact 2040
   cctgaatctg gcccaggtac ttctactgaa ccgtccgagg gcagcgcacc aggtagccct 2100
   gctggctctc caacctccac cgaagaaggt acctctgaaa gcgcaacccc tgaatccggc 2160
   ccaggtagcg aaccggcaac ctccggttct gaaaccccag gtacttctga aagcgctact 2220
   cctgagtccg gcccaggtag cccggctggc tctccgactt ccaccgagga aggtagcccg 2280
   gctggctctc caacttctac tgaagaaggt acttctaccg aaccttccga gggcagcgca 2340
   ccaggtactt ctgaaagcgc tacccctgag tccggcccag gtacttctga aagcgctact 2400
   cctgaatccg gtccaggtac ttctgaaagc gctaccccgg aatctggccc aggtagcgaa 2460
   ccggctactt ctggttctga aaccccaggt agcgaaccgg ctacctccgg ttctgaaact 2520
   ccaggtagcc cagcaggctc tccgacttcc actgaggaag gtacttctac tgaaccttcc 2580
   gaaggcagcg caccaggtac ctctactgaa ccttctgagg gcagcgctcc aggtagcgaa 2640
   cctgcaacct ctggctctga aaccccaggt acctctgaaa gcgctactcc tgaatctggc 2700
   ccaggtactt ctactgaacc gtccgagggc agcgcaccag gttttccgac tattccgctg 2760
   tctcgtctgt ttgataatgc tatgctgcgt gcgcaccgtc tgcaccagct ggcctttgat 2820
   acttaccagg aatttgaaga agcctacatt cctaaagagc agaagtactc tttcctgcaa 2880
   aacccacaga cttctctctg cttcagcgaa tctattccga cgccttccaa tcgcgaggaa 2940
   actcagcaaa agtccaatct ggaactactc cgcatttctc tgcttctgat tcagagctgg 3000
   ctagaaccag tgcaatttct gcgttccgtc ttcgccaata gcctagttta tggcgcatcc 3060
   gacagcaacg tatacgatct cctgaaagat ctcgaggaag gcattcagac cctgatgggt 3120
   cgtctcgagg atggctctcc gcgtactggt cagatcttca agcagactta ctctaaattt 3180
   gatactaaca gccacaatga cgatgcgctt ctaaaaaact atggtctgct gtattgtttt     3240
   cgtaaagata tggacaaagt tgaaaccttc ctgcgtattg ttcagtgtcg ttccgttgag     3300
   ggcagctgtg gtttctaa                                                   3318
<210> 9
   <211> 1250
   <212> PRT
   <213> Artificial Sequence
<220>
   <221> source
   <223> /note="Description of Artificial Sequence: Synthetic polypeptide"
<400> 9
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
                   165                 170                 175
   Glu Ser Gly Pro Gly Thr Ser Thr Glu Pro Ser Glu Gly Ser Ala Pro
               180                 185                 190
   Gly Thr Ser Thr Glu Pro Ser Glu Gly Ser Ala Pro Gly Ser Pro Ala
           195                 200                 205
   Gly Ser Pro Thr Ser Thr Glu Glu Gly Thr Ser Thr Glu Pro Ser Glu
       210                 215                 220
   Gly Ser Ala Pro Gly Thr Ser Thr Glu Pro Ser Glu Gly Ser Ala Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Ser Glu Pro Ala Thr Ser Gly Ser Glu Thr Pro
           275                 280                 285
   Gly Thr Ser Thr Glu Pro Ser Glu Gly Ser Ala Pro Gly Thr Ser Thr
       290                 295                 300
   Glu Pro Ser Glu Gly Ser Ala Pro Gly Thr Ser Glu Ser Ala Thr Pro
   305                 310                 315                 320
   Glu Ser Gly Pro Gly Thr Ser Glu Ser Ala Thr Pro Glu Ser Gly Pro
                   325                 330                 335
   Gly Ser Pro Ala Gly Ser Pro Thr Ser Thr Glu Glu Gly Thr Ser Glu
               340                 345                 350
   Ser Ala Thr Pro Glu Ser Gly Pro Gly Ser Glu Pro Ala Thr Ser Gly
           355                 360                 365
   Ser Glu Thr Pro Gly Thr Ser Glu Ser Ala Thr Pro Glu Ser Gly Pro
       370                 375                 380
   Gly Thr Ser Thr Glu Pro Ser Glu Gly Ser Ala Pro Gly Thr Ser Thr
   385                 390                 395                 400
   Glu Pro Ser Glu Gly Ser Ala Pro Gly Thr Ser Thr Glu Pro Ser Glu
                   405                 410                 415
   Gly Ser Ala Pro Gly Thr Ser Thr Glu Pro Ser Glu Gly Ser Ala Pro
               420                 425                 430
   Gly Thr Ser Thr Glu Pro Ser Glu Gly Ser Ala Pro Gly Thr Ser Thr
   435 440 445
   Glu Pro Ser Glu Gly Ser Ala Pro Gly Ser Pro Ala Gly Ser Pro Thr
   450 455 460
   Ser Thr Glu Glu Gly Thr Ser Thr Glu Pro Ser Glu Gly Ser Ala Pro
   465 470 475 480
   Gly Thr Ser Glu Ser Ala Thr Pro Glu Ser Gly Pro Gly Ser Glu Pro
   485 490 495
   Ala Thr Ser Gly Ser Glu Thr Pro Gly Thr Ser Glu Ser Ala Thr Pro
   500 505 510
   Glu Ser Gly Pro Gly Ser Glu Pro Ala Thr Ser Gly Ser Glu Thr Pro
   515 520 525
   Gly Thr Ser Glu Ser Ala Thr Pro Glu Ser Gly Pro Gly Thr Ser Thr
   530 535 540
   Glu Pro Ser Glu Gly Ser Ala Pro Gly Thr Ser Glu Ser Ala Thr Pro
   545 550 555 560
   Glu Ser Gly Pro Gly Ser Pro Ala Gly Ser Pro Thr Ser Thr Glu Glu
   565 570 575
   Gly Ser Pro Ala Gly Ser Pro Thr Ser Thr Glu Glu Gly Ser Pro Ala
   580 585 590
   Gly Ser Pro Thr Ser Thr Glu Glu Gly Thr Ser Glu Ser Ala Thr Pro
   595 600 605
   Glu Ser Gly Pro Gly Thr Ser Thr Glu Pro Ser Glu Gly Ser Ala Pro
   610 615 620
   Gly Thr Ser Glu Ser Ala Thr Pro Glu Ser Gly Pro Gly Ser Glu Pro
   625 630 635 640
   Ala Thr Ser Gly Ser Glu Thr Pro Gly Thr Ser Glu Ser Ala Thr Pro
   645 650 655
   Glu Ser Gly Pro Gly Ser Glu Pro Ala Thr Ser Gly Ser Glu Thr Pro
   660 665 670
   Gly Thr Ser Glu Ser Ala Thr Pro Glu Ser Gly Pro Gly Thr Ser Thr
   675 680 685
   Glu Pro Ser Glu Gly Ser Ala Pro Gly Ser Pro Ala Gly Ser Pro Thr
       690                 695                 700
   Ser Thr Glu Glu Gly Thr Ser Glu Ser Ala Thr Pro Glu Ser Gly Pro
   705                 710                 715                 720
   Gly Ser Glu Pro Ala Thr Ser Gly Ser Glu Thr Pro Gly Thr Ser Glu
                   725                 730                 735
   Ser Ala Thr Pro Glu Ser Gly Pro Gly Ser Pro Ala Gly Ser Pro Thr
               740                 745                 750
   Ser Thr Glu Glu Gly Ser Pro Ala Gly Ser Pro Thr Ser Thr Glu Glu
           755                 760                 765
   Gly Thr Ser Thr Glu Pro Ser Glu Gly Ser Ala Pro Gly Thr Ser Glu
       770                 775                 780
   Ser Ala Thr Pro Glu Ser Gly Pro Gly Thr Ser Glu Ser Ala Thr Pro
   785                 790                 795                 800
   Glu Ser Gly Pro Gly Thr Ser Glu Ser Ala Thr Pro Glu Ser Gly Pro
                   805                 810                 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865                 870                 875                 880
   Ala Thr Ser Gly Ser Glu Thr Pro Gly Thr Ser Glu Ser Ala Thr Pro
                   885                 890                 895
   Glu Ser Gly Pro Gly Thr Ser Thr Glu Pro Ser Glu Gly Ser Ala Pro
               900                 905                 910
   Gly Phe Pro Thr Ile Pro Leu Ser Arg Leu Phe Asp Asn Ala Met Leu
           915                 920                 925
   Arg Ala His Arg Leu His Gln Leu Ala Phe Asp Thr Tyr Gln Glu Phe
   930 935 940
   Glu Glu Ala Tyr Ile Pro Lys Glu Gln Lys Tyr Ser Phe Leu Gln Asn
   945 950 955 960
   Pro Gln Thr Ser Leu Cys Phe Ser Glu Ser Ile Pro Thr Pro Ser Asn
   965 970 975
   Arg Glu Glu Thr Gln Gln Lys Ser Asn Leu Glu Leu Leu Arg Ile Ser
   980 985 990
   Leu Leu Leu Ile Gln Ser Trp Leu Glu Pro Val Gln Phe Leu Arg Ser
   995 1000 1005
   Val Phe Ala Asn Ser Leu Val Tyr Gly Ala Ser Asp Ser Asn Val
   1010 1015 1020
   Tyr Asp Leu Leu Lys Asp Leu Glu Glu Gly Ile Gln Thr Leu Met
   1025 1030 1035
   Gly Arg Leu Glu Asp Gly Ser Pro Arg Thr Gly Gln Ile Phe Lys
   1040 1045 1050
   Gln Thr Tyr Ser Lys Phe Asp Thr Asn Ser His Asn Asp Asp Ala
   1055 1060 1065
   Leu Leu Lys Asn Tyr Gly Leu Leu Tyr Cys Phe Arg Lys Asp Met
   1070 1075 1080
   Asp Lys Val Glu Thr Phe Leu Arg Ile Val Gln Cys Arg Ser Val
   1085 1090 1095
   Glu Gly Ser Cys Gly Phe Gly Gly Thr Ser Glu Ser Ala Thr Pro
   1100 1105 1110
   Glu Ser Gly Pro Gly Thr Ser Thr Glu Pro Ser Glu Gly Ser Ala
   1115 1120 1125
   Pro Gly Thr Ser Thr Glu Pro Ser Glu Gly Ser Ala Pro Gly Thr
   1130 1135 1140
   Ser Glu Ser Ala Thr Pro Glu Ser Gly Pro Gly Thr Ser Thr Glu
   1145 1150 1155
   Pro Ser Glu Gly Ser Ala Pro Gly Thr Ser Thr Glu Pro Ser Glu
   1160 1165 1170
   Gly Ser  Ala Pro Gly Thr Ser  Glu Ser Ala Thr Pro  Glu Ser Gly
       1175                 1180                 1185
   Pro Gly  Thr Ser Thr Glu Pro  Ser Glu Gly Ser Ala  Pro Gly Thr
       1190                 1195                 1200
   Ser Thr  Glu Pro Ser Glu Gly  Ser Ala Pro Gly Thr  Ser Thr Glu
       1205                 1210                 1215
   Pro Ser  Glu Gly Ser Ala Pro  Gly Ser Pro Ala Gly  Ser Pro Thr
       1220                 1225                 1230
   Ser Thr  Glu Glu Gly Thr Ser  Thr Glu Pro Ser Glu  Gly Ser Ala
       1235                 1240                 1245
   Pro Gly
       1250
<210> 10
   <211> 3753
   <212> DNA
   <213> Artificial Sequence
<220>
   <221> source
   <223> /note="Description of Artificial Sequence: Synthetic polynucleotide"
<400> 10
   atggctgaac ctgctggctc tccaacctcc actgaggaag gtaccccggg tagcggtact      60
   gcttcttcct ctccaggtag ctctacccct tctggtgcaa ccggctctcc aggtgcttct     120
   ccgggcacca gctctaccgg ttctccaggt agcccggctg gctctcctac ctctactgag     180
   gaaggtactt ctgaaagcgc tactcctgag tctggtccag gtacctctac tgaaccgtcc     240
   gaaggtagcg ctccaggtag cccagcaggc tctccgactt ccactgagga aggtacttct     300
   actgaacctt ccgaaggcag cgcaccaggt acctctactg aaccttctga gggcagcgct     360
   ccaggtactt ctgaaagcgc taccccggaa tctggcccag gtagcgaacc ggctacttct     420
   ggttctgaaa ccccaggtag cgaaccggct acctccggtt ctgaaactcc aggtagcccg     480
   gcaggctctc cgacctctac tgaggaaggt acttctgaaa gcgcaacccc ggagtccggc     540
   ccaggtacct ctaccgaacc gtctgagggc agcgcaccag gtacttctac cgaaccgtcc     600
   gagggtagcg caccaggtag cccagcaggt tctcctacct ccaccgagga aggtacttct     660
   accgaaccgt ccgagggtag cgcaccaggt acctctactg aaccttctga gggcagcgct     720
   ccaggtactt ctgaaagcgc taccccggag tccggtccag gtacttctac tgaaccgtcc     780
   gaaggtagcg caccaggtac ttctgaaagc gcaacccctg aatccggtcc aggtagcgaa     840
   ccggctactt ctggctctga gactccaggt acttctaccg aaccgtccga aggtagcgca 900
   ccaggtactt ctactgaacc gtctgaaggt agcgcaccag gtacttctga aagcgcaacc 960
   ccggaatccg gcccaggtac ctctgaaagc gcaaccccgg agtccggccc aggtagccct 1020
   gctggctctc caacctccac cgaagaaggt acctctgaaa gcgcaacccc tgaatccggc 1080
   ccaggtagcg aaccggcaac ctccggttct gaaaccccag gtacctctga aagcgctact 1140
   ccggagtctg gcccaggtac ctctactgaa ccgtctgagg gtagcgctcc aggtacttct 1200
   actgaaccgt ccgaaggtag cgcaccaggt acttctaccg aaccgtccga aggcagcgct 1260
   ccaggtacct ctactgaacc ttccgagggc agcgctccag gtacctctac cgaaccttct 1320
   gaaggtagcg caccaggtac ttctaccgaa ccgtccgagg gtagcgcacc aggtagccca 1380
   gcaggttctc ctacctccac cgaggaaggt acttctaccg aaccgtccga gggtagcgca 1440
   ccaggtacct ctgaaagcgc aactcctgag tctggcccag gtagcgaacc tgctacctcc 1500
   ggctctgaga ctccaggtac ctctgaaagc gcaaccccgg aatctggtcc aggtagcgaa 1560
   cctgcaacct ctggctctga aaccccaggt acctctgaaa gcgctactcc tgaatctggc 1620
   ccaggtactt ctactgaacc gtccgagggc agcgcaccag gtacttctga aagcgctact 1680
   cctgagtccg gcccaggtag cccggctggc tctccgactt ccaccgagga aggtagcccg 1740
   gctggctctc caacttctac tgaagaaggt agcccggcag gctctccgac ctctactgag 1800
   gaaggtactt ctgaaagcgc aaccccggag tccggcccag gtacctctac cgaaccgtct 1860
   gagggcagcg caccaggtac ctctgaaagc gcaactcctg agtctggccc aggtagcgaa 1920
   cctgctacct ccggctctga gactccaggt acctctgaaa gcgcaacccc ggaatctggt 1980
   ccaggtagcg aacctgcaac ctctggctct gaaaccccag gtacctctga aagcgctact 2040
   cctgaatctg gcccaggtac ttctactgaa ccgtccgagg gcagcgcacc aggtagccct 2100
   gctggctctc caacctccac cgaagaaggt acctctgaaa gcgcaacccc tgaatccggc 2160
   ccaggtagcg aaccggcaac ctccggttct gaaaccccag gtacttctga aagcgctact 2220
   cctgagtccg gcccaggtag cccggctggc tctccgactt ccaccgagga aggtagcccg 2280
   gctggctctc caacttctac tgaagaaggt acttctaccg aaccttccga gggcagcgca 2340
   ccaggtactt ctgaaagcgc tacccctgag tccggcccag gtacttctga aagcgctact 2400
   cctgaatccg gtccaggtac ttctgaaagc gctaccccgg aatctggccc aggtagcgaa 2460
   ccggctactt ctggttctga aaccccaggt agcgaaccgg ctacctccgg ttctgaaact 2520
   ccaggtagcc cagcaggctc tccgacttcc actgaggaag gtacttctac tgaaccttcc 2580
   gaaggcagcg caccaggtac ctctactgaa ccttctgagg gcagcgctcc aggtagcgaa 2640
   cctgcaacct ctggctctga aaccccaggt acctctgaaa gcgctactcc tgaatctggc 2700
   ccaggtactt ctactgaacc gtccgagggc agcgcaccag gttttccgac tattccgctg 2760
   tctcgtctgt ttgataatgc tatgctgcgt gcgcaccgtc tgcaccagct ggcctttgat 2820
   acttaccagg aatttgaaga agcctacatt cctaaagagc agaagtactc tttcctgcaa 2880
   aacccacaga cttctctctg cttcagcgaa tctattccga cgccttccaa tcgcgaggaa 2940
   actcagcaaa agtccaatct ggaactactc cgcatttctc tgcttctgat tcagagctgg 3000
   ctagaaccag tgcaatttct gcgttccgtc ttcgccaata gcctagttta tggcgcatcc 3060
   gacagcaacg tatacgatct cctgaaagat ctcgaggaag gcattcagac cctgatgggt 3120
   cgtctcgagg atggctctcc gcgtactggt cagatcttca agcagactta ctctaaattt 3180
   gatactaaca gccacaatga cgatgcgctt ctaaaaaact atggtctgct gtattgtttt 3240
   cgtaaagata tggacaaagt tgaaaccttc ctgcgtattg ttcagtgtcg ttccgttgag 3300
   ggcagctgtg gtttctaagg tggtagcgaa ccggcaactt ccggctctga aaccccaggt 3360
   acttctgaaa gcgctactcc tgagtctggc ccaggtagcg aacctgctac ctctggctct 3420
   gaaaccccag gtagcccggc aggctctccg acttccaccg aggaaggtac ctctactgaa 3480
   ccttctgagg gtagcgctcc aggtagcgaa ccggcaacct ctggctctga aaccccaggt 3540
   agcgaacctg ctacctccgg ctctgaaact ccaggtagcg aaccggctac ttccggttct 3600
   gaaactccag gtacctctac cgaaccttcc gaaggcagcg caccaggtac ttctgaaagc 3660
   gcaacccctg aatccggtcc aggtagcgaa ccggctactt ctggctctga gactccaggt 3720
   acttctaccg aaccgtccga aggtagcgca cca 3753
<210> 11
   <211> 1394
   <212> PRT
   <213> Artificial Sequence
<220>
   <221> source
   <223> /note="Description of Artificial Sequence: Synthetic polypeptide"
<400> 11
   Ala Glu Pro Ala Gly Ser Pro Thr Ser Thr Glu Glu Gly Thr Pro Gly
   1 5 10 15
   Ser Gly Thr Ala Ser Ser Ser Pro Gly Ser Ser Thr Pro Ser Gly Ala
   20 25 30
   Thr Gly Ser Pro Gly Ala Ser Pro Gly Thr Ser Ser Thr Gly Ser Pro
   35 40 45
   Gly Ser Pro Ala Gly Ser Pro Thr Ser Thr Glu Glu Gly Thr Ser Glu
   50 55 60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
                   165                 170                 175
   Glu Ser Gly Pro Gly Thr Ser Thr Glu Pro Ser Glu Gly Ser Ala Pro
               180                 185                 190
   Gly Thr Ser Thr Glu Pro Ser Glu Gly Ser Ala Pro Gly Ser Pro Ala
           195                 200                 205
   Gly Ser Pro Thr Ser Thr Glu Glu Gly Thr Ser Thr Glu Pro Ser Glu
       210                 215                 220
   Gly Ser Ala Pro Gly Thr Ser Thr Glu Pro Ser Glu Gly Ser Ala Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Ser Glu Pro Ala Thr Ser Gly Ser Glu Thr Pro
           275                 280                 285
   Gly Thr Ser Thr Glu Pro Ser Glu Gly Ser Ala Pro Gly Thr Ser Thr
       290                 295                 300
   Glu Pro Ser Glu Gly Ser Ala Pro Gly Thr Ser Glu Ser Ala Thr Pro
   305                 310                 315                 320
   Glu Ser Gly Pro Gly Thr Ser Glu Ser Ala Thr Pro Glu Ser Gly Pro
                   325                 330                 335
   Gly Ser Pro Ala Gly Ser Pro Thr Ser Thr Glu Glu Gly Thr Ser Glu
               340                 345                 350
   Ser Ala Thr Pro Glu Ser Gly Pro Gly Ser Glu Pro Ala Thr Ser Gly
           355                 360                 365
   Ser Glu Thr Pro Gly Thr Ser Glu Ser Ala Thr Pro Glu Ser Gly Pro
       370                 375                 380
   Gly Thr Ser Thr Glu Pro Ser Glu Gly Ser Ala Pro Gly Thr Ser Thr
   385                 390                 395                 400
   Glu Pro Ser Glu Gly Ser Ala Pro Gly Thr Ser Thr Glu Pro Ser Glu
                   405                 410                 415
   Gly Ser Ala Pro Gly Thr Ser Thr Glu Pro Ser Glu Gly Ser Ala Pro
               420                 425                 430
   Gly Thr Ser Thr Glu Pro Ser Glu Gly Ser Ala Pro Gly Thr Ser Thr
           435                 440                 445
   Glu Pro Ser Glu Gly Ser Ala Pro Gly Ser Pro Ala Gly Ser Pro Thr
       450                 455                 460
   Ser Thr Glu Glu Gly Thr Ser Thr Glu Pro Ser Glu Gly Ser Ala Pro
   465                 470                 475                 480
   Gly Thr Ser Glu Ser Ala Thr Pro Glu Ser Gly Pro Gly Ser Glu Pro
                   485                 490                 495
   Ala Thr Ser Gly Ser Glu Thr Pro Gly Thr Ser Glu Ser Ala Thr Pro
               500                 505                 510
   Glu Ser Gly Pro Gly Ser Glu Pro Ala Thr Ser Gly Ser Glu Thr Pro
           515                 520                 525
   Gly Thr Ser Glu Ser Ala Thr Pro Glu Ser Gly Pro Gly Thr Ser Thr
       530                 535                 540
   Glu Pro Ser Glu Gly Ser Ala Pro Gly Thr Ser Glu Ser Ala Thr Pro
   545                 550                 555                 560
   Glu Ser Gly Pro Gly Ser Pro Ala Gly Ser Pro Thr Ser Thr Glu Glu
                   565                 570                 575
   Gly Ser Pro Ala Gly Ser Pro Thr Ser Thr Glu Glu Gly Ser Pro Ala
   580 585 590
   Gly Ser Pro Thr Ser Thr Glu Glu Gly Thr Ser Glu Ser Ala Thr Pro
   595 600 605
   Glu Ser Gly Pro Gly Thr Ser Thr Glu Pro Ser Glu Gly Ser Ala Pro
   610 615 620
   Gly Thr Ser Glu Ser Ala Thr Pro Glu Ser Gly Pro Gly Ser Glu Pro
   625 630 635 640
   Ala Thr Ser Gly Ser Glu Thr Pro Gly Thr Ser Glu Ser Ala Thr Pro
   645 650 655
   Glu Ser Gly Pro Gly Ser Glu Pro Ala Thr Ser Gly Ser Glu Thr Pro
   660 665 670
   Gly Thr Ser Glu Ser Ala Thr Pro Glu Ser Gly Pro Gly Thr Ser Thr
   675 680 685
   Glu Pro Ser Glu Gly Ser Ala Pro Gly Ser Pro Ala Gly Ser Pro Thr
   690 695 700
   Ser Thr Glu Glu Gly Thr Ser Glu Ser Ala Thr Pro Glu Ser Gly Pro
   705 710 715 720
   Gly Ser Glu Pro Ala Thr Ser Gly Ser Glu Thr Pro Gly Thr Ser Glu
   725 730 735
   Ser Ala Thr Pro Glu Ser Gly Pro Gly Ser Pro Ala Gly Ser Pro Thr
   740 745 750
   Ser Thr Glu Glu Gly Ser Pro Ala Gly Ser Pro Thr Ser Thr Glu Glu
   755 760 765
   Gly Thr Ser Thr Glu Pro Ser Glu Gly Ser Ala Pro Gly Thr Ser Glu
   770 775 780
   Ser Ala Thr Pro Glu Ser Gly Pro Gly Thr Ser Glu Ser Ala Thr Pro
   785 790 795 800
   Glu Ser Gly Pro Gly Thr Ser Glu Ser Ala Thr Pro Glu Ser Gly Pro
   805 810 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865                 870                 875                 880
   Ala Thr Ser Gly Ser Glu Thr Pro Gly Thr Ser Glu Ser Ala Thr Pro
                   885                 890                 895
   Glu Ser Gly Pro Gly Thr Ser Thr Glu Pro Ser Glu Gly Ser Ala Pro
               900                 905                 910
   Gly Phe Pro Thr Ile Pro Leu Ser Arg Leu Phe Asp Asn Ala Met Leu
           915                 920                 925
   Arg Ala His Arg Leu His Gln Leu Ala Phe Asp Thr Tyr Gln Glu Phe
       930                 935                 940
   Glu Glu Ala Tyr Ile Pro Lys Glu Gln Lys Tyr Ser Phe Leu Gln Asn
   945                 950                 955                 960
   Pro Gln Thr Ser Leu Cys Phe Ser Glu Ser Ile Pro Thr Pro Ser Asn
                   965                 970                 975
   Arg Glu Glu Thr Gln Gln Lys Ser Asn Leu Glu Leu Leu Arg Ile Ser
               980                 985                 990
   Leu Leu Leu Ile Gln Ser Trp Leu  Glu Pro Val Gln Phe  Leu Arg Ser
           995                 1000                 1005
   Val Phe  Ala Asn Ser Leu Val  Tyr Gly Ala Ser Asp  Ser Asn Val
       1010                 1015                 1020
   Tyr Asp  Leu Leu Lys Asp Leu  Glu Glu Gly Ile Gln  Thr Leu Met
       1025                 1030                 1035
   Gly Arg  Leu Glu Asp Gly Ser  Pro Arg Thr Gly Gln  Ile Phe Lys
       1040                 1045                 1050
   Gln Thr  Tyr Ser Lys Phe Asp  Thr Asn Ser His Asn  Asp Asp Ala
       1055                 1060                 1065
   Leu Leu Lys Asn Tyr Gly Leu Leu Tyr Cys Phe Arg Lys Asp Met
   1070 1075 1080
   Asp Lys Val Glu Thr Phe Leu Arg Ile Val Gln Cys Arg Ser Val
   1085 1090 1095
   Glu Gly Ser Cys Gly Phe Gly Gly Thr Ser Glu Ser Ala Thr Pro
   1100 1105 1110
   Glu Ser Gly Pro Gly Ser Glu Pro Ala Thr Ser Gly Ser Glu Thr
   1115 1120 1125
   Pro Gly Thr Ser Glu Ser Ala Thr Pro Glu Ser Gly Pro Gly Ser
   1130 1135 1140
   Glu Pro Ala Thr Ser Gly Ser Glu Thr Pro Gly Thr Ser Glu Ser
   1145 1150 1155
   Ala Thr Pro Glu Ser Gly Pro Gly Thr Ser Thr Glu Pro Ser Glu
   1160 1165 1170
   Gly Ser Ala Pro Gly Ser Pro Ala Gly Ser Pro Thr Ser Thr Glu
   1175 1180 1185
   Glu Gly Thr Ser Glu Ser Ala Thr Pro Glu Ser Gly Pro Gly Ser
   1190 1195 1200
   Glu Pro Ala Thr Ser Gly Ser Glu Thr Pro Gly Thr Ser Glu Ser
   1205 1210 1215
   Ala Thr Pro Glu Ser Gly Pro Gly Ser Pro Ala Gly Ser Pro Thr
   1220 1225 1230
   Ser Thr Glu Glu Gly Ser Pro Ala Gly Ser Pro Thr Ser Thr Glu
   1235 1240 1245
   Glu Gly Thr Ser Thr Glu Pro Ser Glu Gly Ser Ala Pro Gly Thr
   1250 1255 1260
   Ser Glu Ser Ala Thr Pro Glu Ser Gly Pro Gly Thr Ser Glu Ser
   1265 1270 1275
   Ala Thr Pro Glu Ser Gly Pro Gly Thr Ser Glu Ser Ala Thr Pro
   1280 1285 1290
   Glu Ser Gly Pro Gly Ser Glu Pro Ala Thr Ser Gly Ser Glu Thr
   1295 1300 1305
   Pro Gly  Ser Glu Pro Ala Thr  Ser Gly Ser Glu Thr  Pro Gly Ser
       1310                 1315                 1320
   Pro Ala  Gly Ser Pro Thr Ser  Thr Glu Glu Gly Thr  Ser Thr Glu
       1325                 1330                 1335
   Pro Ser  Glu Gly Ser Ala Pro  Gly Thr Ser Thr Glu  Pro Ser Glu
       1340                 1345                 1350
   Gly Ser  Ala Pro Gly Ser Glu  Pro Ala Thr Ser Gly  Ser Glu Thr
       1355                 1360                 1365
   Pro Gly  Thr Ser Glu Ser Ala  Thr Pro Glu Ser Gly  Pro Gly Thr
       1370                 1375                 1380
   Ser Thr  Glu Pro Ser Glu Gly  Ser Ala Pro Gly
       1385                 1390
<210> 12
   <211> 4185
   <212> DNA
   <213> Artificial Sequence
<220>
   <221> source
   <223> /note="Description of Artificial Sequence: Synthetic polynucleotide"
<400> 12
   atggctgaac ctgctggctc tccaacctcc actgaggaag gtaccccggg tagcggtact      60
   gcttcttcct ctccaggtag ctctacccct tctggtgcaa ccggctctcc aggtgcttct     120
   ccgggcacca gctctaccgg ttctccaggt agcccggctg gctctcctac ctctactgag     180
   gaaggtactt ctgaaagcgc tactcctgag tctggtccag gtacctctac tgaaccgtcc     240
   gaaggtagcg ctccaggtag cccagcaggc tctccgactt ccactgagga aggtacttct     300
   actgaacctt ccgaaggcag cgcaccaggt acctctactg aaccttctga gggcagcgct     360
   ccaggtactt ctgaaagcgc taccccggaa tctggcccag gtagcgaacc ggctacttct     420
   ggttctgaaa ccccaggtag cgaaccggct acctccggtt ctgaaactcc aggtagcccg     480
   gcaggctctc cgacctctac tgaggaaggt acttctgaaa gcgcaacccc ggagtccggc     540
   ccaggtacct ctaccgaacc gtctgagggc agcgcaccag gtacttctac cgaaccgtcc     600
   gagggtagcg caccaggtag cccagcaggt tctcctacct ccaccgagga aggtacttct     660
   accgaaccgt ccgagggtag cgcaccaggt acctctactg aaccttctga gggcagcgct     720
   ccaggtactt ctgaaagcgc taccccggag tccggtccag gtacttctac tgaaccgtcc     780
   gaaggtagcg caccaggtac ttctgaaagc gcaacccctg aatccggtcc aggtagcgaa     840
   ccggctactt ctggctctga gactccaggt acttctaccg aaccgtccga aggtagcgca     900
   ccaggtactt ctactgaacc gtctgaaggt agcgcaccag gtacttctga aagcgcaacc     960
   ccggaatccg gcccaggtac ctctgaaagc gcaaccccgg agtccggccc aggtagccct    1020
   gctggctctc caacctccac cgaagaaggt acctctgaaa gcgcaacccc tgaatccggc    1080
   ccaggtagcg aaccggcaac ctccggttct gaaaccccag gtacctctga aagcgctact    1140
   ccggagtctg gcccaggtac ctctactgaa ccgtctgagg gtagcgctcc aggtacttct    1200
   actgaaccgt ccgaaggtag cgcaccaggt acttctaccg aaccgtccga aggcagcgct    1260
   ccaggtacct ctactgaacc ttccgagggc agcgctccag gtacctctac cgaaccttct    1320
   gaaggtagcg caccaggtac ttctaccgaa ccgtccgagg gtagcgcacc aggtagccca    1380
   gcaggttctc ctacctccac cgaggaaggt acttctaccg aaccgtccga gggtagcgca    1440
   ccaggtacct ctgaaagcgc aactcctgag tctggcccag gtagcgaacc tgctacctcc    1500
   ggctctgaga ctccaggtac ctctgaaagc gcaaccccgg aatctggtcc aggtagcgaa    1560
   cctgcaacct ctggctctga aaccccaggt acctctgaaa gcgctactcc tgaatctggc    1620
   ccaggtactt ctactgaacc gtccgagggc agcgcaccag gtacttctga aagcgctact    1680
   cctgagtccg gcccaggtag cccggctggc tctccgactt ccaccgagga aggtagcccg    1740
   gctggctctc caacttctac tgaagaaggt agcccggcag gctctccgac ctctactgag    1800
   gaaggtactt ctgaaagcgc aaccccggag tccggcccag gtacctctac cgaaccgtct    1860
   gagggcagcg caccaggtac ctctgaaagc gcaactcctg agtctggccc aggtagcgaa    1920
   cctgctacct ccggctctga gactccaggt acctctgaaa gcgcaacccc ggaatctggt    1980
   ccaggtagcg aacctgcaac ctctggctct gaaaccccag gtacctctga aagcgctact    2040
   cctgaatctg gcccaggtac ttctactgaa ccgtccgagg gcagcgcacc aggtagccct    2100
   gctggctctc caacctccac cgaagaaggt acctctgaaa gcgcaacccc tgaatccggc    2160
   ccaggtagcg aaccggcaac ctccggttct gaaaccccag gtacttctga aagcgctact    2220
   cctgagtccg gcccaggtag cccggctggc tctccgactt ccaccgagga aggtagcccg    2280
   gctggctctc caacttctac tgaagaaggt acttctaccg aaccttccga gggcagcgca    2340
   ccaggtactt ctgaaagcgc tacccctgag tccggcccag gtacttctga aagcgctact    2400
   cctgaatccg gtccaggtac ttctgaaagc gctaccccgg aatctggccc aggtagcgaa    2460
   ccggctactt ctggttctga aaccccaggt agcgaaccgg ctacctccgg ttctgaaact    2520
   ccaggtagcc cagcaggctc tccgacttcc actgaggaag gtacttctac tgaaccttcc    2580
   gaaggcagcg caccaggtac ctctactgaa ccttctgagg gcagcgctcc aggtagcgaa    2640
   cctgcaacct ctggctctga aaccccaggt acctctgaaa gcgctactcc tgaatctggc    2700
   ccaggtactt ctactgaacc gtccgagggc agcgcaccag gttttccgac tattccgctg 2760
   tctcgtctgt ttgataatgc tatgctgcgt gcgcaccgtc tgcaccagct ggcctttgat 2820
   acttaccagg aatttgaaga agcctacatt cctaaagagc agaagtactc tttcctgcaa 2880
   aacccacaga cttctctctg cttcagcgaa tctattccga cgccttccaa tcgcgaggaa 2940
   actcagcaaa agtccaatct ggaactactc cgcatttctc tgcttctgat tcagagctgg 3000
   ctagaaccag tgcaatttct gcgttccgtc ttcgccaata gcctagttta tggcgcatcc 3060
   gacagcaacg tatacgatct cctgaaagat ctcgaggaag gcattcagac cctgatgggt 3120
   cgtctcgagg atggctctcc gcgtactggt cagatcttca agcagactta ctctaaattt 3180
   gatactaaca gccacaatga cgatgcgctt ctaaaaaact atggtctgct gtattgtttt 3240
   cgtaaagata tggacaaagt tgaaaccttc ctgcgtattg ttcagtgtcg ttccgttgag 3300
   ggcagctgtg gtttctaagg tggtacctct gaaagcgcaa ctcctgagtc tggcccaggt 3360
   agcgaacctg ctacctccgg ctctgagact ccaggtacct ctgaaagcgc aaccccggaa 3420
   tctggtccag gtagcgaacc tgcaacctct ggctctgaaa ccccaggtac ctctgaaagc 3480
   gctactcctg aatctggccc aggtacttct actgaaccgt ccgagggcag cgcaccaggt 3540
   agccctgctg gctctccaac ctccaccgaa gaaggtacct ctgaaagcgc aacccctgaa 3600
   tccggcccag gtagcgaacc ggcaacctcc ggttctgaaa ccccaggtac ttctgaaagc 3660
   gctactcctg agtccggccc aggtagcccg gctggctctc cgacttccac cgaggaaggt 3720
   agcccggctg gctctccaac ttctactgaa gaaggtactt ctaccgaacc ttccgagggc 3780
   agcgcaccag gtacttctga aagcgctacc cctgagtccg gcccaggtac ttctgaaagc 3840
   gctactcctg aatccggtcc aggtacttct gaaagcgcta ccccggaatc tggcccaggt 3900
   agcgaaccgg ctacttctgg ttctgaaacc ccaggtagcg aaccggctac ctccggttct 3960
   gaaactccag gtagcccagc aggctctccg acttccactg aggaaggtac ttctactgaa 4020
   ccttccgaag gcagcgcacc aggtacctct actgaacctt ctgagggcag cgctccaggt 4080
   agcgaacctg caacctctgg ctctgaaacc ccaggtacct ctgaaagcgc tactcctgaa 4140
   tctggcccag gtacttctac tgaaccgtcc gagggcagcg cacca 4185
<210> 13
   <211> 1067
   <212> PRT
   <213> Artificial Sequence
<220>
   <221> source
   <223> /note="Description of Artificial Sequence: Synthetic polypeptide"
<400> 13
   Gly Thr Ser Thr Glu Pro Ser Glu Gly Ser Ala Pro Gly Ser Glu Pro
   1               5                   10                  15
   Ala Thr Ser Gly Ser Glu Thr Pro Gly Ser Pro Ala Gly Ser Pro Thr
               20                  25                  30
   Ser Thr Glu Glu Gly Ser Thr Ser Ser Thr Ala Glu Ser Pro Gly Pro
           35                  40                  45
   Gly Thr Ser Thr Pro Glu Ser Gly Ser Ala Ser Pro Gly Ser Thr Ser
       50                  55                  60
   Glu Ser Pro Ser Gly Thr Ala Pro Gly Ser Thr Ser Glu Ser Pro Ser
   65                  70                  75                  80
   Gly Thr Ala Pro Gly Thr Ser Thr Pro Glu Ser Gly Ser Ala Ser Pro
                   85                  90                  95
   Gly Thr Ser Thr Pro Glu Ser Gly Ser Ala Ser Pro Gly Ser Glu Pro
               100                 105                 110
   Ala Thr Ser Gly Ser Glu Thr Pro Gly Thr Ser Glu Ser Ala Thr Pro
           115                 120                 125
   Glu Ser Gly Pro Gly Ser Pro Ala Gly Ser Pro Thr Ser Thr Glu Glu
       130                 135                 140
   Gly Thr Ser Thr Glu Pro Ser Glu Gly Ser Ala Pro Gly Thr Ser Glu
   145                 150                 155                 160
   Ser Ala Thr Pro Glu Ser Gly Pro Gly Thr Ser Thr Glu Pro Ser Glu
                   165                 170                 175
   Gly Ser Ala Pro Gly Thr Ser Thr Glu Pro Ser Glu Gly Ser Ala Pro
               180                 185                 190
   Gly Ser Pro Ala Gly Ser Pro Thr Ser Thr Glu Glu Gly Thr Ser Thr
           195                 200                 205
   Glu Pro Ser Glu Gly Ser Ala Pro Gly Thr Ser Thr Glu Pro Ser Glu
       210                 215                 220
   Gly Ser Ala Pro Gly Thr Ser Glu Ser Ala Thr Pro Glu Ser Gly Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Thr Glu Pro Ser Glu
   260 265 270
   Gly Ser Ala Pro Gly Thr Ser Glu Ser Ala Thr Pro Glu Ser Gly Pro
   275 280 285
   Gly Thr Ser Thr Glu Pro Ser Glu Gly Ser Ala Pro Gly Ser Glu Pro
   290 295 300
   Ala Thr Ser Gly Ser Glu Thr Pro Gly Ser Pro Ala Gly Ser Pro Thr
   305 310 315 320
   Ser Thr Glu Glu Gly Ser Ser Thr Pro Ser Gly Ala Thr Gly Ser Pro
   325 330 335
   Gly Thr Pro Gly Ser Gly Thr Ala Ser Ser Ser Pro Gly Ser Ser Thr
   340 345 350
   Pro Ser Gly Ala Thr Gly Ser Pro Gly Thr Ser Thr Glu Pro Ser Glu
   355 360 365
   Gly Ser Ala Pro Gly Thr Ser Thr Glu Pro Ser Glu Gly Ser Ala Pro
   370 375 380
   Gly Ser Glu Pro Ala Thr Ser Gly Ser Glu Thr Pro Gly Ser Pro Ala
   385 390 395 400
   Gly Ser Pro Thr Ser Thr Glu Glu Gly Ser Pro Ala Gly Ser Pro Thr
   405 410 415
   Ser Thr Glu Glu Gly Thr Ser Thr Glu Pro Ser Glu Gly Ser Ala Pro
   420 425 430
   Gly Ala Ser Ala Ser Gly Ala Pro Ser Thr Gly Gly Thr Ser Glu Ser
   435 440 445
   Ala Thr Pro Glu Ser Gly Pro Gly Ser Pro Ala Gly Ser Pro Thr Ser
   450 455 460
   Thr Glu Glu Gly Ser Pro Ala Gly Ser Pro Thr Ser Thr Glu Glu Gly
   465 470 475 480
   Ser Thr Ser Ser Thr Ala Glu Ser Pro Gly Pro Gly Ser Thr Ser Glu
   485 490 495
   Ser Pro Ser Gly Thr Ala Pro Gly Thr Ser Pro Ser Gly Glu Ser Ser
   500 505 510
   Thr Ala Pro Gly Thr Pro Gly Ser Gly Thr Ala Ser Ser Ser Pro Gly
   515 520 525
   Ser Ser Thr Pro Ser Gly Ala Thr Gly Ser Pro Gly Ser Ser Pro Ser
   530 535 540
   Ala Ser Thr Gly Thr Gly Pro Gly Ser Glu Pro Ala Thr Ser Gly Ser
   545 550 555 560
   Glu Thr Pro Gly Thr Ser Glu Ser Ala Thr Pro Glu Ser Gly Pro Gly
   565 570 575
   Ser Glu Pro Ala Thr Ser Gly Ser Glu Thr Pro Gly Ser Thr Ser Ser
   580 585 590
   Thr Ala Glu Ser Pro Gly Pro Gly Ser Thr Ser Ser Thr Ala Glu Ser
   595 600 605
   Pro Gly Pro Gly Thr Ser Pro Ser Gly Glu Ser Ser Thr Ala Pro Gly
   610 615 620
   Ser Glu Pro Ala Thr Ser Gly Ser Glu Thr Pro Gly Ser Glu Pro Ala
   625 630 635 640
   Thr Ser Gly Ser Glu Thr Pro Gly Thr Ser Thr Glu Pro Ser Glu Gly
   645 650 655
   Ser Ala Pro Gly Ser Thr Ser Ser Thr Ala Glu Ser Pro Gly Pro Gly
   660 665 670
   Thr Ser Thr Pro Glu Ser Gly Ser Ala Ser Pro Gly Ser Thr Ser Glu
   675 680 685
   Ser Pro Ser Gly Thr Ala Pro Gly Thr Ser Thr Glu Pro Ser Glu Gly
   690 695 700
   Ser Ala Pro Gly Thr Ser Thr Glu Pro Ser Glu Gly Ser Ala Pro Gly
   705 710 715 720
   Thr Ser Thr Glu Pro Ser Glu Gly Ser Ala Pro Gly Ser Ser Thr Pro
   725 730 735
   Ser Gly Ala Thr Gly Ser Pro Gly Ser Ser Pro Ser Ala Ser Thr Gly
   740 745 750
   Thr Gly Pro Gly Ala Ser Pro Gly Thr Ser Ser Thr Gly Ser Pro Gly
   755 760 765
   Ser Glu Pro Ala Thr Ser Gly Ser Glu Thr Pro Gly Thr Ser Glu Ser
   770 775 780
   Ala Thr Pro Glu Ser Gly Pro Gly Ser Pro Ala Gly Ser Pro Thr Ser
   785 790 795 800
   Thr Glu Glu Gly Ser Ser Thr Pro Ser Gly Ala Thr Gly Ser Pro Gly
   805 810 815
   Ser Ser Pro Ser Ala Ser Thr Gly Thr Gly Pro Gly Ala Ser Pro Gly
   820 825 830
   Thr Ser Ser Thr Gly Ser Pro Gly Thr Ser Glu Ser Ala Thr Pro Glu
   835 840 845
   Ser Gly Pro Gly Thr Ser Thr Glu Pro Ser Glu Gly Ser Ala Pro Gly
   850 855 860
   Thr Ser Thr Glu Pro Ser Glu Gly Ser Ala Pro Gly Phe Pro Thr Ile
   865 870 875 880
   Pro Leu Ser Arg Leu Phe Asp Asn Ala Met Leu Arg Ala His Arg Leu
   885 890 895
   His Gln Leu Ala Phe Asp Thr Tyr Gln Glu Phe Glu Glu Ala Tyr Ile
   900 905 910
   Pro Lys Glu Gln Lys Tyr Ser Phe Leu Gln Asn Pro Gln Thr Ser Leu
   915 920 925
   Cys Phe Ser Glu Ser Ile Pro Thr Pro Ser Asn Arg Glu Glu Thr Gln
   930 935 940
   Gln Lys Ser Asn Leu Glu Leu Leu Arg Ile Ser Leu Leu Leu Ile Gln
   945 950 955 960
   Ser Trp Leu Glu Pro Val Gln Phe Leu Arg Ser Val Phe Ala Asn Ser
   965 970 975
   Leu Val Tyr Gly Ala Ser Asp Ser Asn Val Tyr Asp Leu Leu Lys Asp
   980 985 990
   Leu Glu Glu Gly Ile Gln Thr Leu Met Gly Arg Leu Glu Asp Gly Ser
   995 1000 1005
   Pro Arg Thr Gly Gln Ile Phe Lys Gln Thr Tyr Ser Lys Phe Asp
           1010                1015                1020
   Thr Asn  Ser His Asn Asp Asp  Ala Leu Leu Lys Asn  Tyr Gly Leu
       1025                1030                1035
   Leu Tyr  Cys Phe Arg Lys Asp  Met Asp Lys Val Glu  Thr Phe Leu
       1040                1045                1050
   Arg Ile  Val Gln Cys Arg Ser  Val Glu Gly Ser Cys  Gly Phe
       1055                1060                1065
<210> 14
   <211> 3204
   <212> DNA
   <213> Artificial Sequence
<220>
   <221> source
   <223> /note="Description of Artificial Sequence: Synthetic polynucleotide"
<400> 14
   ggtacttcta ctgaaccgtc tgaaggcagc gcaccaggta gcgaaccggc tacttccggt 60
   tctgaaaccc caggtagccc agcaggttct ccaacttcta ctgaagaagg ttctaccagc 120
   tctaccgcag aatctcctgg tccaggtacc tctactccgg aaagcggctc tgcatctcca 180
   ggttctacta gcgaatctcc ttctggcact gcaccaggtt ctactagcga atccccgtct 240
   ggtactgctc caggtacttc tactcctgaa agcggttccg cttctccagg tacctctact 300
   ccggaaagcg gttctgcatc tccaggtagc gaaccggcaa cctccggctc tgaaacccca 360
   ggtacctctg aaagcgctac tcctgaatcc ggcccaggta gcccggcagg ttctccgact 420
   tccactgagg aaggtacctc tactgaacct tctgagggca gcgctccagg tacttctgaa 480
   agcgctaccc cggagtccgg tccaggtact tctactgaac cgtccgaagg tagcgcacca 540
   ggtacttcta ccgaaccgtc cgagggtagc gcaccaggta gcccagcagg ttctcctacc 600
   tccaccgagg aaggtacttc taccgaaccg tccgagggta gcgcaccagg tacttctacc 660
   gaaccttccg agggcagcgc accaggtact tctgaaagcg ctacccctga gtccggccca 720
   ggtacttctg aaagcgctac tcctgaatcc ggtccaggta cctctactga accttccgaa 780
   ggcagcgctc caggtacctc taccgaaccg tccgagggca gcgcaccagg tacttctgaa 840
   agcgcaaccc ctgaatccgg tccaggtact tctactgaac cttccgaagg tagcgctcca 900
   ggtagcgaac ctgctacttc tggttctgaa accccaggta gcccggctgg ctctccgacc 960
   tccaccgagg aaggtagctc taccccgtct ggtgctactg gttctccagg tactccgggc 1020
   agcggtactg cttcttcctc tccaggtagc tctacccctt ctggtgctac tggctctcca 1080
   ggtacctcta ccgaaccgtc cgagggtagc gcaccaggta cctctactga accgtctgag 1140
   ggtagcgctc caggtagcga accggcaacc tccggttctg aaactccagg tagccctgct 1200
   ggctctccga cttctactga ggaaggtagc ccggctggtt ctccgacttc tactgaggaa 1260
   ggtacttcta ccgaaccttc cgaaggtagc gctccaggtg caagcgcaag cggcgcgcca 1320
   agcacgggag gtacttctga aagcgctact cctgagtccg gcccaggtag cccggctggc 1380
   tctccgactt ccaccgagga aggtagcccg gctggctctc caacttctac tgaagaaggt 1440
   tctaccagct ctaccgctga atctcctggc ccaggttcta ctagcgaatc tccgtctggc 1500
   accgcaccag gtacttcccc tagcggtgaa tcttctactg caccaggtac ccctggcagc 1560
   ggtaccgctt cttcctctcc aggtagctct accccgtctg gtgctactgg ctctccaggt 1620
   tctagcccgt ctgcatctac cggtaccggc ccaggtagcg aaccggcaac ctccggctct 1680
   gaaactccag gtacttctga aagcgctact ccggaatccg gcccaggtag cgaaccggct 1740
   acttccggct ctgaaacccc aggttccacc agctctactg cagaatctcc gggcccaggt 1800
   tctactagct ctactgcaga atctccgggt ccaggtactt ctcctagcgg cgaatcttct 1860
   accgctccag gtagcgaacc ggcaacctct ggctctgaaa ctccaggtag cgaacctgca 1920
   acctccggct ctgaaacccc aggtacttct actgaacctt ctgagggcag cgcaccaggt 1980
   tctaccagct ctaccgcaga atctcctggt ccaggtacct ctactccgga aagcggctct 2040
   gcatctccag gttctactag cgaatctcct tctggcactg caccaggtac ttctaccgaa 2100
   ccgtccgaag gcagcgctcc aggtacctct actgaacctt ccgagggcag cgctccaggt 2160
   acctctaccg aaccttctga aggtagcgca ccaggtagct ctactccgtc tggtgcaacc 2220
   ggctccccag gttctagccc gtctgcttcc actggtactg gcccaggtgc ttccccgggc 2280
   accagctcta ctggttctcc aggtagcgaa cctgctacct ccggttctga aaccccaggt 2340
   acctctgaaa gcgcaactcc ggagtctggt ccaggtagcc ctgcaggttc tcctacctcc 2400
   actgaggaag gtagctctac tccgtctggt gcaaccggct ccccaggttc tagcccgtct 2460
   gcttccactg gtactggccc aggtgcttcc ccgggcacca gctctactgg ttctccaggt 2520
   acctctgaaa gcgctactcc ggagtctggc ccaggtacct ctactgaacc gtctgagggt 2580
   agcgctccag gtacttctac tgaaccgtcc gaaggtagcg caccaggttt tccgactatt 2640
   ccgctgtctc gtctgtttga taatgctatg ctgcgtgcgc accgtctgca ccagctggcc 2700
   tttgatactt accaggaatt tgaagaagcc tacattccta aagagcagaa gtactctttc 2760
   ctgcaaaacc cacagacttc tctctgcttc agcgaatcta ttccgacgcc ttccaatcgc 2820
   gaggaaactc agcaaaagtc caatctggaa ctactccgca tttctctgct tctgattcag 2880
   agctggctag aaccagtgca atttctgcgt tccgtcttcg ccaatagcct agtttatggc 2940
   gcatccgaca gcaacgtata cgatctcctg aaagatctcg aggaaggcat tcagaccctg 3000
   atgggtcgtc tcgaggatgg ctctccgcgt actggtcaga tcttcaagca gacttactct 3060
   aaatttgata ctaacagcca caatgacgat gcgcttctaa aaaactatgg tctgctgtat 3120
   tgttttcgta aagatatgga caaagttgaa accttcctgc gtattgttca gtgtcgttcc 3180
   gttgagggca gctgtggttt ctaa 3204
<210> 15
   <211> 12
   <212> PRT
   <213> Artificial Sequence
<220>
   <221> source
   <223> /note="Description of Artificial Sequence: Synthetic peptide"
<400> 15
   Gly Glu Ser Pro Gly Gly Ser Ser Gly Ser Glu Ser
   1 5 10
<210> 16
   <211> 12
   <212> PRT
   <213> Artificial Sequence
<220>
   <221> source
   <223> /note="Description of Artificial Sequence: Synthetic peptide"
<400> 16
   Gly Ser Glu Gly Ser Ser Gly Pro Gly Glu Ser Ser
   1 5 10
<210> 17
   <211> 12
   <212> PRT
   <213> Artificial Sequence
<220>
   <221> source
   <223> /note="Description of Artificial Sequence: Synthetic peptide"
<400> 17
   Gly Ser Ser Glu Ser Gly Ser Ser Glu Gly Gly Pro
   1 5 10
<210> 18
   <211> 12
   <212> PRT
   <213> Artificial Sequence
<220>
   <221> source
   <223> /note="Description of Artificial Sequence: Synthetic peptide"
<400> 18
   Gly Ser Gly Gly Glu Pro Ser Glu Ser Gly Ser Ser
   1               5                   10
<210> 19
   <211> 12
   <212> PRT
   <213> Artificial Sequence
<220>
   <221> source
   <223> /note="Description of Artificial Sequence: Synthetic peptide"
<400> 19
   Gly Ser Pro Ala Gly Ser Pro Thr Ser Thr Glu Glu
   1               5                   10
<210> 20
   <211> 12
   <212> PRT
   <213> Artificial Sequence
<220>
   <221> source
   <223> /note="Description of Artificial Sequence: Synthetic peptide"
<400> 20
   Gly Ser Glu Pro Ala Thr Ser Gly Ser Glu Thr Pro
   1               5                   10
<210> 21
   <211> 12
   <212> PRT
   <213> Artificial Sequence
<220>
   <221> source
   <223> /note="Description of Artificial Sequence: Synthetic peptide"
<400> 21
   Gly Thr Ser Glu Ser Ala Thr Pro Glu Ser Gly Pro
   1               5                   10
<210> 22
   <211> 12
   <212> PRT
   <213> Artificial Sequence
<220>
   <221> source
   <223> /note="Description of Artificial Sequence: Synthetic peptide"
<400> 22
   Gly Thr Ser Thr Glu Pro Ser Glu Gly Ser Ala Pro
       1               5                   10
<210> 23
   <211> 12
   <212> PRT
   <213> Artificial Sequence
<220>
   <221> source
   <223> /note="Description of Artificial Sequence: Synthetic peptide"
<400> 23
   Gly Ser Thr Ser Glu Ser Pro Ser Gly Thr Ala Pro
   1               5                   10
<210> 24
   <211> 12
   <212> PRT
   <213> Artificial Sequence
<220>
   <221> source
   <223> /note="Description of Artificial Sequence: Synthetic peptide"
<400> 24
   Gly Thr Ser Thr Pro Glu Ser Gly Ser Ala Ser Pro
   1               5                   10
<210> 25
   <211> 12
   <212> PRT
   <213> Artificial Sequence
<220>
   <221> source
   <223> /note="Description of Artificial Sequence: Synthetic peptide"
<400> 25
   Gly Thr Ser Pro Ser Gly Glu Ser Ser Thr Ala Pro
   1               5                   10
<210> 26
   <211> 12
   <212> PRT
   <213> Artificial Sequence
<220>
   <221> source
   <223> /note="Description of Artificial Sequence: Synthetic peptide"
<400> 26
   Gly Ser Thr Ser Ser Thr Ala Glu Ser Pro Gly Pro
   1               5                   10
<210> 27
   <211> 12
   <212> PRT
   <213> Artificial Sequence
<220>
   <221> source
   <223> /note="Description of Artificial Sequence: Synthetic peptide"
<400> 27
   Gly Thr Pro Gly Ser Gly Thr Ala Ser Ser Ser Pro
   1               5                   10
<210> 28
   <211> 12
   <212> PRT
   <213> Artificial Sequence
<220>
   <221> source
   <223> /note="Description of Artificial Sequence: Synthetic peptide"
<400> 28
   Gly Ser Ser Thr Pro Ser Gly Ala Thr Gly Ser Pro
   1               5                   10
<210> 29
   <211> 12
   <212> PRT
   <213> Artificial Sequence
<220>
   <221> source
   <223> /note="Description of Artificial Sequence: Synthetic peptide"
<400> 29
   Gly Ser Ser Pro Ser Ala Ser Thr Gly Thr Gly Pro
   1               5                   10
<210> 30
   <211> 12
   <212> PRT
   <213> Artificial Sequence
<220>
   <221> source
   <223> /note="Description of Artificial Sequence: Synthetic peptide"
<400> 30
   Gly Ala Ser Pro Gly Thr Ser Ser Thr Gly Ser Pro
   1               5                   10
<210> 31
   <211> 48
   <212> PRT
   <213> Artificial Sequence
<220>
   <221> source
   <223> /note="Description of Artificial Sequence: Synthetic polypeptide"
<400> 31
   Met Ala Glu Pro Ala Gly Ser Pro Thr Ser Thr Glu Glu Gly Thr Pro
   1               5                   10                  15
   Gly Ser Gly Thr Ala Ser Ser Ser Pro Gly Ser Ser Thr Pro Ser Gly
               20                  25                  30
   Ala Thr Gly Ser Pro Gly Ala Ser Pro Gly Thr Ser Ser Thr Gly Ser
           35                  40                  45
<210> 32
   <211> 48
   <212> PRT
   <213> Artificial Sequence
<220>
   <221> source
   <223> /note="Description of Artificial Sequence: Synthetic polypeptide"
<400> 32
   Met Ala Glu Pro Ala Gly Ser Pro Thr Ser Thr Glu Glu Gly Ala Ser
   1               5                   10                  15
   Pro Gly Thr Ser Ser Thr Gly Ser Pro Gly Ser Ser Thr Pro Ser Gly
               20                  25                  30
   Ala Thr Gly Ser Pro Gly Ser Ser Thr Pro Ser Gly Ala Thr Gly Ser
           35                  40                  45
<210> 33
   <211> 144
   <212> PRT
   <213> Artificial Sequence
<220>
   <221> source
   <223> /note="Description of Artificial Sequence: Synthetic polypeptide"
<400> 33
   Gly Ser Glu Pro Ala Thr Ser Gly Ser Glu Thr Pro Gly Thr Ser Glu
   1               5                   10                  15
   Ser Ala Thr Pro Glu Ser Gly Pro Gly Ser Glu Pro Ala Thr Ser Gly
               20                  25                  30
   Ser Glu Thr Pro Gly Ser Pro Ala Gly Ser Pro Thr Ser Thr Glu Glu
           35                  40                  45
   Gly Thr Ser Thr Glu Pro Ser Glu Gly Ser Ala Pro Gly Ser Glu Pro
       50                  55                  60
   Ala Thr Ser Gly Ser Glu Thr Pro Gly Ser Glu Pro Ala Thr Ser Gly
   65                  70                  75                  80
   Ser Glu Thr Pro Gly Ser Glu Pro Ala Thr Ser Gly Ser Glu Thr Pro
                   85                  90                  95
   Gly Thr Ser Thr Glu Pro Ser Glu Gly Ser Ala Pro Gly Thr Ser Glu
               100                 105                 110
   Ser Ala Thr Pro Glu Ser Gly Pro Gly Ser Glu Pro Ala Thr Ser Gly
           115                 120                 125
   Ser Glu Thr Pro Gly Thr Ser Thr Glu Pro Ser Glu Gly Ser Ala Pro
       130                 135                 140
<210> 34
   <211> 144
   <212> PRT
   <213> Artificial Sequence
<220>
   <221> source
   <223> /note="Description of Artificial Sequence: Synthetic polypeptide"
<400> 34
   Gly Thr Ser Thr Pro Glu Ser Gly Ser Ala Ser Pro Gly Thr Ser Pro
   1               5                   10                  15
   Ser Gly Glu Ser Ser Thr Ala Pro Gly Thr Ser Pro Ser Gly Glu Ser
               20                  25                  30
   Ser Thr Ala Pro Gly Ser Thr Ser Ser Thr Ala Glu Ser Pro Gly Pro
           35                  40                  45
   Gly Ser Thr Ser Glu Ser Pro Ser Gly Thr Ala Pro Gly Ser Thr Ser
       50                  55                  60
   Ser Thr Ala Glu Ser Pro Gly Pro Gly Thr Ser Pro Ser Gly Glu Ser
   65                  70                  75                  80
   Ser Thr Ala Pro Gly Thr Ser Thr Pro Glu Ser Gly Ser Ala Ser Pro
                   85                  90                  95
   Gly Ser Thr Ser Ser Thr Ala Glu Ser Pro Gly Pro Gly Thr Ser Pro
               100                 105                 110
   Ser Gly Glu Ser Ser Thr Ala Pro Gly Thr Ser Pro Ser Gly Glu Ser
           115                 120                 125
   Ser Thr Ala Pro Gly Thr Ser Pro Ser Gly Glu Ser Ser Thr Ala Pro
       130                 135                 140
<210> 35
   <211> 288
   <212> PRT
   <213> Artificial Sequence
<220>
   <221> source
   <223> /note="Description of Artificial Sequence: Synthetic polypeptide"
<400> 35
   Gly Thr Ser Glu Ser Ala Thr Pro Glu Ser Gly Pro Gly Ser Glu Pro
   1               5                   10                  15
   Ala Thr Ser Gly Ser Glu Thr Pro Gly Thr Ser Glu Ser Ala Thr Pro
               20                  25                  30
   Glu Ser Gly Pro Gly Ser Glu Pro Ala Thr Ser Gly Ser Glu Thr Pro
           35                  40                  45
   Gly Thr Ser Glu Ser Ala Thr Pro Glu Ser Gly Pro Gly Thr Ser Thr
       50                  55                  60
   Glu Pro Ser Glu Gly Ser Ala Pro Gly Ser Pro Ala Gly Ser Pro Thr
   65                  70                  75                  80
   Ser Thr Glu Glu Gly Thr Ser Glu Ser Ala Thr Pro Glu Ser Gly Pro
                   85                  90                  95
   Gly Ser Glu Pro Ala Thr Ser Gly Ser Glu Thr Pro Gly Thr Ser Glu
               100                 105                 110
   Ser Ala Thr Pro Glu Ser Gly Pro Gly Ser Pro Ala Gly Ser Pro Thr
           115                 120                 125
   Ser Thr Glu Glu Gly Ser Pro Ala Gly Ser Pro Thr Ser Thr Glu Glu
       130                 135                 140
   Gly Thr Ser Thr Glu Pro Ser Glu Gly Ser Ala Pro Gly Thr Ser Glu
   145                 150                 155                 160
   Ser Ala Thr Pro Glu Ser Gly Pro Gly Thr Ser Glu Ser Ala Thr Pro
                   165                 170                 175
   Glu Ser Gly Pro Gly Thr Ser Glu Ser Ala Thr Pro Glu Ser Gly Pro
               180                 185                 190
   Gly Ser Glu Pro Ala Thr Ser Gly Ser Glu Thr Pro Gly Ser Glu Pro
           195                 200                 205
   Ala Thr Ser Gly Ser Glu Thr Pro Gly Ser Pro Ala Gly Ser Pro Thr
       210                 215                 220
   Ser Thr Glu Glu Gly Thr Ser Thr Glu Pro Ser Glu Gly Ser Ala Pro
   225                 230                 235                 240
   Gly Thr Ser Thr Glu Pro Ser Glu Gly Ser Ala Pro Gly Ser Glu Pro
                   245                 250                 255
   Ala Thr Ser Gly Ser Glu Thr Pro Gly Thr Ser Glu Ser Ala Thr Pro
               260                 265                 270
   Glu Ser Gly Pro Gly Thr Ser Thr Glu Pro Ser Glu Gly Ser Ala Pro
           275                 280                 285
<210> 36
   <211> 504
   <212> PRT
   <213> Artificial Sequence
<220>
   <221> source
   <223> /note="Description of Artificial Sequence: Synthetic polypeptide"
<220>
   <221> MOD_RES
   <222> (63)..(63)
   <223> Any amino acid
<220>
   <221> MOD_RES
   <222> (207)..(207)
   <223> Any amino acid
<400> 36
   Gly Ala Ser Pro Gly Thr Ser Ser Thr Gly Ser Pro Gly Ser Ser Pro
   1               5                   10                  15
   Ser Ala Ser Thr Gly Thr Gly Pro Gly Ser Ser Pro Ser Ala Ser Thr
               20                  25                  30
   Gly Thr Gly Pro Gly Thr Pro Gly Ser Gly Thr Ala Ser Ser Ser Pro
           35                  40                  45
   Gly Ser Ser Thr Pro Ser Gly Ala Thr Gly Ser Pro Gly Ser Xaa Pro
   50 55 60
   Ser Ala Ser Thr Gly Thr Gly Pro Gly Ala Ser Pro Gly Thr Ser Ser
   65 70 75 80
   Thr Gly Ser Pro Gly Thr Pro Gly Ser Gly Thr Ala Ser Ser Ser Pro
   85 90 95
   Gly Ser Ser Thr Pro Ser Gly Ala Thr Gly Ser Pro Gly Thr Pro Gly
   100 105 110
   Ser Gly Thr Ala Ser Ser Ser Pro Gly Ala Ser Pro Gly Thr Ser Ser
   115 120 125
   Thr Gly Ser Pro Gly Ala Ser Pro Gly Thr Ser Ser Thr Gly Ser Pro
   130 135 140
   Gly Thr Pro Gly Ser Gly Thr Ala Ser Ser Ser Pro Gly Ser Ser Thr
   145 150 155 160
   Pro Ser Gly Ala Thr Gly Ser Pro Gly Ala Ser Pro Gly Thr Ser Ser
   165 170 175
   Thr Gly Ser Pro Gly Thr Pro Gly Ser Gly Thr Ala Ser Ser Ser Pro
   180 185 190
   Gly Ser Ser Thr Pro Ser Gly Ala Thr Gly Ser Pro Gly Ser Xaa Pro
   195 200 205
   Ser Ala Ser Thr Gly Thr Gly Pro Gly Ser Ser Pro Ser Ala Ser Thr
   210 215 220
   Gly Thr Gly Pro Gly Ser Ser Thr Pro Ser Gly Ala Thr Gly Ser Pro
   225 230 235 240
   Gly Ser Ser Thr Pro Ser Gly Ala Thr Gly Ser Pro Gly Ala Ser Pro
   245 250 255
   Gly Thr Ser Ser Thr Gly Ser Pro Gly Ala Ser Pro Gly Thr Ser Ser
   260 265 270
   Thr Gly Ser Pro Gly Ala Ser Pro Gly Thr Ser Ser Thr Gly Ser Pro
   275 280 285
   Gly Thr Pro Gly Ser Gly Thr Ala Ser Ser Ser Pro Gly Ala Ser Pro
   290 295 300
   Gly Thr Ser Ser Thr Gly Ser Pro Gly Ala Ser Pro Gly Thr Ser Ser
   305 310 315 320
   Thr Gly Ser Pro Gly Ala Ser Pro Gly Thr Ser Ser Thr Gly Ser Pro
   325 330 335
   Gly Ser Ser Pro Ser Ala Ser Thr Gly Thr Gly Pro Gly Thr Pro Gly
   340 345 350
   Ser Gly Thr Ala Ser Ser Ser Pro Gly Ala Ser Pro Gly Thr Ser Ser
   355 360 365
   Thr Gly Ser Pro Gly Ala Ser Pro Gly Thr Ser Ser Thr Gly Ser Pro
   370 375 380
   Gly Ala Ser Pro Gly Thr Ser Ser Thr Gly Ser Pro Gly Ser Ser Thr
   385 390 395 400
   Pro Ser Gly Ala Thr Gly Ser Pro Gly Ser Ser Thr Pro Ser Gly Ala
   405 410 415
   Thr Gly Ser Pro Gly Ala Ser Pro Gly Thr Ser Ser Thr Gly Ser Pro
   420 425 430
   Gly Thr Pro Gly Ser Gly Thr Ala Ser Ser Ser Pro Gly Ser Ser Thr
   435 440 445
   Pro Ser Gly Ala Thr Gly Ser Pro Gly Ser Ser Thr Pro Ser Gly Ala
   450 455 460
   Thr Gly Ser Pro Gly Ser Ser Thr Pro Ser Gly Ala Thr Gly Ser Pro
   465 470 475 480
   Gly Ser Ser Pro Ser Ala Ser Thr Gly Thr Gly Pro Gly Ala Ser Pro
   485 490 495
   Gly Thr Ser Ser Thr Gly Ser Pro
   500
<210> 37
   <211> 540
   <212> PRT
   <213> Artificial Sequence
<220>
   <221> source
   <223> /note="Description of Artificial Sequence: Synthetic polypeptide"
<400> 37
   Gly Ser Thr Ser Ser Thr Ala Glu Ser Pro Gly Pro Gly Ser Thr Ser
   1               5                   10                  15
   Ser Thr Ala Glu Ser Pro Gly Pro Gly Ser Thr Ser Glu Ser Pro Ser
               20                  25                  30
   Gly Thr Ala Pro Gly Ser Thr Ser Ser Thr Ala Glu Ser Pro Gly Pro
           35                  40                  45
   Gly Ser Thr Ser Ser Thr Ala Glu Ser Pro Gly Pro Gly Thr Ser Thr
       50                  55                  60
   Pro Glu Ser Gly Ser Ala Ser Pro Gly Ser Thr Ser Glu Ser Pro Ser
   65                  70                  75                  80
   Gly Thr Ala Pro Gly Thr Ser Pro Ser Gly Glu Ser Ser Thr Ala Pro
                   85                  90                  95
   Gly Ser Thr Ser Glu Ser Pro Ser Gly Thr Ala Pro Gly Ser Thr Ser
               100                 105                 110
   Glu Ser Pro Ser Gly Thr Ala Pro Gly Thr Ser Pro Ser Gly Glu Ser
           115                 120                 125
   Ser Thr Ala Pro Gly Ser Thr Ser Glu Ser Pro Ser Gly Thr Ala Pro
       130                 135                 140
   Gly Ser Thr Ser Glu Ser Pro Ser Gly Thr Ala Pro Gly Thr Ser Pro
   145                 150                 155                 160
   Ser Gly Glu Ser Ser Thr Ala Pro Gly Ser Thr Ser Glu Ser Pro Ser
                   165                 170                 175
   Gly Thr Ala Pro Gly Ser Thr Ser Glu Ser Pro Ser Gly Thr Ala Pro
               180                 185                 190
   Gly Ser Thr Ser Glu Ser Pro Ser Gly Thr Ala Pro Gly Thr Ser Thr
           195                 200                 205
   Pro Glu Ser Gly Ser Ala Ser Pro Gly Ser Thr Ser Glu Ser Pro Ser
       210                 215                 220
   Gly Thr Ala Pro Gly Thr Ser Thr Pro Glu Ser Gly Ser Ala Ser Pro
   225                 230                 235                 240
   Gly Ser Thr Ser Ser Thr Ala Glu Ser Pro Gly Pro Gly Ser Thr Ser
   245 250 255
   Ser Thr Ala Glu Ser Pro Gly Pro Gly Thr Ser Thr Pro Glu Ser Gly
   260 265 270
   Ser Ala Ser Pro Gly Thr Ser Thr Pro Glu Ser Gly Ser Ala Ser Pro
   275 280 285
   Gly Ser Thr Ser Glu Ser Pro Ser Gly Thr Ala Pro Gly Thr Ser Thr
   290 295 300
   Pro Glu Ser Gly Ser Ala Ser Pro Gly Thr Ser Thr Pro Glu Ser Gly
   305 310 315 320
   Ser Ala Ser Pro Gly Ser Thr Ser Glu Ser Pro Ser Gly Thr Ala Pro
   325 330 335
   Gly Ser Thr Ser Glu Ser Pro Ser Gly Thr Ala Pro Gly Ser Thr Ser
   340 345 350
   Glu Ser Pro Ser Gly Thr Ala Pro Gly Ser Thr Ser Ser Thr Ala Glu
   355 360 365
   Ser Pro Gly Pro Gly Thr Ser Thr Pro Glu Ser Gly Ser Ala Ser Pro
   370 375 380
   Gly Thr Ser Thr Pro Glu Ser Gly Ser Ala Ser Pro Gly Ser Thr Ser
   385 390 395 400
   Glu Ser Pro Ser Gly Thr Ala Pro Gly Ser Thr Ser Glu Ser Pro Ser
   405 410 415
   Gly Thr Ala Pro Gly Thr Ser Thr Pro Glu Ser Gly Ser Ala Ser Pro
   420 425 430
   Gly Ser Thr Ser Glu Ser Pro Ser Gly Thr Ala Pro Gly Ser Thr Ser
   435 440 445
   Glu Ser Pro Ser Gly Thr Ala Pro Gly Thr Ser Thr Pro Glu Ser Gly
   450 455 460
   Ser Ala Ser Pro Gly Thr Ser Pro Ser Gly Glu Ser Ser Thr Ala Pro
   465 470 475 480
   Gly Ser Thr Ser Ser Thr Ala Glu Ser Pro Gly Pro Gly Thr Ser Pro
   485 490 495
   Ser Gly Glu Ser Ser Thr Ala Pro Gly Ser Thr Ser Ser Thr Ala Glu
   500 505 510
   Ser Pro Gly Pro Gly Thr Ser Thr Pro Glu Ser Gly Ser Ala Ser Pro
   515 520 525
   Gly Ser Thr Ser Glu Ser Pro Ser Gly Thr Ala Pro
   530 535 540
<210> 38
   <211> 576
   <212> PRT
   <213> Artificial Sequence
<220>
   <221> source
   <223> /note="Description of Artificial Sequence: Synthetic polypeptide"
<400> 38
   Gly Ser Ser Glu Ser Gly Ser Ser Glu Gly Gly Pro Gly Ser Gly Gly
   1 5 10 15
   Glu Pro Ser Glu Ser Gly Ser Ser Gly Ser Ser Glu Ser Gly Ser Ser
   20 25 30
   Glu Gly Gly Pro Gly Ser Ser Glu Ser Gly Ser Ser Glu Gly Gly Pro
   35 40 45
   Gly Ser Ser Glu Ser Gly Ser Ser Glu Gly Gly Pro Gly Ser Ser Glu
   50 55 60
   Ser Gly Ser Ser Glu Gly Gly Pro Gly Ser Ser Glu Ser Gly Ser Ser
   65 70 75 80
   Glu Gly Gly Pro Gly Glu Ser Pro Gly Gly Ser Ser Gly Ser Glu Ser
   85 90 95
   Gly Ser Glu Gly Ser Ser Gly Pro Gly Glu Ser Ser Gly Ser Ser Glu
   100 105 110
   Ser Gly Ser Ser Glu Gly Gly Pro Gly Ser Ser Glu Ser Gly Ser Ser
   115 120 125
   Glu Gly Gly Pro Gly Ser Ser Glu Ser Gly Ser Ser Glu Gly Gly Pro
   130 135 140
   Gly Ser Gly Gly Glu Pro Ser Glu Ser Gly Ser Ser Gly Glu Ser Pro
   145 150 155 160
   Gly Gly Ser Ser Gly Ser Glu Ser Gly Glu Ser Pro Gly Gly Ser Ser
                   165                 170                 175
   Gly Ser Glu Ser Gly Ser Gly Gly Glu Pro Ser Glu Ser Gly Ser Ser
               180                 185                 190
   Gly Ser Ser Glu Ser Gly Ser Ser Glu Gly Gly Pro Gly Ser Gly Gly
           195                 200                 205
   Glu Pro Ser Glu Ser Gly Ser Ser Gly Ser Gly Gly Glu Pro Ser Glu
       210                 215                 220
   Ser Gly Ser Ser Gly Ser Glu Gly Ser Ser Gly Pro Gly Glu Ser Ser
   225                 230                 235                 240
   Gly Glu Ser Pro Gly Gly Ser Ser Gly Ser Glu Ser Gly Ser Gly Gly
                   245                 250                 255
   Glu Pro Ser Glu Ser Gly Ser Ser Gly Ser Gly Gly Glu Pro Ser Glu
               260                 265                 270
   Ser Gly Ser Ser Gly Ser Gly Gly Glu Pro Ser Glu Ser Gly Ser Ser
           275                 280                 285
   Gly Ser Ser Glu Ser Gly Ser Ser Glu Gly Gly Pro Gly Glu Ser Pro
       290                 295                 300
   Gly Gly Ser Ser Gly Ser Glu Ser Gly Glu Ser Pro Gly Gly Ser Ser
   305                 310                 315                 320
   Gly Ser Glu Ser Gly Glu Ser Pro Gly Gly Ser Ser Gly Ser Glu Ser
                   325                 330                 335
   Gly Glu Ser Pro Gly Gly Ser Ser Gly Ser Glu Ser Gly Glu Ser Pro
               340                 345                 350
   Gly Gly Ser Ser Gly Ser Glu Ser Gly Ser Ser Glu Ser Gly Ser Ser
           355                 360                 365
   Glu Gly Gly Pro Gly Ser Gly Gly Glu Pro Ser Glu Ser Gly Ser Ser
       370                 375                 380
   Gly Ser Glu Gly Ser Ser Gly Pro Gly Glu Ser Ser Gly Ser Ser Glu
   385                 390                 395                 400
   Ser Gly Ser Ser Glu Gly Gly Pro Gly Ser Gly Gly Glu Pro Ser Glu
                   405                 410                 415
   Ser Gly Ser Ser Gly Ser Ser Glu Ser Gly Ser Ser Glu Gly Gly Pro
   420 425 430
   Gly Ser Gly Gly Glu Pro Ser Glu Ser Gly Ser Ser Gly Glu Ser Pro
   435 440 445
   Gly Gly Ser Ser Gly Ser Glu Ser Gly Glu Ser Pro Gly Gly Ser Ser
   450 455 460
   Gly Ser Glu Ser Gly Ser Ser Glu Ser Gly Ser Ser Glu Gly Gly Pro
   465 470 475 480
   Gly Ser Gly Gly Glu Pro Ser Glu Ser Gly Ser Ser Gly Ser Ser Glu
   485 490 495
   Ser Gly Ser Ser Glu Gly Gly Pro Gly Ser Gly Gly Glu Pro Ser Glu
   500 505 510
   Ser Gly Ser Ser Gly Ser Gly Gly Glu Pro Ser Glu Ser Gly Ser Ser
   515 520 525
   Gly Glu Ser Pro Gly Gly Ser Ser Gly Ser Glu Ser Gly Ser Glu Gly
   530 535 540
   Ser Ser Gly Pro Gly Glu Ser Ser Gly Ser Ser Glu Ser Gly Ser Ser
   545 550 555 560
   Glu Gly Gly Pro Gly Ser Glu Gly Ser Ser Gly Pro Gly Glu Ser Ser
   565 570 575
<210> 39
   <211> 576
   <212> PRT
   <213> Artificial Sequence
<220>
   <221> source
   <223> /note="Description of Artificial Sequence: Synthetic polypeptide"
<400> 39
   Gly Ser Pro Ala Gly Ser Pro Thr Ser Thr Glu Glu Gly Thr Ser Glu
   1 5 10 15
   Ser Ala Thr Pro Glu Ser Gly Pro Gly Thr Ser Thr Glu Pro Ser Glu
   20 25 30
   Gly Ser Ala Pro Gly Ser Pro Ala Gly Ser Pro Thr Ser Thr Glu Glu
   35 40 45
   Gly Thr Ser Thr Glu Pro Ser Glu Gly Ser Ala Pro Gly Thr Ser Thr
       50                  55                  60
   Glu Pro Ser Glu Gly Ser Ala Pro Gly Thr Ser Glu Ser Ala Thr Pro
   65                  70                  75                  80
   Glu Ser Gly Pro Gly Ser Glu Pro Ala Thr Ser Gly Ser Glu Thr Pro
                   85                  90                  95
   Gly Ser Glu Pro Ala Thr Ser Gly Ser Glu Thr Pro Gly Ser Pro Ala
               100                 105                 110
   Gly Ser Pro Thr Ser Thr Glu Glu Gly Thr Ser Glu Ser Ala Thr Pro
           115                 120                 125
   Glu Ser Gly Pro Gly Thr Ser Thr Glu Pro Ser Glu Gly Ser Ala Pro
       130                 135                 140
   Gly Thr Ser Thr Glu Pro Ser Glu Gly Ser Ala Pro Gly Ser Pro Ala
   145                 150                 155                 160
   Gly Ser Pro Thr Ser Thr Glu Glu Gly Thr Ser Thr Glu Pro Ser Glu
                   165                 170                 175
   Gly Ser Ala Pro Gly Thr Ser Thr Glu Pro Ser Glu Gly Ser Ala Pro
               180                 185                 190
   Gly Thr Ser Glu Ser Ala Thr Pro Glu Ser Gly Pro Gly Thr Ser Thr
           195                 200                 205
   Glu Pro Ser Glu Gly Ser Ala Pro Gly Thr Ser Glu Ser Ala Thr Pro
       210                 215                 220
   Glu Ser Gly Pro Gly Ser Glu Pro Ala Thr Ser Gly Ser Glu Thr Pro
   225                 230                 235                 240
   Gly Thr Ser Thr Glu Pro Ser Glu Gly Ser Ala Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Thr Ser Glu Ser Ala Thr Pro Glu Ser Gly Pro
           275                 280                 285
   Gly Ser Pro Ala Gly Ser Pro Thr Ser Thr Glu Glu Gly Thr Ser Glu
   290 295 300
   Ser Ala Thr Pro Glu Ser Gly Pro Gly Ser Glu Pro Ala Thr Ser Gly
   305 310 315 320
   Ser Glu Thr Pro Gly Thr Ser Glu Ser Ala Thr Pro Glu Ser Gly Pro
   325 330 335
   Gly Thr Ser Thr Glu Pro Ser Glu Gly Ser Ala Pro Gly Thr Ser Thr
   340 345 350
   Glu Pro Ser Glu Gly Ser Ala Pro Gly Thr Ser Thr Glu Pro Ser Glu
   355 360 365
   Gly Ser Ala Pro Gly Thr Ser Thr Glu Pro Ser Glu Gly Ser Ala Pro
   370 375 380
   Gly Thr Ser Thr Glu Pro Ser Glu Gly Ser Ala Pro Gly Thr Ser Thr
   385 390 395 400
   Glu Pro Ser Glu Gly Ser Ala Pro Gly Ser Pro Ala Gly Ser Pro Thr
   405 410 415
   Ser Thr Glu Glu Gly Thr Ser Thr Glu Pro Ser Glu Gly Ser Ala Pro
   420 425 430
   Gly Thr Ser Glu Ser Ala Thr Pro Glu Ser Gly Pro Gly Ser Glu Pro
   435 440 445
   Ala Thr Ser Gly Ser Glu Thr Pro Gly Thr Ser Glu Ser Ala Thr Pro
   450 455 460
   Glu Ser Gly Pro Gly Ser Glu Pro Ala Thr Ser Gly Ser Glu Thr Pro
   465 470 475 480
   Gly Thr Ser Glu Ser Ala Thr Pro Glu Ser Gly Pro Gly Thr Ser Thr
   485 490 495
   Glu Pro Ser Glu Gly Ser Ala Pro Gly Thr Ser Glu Ser Ala Thr Pro
   500 505 510
   Glu Ser Gly Pro Gly Ser Pro Ala Gly Ser Pro Thr Ser Thr Glu Glu
   515 520 525
   Gly Ser Pro Ala Gly Ser Pro Thr Ser Thr Glu Glu Gly Ser Pro Ala
   530 535 540
   Gly Ser Pro Thr Ser Thr Glu Glu Gly Thr Ser Glu Ser Ala Thr Pro
   545 550 555 560
   Glu Ser Gly Pro Gly Thr Ser Thr Glu Pro Ser Glu Gly Ser Ala Pro
   565 570 575
<210> 40
   <211> 576
   <212> PRT
   <213> Artificial Sequence
<220>
   <221> source
   <223> /note="Description of Artificial Sequence: Synthetic polypeptide"
<400> 40
   Gly Ser Thr Ser Ser Thr Ala Glu Ser Pro Gly Pro Gly Ser Thr Ser
   1 5 10 15
   Ser Thr Ala Glu Ser Pro Gly Pro Gly Ser Thr Ser Glu Ser Pro Ser
   20 25 30
   Gly Thr Ala Pro Gly Ser Thr Ser Ser Thr Ala Glu Ser Pro Gly Pro
   35 40 45
   Gly Ser Thr Ser Ser Thr Ala Glu Ser Pro Gly Pro Gly Thr Ser Thr
   50 55 60
   Pro Glu Ser Gly Ser Ala Ser Pro Gly Ser Thr Ser Glu Ser Pro Ser
   65 70 75 80
   Gly Thr Ala Pro Gly Thr Ser Pro Ser Gly Glu Ser Ser Thr Ala Pro
   85 90 95
   Gly Ser Thr Ser Glu Ser Pro Ser Gly Thr Ala Pro Gly Ser Thr Ser
   100 105 110
   Glu Ser Pro Ser Gly Thr Ala Pro Gly Thr Ser Pro Ser Gly Glu Ser
   115 120 125
   Ser Thr Ala Pro Gly Ser Thr Ser Glu Ser Pro Ser Gly Thr Ala Pro
   130 135 140
   Gly Ser Thr Ser Glu Ser Pro Ser Gly Thr Ala Pro Gly Thr Ser Pro
   145 150 155 160
   Ser Gly Glu Ser Ser Thr Ala Pro Gly Ser Thr Ser Glu Ser Pro Ser
   165 170 175
   Gly Thr Ala Pro Gly Ser Thr Ser Glu Ser Pro Ser Gly Thr Ala Pro
               180                 185                 190
   Gly Ser Thr Ser Glu Ser Pro Ser Gly Thr Ala Pro Gly Thr Ser Thr
           195                 200                 205
   Pro Glu Ser Gly Ser Ala Ser Pro Gly Ser Thr Ser Glu Ser Pro Ser
       210                 215                 220
   Gly Thr Ala Pro Gly Thr Ser Thr Pro Glu Ser Gly Ser Ala Ser Pro
   225                 230                 235                 240
   Gly Ser Thr Ser Ser Thr Ala Glu Ser Pro Gly Pro Gly Ser Thr Ser
                   245                 250                 255
   Ser Thr Ala Glu Ser Pro Gly Pro Gly Thr Ser Thr Pro Glu Ser Gly
               260                 265                 270
   Ser Ala Ser Pro Gly Thr Ser Thr Pro Glu Ser Gly Ser Ala Ser Pro
           275                 280                 285
   Gly Ser Thr Ser Glu Ser Pro Ser Gly Thr Ala Pro Gly Thr Ser Thr
       290                 295                 300
   Pro Glu Ser Gly Ser Ala Ser Pro Gly Thr Ser Thr Pro Glu Ser Gly
   305                 310                 315                 320
   Ser Ala Ser Pro Gly Ser Thr Ser Glu Ser Pro Ser Gly Thr Ala Pro
                   325                 330                 335
   Gly Ser Thr Ser Glu Ser Pro Ser Gly Thr Ala Pro Gly Ser Thr Ser
               340                 345                 350
   Glu Ser Pro Ser Gly Thr Ala Pro Gly Ser Thr Ser Ser Thr Ala Glu
           355                 360                 365
   Ser Pro Gly Pro Gly Thr Ser Thr Pro Glu Ser Gly Ser Ala Ser Pro
       370                 375                 380
   Gly Thr Ser Thr Pro Glu Ser Gly Ser Ala Ser Pro Gly Ser Thr Ser
   385                 390                 395                 400
   Glu Ser Pro Ser Gly Thr Ala Pro Gly Ser Thr Ser Glu Ser Pro Ser
                   405                 410                 415
   Gly Thr Ala Pro Gly Thr Ser Thr Pro Glu Ser Gly Ser Ala Ser Pro
               420                 425                 430
   Gly Ser Thr Ser Glu Ser Pro Ser Gly Thr Ala Pro Gly Ser Thr Ser
   435 440 445
   Glu Ser Pro Ser Gly Thr Ala Pro Gly Thr Ser Thr Pro Glu Ser Gly
   450 455 460
   Ser Ala Ser Pro Gly Thr Ser Pro Ser Gly Glu Ser Ser Thr Ala Pro
   465 470 475 480
   Gly Ser Thr Ser Ser Thr Ala Glu Ser Pro Gly Pro Gly Thr Ser Pro
   485 490 495
   Ser Gly Glu Ser Ser Thr Ala Pro Gly Ser Thr Ser Ser Thr Ala Glu
   500 505 510
   Ser Pro Gly Pro Gly Thr Ser Thr Pro Glu Ser Gly Ser Ala Ser Pro
   515 520 525
   Gly Ser Thr Ser Glu Ser Pro Ser Gly Thr Ala Pro Gly Ser Thr Ser
   530 535 540
   Ser Thr Ala Glu Ser Pro Gly Pro Gly Thr Ser Thr Pro Glu Ser Gly
   545 550 555 560
   Ser Ala Ser Pro Gly Thr Ser Thr Pro Glu Ser Gly Ser Ala Ser Pro
   565 570 575
<210> 41
   <211> 625
   <212> PRT
   <213> Artificial Sequence
<220>
   <221> source
   <223> /note="Description of Artificial Sequence: Synthetic polypeptide"
<400> 41
   Met Ala Glu Pro Ala Gly Ser Pro Thr Ser Thr Glu Glu Gly Thr Pro
   1 5 10 15
   Gly Ser Gly Thr Ala Ser Ser Ser Pro Gly Ser Ser Thr Pro Ser Gly
   20 25 30
   Ala Thr Gly Ser Pro Gly Ala Ser Pro Gly Thr Ser Ser Thr Gly Ser
   35 40 45
   Pro Gly Ser Pro Ala Gly Ser Pro Thr Ser Thr Glu Glu Gly Thr Ser
   50 55 60
   Glu Ser Ala Thr Pro Glu Ser Gly Pro Gly Thr Ser Thr Glu Pro Ser
   65 70 75 80
   Glu Gly Ser Ala Pro Gly Ser Pro Ala Gly Ser Pro Thr Ser Thr Glu
   85 90 95
   Glu Gly Thr Ser Thr Glu Pro Ser Glu Gly Ser Ala Pro Gly Thr Ser
   100 105 110
   Thr Glu Pro Ser Glu Gly Ser Ala Pro Gly Thr Ser Glu Ser Ala Thr
   115 120 125
   Pro Glu Ser Gly Pro Gly Ser Glu Pro Ala Thr Ser Gly Ser Glu Thr
   130 135 140
   Pro Gly Ser Glu Pro Ala Thr Ser Gly Ser Glu Thr Pro Gly Ser Pro
   145 150 155 160
   Ala Gly Ser Pro Thr Ser Thr Glu Glu Gly Thr Ser Glu Ser Ala Thr
   165 170 175
   Pro Glu Ser Gly Pro Gly Thr Ser Thr Glu Pro Ser Glu Gly Ser Ala
   180 185 190
   Pro Gly Thr Ser Thr Glu Pro Ser Glu Gly Ser Ala Pro Gly Ser Pro
   195 200 205
   Ala Gly Ser Pro Thr Ser Thr Glu Glu Gly Thr Ser Thr Glu Pro Ser
   210 215 220
   Glu Gly Ser Ala Pro Gly Thr Ser Thr Glu Pro Ser Glu Gly Ser Ala
   225 230 235 240
   Pro Gly Thr Ser Glu Ser Ala Thr Pro Glu Ser Gly Pro Gly Thr Ser
   245 250 255
   Thr Glu Pro Ser Glu Gly Ser Ala Pro Gly Thr Ser Glu Ser Ala Thr
   260 265 270
   Pro Glu Ser Gly Pro Gly Ser Glu Pro Ala Thr Ser Gly Ser Glu Thr
   275 280 285
   Pro Gly Thr Ser Thr Glu Pro Ser Glu Gly Ser Ala Pro Gly Thr Ser
   290 295 300
   Thr Glu Pro Ser Glu Gly Ser Ala Pro Gly Thr Ser Glu Ser Ala Thr
   305 310 315 320
   Pro Glu Ser Gly Pro Gly Thr Ser Glu Ser Ala Thr Pro Glu Ser Gly
                   325                 330                 335
   Pro Gly Ser Pro Ala Gly Ser Pro Thr Ser Thr Glu Glu Gly Thr Ser
               340                 345                 350
   Glu Ser Ala Thr Pro Glu Ser Gly Pro Gly Ser Glu Pro Ala Thr Ser
           355                 360                 365
   Gly Ser Glu Thr Pro Gly Thr Ser Glu Ser Ala Thr Pro Glu Ser Gly
       370                 375                 380
   Pro Gly Thr Ser Thr Glu Pro Ser Glu Gly Ser Ala Pro Gly Thr Ser
   385                 390                 395                 400
   Thr Glu Pro Ser Glu Gly Ser Ala Pro Gly Thr Ser Thr Glu Pro Ser
                   405                 410                 415
   Glu Gly Ser Ala Pro Gly Thr Ser Thr Glu Pro Ser Glu Gly Ser Ala
               420                 425                 430
   Pro Gly Thr Ser Thr Glu Pro Ser Glu Gly Ser Ala Pro Gly Thr Ser
           435                 440                 445
   Thr Glu Pro Ser Glu Gly Ser Ala Pro Gly Ser Pro Ala Gly Ser Pro
       450                 455                 460
   Thr Ser Thr Glu Glu Gly Thr Ser Thr Glu Pro Ser Glu Gly Ser Ala
   465                 470                 475                 480
   Pro Gly Thr Ser Glu Ser Ala Thr Pro Glu Ser Gly Pro Gly Ser Glu
                   485                 490                 495
   Pro Ala Thr Ser Gly Ser Glu Thr Pro Gly Thr Ser Glu Ser Ala Thr
               500                 505                 510
   Pro Glu Ser Gly Pro Gly Ser Glu Pro Ala Thr Ser Gly Ser Glu Thr
           515                 520                 525
   Pro Gly Thr Ser Glu Ser Ala Thr Pro Glu Ser Gly Pro Gly Thr Ser
       530                 535                 540
   Thr Glu Pro Ser Glu Gly Ser Ala Pro Gly Thr Ser Glu Ser Ala Thr
   545                 550                 555                 560
   Pro Glu Ser Gly Pro Gly Ser Pro Ala Gly Ser Pro Thr Ser Thr Glu
                   565                 570                 575
   Glu Gly Ser Pro Ala Gly Ser Pro Thr Ser Thr Glu Glu Gly Ser Pro
               580                 585                 590
   Ala Gly Ser Pro Thr Ser Thr Glu Glu Gly Thr Ser Glu Ser Ala Thr
           595                 600                 605
   Pro Glu Ser Gly Pro Gly Thr Ser Thr Glu Pro Ser Glu Gly Ser Ala
       610                 615                 620
   Pro
   625
<210> 42
   <211> 836
   <212> PRT
   <213> Artificial Sequence
<220>
   <221> source
   <223> /note="Description of Artificial Sequence: Synthetic polypeptide"
<400> 42
   Gly Ser Ser Glu Ser Gly Ser Ser Glu Gly Gly Pro Gly Ser Ser Glu
   1               5                   10                  15
   Ser Gly Ser Ser Glu Gly Gly Pro Gly Glu Ser Pro Gly Gly Ser Ser
               20                  25                  30
   Gly Ser Glu Ser Gly Ser Gly Gly Glu Pro Ser Glu Ser Gly Ser Ser
           35                  40                  45
   Gly Glu Ser Pro Gly Gly Ser Ser Gly Ser Glu Ser Gly Glu Ser Pro
       50                  55                  60
   Gly Gly Ser Ser Gly Ser Glu Ser Gly Ser Ser Glu Ser Gly Ser Ser
   65                  70                  75                  80
   Glu Gly Gly Pro Gly Ser Ser Glu Ser Gly Ser Ser Glu Gly Gly Pro
                   85                  90                  95
   Gly Ser Ser Glu Ser Gly Ser Ser Glu Gly Gly Pro Gly Glu Ser Pro
               100                 105                 110
   Gly Gly Ser Ser Gly Ser Glu Ser Gly Glu Ser Pro Gly Gly Ser Ser
           115                 120                 125
   Gly Ser Glu Ser Gly Glu Ser Pro Gly Gly Ser Ser Gly Ser Glu Ser
   130 135 140
   Gly Ser Ser Glu Ser Gly Ser Ser Glu Gly Gly Pro Gly Ser Ser Glu
   145 150 155 160
   Ser Gly Ser Ser Glu Gly Gly Pro Gly Ser Ser Glu Ser Gly Ser Ser
   165 170 175
   Glu Gly Gly Pro Gly Ser Ser Glu Ser Gly Ser Ser Glu Gly Gly Pro
   180 185 190
   Gly Ser Ser Glu Ser Gly Ser Ser Glu Gly Gly Pro Gly Ser Ser Glu
   195 200 205
   Ser Gly Ser Ser Glu Gly Gly Pro Gly Ser Gly Gly Glu Pro Ser Glu
   210 215 220
   Ser Gly Ser Ser Gly Glu Ser Pro Gly Gly Ser Ser Gly Ser Glu Ser
   225 230 235 240
   Gly Glu Ser Pro Gly Gly Ser Ser Gly Ser Glu Ser Gly Ser Gly Gly
   245 250 255
   Glu Pro Ser Glu Ser Gly Ser Ser Gly Ser Glu Gly Ser Ser Gly Pro
   260 265 270
   Gly Glu Ser Ser Gly Ser Ser Glu Ser Gly Ser Ser Glu Gly Gly Pro
   275 280 285
   Gly Ser Gly Gly Glu Pro Ser Glu Ser Gly Ser Ser Gly Ser Glu Gly
   290 295 300
   Ser Ser Gly Pro Gly Glu Ser Ser Gly Ser Ser Glu Ser Gly Ser Ser
   305 310 315 320
   Glu Gly Gly Pro Gly Ser Gly Gly Glu Pro Ser Glu Ser Gly Ser Ser
   325 330 335
   Gly Glu Ser Pro Gly Gly Ser Ser Gly Ser Glu Ser Gly Ser Gly Gly
   340 345 350
   Glu Pro Ser Glu Ser Gly Ser Ser Gly Ser Gly Gly Glu Pro Ser Glu
   355 360 365
   Ser Gly Ser Ser Gly Ser Ser Glu Ser Gly Ser Ser Glu Gly Gly Pro
   370 375 380
   Gly Ser Gly Gly Glu Pro Ser Glu Ser Gly Ser Ser Gly Ser Gly Gly
   385                 390                 395                 400
   Glu Pro Ser Glu Ser Gly Ser Ser Gly Ser Glu Gly Ser Ser Gly Pro
                   405                 410                 415
   Gly Glu Ser Ser Gly Glu Ser Pro Gly Gly Ser Ser Gly Ser Glu Ser
               420                 425                 430
   Gly Ser Glu Gly Ser Ser Gly Pro Gly Glu Ser Ser Gly Ser Glu Gly
           435                 440                 445
   Ser Ser Gly Pro Gly Glu Ser Ser Gly Ser Gly Gly Glu Pro Ser Glu
       450                 455                 460
   Ser Gly Ser Ser Gly Ser Ser Glu Ser Gly Ser Ser Glu Gly Gly Pro
   465                 470                 475                 480
   Gly Ser Ser Glu Ser Gly Ser Ser Glu Gly Gly Pro Gly Glu Ser Pro
                   485                 490                 495
   Gly Gly Ser Ser Gly Ser Glu Ser Gly Ser Gly Gly Glu Pro Ser Glu
               500                 505                 510
   Ser Gly Ser Ser Gly Ser Glu Gly Ser Ser Gly Pro Gly Glu Ser Ser
           515                 520                 525
   Gly Glu Ser Pro Gly Gly Ser Ser Gly Ser Glu Ser Gly Ser Glu Gly
       530                 535                 540
   Ser Ser Gly Pro Gly Ser Ser Glu Ser Gly Ser Ser Glu Gly Gly Pro
   545                 550                 555                 560
   Gly Ser Gly Gly Glu Pro Ser Glu Ser Gly Ser Ser Gly Ser Glu Gly
                   565                 570                 575
   Ser Ser Gly Pro Gly Glu Ser Ser Gly Ser Glu Gly Ser Ser Gly Pro
               580                 585                 590
   Gly Glu Ser Ser Gly Ser Glu Gly Ser Ser Gly Pro Gly Glu Ser Ser
           595                 600                 605
   Gly Ser Gly Gly Glu Pro Ser Glu Ser Gly Ser Ser Gly Ser Gly Gly
       610                 615                 620
   Glu Pro Ser Glu Ser Gly Ser Ser Gly Glu Ser Pro Gly Gly Ser Ser
   625                 630                 635                 640
   Gly Ser Glu Ser Gly Glu Ser Pro Gly Gly Ser Ser Gly Ser Glu Ser
                   645                 650                 655
   Gly Ser Gly Gly Glu Pro Ser Glu Ser Gly Ser Ser Gly Ser Glu Gly
               660                 665                 670
   Ser Ser Gly Pro Gly Glu Ser Ser Gly Glu Ser Pro Gly Gly Ser Ser
           675                 680                 685
   Gly Ser Glu Ser Gly Ser Ser Glu Ser Gly Ser Ser Glu Gly Gly Pro
       690                 695                 700
   Gly Ser Ser Glu Ser Gly Ser Ser Glu Gly Gly Pro Gly Ser Ser Glu
   705                 710                 715                 720
   Ser Gly Ser Ser Glu Gly Gly Pro Gly Ser Gly Gly Glu Pro Ser Glu
                   725                 730                 735
   Ser Gly Ser Ser Gly Ser Ser Glu Ser Gly Ser Ser Glu Gly Gly Pro
               740                 745                 750
   Gly Glu Ser Pro Gly Gly Ser Ser Gly Ser Glu Ser Gly Ser Gly Gly
           755                 760                 765
   Glu Pro Ser Glu Ser Gly Ser Ser Gly Ser Ser Glu Ser Gly Ser Ser
       770                 775                 780
   Glu Gly Gly Pro Gly Glu Ser Pro Gly Gly Ser Ser Gly Ser Glu Ser
   785                 790                 795                 800
   Gly Ser Gly Gly Glu Pro Ser Glu Ser Gly Ser Ser Gly Glu Ser Pro
                   805                 810                 815
   Gly Gly Ser Ser Gly Ser Glu Ser Gly Ser Gly Gly Glu Pro Ser Glu
               820                 825                 830
   Ser Gly Ser Ser
           835
<210> 43
   <211> 864
   <212> PRT
   <213> Artificial Sequence
<220>
   <221> source
   <223> /note="Description of Artificial Sequence: Synthetic polypeptide"
<400> 43
   Gly Ser Pro Ala Gly Ser Pro Thr Ser Thr Glu Glu Gly Thr Ser Glu
   1               5                   10                  15
   Ser Ala Thr Pro Glu Ser Gly Pro Gly Thr Ser Thr Glu Pro Ser Glu
               20                  25                  30
   Gly Ser Ala Pro Gly Ser Pro Ala Gly Ser Pro Thr Ser Thr Glu Glu
           35                  40                  45
   Gly Thr Ser Thr Glu Pro Ser Glu Gly Ser Ala Pro Gly Thr Ser Thr
       50                  55                  60
   Glu Pro Ser Glu Gly Ser Ala Pro Gly Thr Ser Glu Ser Ala Thr Pro
   65                  70                  75                  80
   Glu Ser Gly Pro Gly Ser Glu Pro Ala Thr Ser Gly Ser Glu Thr Pro
                   85                  90                  95
   Gly Ser Glu Pro Ala Thr Ser Gly Ser Glu Thr Pro Gly Ser Pro Ala
               100                 105                 110
   Gly Ser Pro Thr Ser Thr Glu Glu Gly Thr Ser Glu Ser Ala Thr Pro
           115                 120                 125
   Glu Ser Gly Pro Gly Thr Ser Thr Glu Pro Ser Glu Gly Ser Ala Pro
       130                 135                 140
   Gly Thr Ser Thr Glu Pro Ser Glu Gly Ser Ala Pro Gly Ser Pro Ala
   145                 150                 155                 160
   Gly Ser Pro Thr Ser Thr Glu Glu Gly Thr Ser Thr Glu Pro Ser Glu
                   165                 170                 175
   Gly Ser Ala Pro Gly Thr Ser Thr Glu Pro Ser Glu Gly Ser Ala Pro
               180                 185                 190
   Gly Thr Ser Glu Ser Ala Thr Pro Glu Ser Gly Pro Gly Thr Ser Thr
           195                 200                 205
   Glu Pro Ser Glu Gly Ser Ala Pro Gly Thr Ser Glu Ser Ala Thr Pro
       210                 215                 220
   Glu Ser Gly Pro Gly Ser Glu Pro Ala Thr Ser Gly Ser Glu Thr Pro
   225                 230                 235                 240
   Gly Thr Ser Thr Glu Pro Ser Glu Gly Ser Ala Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Thr Ser Glu Ser Ala Thr Pro Glu Ser Gly Pro
           275                 280                 285
   Gly Ser Pro Ala Gly Ser Pro Thr Ser Thr Glu Glu Gly Thr Ser Glu
       290                 295                 300
   Ser Ala Thr Pro Glu Ser Gly Pro Gly Ser Glu Pro Ala Thr Ser Gly
   305                 310                 315                 320
   Ser Glu Thr Pro Gly Thr Ser Glu Ser Ala Thr Pro Glu Ser Gly Pro
                   325                 330                 335
   Gly Thr Ser Thr Glu Pro Ser Glu Gly Ser Ala Pro Gly Thr Ser Thr
               340                 345                 350
   Glu Pro Ser Glu Gly Ser Ala Pro Gly Thr Ser Thr Glu Pro Ser Glu
           355                 360                 365
   Gly Ser Ala Pro Gly Thr Ser Thr Glu Pro Ser Glu Gly Ser Ala Pro
       370                 375                 380
   Gly Thr Ser Thr Glu Pro Ser Glu Gly Ser Ala Pro Gly Thr Ser Thr
   385                 390                 395                 400
   Glu Pro Ser Glu Gly Ser Ala Pro Gly Ser Pro Ala Gly Ser Pro Thr
                   405                 410                 415
   Ser Thr Glu Glu Gly Thr Ser Thr Glu Pro Ser Glu Gly Ser Ala Pro
               420                 425                 430
   Gly Thr Ser Glu Ser Ala Thr Pro Glu Ser Gly Pro Gly Ser Glu Pro
           435                 440                 445
   Ala Thr Ser Gly Ser Glu Thr Pro Gly Thr Ser Glu Ser Ala Thr Pro
       450                 455                 460
   Glu Ser Gly Pro Gly Ser Glu Pro Ala Thr Ser Gly Ser Glu Thr Pro
   465                 470                 475                 480
   Gly Thr Ser Glu Ser Ala Thr Pro Glu Ser Gly Pro Gly Thr Ser Thr
                   485                 490                 495
   Glu Pro Ser Glu Gly Ser Ala Pro Gly Thr Ser Glu Ser Ala Thr Pro
   500 505 510
   Glu Ser Gly Pro Gly Ser Pro Ala Gly Ser Pro Thr Ser Thr Glu Glu
   515 520 525
   Gly Ser Pro Ala Gly Ser Pro Thr Ser Thr Glu Glu Gly Ser Pro Ala
   530 535 540
   Gly Ser Pro Thr Ser Thr Glu Glu Gly Thr Ser Glu Ser Ala Thr Pro
   545 550 555 560
   Glu Ser Gly Pro Gly Thr Ser Thr Glu Pro Ser Glu Gly Ser Ala Pro
   565 570 575
   Gly Thr Ser Glu Ser Ala Thr Pro Glu Ser Gly Pro Gly Ser Glu Pro
   580 585 590
   Ala Thr Ser Gly Ser Glu Thr Pro Gly Thr Ser Glu Ser Ala Thr Pro
   595 600 605
   Glu Ser Gly Pro Gly Ser Glu Pro Ala Thr Ser Gly Ser Glu Thr Pro
   610 615 620
   Gly Thr Ser Glu Ser Ala Thr Pro Glu Ser Gly Pro Gly Thr Ser Thr
   625 630 635 640
   Glu Pro Ser Glu Gly Ser Ala Pro Gly Ser Pro Ala Gly Ser Pro Thr
   645 650 655
   Ser Thr Glu Glu Gly Thr Ser Glu Ser Ala Thr Pro Glu Ser Gly Pro
   660 665 670
   Gly Ser Glu Pro Ala Thr Ser Gly Ser Glu Thr Pro Gly Thr Ser Glu
   675 680 685
   Ser Ala Thr Pro Glu Ser Gly Pro Gly Ser Pro Ala Gly Ser Pro Thr
   690 695 700
   Ser Thr Glu Glu Gly Ser Pro Ala Gly Ser Pro Thr Ser Thr Glu Glu
   705 710 715 720
   Gly Thr Ser Thr Glu Pro Ser Glu Gly Ser Ala Pro Gly Thr Ser Glu
   725 730 735
   Ser Ala Thr Pro Glu Ser Gly Pro Gly Thr Ser Glu Ser Ala Thr Pro
   740 745 750
   Glu Ser Gly Pro Gly Thr Ser Glu Ser Ala Thr Pro Glu Ser Gly Pro
               755                 760                 765
   Gly Ser Glu Pro Ala Thr Ser Gly Ser Glu Thr Pro Gly Ser Glu Pro
           770                 775                 780
   Ala Thr Ser Gly Ser Glu Thr Pro Gly Ser Pro Ala Gly Ser Pro Thr
   785                 790                 795                 800
   Ser Thr Glu Glu Gly Thr Ser Thr Glu Pro Ser Glu Gly Ser Ala Pro
                   805                 810                 815
   Gly Thr Ser Thr Glu Pro Ser Glu Gly Ser Ala Pro Gly Ser Glu Pro
               820                 825                 830
   Ala Thr Ser Gly Ser Glu Thr Pro Gly Thr Ser Glu Ser Ala Thr Pro
           835                 840                 845
   Glu Ser Gly Pro Gly Thr Ser Thr Glu Pro Ser Glu Gly Ser Ala Pro
       850                 855                 860
<210> 44
   <211> 875
   <212> PRT
   <213> Artificial Sequence
<220>
   <221> source
   <223> /note="Description of Artificial Sequence: Synthetic polypeptide"
<220>
   <221> MOD_RES
   <222> (430)..(432)
   <223> Any amino acid
<220>
   <221> MOD_RES
   <222> (443)..(446)
   <223> Any amino acid
<400> 44
   Gly Ser Thr Ser Glu Ser Pro Ser Gly Thr Ala Pro Gly Thr Ser Pro
   1               5                   10                  15
   Ser Gly Glu Ser Ser Thr Ala Pro Gly Ser Thr Ser Glu Ser Pro Ser
               20                  25                  30
   Gly Thr Ala Pro Gly Ser Thr Ser Glu Ser Pro Ser Gly Thr Ala Pro
           35                  40                  45
   Gly Thr Ser Thr Pro Glu Ser Gly Ser Ala Ser Pro Gly Thr Ser Thr
   50 55 60
   Pro Glu Ser Gly Ser Ala Ser Pro Gly Ser Thr Ser Glu Ser Pro Ser
   65 70 75 80
   Gly Thr Ala Pro Gly Ser Thr Ser Glu Ser Pro Ser Gly Thr Ala Pro
   85 90 95
   Gly Thr Ser Pro Ser Gly Glu Ser Ser Thr Ala Pro Gly Ser Thr Ser
   100 105 110
   Glu Ser Pro Ser Gly Thr Ala Pro Gly Thr Ser Pro Ser Gly Glu Ser
   115 120 125
   Ser Thr Ala Pro Gly Thr Ser Pro Ser Gly Glu Ser Ser Thr Ala Pro
   130 135 140
   Gly Ser Thr Ser Ser Thr Ala Glu Ser Pro Gly Pro Gly Thr Ser Pro
   145 150 155 160
   Ser Gly Glu Ser Ser Thr Ala Pro Gly Thr Ser Pro Ser Gly Glu Ser
   165 170 175
   Ser Thr Ala Pro Gly Ser Thr Ser Ser Thr Ala Glu Ser Pro Gly Pro
   180 185 190
   Gly Thr Ser Thr Pro Glu Ser Gly Ser Ala Ser Pro Gly Thr Ser Thr
   195 200 205
   Pro Glu Ser Gly Ser Ala Ser Pro Gly Ser Thr Ser Glu Ser Pro Ser
   210 215 220
   Gly Thr Ala Pro Gly Ser Thr Ser Glu Ser Pro Ser Gly Thr Ala Pro
   225 230 235 240
   Gly Thr Ser Thr Pro Glu Ser Gly Ser Ala Ser Pro Gly Ser Thr Ser
   245 250 255
   Ser Thr Ala Glu Ser Pro Gly Pro Gly Thr Ser Thr Pro Glu Ser Gly
   260 265 270
   Ser Ala Ser Pro Gly Ser Thr Ser Glu Ser Pro Ser Gly Thr Ala Pro
   275 280 285
   Gly Thr Ser Pro Ser Gly Glu Ser Ser Thr Ala Pro Gly Ser Thr Ser
   290 295 300
   Ser Thr Ala Glu Ser Pro Gly Pro Gly Thr Ser Pro Ser Gly Glu Ser
   305                 310                 315                 320
   Ser Thr Ala Pro Gly Thr Ser Thr Pro Glu Ser Gly Ser Ala Ser Pro
                   325                 330                 335
   Gly Ser Thr Ser Ser Thr Ala Glu Ser Pro Gly Pro Gly Ser Thr Ser
               340                 345                 350
   Ser Thr Ala Glu Ser Pro Gly Pro Gly Ser Thr Ser Ser Thr Ala Glu
           355                 360                 365
   Ser Pro Gly Pro Gly Ser Thr Ser Ser Thr Ala Glu Ser Pro Gly Pro
       370                 375                 380
   Gly Thr Ser Pro Ser Gly Glu Ser Ser Thr Ala Pro Gly Ser Thr Ser
   385                 390                 395                 400
   Glu Ser Pro Ser Gly Thr Ala Pro Gly Ser Thr Ser Glu Ser Pro Ser
                   405                 410                 415
   Gly Thr Ala Pro Gly Thr Ser Thr Pro Glu Ser Gly Pro Xaa Xaa Xaa
               420                 425                 430
   Gly Ala Ser Ala Ser Gly Ala Pro Ser Thr Xaa Xaa Xaa Xaa Ser Glu
           435                 440                 445
   Ser Pro Ser Gly Thr Ala Pro Gly Ser Thr Ser Glu Ser Pro Ser Gly
       450                 455                 460
   Thr Ala Pro Gly Ser Thr Ser Glu Ser Pro Ser Gly Thr Ala Pro Gly
   465                 470                 475                 480
   Ser Thr Ser Glu Ser Pro Ser Gly Thr Ala Pro Gly Ser Thr Ser Glu
                   485                 490                 495
   Ser Pro Ser Gly Thr Ala Pro Gly Ser Thr Ser Glu Ser Pro Ser Gly
               500                 505                 510
   Thr Ala Pro Gly Thr Ser Thr Pro Glu Ser Gly Ser Ala Ser Pro Gly
           515                 520                 525
   Thr Ser Pro Ser Gly Glu Ser Ser Thr Ala Pro Gly Thr Ser Pro Ser
       530                 535                 540
   Gly Glu Ser Ser Thr Ala Pro Gly Ser Thr Ser Ser Thr Ala Glu Ser
   545                 550                 555                 560
   Pro Gly Pro Gly Thr Ser Pro Ser Gly Glu Ser Ser Thr Ala Pro Gly
                   565                 570                 575
   Thr Ser Thr Pro Glu Ser Gly Ser Ala Ser Pro Gly Ser Thr Ser Glu
               580                 585                 590
   Ser Pro Ser Gly Thr Ala Pro Gly Ser Thr Ser Glu Ser Pro Ser Gly
           595                 600                 605
   Thr Ala Pro Gly Thr Ser Pro Ser Gly Glu Ser Ser Thr Ala Pro Gly
       610                 615                 620
   Ser Thr Ser Glu Ser Pro Ser Gly Thr Ala Pro Gly Thr Ser Thr Pro
   625                 630                 635                 640
   Glu Ser Gly Ser Ala Ser Pro Gly Thr Ser Thr Pro Glu Ser Gly Ser
                   645                 650                 655
   Ala Ser Pro Gly Ser Thr Ser Glu Ser Pro Ser Gly Thr Ala Pro Gly
               660                 665                 670
   Thr Ser Thr Pro Glu Ser Gly Ser Ala Ser Pro Gly Ser Thr Ser Ser
           675                 680                 685
   Thr Ala Glu Ser Pro Gly Pro Gly Ser Thr Ser Glu Ser Pro Ser Gly
       690                 695                 700
   Thr Ala Pro Gly Ser Thr Ser Glu Ser Pro Ser Gly Thr Ala Pro Gly
   705                 710                 715                 720
   Thr Ser Pro Ser Gly Glu Ser Ser Thr Ala Pro Gly Ser Thr Ser Ser
                   725                 730                 735
   Thr Ala Glu Ser Pro Gly Pro Gly Thr Ser Pro Ser Gly Glu Ser Ser
               740                 745                 750
   Thr Ala Pro Gly Thr Ser Thr Pro Glu Ser Gly Ser Ala Ser Pro Gly
           755                 760                 765
   Thr Ser Pro Ser Gly Glu Ser Ser Thr Ala Pro Gly Thr Ser Pro Ser
       770                 775                 780
   Gly Glu Ser Ser Thr Ala Pro Gly Thr Ser Pro Ser Gly Glu Ser Ser
   785                 790                 795                 800
   Thr Ala Pro Gly Ser Thr Ser Ser Thr Ala Glu Ser Pro Gly Pro Gly
                   805                 810                 815
   Ser Thr Ser Ser Thr Ala Glu Ser Pro Gly Pro Gly Thr Ser Pro Ser
               820                 825                 830
   Gly Glu Ser Ser Thr Ala Pro Gly Ser Ser Pro Ser Ala Ser Thr Gly
           835                 840                 845
   Thr Gly Pro Gly Ser Ser Thr Pro Ser Gly Ala Thr Gly Ser Pro Gly
       850                 855                 860
   Ser Ser Thr Pro Ser Gly Ala Thr Gly Ser Pro
   865                 870                 875
<210> 45
   <211> 864
   <212> PRT
   <213> Artificial Sequence
<220>
   <221> source
   <223> /note="Description of Artificial Sequence: Synthetic polypeptide"
<400> 45
   Gly Ala Ser Pro Gly Thr Ser Ser Thr Gly Ser Pro Gly Ser Ser Pro
   1               5                   10                  15
   Ser Ala Ser Thr Gly Thr Gly Pro Gly Ser Ser Pro Ser Ala Ser Thr
               20                  25                  30
   Gly Thr Gly Pro Gly Thr Pro Gly Ser Gly Thr Ala Ser Ser Ser Pro
           35                  40                  45
   Gly Ser Ser Thr Pro Ser Gly Ala Thr Gly Ser Pro Gly Ser Ser Pro
       50                  55                  60
   Ser Ala Ser Thr Gly Thr Gly Pro Gly Ala Ser Pro Gly Thr Ser Ser
   65                  70                  75                  80
   Thr Gly Ser Pro Gly Thr Pro Gly Ser Gly Thr Ala Ser Ser Ser Pro
                   85                  90                  95
   Gly Ser Ser Thr Pro Ser Gly Ala Thr Gly Ser Pro Gly Thr Pro Gly
               100                 105                 110
   Ser Gly Thr Ala Ser Ser Ser Pro Gly Ala Ser Pro Gly Thr Ser Ser
           115                 120                 125
   Thr Gly Ser Pro Gly Ala Ser Pro Gly Thr Ser Ser Thr Gly Ser Pro
       130                 135                 140
   Gly Thr Pro Gly Ser Gly Thr Ala Ser Ser Ser Pro Gly Ser Ser Thr
   145                 150                 155                 160
   Pro Ser Gly Ala Thr Gly Ser Pro Gly Ala Ser Pro Gly Thr Ser Ser
                   165                 170                 175
   Thr Gly Ser Pro Gly Thr Pro Gly Ser Gly Thr Ala Ser Ser Ser Pro
               180                 185                 190
   Gly Ser Ser Thr Pro Ser Gly Ala Thr Gly Ser Pro Gly Ser Ser Pro
           195                 200                 205
   Ser Ala Ser Thr Gly Thr Gly Pro Gly Ser Ser Pro Ser Ala Ser Thr
       210                 215                 220
   Gly Thr Gly Pro Gly Ser Ser Thr Pro Ser Gly Ala Thr Gly Ser Pro
   225                 230                 235                 240
   Gly Ser Ser Thr Pro Ser Gly Ala Thr Gly Ser Pro Gly Ala Ser Pro
                   245                 250                 255
   Gly Thr Ser Ser Thr Gly Ser Pro Gly Ala Ser Pro Gly Thr Ser Ser
               260                 265                 270
   Thr Gly Ser Pro Gly Ala Ser Pro Gly Thr Ser Ser Thr Gly Ser Pro
           275                 280                 285
   Gly Thr Pro Gly Ser Gly Thr Ala Ser Ser Ser Pro Gly Ala Ser Pro
       290                 295                 300
   Gly Thr Ser Ser Thr Gly Ser Pro Gly Ala Ser Pro Gly Thr Ser Ser
   305                 310                 315                 320
   Thr Gly Ser Pro Gly Ala Ser Pro Gly Thr Ser Ser Thr Gly Ser Pro
                   325                 330                 335
   Gly Ser Ser Pro Ser Ala Ser Thr Gly Thr Gly Pro Gly Thr Pro Gly
               340                 345                 350
   Ser Gly Thr Ala Ser Ser Ser Pro Gly Ala Ser Pro Gly Thr Ser Ser
           355                 360                 365
   Thr Gly Ser Pro Gly Ala Ser Pro Gly Thr Ser Ser Thr Gly Ser Pro
       370                 375                 380
   Gly Ala Ser Pro Gly Thr Ser Ser Thr Gly Ser Pro Gly Ser Ser Thr
   385 390 395 400
   Pro Ser Gly Ala Thr Gly Ser Pro Gly Ser Ser Thr Pro Ser Gly Ala
   405 410 415
   Thr Gly Ser Pro Gly Ala Ser Pro Gly Thr Ser Ser Thr Gly Ser Pro
   420 425 430
   Gly Thr Pro Gly Ser Gly Thr Ala Ser Ser Ser Pro Gly Ser Ser Thr
   435 440 445
   Pro Ser Gly Ala Thr Gly Ser Pro Gly Ser Ser Thr Pro Ser Gly Ala
   450 455 460
   Thr Gly Ser Pro Gly Ser Ser Thr Pro Ser Gly Ala Thr Gly Ser Pro
   465 470 475 480
   Gly Ser Ser Pro Ser Ala Ser Thr Gly Thr Gly Pro Gly Ala Ser Pro
   485 490 495
   Gly Thr Ser Ser Thr Gly Ser Pro Gly Ala Ser Pro Gly Thr Ser Ser
   500 505 510
   Thr Gly Ser Pro Gly Thr Pro Gly Ser Gly Thr Ala Ser Ser Ser Pro
   515 520 525
   Gly Ala Ser Pro Gly Thr Ser Ser Thr Gly Ser Pro Gly Ala Ser Pro
   530 535 540
   Gly Thr Ser Ser Thr Gly Ser Pro Gly Ala Ser Pro Gly Thr Ser Ser
   545 550 555 560
   Thr Gly Ser Pro Gly Ala Ser Pro Gly Thr Ser Ser Thr Gly Ser Pro
   565 570 575
   Gly Thr Pro Gly Ser Gly Thr Ala Ser Ser Ser Pro Gly Ser Ser Thr
   580 585 590
   Pro Ser Gly Ala Thr Gly Ser Pro Gly Thr Pro Gly Ser Gly Thr Ala
   595 600 605
   Ser Ser Ser Pro Gly Ser Ser Thr Pro Ser Gly Ala Thr Gly Ser Pro
   610 615 620
   Gly Thr Pro Gly Ser Gly Thr Ala Ser Ser Ser Pro Gly Ser Ser Thr
   625 630 635 640
   Pro Ser Gly Ala Thr Gly Ser Pro Gly Ser Ser Thr Pro Ser Gly Ala
   645 650 655
   Thr Gly Ser Pro Gly Ser Ser Pro Ser Ala Ser Thr Gly Thr Gly Pro
   660 665 670
   Gly Ser Ser Pro Ser Ala Ser Thr Gly Thr Gly Pro Gly Ala Ser Pro
   675 680 685
   Gly Thr Ser Ser Thr Gly Ser Pro Gly Thr Pro Gly Ser Gly Thr Ala
   690 695 700
   Ser Ser Ser Pro Gly Ser Ser Thr Pro Ser Gly Ala Thr Gly Ser Pro
   705 710 715 720
   Gly Ser Ser Pro Ser Ala Ser Thr Gly Thr Gly Pro Gly Ser Ser Pro
   725 730 735
   Ser Ala Ser Thr Gly Thr Gly Pro Gly Ala Ser Pro Gly Thr Ser Ser
   740 745 750
   Thr Gly Ser Pro Gly Ala Ser Pro Gly Thr Ser Ser Thr Gly Ser Pro
   755 760 765
   Gly Ser Ser Thr Pro Ser Gly Ala Thr Gly Ser Pro Gly Ser Ser Pro
   770 775 780
   Ser Ala Ser Thr Gly Thr Gly Pro Gly Ala Ser Pro Gly Thr Ser Ser
   785 790 795 800
   Thr Gly Ser Pro Gly Ser Ser Pro Ser Ala Ser Thr Gly Thr Gly Pro
   805 810 815
   Gly Thr Pro Gly Ser Gly Thr Ala Ser Ser Ser Pro Gly Ser Ser Thr
   820 825 830
   Pro Ser Gly Ala Thr Gly Ser Pro Gly Ser Ser Thr Pro Ser Gly Ala
   835 840 845
   Thr Gly Ser Pro Gly Ala Ser Pro Gly Thr Ser Ser Thr Gly Ser Pro
   850 855 860
<210> 46
   <211> 875
   <212> PRT
   <213> Artificial Sequence
<220>
   <221> source
   <223> /note="Description of Artificial Sequence: Synthetic polypeptide"
<400> 46
   Gly Thr Ser Thr Glu Pro Ser Glu Gly Ser Ala Pro Gly Ser Glu Pro
   1               5                   10                  15
   Ala Thr Ser Gly Ser Glu Thr Pro Gly Ser Pro Ala Gly Ser Pro Thr
               20                  25                  30
   Ser Thr Glu Glu Gly Ser Thr Ser Ser Thr Ala Glu Ser Pro Gly Pro
           35                  40                  45
   Gly Thr Ser Thr Pro Glu Ser Gly Ser Ala Ser Pro Gly Ser Thr Ser
       50                  55                  60
   Glu Ser Pro Ser Gly Thr Ala Pro Gly Ser Thr Ser Glu Ser Pro Ser
   65                  70                  75                  80
   Gly Thr Ala Pro Gly Thr Ser Thr Pro Glu Ser Gly Ser Ala Ser Pro
                   85                  90                  95
   Gly Thr Ser Thr Pro Glu Ser Gly Ser Ala Ser Pro Gly Ser Glu Pro
               100                 105                 110
   Ala Thr Ser Gly Ser Glu Thr Pro Gly Thr Ser Glu Ser Ala Thr Pro
           115                 120                 125
   Glu Ser Gly Pro Gly Ser Pro Ala Gly Ser Pro Thr Ser Thr Glu Glu
       130                 135                 140
   Gly Thr Ser Thr Glu Pro Ser Glu Gly Ser Ala Pro Gly Thr Ser Glu
   145                 150                 155                 160
   Ser Ala Thr Pro Glu Ser Gly Pro Gly Thr Ser Thr Glu Pro Ser Glu
                   165                 170                 175
   Gly Ser Ala Pro Gly Thr Ser Thr Glu Pro Ser Glu Gly Ser Ala Pro
               180                 185                 190
   Gly Ser Pro Ala Gly Ser Pro Thr Ser Thr Glu Glu Gly Thr Ser Thr
           195                 200                 205
   Glu Pro Ser Glu Gly Ser Ala Pro Gly Thr Ser Thr Glu Pro Ser Glu
       210                 215                 220
   Gly Ser Ala Pro Gly Thr Ser Glu Ser Ala Thr Pro Glu Ser Gly Pro
   225                 230                 235                 240
   Gly Thr Ser Glu Ser Ala Thr Pro Glu Ser Gly Pro Gly Thr Ser Thr
   245 250 255
   Glu Pro Ser Glu Gly Ser Ala Pro Gly Thr Ser Thr Glu Pro Ser Glu
   260 265 270
   Gly Ser Ala Pro Gly Thr Ser Glu Ser Ala Thr Pro Glu Ser Gly Pro
   275 280 285
   Gly Thr Ser Thr Glu Pro Ser Glu Gly Ser Ala Pro Gly Ser Glu Pro
   290 295 300
   Ala Thr Ser Gly Ser Glu Thr Pro Gly Ser Pro Ala Gly Ser Pro Thr
   305 310 315 320
   Ser Thr Glu Glu Gly Ser Ser Thr Pro Ser Gly Ala Thr Gly Ser Pro
   325 330 335
   Gly Thr Pro Gly Ser Gly Thr Ala Ser Ser Ser Pro Gly Ser Ser Thr
   340 345 350
   Pro Ser Gly Ala Thr Gly Ser Pro Gly Thr Ser Thr Glu Pro Ser Glu
   355 360 365
   Gly Ser Ala Pro Gly Thr Ser Thr Glu Pro Ser Glu Gly Ser Ala Pro
   370 375 380
   Gly Ser Glu Pro Ala Thr Ser Gly Ser Glu Thr Pro Gly Ser Pro Ala
   385 390 395 400
   Gly Ser Pro Thr Ser Thr Glu Glu Gly Ser Pro Ala Gly Ser Pro Thr
   405 410 415
   Ser Thr Glu Glu Gly Thr Ser Thr Glu Pro Ser Glu Gly Ser Ala Pro
   420 425 430
   Gly Ala Ser Ala Ser Gly Ala Pro Ser Thr Gly Gly Thr Ser Glu Ser
   435 440 445
   Ala Thr Pro Glu Ser Gly Pro Gly Ser Pro Ala Gly Ser Pro Thr Ser
   450 455 460
   Thr Glu Glu Gly Ser Pro Ala Gly Ser Pro Thr Ser Thr Glu Glu Gly
   465 470 475 480
   Ser Thr Ser Ser Thr Ala Glu Ser Pro Gly Pro Gly Ser Thr Ser Glu
   485 490 495
   Ser Pro Ser Gly Thr Ala Pro Gly Thr Ser Pro Ser Gly Glu Ser Ser
   500 505 510
   Thr Ala Pro Gly Thr Pro Gly Ser Gly Thr Ala Ser Ser Ser Pro Gly
   515 520 525
   Ser Ser Thr Pro Ser Gly Ala Thr Gly Ser Pro Gly Ser Ser Pro Ser
   530 535 540
   Ala Ser Thr Gly Thr Gly Pro Gly Ser Glu Pro Ala Thr Ser Gly Ser
   545 550 555 560
   Glu Thr Pro Gly Thr Ser Glu Ser Ala Thr Pro Glu Ser Gly Pro Gly
   565 570 575
   Ser Glu Pro Ala Thr Ser Gly Ser Glu Thr Pro Gly Ser Thr Ser Ser
   580 585 590
   Thr Ala Glu Ser Pro Gly Pro Gly Ser Thr Ser Ser Thr Ala Glu Ser
   595 600 605
   Pro Gly Pro Gly Thr Ser Pro Ser Gly Glu Ser Ser Thr Ala Pro Gly
   610 615 620
   Ser Glu Pro Ala Thr Ser Gly Ser Glu Thr Pro Gly Ser Glu Pro Ala
   625 630 635 640
   Thr Ser Gly Ser Glu Thr Pro Gly Thr Ser Thr Glu Pro Ser Glu Gly
   645 650 655
   Ser Ala Pro Gly Ser Thr Ser Ser Thr Ala Glu Ser Pro Gly Pro Gly
   660 665 670
   Thr Ser Thr Pro Glu Ser Gly Ser Ala Ser Pro Gly Ser Thr Ser Glu
   675 680 685
   Ser Pro Ser Gly Thr Ala Pro Gly Thr Ser Thr Glu Pro Ser Glu Gly
   690 695 700
   Ser Ala Pro Gly Thr Ser Thr Glu Pro Ser Glu Gly Ser Ala Pro Gly
   705 710 715 720
   Thr Ser Thr Glu Pro Ser Glu Gly Ser Ala Pro Gly Ser Ser Thr Pro
   725 730 735
   Ser Gly Ala Thr Gly Ser Pro Gly Ser Ser Pro Ser Ala Ser Thr Gly
   740 745 750
   Thr Gly Pro Gly Ala Ser Pro Gly Thr Ser Ser Thr Gly Ser Pro Gly
   755 760 765
   Ser Glu Pro Ala Thr Ser Gly Ser Glu Thr Pro Gly Thr Ser Glu Ser
   770 775 780
   Ala Thr Pro Glu Ser Gly Pro Gly Ser Pro Ala Gly Ser Pro Thr Ser
   785 790 795 800
   Thr Glu Glu Gly Ser Ser Thr Pro Ser Gly Ala Thr Gly Ser Pro Gly
   805 810 815
   Ser Ser Pro Ser Ala Ser Thr Gly Thr Gly Pro Gly Ala Ser Pro Gly
   820 825 830
   Thr Ser Ser Thr Gly Ser Pro Gly Thr Ser Glu Ser Ala Thr Pro Glu
   835 840 845
   Ser Gly Pro Gly Thr Ser Thr Glu Pro Ser Glu Gly Ser Ala Pro Gly
   850 855 860
   Thr Ser Thr Glu Pro Ser Glu Gly Ser Ala Pro
   865 870 875
<210> 47
   <211> 913
   <212> PRT
   <213> Artificial Sequence
<220>
   <221> source
   <223> /note="Description of Artificial Sequence: Synthetic polypeptide"
<400> 47
   Met Ala Glu Pro Ala Gly Ser Pro Thr Ser Thr Glu Glu Gly Thr Pro
   1               5                   10                  15
   Gly Ser Gly Thr Ala Ser Ser Ser Pro Gly Ser Ser Thr Pro Ser Gly
               20                  25                  30
   Ala Thr Gly Ser Pro Gly Ala Ser Pro Gly Thr Ser Ser Thr Gly Ser
           35                  40                  45
   Pro Gly Ser Pro Ala Gly Ser Pro Thr Ser Thr Glu Glu Gly Thr Ser
       50                  55                  60
   Glu Ser Ala Thr Pro Glu Ser Gly Pro Gly Thr Ser Thr Glu Pro Ser
   65 70 75 80
   Glu Gly Ser Ala Pro Gly Ser Pro Ala Gly Ser Pro Thr Ser Thr Glu
   85 90 95
   Glu Gly Thr Ser Thr Glu Pro Ser Glu Gly Ser Ala Pro Gly Thr Ser
   100 105 110
   Thr Glu Pro Ser Glu Gly Ser Ala Pro Gly Thr Ser Glu Ser Ala Thr
   115 120 125
   Pro Glu Ser Gly Pro Gly Ser Glu Pro Ala Thr Ser Gly Ser Glu Thr
   130 135 140
   Pro Gly Ser Glu Pro Ala Thr Ser Gly Ser Glu Thr Pro Gly Ser Pro
   145 150 155 160
   Ala Gly Ser Pro Thr Ser Thr Glu Glu Gly Thr Ser Glu Ser Ala Thr
   165 170 175
   Pro Glu Ser Gly Pro Gly Thr Ser Thr Glu Pro Ser Glu Gly Ser Ala
   180 185 190
   Pro Gly Thr Ser Thr Glu Pro Ser Glu Gly Ser Ala Pro Gly Ser Pro
   195 200 205
   Ala Gly Ser Pro Thr Ser Thr Glu Glu Gly Thr Ser Thr Glu Pro Ser
   210 215 220
   Glu Gly Ser Ala Pro Gly Thr Ser Thr Glu Pro Ser Glu Gly Ser Ala
   225 230 235 240
   Pro Gly Thr Ser Glu Ser Ala Thr Pro Glu Ser Gly Pro Gly Thr Ser
   245 250 255
   Thr Glu Pro Ser Glu Gly Ser Ala Pro Gly Thr Ser Glu Ser Ala Thr
   260 265 270
   Pro Glu Ser Gly Pro Gly Ser Glu Pro Ala Thr Ser Gly Ser Glu Thr
   275 280 285
   Pro Gly Thr Ser Thr Glu Pro Ser Glu Gly Ser Ala Pro Gly Thr Ser
   290 295 300
   Thr Glu Pro Ser Glu Gly Ser Ala Pro Gly Thr Ser Glu Ser Ala Thr
   305 310 315 320
   Pro Glu Ser Gly Pro Gly Thr Ser Glu Ser Ala Thr Pro Glu Ser Gly
                   325                 330                 335
   Pro Gly Ser Pro Ala Gly Ser Pro Thr Ser Thr Glu Glu Gly Thr Ser
               340                 345                 350
   Glu Ser Ala Thr Pro Glu Ser Gly Pro Gly Ser Glu Pro Ala Thr Ser
           355                 360                 365
   Gly Ser Glu Thr Pro Gly Thr Ser Glu Ser Ala Thr Pro Glu Ser Gly
       370                 375                 380
   Pro Gly Thr Ser Thr Glu Pro Ser Glu Gly Ser Ala Pro Gly Thr Ser
   385                 390                 395                 400
   Thr Glu Pro Ser Glu Gly Ser Ala Pro Gly Thr Ser Thr Glu Pro Ser
                   405                 410                 415
   Glu Gly Ser Ala Pro Gly Thr Ser Thr Glu Pro Ser Glu Gly Ser Ala
               420                 425                 430
   Pro Gly Thr Ser Thr Glu Pro Ser Glu Gly Ser Ala Pro Gly Thr Ser
           435                 440                 445
   Thr Glu Pro Ser Glu Gly Ser Ala Pro Gly Ser Pro Ala Gly Ser Pro
       450                 455                 460
   Thr Ser Thr Glu Glu Gly Thr Ser Thr Glu Pro Ser Glu Gly Ser Ala
   465                 470                 475                 480
   Pro Gly Thr Ser Glu Ser Ala Thr Pro Glu Ser Gly Pro Gly Ser Glu
                   485                 490                 495
   Pro Ala Thr Ser Gly Ser Glu Thr Pro Gly Thr Ser Glu Ser Ala Thr
               500                 505                 510
   Pro Glu Ser Gly Pro Gly Ser Glu Pro Ala Thr Ser Gly Ser Glu Thr
           515                 520                 525
   Pro Gly Thr Ser Glu Ser Ala Thr Pro Glu Ser Gly Pro Gly Thr Ser
       530                 535                 540
   Thr Glu Pro Ser Glu Gly Ser Ala Pro Gly Thr Ser Glu Ser Ala Thr
   545                 550                 555                 560
   Pro Glu Ser Gly Pro Gly Ser Pro Ala Gly Ser Pro Thr Ser Thr Glu
                   565                 570                 575
   Glu Gly Ser Pro Ala Gly Ser Pro Thr Ser Thr Glu Glu Gly Ser Pro
   580 585 590
   Ala Gly Ser Pro Thr Ser Thr Glu Glu Gly Thr Ser Glu Ser Ala Thr
   595 600 605
   Pro Glu Ser Gly Pro Gly Thr Ser Thr Glu Pro Ser Glu Gly Ser Ala
   610 615 620
   Pro Gly Thr Ser Glu Ser Ala Thr Pro Glu Ser Gly Pro Gly Ser Glu
   625 630 635 640
   Pro Ala Thr Ser Gly Ser Glu Thr Pro Gly Thr Ser Glu Ser Ala Thr
   645 650 655
   Pro Glu Ser Gly Pro Gly Ser Glu Pro Ala Thr Ser Gly Ser Glu Thr
   660 665 670
   Pro Gly Thr Ser Glu Ser Ala Thr Pro Glu Ser Gly Pro Gly Thr Ser
   675 680 685
   Thr Glu Pro Ser Glu Gly Ser Ala Pro Gly Ser Pro Ala Gly Ser Pro
   690 695 700
   Thr Ser Thr Glu Glu Gly Thr Ser Glu Ser Ala Thr Pro Glu Ser Gly
   705 710 715 720
   Pro Gly Ser Glu Pro Ala Thr Ser Gly Ser Glu Thr Pro Gly Thr Ser
   725 730 735
   Glu Ser Ala Thr Pro Glu Ser Gly Pro Gly Ser Pro Ala Gly Ser Pro
   740 745 750
   Thr Ser Thr Glu Glu Gly Ser Pro Ala Gly Ser Pro Thr Ser Thr Glu
   755 760 765
   Glu Gly Thr Ser Thr Glu Pro Ser Glu Gly Ser Ala Pro Gly Thr Ser
   770 775 780
   Glu Ser Ala Thr Pro Glu Ser Gly Pro Gly Thr Ser Glu Ser Ala Thr
   785 790 795 800
   Pro Glu Ser Gly Pro Gly Thr Ser Glu Ser Ala Thr Pro Glu Ser Gly
   805 810 815
   Pro Gly Ser Glu Pro Ala Thr Ser Gly Ser Glu Thr Pro Gly Ser Glu
   820 825 830
   Pro Ala Thr Ser Gly Ser Glu Thr Pro Gly Ser Pro Ala Gly Ser Pro
   835 840 845
   Thr Ser Thr Glu Glu Gly Thr Ser Thr Glu Pro Ser Glu Gly Ser Ala
   850 855 860
   Pro Gly Thr Ser Thr Glu Pro Ser Glu Gly Ser Ala Pro Gly Ser Glu
   865 870 875 880
   Pro Ala Thr Ser Gly Ser Glu Thr Pro Gly Thr Ser Glu Ser Ala Thr
   885 890 895
   Pro Glu Ser Gly Pro Gly Thr Ser Thr Glu Pro Ser Glu Gly Ser Ala
   900 905 910
   Pro
<210> 48
   <211> 924
   <212> PRT
   <213> Artificial Sequence
<220>
   <221> source
   <223> /note="Description of Artificial Sequence: Synthetic polypeptide"
<400> 48
   Met Ala Glu Pro Ala Gly Ser Pro Thr Ser Thr Glu Glu Gly Ala Ser
   1 5 10 15
   Pro Gly Thr Ser Ser Thr Gly Ser Pro Gly Ser Ser Thr Pro Ser Gly
   20 25 30
   Ala Thr Gly Ser Pro Gly Ser Ser Thr Pro Ser Gly Ala Thr Gly Ser
   35 40 45
   Pro Gly Thr Ser Thr Glu Pro Ser Glu Gly Ser Ala Pro Gly Ser Glu
   50 55 60
   Pro Ala Thr Ser Gly Ser Glu Thr Pro Gly Ser Pro Ala Gly Ser Pro
   65 70 75 80
   Thr Ser Thr Glu Glu Gly Ser Thr Ser Ser Thr Ala Glu Ser Pro Gly
   85 90 95
   Pro Gly Thr Ser Thr Pro Glu Ser Gly Ser Ala Ser Pro Gly Ser Thr
   100 105 110
   Ser Glu Ser Pro Ser Gly Thr Ala Pro Gly Ser Thr Ser Glu Ser Pro
           115                 120                 125
   Ser Gly Thr Ala Pro Gly Thr Ser Thr Pro Glu Ser Gly Ser Ala Ser
       130                 135                 140
   Pro Gly Thr Ser Thr Pro Glu Ser Gly Ser Ala Ser Pro Gly Ser Glu
   145                 150                 155                 160
   Pro Ala Thr Ser Gly Ser Glu Thr Pro Gly Thr Ser Glu Ser Ala Thr
                   165                 170                 175
   Pro Glu Ser Gly Pro Gly Ser Pro Ala Gly Ser Pro Thr Ser Thr Glu
               180                 185                 190
   Glu Gly Thr Ser Thr Glu Pro Ser Glu Gly Ser Ala Pro Gly Thr Ser
           195                 200                 205
   Glu Ser Ala Thr Pro Glu Ser Gly Pro Gly Thr Ser Thr Glu Pro Ser
       210                 215                 220
   Glu Gly Ser Ala Pro Gly Thr Ser Thr Glu Pro Ser Glu Gly Ser Ala
   225                 230                 235                 240
   Pro Gly Ser Pro Ala Gly Ser Pro Thr Ser Thr Glu Glu Gly Thr Ser
                   245                 250                 255
   Thr Glu Pro Ser Glu Gly Ser Ala Pro Gly Thr Ser Thr Glu Pro Ser
               260                 265                 270
   Glu Gly Ser Ala Pro Gly Thr Ser Glu Ser Ala Thr Pro Glu Ser Gly
           275                 280                 285
   Pro Gly Thr Ser Glu Ser Ala Thr Pro Glu Ser Gly Pro Gly Thr Ser
       290                 295                 300
   Thr Glu Pro Ser Glu Gly Ser Ala Pro Gly Thr Ser Thr Glu Pro Ser
   305                 310                 315                 320
   Glu Gly Ser Ala Pro Gly Thr Ser Glu Ser Ala Thr Pro Glu Ser Gly
                   325                 330                 335
   Pro Gly Thr Ser Thr Glu Pro Ser Glu Gly Ser Ala Pro Gly Ser Glu
               340                 345                 350
   Pro Ala Thr Ser Gly Ser Glu Thr Pro Gly Ser Pro Ala Gly Ser Pro
           355                 360                 365
   Thr Ser Thr Glu Glu Gly Ser Ser Thr Pro Ser Gly Ala Thr Gly Ser
       370                 375                 380
   Pro Gly Thr Pro Gly Ser Gly Thr Ala Ser Ser Ser Pro Gly Ser Ser
   385                 390                 395                 400
   Thr Pro Ser Gly Ala Thr Gly Ser Pro Gly Thr Ser Thr Glu Pro Ser
                   405                 410                 415
   Glu Gly Ser Ala Pro Gly Thr Ser Thr Glu Pro Ser Glu Gly Ser Ala
               420                 425                 430
   Pro Gly Ser Glu Pro Ala Thr Ser Gly Ser Glu Thr Pro Gly Ser Pro
           435                 440                 445
   Ala Gly Ser Pro Thr Ser Thr Glu Glu Gly Ser Pro Ala Gly Ser Pro
       450                 455                 460
   Thr Ser Thr Glu Glu Gly Thr Ser Thr Glu Pro Ser Glu Gly Ser Ala
   465                 470                 475                 480
   Pro Gly Ala Ser Ala Ser Gly Ala Pro Ser Thr Gly Gly Thr Ser Glu
                   485                 490                 495
   Ser Ala Thr Pro Glu Ser Gly Pro Gly Ser Pro Ala Gly Ser Pro Thr
               500                 505                 510
   Ser Thr Glu Glu Gly Ser Pro Ala Gly Ser Pro Thr Ser Thr Glu Glu
           515                 520                 525
   Gly Ser Thr Ser Ser Thr Ala Glu Ser Pro Gly Pro Gly Ser Thr Ser
       530                 535                 540
   Glu Ser Pro Ser Gly Thr Ala Pro Gly Thr Ser Pro Ser Gly Glu Ser
   545                 550                 555                 560
   Ser Thr Ala Pro Gly Thr Pro Gly Ser Gly Thr Ala Ser Ser Ser Pro
                   565                 570                 575
   Gly Ser Ser Thr Pro Ser Gly Ala Thr Gly Ser Pro Gly Ser Ser Pro
               580                 585                 590
   Ser Ala Ser Thr Gly Thr Gly Pro Gly Ser Glu Pro Ala Thr Ser Gly
           595                 600                 605
   Ser Glu Thr Pro Gly Thr Ser Glu Ser Ala Thr Pro Glu Ser Gly Pro
       610                 615                 620
   Gly Ser Glu Pro Ala Thr Ser Gly Ser Glu Thr Pro Gly Ser Thr Ser
   625                 630                 635                 640
   Ser Thr Ala Glu Ser Pro Gly Pro Gly Ser Thr Ser Ser Thr Ala Glu
                   645                 650                 655
   Ser Pro Gly Pro Gly Thr Ser Pro Ser Gly Glu Ser Ser Thr Ala Pro
               660                 665                 670
   Gly Ser Glu Pro Ala Thr Ser Gly Ser Glu Thr Pro Gly Ser Glu Pro
           675                 680                 685
   Ala Thr Ser Gly Ser Glu Thr Pro Gly Thr Ser Thr Glu Pro Ser Glu
       690                 695                 700
   Gly Ser Ala Pro Gly Ser Thr Ser Ser Thr Ala Glu Ser Pro Gly Pro
   705                 710                 715                 720
   Gly Thr Ser Thr Pro Glu Ser Gly Ser Ala Ser Pro Gly Ser Thr Ser
                   725                 730                 735
   Glu Ser Pro Ser Gly Thr Ala Pro Gly Thr Ser Thr Glu Pro Ser Glu
               740                 745                 750
   Gly Ser Ala Pro Gly Thr Ser Thr Glu Pro Ser Glu Gly Ser Ala Pro
           755                 760                 765
   Gly Thr Ser Thr Glu Pro Ser Glu Gly Ser Ala Pro Gly Ser Ser Thr
       770                 775                 780
   Pro Ser Gly Ala Thr Gly Ser Pro Gly Ser Ser Pro Ser Ala Ser Thr
   785                 790                 795                 800
   Gly Thr Gly Pro Gly Ala Ser Pro Gly Thr Ser Ser Thr Gly Ser Pro
                   805                 810                 815
   Gly Ser Glu Pro Ala Thr Ser Gly Ser Glu Thr Pro Gly Thr Ser Glu
               820                 825                 830
   Ser Ala Thr Pro Glu Ser Gly Pro Gly Ser Pro Ala Gly Ser Pro Thr
           835                 840                 845
   Ser Thr Glu Glu Gly Ser Ser Thr Pro Ser Gly Ala Thr Gly Ser Pro
       850                 855                 860
   Gly Ser Ser Pro Ser Ala Ser Thr Gly Thr Gly Pro Gly Ala Ser Pro
   865 870 875 880
   Gly Thr Ser Ser Thr Gly Ser Pro Gly Thr Ser Glu Ser Ala Thr Pro
   885 890 895
   Glu Ser Gly Pro Gly Thr Ser Thr Glu Pro Ser Glu Gly Ser Ala Pro
   900 905 910
   Gly Thr Ser Thr Glu Pro Ser Glu Gly Ser Ala Pro
   915 920
<210> 49
   <211> 1318
   <212> PRT
   <213> Artificial Sequence
<220>
   <221> source
   <223> /note="Description of Artificial Sequence: Synthetic polypeptide"
<400> 49
   Gly Thr Ser Thr Glu Pro Ser Glu Gly Ser Ala Pro Gly Ser Glu Pro
   1 5 10 15
   Ala Thr Ser Gly Ser Glu Thr Pro Gly Ser Pro Ala Gly Ser Pro Thr
   20 25 30
   Ser Thr Glu Glu Gly Ser Thr Ser Ser Thr Ala Glu Ser Pro Gly Pro
   35 40 45
   Gly Thr Ser Thr Pro Glu Ser Gly Ser Ala Ser Pro Gly Ser Thr Ser
   50 55 60
   Glu Ser Pro Ser Gly Thr Ala Pro Gly Ser Thr Ser Glu Ser Pro Ser
   65 70 75 80
   Gly Thr Ala Pro Gly Thr Ser Thr Pro Glu Ser Gly Ser Ala Ser Pro
   85 90 95
   Gly Thr Ser Thr Pro Glu Ser Gly Ser Ala Ser Pro Gly Ser Glu Pro
   100 105 110
   Ala Thr Ser Gly Ser Glu Thr Pro Gly Thr Ser Glu Ser Ala Thr Pro
   115 120 125
   Glu Ser Gly Pro Gly Ser Pro Ala Gly Ser Pro Thr Ser Thr Glu Glu
   130 135 140
   Gly Thr Ser Thr Glu Pro Ser Glu Gly Ser Ala Pro Gly Thr Ser Glu
   145                 150                 155                 160
   Ser Ala Thr Pro Glu Ser Gly Pro Gly Thr Ser Thr Glu Pro Ser Glu
                   165                 170                 175
   Gly Ser Ala Pro Gly Thr Ser Thr Glu Pro Ser Glu Gly Ser Ala Pro
               180                 185                 190
   Gly Ser Pro Ala Gly Ser Pro Thr Ser Thr Glu Glu Gly Thr Ser Thr
           195                 200                 205
   Glu Pro Ser Glu Gly Ser Ala Pro Gly Thr Ser Thr Glu Pro Ser Glu
       210                 215                 220
   Gly Ser Ala Pro Gly Thr Ser Glu Ser Ala Thr Pro Glu Ser Gly Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Thr Glu Pro Ser Glu
               260                 265                 270
   Gly Ser Ala Pro Gly Thr Ser Glu Ser Ala Thr Pro Glu Ser Gly Pro
           275                 280                 285
   Gly Thr Ser Thr Glu Pro Ser Glu Gly Ser Ala Pro Gly Ser Glu Pro
       290                 295                 300
   Ala Thr Ser Gly Ser Glu Thr Pro Gly Ser Pro Ala Gly Ser Pro Thr
   305                 310                 315                 320
   Ser Thr Glu Glu Gly Ser Ser Thr Pro Ser Gly Ala Thr Gly Ser Pro
                   325                 330                 335
   Gly Thr Pro Gly Ser Gly Thr Ala Ser Ser Ser Pro Gly Ser Ser Thr
               340                 345                 350
   Pro Ser Gly Ala Thr Gly Ser Pro Gly Thr Ser Thr Glu Pro Ser Glu
           355                 360                 365
   Gly Ser Ala Pro Gly Thr Ser Thr Glu Pro Ser Glu Gly Ser Ala Pro
       370                 375                 380
   Gly Ser Glu Pro Ala Thr Ser Gly Ser Glu Thr Pro Gly Ser Pro Ala
   385                 390                 395                 400
   Gly Ser Pro Thr Ser Thr Glu Glu Gly Ser Pro Ala Gly Ser Pro Thr
   405 410 415
   Ser Thr Glu Glu Gly Thr Ser Thr Glu Pro Ser Glu Gly Ser Ala Pro
   420 425 430
   Gly Pro Glu Pro Thr Gly Pro Ala Pro Ser Gly Gly Ser Glu Pro Ala
   435 440 445
   Thr Ser Gly Ser Glu Thr Pro Gly Thr Ser Glu Ser Ala Thr Pro Glu
   450 455 460
   Ser Gly Pro Gly Ser Pro Ala Gly Ser Pro Thr Ser Thr Glu Glu Gly
   465 470 475 480
   Thr Ser Glu Ser Ala Thr Pro Glu Ser Gly Pro Gly Ser Pro Ala Gly
   485 490 495
   Ser Pro Thr Ser Thr Glu Glu Gly Ser Pro Ala Gly Ser Pro Thr Ser
   500 505 510
   Thr Glu Glu Gly Thr Ser Glu Ser Ala Thr Pro Glu Ser Gly Pro Gly
   515 520 525
   Ser Pro Ala Gly Ser Pro Thr Ser Thr Glu Glu Gly Ser Pro Ala Gly
   530 535 540
   Ser Pro Thr Ser Thr Glu Glu Gly Ser Thr Ser Ser Thr Ala Glu Ser
   545 550 555 560
   Pro Gly Pro Gly Ser Thr Ser Glu Ser Pro Ser Gly Thr Ala Pro Gly
   565 570 575
   Thr Ser Pro Ser Gly Glu Ser Ser Thr Ala Pro Gly Ser Thr Ser Glu
   580 585 590
   Ser Pro Ser Gly Thr Ala Pro Gly Ser Thr Ser Glu Ser Pro Ser Gly
   595 600 605
   Thr Ala Pro Gly Thr Ser Pro Ser Gly Glu Ser Ser Thr Ala Pro Gly
   610 615 620
   Thr Ser Thr Glu Pro Ser Glu Gly Ser Ala Pro Gly Thr Ser Glu Ser
   625 630 635 640
   Ala Thr Pro Glu Ser Gly Pro Gly Thr Ser Glu Ser Ala Thr Pro Glu
   645 650 655
   Ser Gly Pro Gly Ser Glu Pro Ala Thr Ser Gly Ser Glu Thr Pro Gly
   660 665 670
   Thr Ser Glu Ser Ala Thr Pro Glu Ser Gly Pro Gly Thr Ser Glu Ser
   675 680 685
   Ala Thr Pro Glu Ser Gly Pro Gly Thr Ser Thr Glu Pro Ser Glu Gly
   690 695 700
   Ser Ala Pro Gly Thr Ser Glu Ser Ala Thr Pro Glu Ser Gly Pro Gly
   705 710 715 720
   Thr Ser Thr Glu Pro Ser Glu Gly Ser Ala Pro Gly Thr Ser Pro Ser
   725 730 735
   Gly Glu Ser Ser Thr Ala Pro Gly Thr Ser Pro Ser Gly Glu Ser Ser
   740 745 750
   Thr Ala Pro Gly Thr Ser Pro Ser Gly Glu Ser Ser Thr Ala Pro Gly
   755 760 765
   Thr Ser Thr Glu Pro Ser Glu Gly Ser Ala Pro Gly Ser Pro Ala Gly
   770 775 780
   Ser Pro Thr Ser Thr Glu Glu Gly Thr Ser Thr Glu Pro Ser Glu Gly
   785 790 795 800
   Ser Ala Pro Gly Ser Ser Pro Ser Ala Ser Thr Gly Thr Gly Pro Gly
   805 810 815
   Ser Ser Thr Pro Ser Gly Ala Thr Gly Ser Pro Gly Ser Ser Thr Pro
   820 825 830
   Ser Gly Ala Thr Gly Ser Pro Gly Ser Ser Thr Pro Ser Gly Ala Thr
   835 840 845
   Gly Ser Pro Gly Ser Ser Thr Pro Ser Gly Ala Thr Gly Ser Pro Gly
   850 855 860
   Ala Ser Pro Gly Thr Ser Ser Thr Gly Ser Pro Gly Ala Ser Ala Ser
   865 870 875 880
   Gly Ala Pro Ser Thr Gly Gly Thr Ser Pro Ser Gly Glu Ser Ser Thr
   885 890 895
   Ala Pro Gly Ser Thr Ser Ser Thr Ala Glu Ser Pro Gly Pro Gly Thr
               900                 905                 910
   Ser Pro Ser Gly Glu Ser Ser Thr Ala Pro Gly Thr Ser Glu Ser Ala
           915                 920                 925
   Thr Pro Glu Ser Gly Pro Gly Thr Ser Thr Glu Pro Ser Glu Gly Ser
       930                 935                 940
   Ala Pro Gly Thr Ser Thr Glu Pro Ser Glu Gly Ser Ala Pro Gly Ser
   945                 950                 955                 960
   Ser Pro Ser Ala Ser Thr Gly Thr Gly Pro Gly Ser Ser Thr Pro Ser
                   965                 970                 975
   Gly Ala Thr Gly Ser Pro Gly Ala Ser Pro Gly Thr Ser Ser Thr Gly
               980                 985                 990
   Ser Pro Gly Thr Ser Thr Pro Glu  Ser Gly Ser Ala Ser  Pro Gly Thr
           995                 1000                 1005
   Ser Pro  Ser Gly Glu Ser Ser  Thr Ala Pro Gly Thr  Ser Pro Ser
       1010                 1015                 1020
   Gly Glu  Ser Ser Thr Ala Pro  Gly Thr Ser Glu Ser  Ala Thr Pro
       1025                 1030                 1035
   Glu Ser  Gly Pro Gly Ser Glu  Pro Ala Thr Ser Gly  Ser Glu Thr
       1040                 1045                 1050
   Pro Gly  Thr Ser Thr Glu Pro  Ser Glu Gly Ser Ala  Pro Gly Ser
       1055                 1060                 1065
   Thr Ser  Glu Ser Pro Ser Gly  Thr Ala Pro Gly Ser  Thr Ser Glu
       1070                 1075                 1080
   Ser Pro  Ser Gly Thr Ala Pro  Gly Thr Ser Thr Pro  Glu Ser Gly
       1085                 1090                 1095
   Ser Ala  Ser Pro Gly Ser Pro  Ala Gly Ser Pro Thr  Ser Thr Glu
       1100                 1105                 1110
   Glu Gly  Thr Ser Glu Ser Ala  Thr Pro Glu Ser Gly  Pro Gly Thr
       1115                 1120                 1125
   Ser Thr  Glu Pro Ser Glu Gly  Ser Ala Pro Gly Ser  Pro Ala Gly
       1130                 1135                 1140
   Ser Pro Thr Ser Thr Glu Glu Gly Thr Ser Glu Ser Ala Thr Pro
   1145 1150 1155
   Glu Ser Gly Pro Gly Ser Glu Pro Ala Thr Ser Gly Ser Glu Thr
   1160 1165 1170
   Pro Gly Ser Ser Thr Pro Ser Gly Ala Thr Gly Ser Pro Gly Ala
   1175 1180 1185
   Ser Pro Gly Thr Ser Ser Thr Gly Ser Pro Gly Ser Ser Thr Pro
   1190 1195 1200
   Ser Gly Ala Thr Gly Ser Pro Gly Ser Thr Ser Glu Ser Pro Ser
   1205 1210 1215
   Gly Thr Ala Pro Gly Thr Ser Pro Ser Gly Glu Ser Ser Thr Ala
   1220 1225 1230
   Pro Gly Ser Thr Ser Ser Thr Ala Glu Ser Pro Gly Pro Gly Ser
   1235 1240 1245
   Ser Thr Pro Ser Gly Ala Thr Gly Ser Pro Gly Ala Ser Pro Gly
   1250 1255 1260
   Thr Ser Ser Thr Gly Ser Pro Gly Thr Pro Gly Ser Gly Thr Ala
   1265 1270 1275
   Ser Ser Ser Pro Gly Ser Pro Ala Gly Ser Pro Thr Ser Thr Glu
   1280 1285 1290
   Glu Gly Ser Pro Ala Gly Ser Pro Thr Ser Thr Glu Glu Gly Thr
   1295 1300 1305
   Ser Thr Glu Pro Ser Glu Gly Ser Ala Pro
   1310 1315
<210> 50
   <211> 864
   <212> PRT
   <213> Artificial Sequence
<220>
   <221> source
   <223> /note="Description of Artificial Sequence: Synthetic polypeptide"
<400> 50
   Gly Thr Ser Thr Glu Pro Ser Glu Pro Gly Ser Ala Gly Thr Ser Thr
   1 5 10 15
   Glu Pro Ser Glu Pro Gly Ser Ala Gly Ser Glu Pro Ala Thr Ser Gly
               20                  25                  30
   Thr Glu Pro Ser Gly Ser Gly Ala Ser Glu Pro Thr Ser Thr Glu Pro
           35                  40                  45
   Gly Ser Glu Pro Ala Thr Ser Gly Thr Glu Pro Ser Gly Ser Glu Pro
       50                  55                  60
   Ala Thr Ser Gly Thr Glu Pro Ser Gly Ser Glu Pro Ala Thr Ser Gly
   65                  70                  75                  80
   Thr Glu Pro Ser Gly Ser Gly Ala Ser Glu Pro Thr Ser Thr Glu Pro
                   85                  90                  95
   Gly Thr Ser Thr Glu Pro Ser Glu Pro Gly Ser Ala Gly Ser Glu Pro
               100                 105                 110
   Ala Thr Ser Gly Thr Glu Pro Ser Gly Thr Ser Thr Glu Pro Ser Glu
           115                 120                 125
   Pro Gly Ser Ala Gly Ser Glu Pro Ala Thr Ser Gly Thr Glu Pro Ser
       130                 135                 140
   Gly Ser Glu Pro Ala Thr Ser Gly Thr Glu Pro Ser Gly Thr Ser Thr
   145                 150                 155                 160
   Glu Pro Ser Glu Pro Gly Ser Ala Gly Thr Ser Thr Glu Pro Ser Glu
                   165                 170                 175
   Pro Gly Ser Ala Gly Ser Glu Pro Ala Thr Ser Gly Thr Glu Pro Ser
               180                 185                 190
   Gly Ser Glu Pro Ala Thr Ser Gly Thr Glu Pro Ser Gly Thr Ser Glu
           195                 200                 205
   Pro Ser Thr Ser Glu Pro Gly Ala Gly Ser Gly Ala Ser Glu Pro Thr
       210                 215                 220
   Ser Thr Glu Pro Gly Thr Ser Glu Pro Ser Thr Ser Glu Pro Gly Ala
   225                 230                 235                 240
   Gly Ser Glu Pro Ala Thr Ser Gly Thr Glu Pro Ser Gly Ser Glu Pro
                   245                 250                 255
   Ala Thr Ser Gly Thr Glu Pro Ser Gly Thr Ser Thr Glu Pro Ser Glu
               260                 265                 270
   Pro Gly Ser Ala Gly Thr Ser Thr Glu Pro Ser Glu Pro Gly Ser Ala
   275 280 285
   Gly Ser Gly Ala Ser Glu Pro Thr Ser Thr Glu Pro Gly Ser Glu Pro
   290 295 300
   Ala Thr Ser Gly Thr Glu Pro Ser Gly Ser Glu Pro Ala Thr Ser Gly
   305 310 315 320
   Thr Glu Pro Ser Gly Ser Glu Pro Ala Thr Ser Gly Thr Glu Pro Ser
   325 330 335
   Gly Ser Glu Pro Ala Thr Ser Gly Thr Glu Pro Ser Gly Thr Ser Thr
   340 345 350
   Glu Pro Ser Glu Pro Gly Ser Ala Gly Ser Glu Pro Ala Thr Ser Gly
   355 360 365
   Thr Glu Pro Ser Gly Ser Gly Ala Ser Glu Pro Thr Ser Thr Glu Pro
   370 375 380
   Gly Thr Ser Thr Glu Pro Ser Glu Pro Gly Ser Ala Gly Ser Glu Pro
   385 390 395 400
   Ala Thr Ser Gly Thr Glu Pro Ser Gly Ser Gly Ala Ser Glu Pro Thr
   405 410 415
   Ser Thr Glu Pro Gly Thr Ser Thr Glu Pro Ser Glu Pro Gly Ser Ala
   420 425 430
   Gly Ser Gly Ala Ser Glu Pro Thr Ser Thr Glu Pro Gly Ser Glu Pro
   435 440 445
   Ala Thr Ser Gly Thr Glu Pro Ser Gly Ser Gly Ala Ser Glu Pro Thr
   450 455 460
   Ser Thr Glu Pro Gly Ser Glu Pro Ala Thr Ser Gly Thr Glu Pro Ser
   465 470 475 480
   Gly Ser Gly Ala Ser Glu Pro Thr Ser Thr Glu Pro Gly Thr Ser Thr
   485 490 495
   Glu Pro Ser Glu Pro Gly Ser Ala Gly Ser Glu Pro Ala Thr Ser Gly
   500 505 510
   Thr Glu Pro Ser Gly Ser Gly Ala Ser Glu Pro Thr Ser Thr Glu Pro
               515                 520                 525
   Gly Thr Ser Thr Glu Pro Ser Glu Pro Gly Ser Ala Gly Ser Glu Pro
       530                 535                 540
   Ala Thr Ser Gly Thr Glu Pro Ser Gly Thr Ser Thr Glu Pro Ser Glu
   545                 550                 555                 560
   Pro Gly Ser Ala Gly Ser Glu Pro Ala Thr Ser Gly Thr Glu Pro Ser
                   565                 570                 575
   Gly Thr Ser Thr Glu Pro Ser Glu Pro Gly Ser Ala Gly Thr Ser Thr
               580                 585                 590
   Glu Pro Ser Glu Pro Gly Ser Ala Gly Thr Ser Thr Glu Pro Ser Glu
           595                 600                 605
   Pro Gly Ser Ala Gly Thr Ser Thr Glu Pro Ser Glu Pro Gly Ser Ala
       610                 615                 620
   Gly Thr Ser Thr Glu Pro Ser Glu Pro Gly Ser Ala Gly Thr Ser Thr
   625                 630                 635                 640
   Glu Pro Ser Glu Pro Gly Ser Ala Gly Thr Ser Glu Pro Ser Thr Ser
                   645                 650                 655
   Glu Pro Gly Ala Gly Ser Gly Ala Ser Glu Pro Thr Ser Thr Glu Pro
               660                 665                 670
   Gly Thr Ser Thr Glu Pro Ser Glu Pro Gly Ser Ala Gly Thr Ser Thr
           675                 680                 685
   Glu Pro Ser Glu Pro Gly Ser Ala Gly Thr Ser Thr Glu Pro Ser Glu
       690                 695                 700
   Pro Gly Ser Ala Gly Ser Glu Pro Ala Thr Ser Gly Thr Glu Pro Ser
   705                 710                 715                 720
   Gly Ser Gly Ala Ser Glu Pro Thr Ser Thr Glu Pro Gly Ser Glu Pro
                   725                 730                 735
   Ala Thr Ser Gly Thr Glu Pro Ser Gly Ser Glu Pro Ala Thr Ser Gly
               740                 745                 750
   Thr Glu Pro Ser Gly Ser Glu Pro Ala Thr Ser Gly Thr Glu Pro Ser
           755                 760                 765
   Gly Ser Glu Pro Ala Thr Ser Gly Thr Glu Pro Ser Gly Thr Ser Glu
       770                 775                 780
   Pro Ser Thr Ser Glu Pro Gly Ala Gly Ser Glu Pro Ala Thr Ser Gly
   785                 790                 795                 800
   Thr Glu Pro Ser Gly Ser Gly Ala Ser Glu Pro Thr Ser Thr Glu Pro
                   805                 810                 815
   Gly Thr Ser Thr Glu Pro Ser Glu Pro Gly Ser Ala Gly Ser Glu Pro
               820                 825                 830
   Ala Thr Ser Gly Thr Glu Pro Ser Gly Ser Gly Ala Ser Glu Pro Thr
           835                 840                 845
   Ser Thr Glu Pro Gly Thr Ser Thr Glu Pro Ser Glu Pro Gly Ser Ala
       850                 855                 860
<210> 51
   <211> 864
   <212> PRT
   <213> Artificial Sequence
<220>
   <221> source
   <223> /note="Description of Artificial Sequence: Synthetic polypeptide"
<400> 51
   Gly Ser Glu Thr Ala Thr Ser Gly Ser Glu Thr Ala Gly Thr Ser Glu
   1               5                   10                  15
   Ser Ala Thr Ser Glu Ser Gly Ala Gly Ser Thr Ala Gly Ser Glu Thr
               20                  25                  30
   Ser Thr Glu Ala Gly Thr Ser Glu Ser Ala Thr Ser Glu Ser Gly Ala
           35                  40                  45
   Gly Ser Glu Thr Ala Thr Ser Gly Ser Glu Thr Ala Gly Ser Glu Thr
       50                  55                  60
   Ala Thr Ser Gly Ser Glu Thr Ala Gly Thr Ser Thr Glu Ala Ser Glu
   65                  70                  75                  80
   Gly Ser Ala Ser Gly Thr Ser Thr Glu Ala Ser Glu Gly Ser Ala Ser
                   85                  90                  95
   Gly Thr Ser Glu Ser Ala Thr Ser Glu Ser Gly Ala Gly Ser Glu Thr
               100                 105                 110
   Ala Thr Ser Gly Ser Glu Thr Ala Gly Thr Ser Thr Glu Ala Ser Glu
           115                 120                 125
   Gly Ser Ala Ser Gly Ser Thr Ala Gly Ser Glu Thr Ser Thr Glu Ala
       130                 135                 140
   Gly Thr Ser Glu Ser Ala Thr Ser Glu Ser Gly Ala Gly Thr Ser Glu
   145                 150                 155                 160
   Ser Ala Thr Ser Glu Ser Gly Ala Gly Ser Glu Thr Ala Thr Ser Gly
                   165                 170                 175
   Ser Glu Thr Ala Gly Thr Ser Glu Ser Ala Thr Ser Glu Ser Gly Ala
               180                 185                 190
   Gly Thr Ser Thr Glu Ala Ser Glu Gly Ser Ala Ser Gly Ser Glu Thr
           195                 200                 205
   Ala Thr Ser Gly Ser Glu Thr Ala Gly Ser Glu Thr Ala Thr Ser Gly
       210                 215                 220
   Ser Glu Thr Ala Gly Thr Ser Thr Glu Ala Ser Glu Gly Ser Ala Ser
   225                 230                 235                 240
   Gly Ser Thr Ala Gly Ser Glu Thr Ser Thr Glu Ala Gly Thr Ser Glu
                   245                 250                 255
   Ser Ala Thr Ser Glu Ser Gly Ala Gly Thr Ser Thr Glu Ala Ser Glu
               260                 265                 270
   Gly Ser Ala Ser Gly Ser Glu Thr Ala Thr Ser Gly Ser Glu Thr Ala
           275                 280                 285
   Gly Ser Thr Ala Gly Ser Glu Thr Ser Thr Glu Ala Gly Ser Thr Ala
       290                 295                 300
   Gly Ser Glu Thr Ser Thr Glu Ala Gly Ser Glu Thr Ala Thr Ser Gly
   305                 310                 315                 320
   Ser Glu Thr Ala Gly Thr Ser Glu Ser Ala Thr Ser Glu Ser Gly Ala
                   325                 330                 335
   Gly Thr Ser Glu Ser Ala Thr Ser Glu Ser Gly Ala Gly Ser Glu Thr
               340                 345                 350
   Ala Thr Ser Gly Ser Glu Thr Ala Gly Thr Ser Glu Ser Ala Thr Ser
           355                 360                 365
   Glu Ser Gly Ala Gly Thr Ser Glu Ser Ala Thr Ser Glu Ser Gly Ala
       370                 375                 380
   Gly Ser Glu Thr Ala Thr Ser Gly Ser Glu Thr Ala Gly Ser Glu Thr
   385                 390                 395                 400
   Ala Thr Ser Gly Ser Glu Thr Ala Gly Thr Ser Thr Glu Ala Ser Glu
                   405                 410                 415
   Gly Ser Ala Ser Gly Ser Thr Ala Gly Ser Glu Thr Ser Thr Glu Ala
               420                 425                 430
   Gly Ser Glu Thr Ala Thr Ser Gly Ser Glu Thr Ala Gly Thr Ser Glu
           435                 440                 445
   Ser Ala Thr Ser Glu Ser Gly Ala Gly Ser Thr Ala Gly Ser Glu Thr
       450                 455                 460
   Ser Thr Glu Ala Gly Ser Thr Ala Gly Ser Glu Thr Ser Thr Glu Ala
   465                 470                 475                 480
   Gly Ser Thr Ala Gly Ser Glu Thr Ser Thr Glu Ala Gly Thr Ser Thr
                   485                 490                 495
   Glu Ala Ser Glu Gly Ser Ala Ser Gly Ser Thr Ala Gly Ser Glu Thr
               500                 505                 510
   Ser Thr Glu Ala Gly Ser Thr Ala Gly Ser Glu Thr Ser Thr Glu Ala
           515                 520                 525
   Gly Thr Ser Thr Glu Ala Ser Glu Gly Ser Ala Ser Gly Ser Thr Ala
       530                 535                 540
   Gly Ser Glu Thr Ser Thr Glu Ala Gly Ser Glu Thr Ala Thr Ser Gly
   545                 550                 555                 560
   Ser Glu Thr Ala Gly Thr Ser Thr Glu Ala Ser Glu Gly Ser Ala Ser
                   565                 570                 575
   Gly Thr Ser Glu Ser Ala Thr Ser Glu Ser Gly Ala Gly Ser Glu Thr
               580                 585                 590
   Ala Thr Ser Gly Ser Glu Thr Ala Gly Thr Ser Glu Ser Ala Thr Ser
           595                 600                 605
   Glu Ser Gly Ala Gly Thr Ser Glu Ser Ala Thr Ser Glu Ser Gly Ala
       610                 615                 620
   Gly Ser Glu Thr Ala Thr Ser Gly Ser Glu Thr Ala Gly Thr Ser Glu
   625 630 635 640
   Ser Ala Thr Ser Glu Ser Gly Ala Gly Ser Glu Thr Ala Thr Ser Gly
   645 650 655
   Ser Glu Thr Ala Gly Thr Ser Thr Glu Ala Ser Glu Gly Ser Ala Ser
   660 665 670
   Gly Thr Ser Thr Glu Ala Ser Glu Gly Ser Ala Ser Gly Ser Thr Ala
   675 680 685
   Gly Ser Glu Thr Ser Thr Glu Ala Gly Ser Thr Ala Gly Ser Glu Thr
   690 695 700
   Ser Thr Glu Ala Gly Ser Glu Thr Ala Thr Ser Gly Ser Glu Thr Ala
   705 710 715 720
   Gly Thr Ser Glu Ser Ala Thr Ser Glu Ser Gly Ala Gly Thr Ser Glu
   725 730 735
   Ser Ala Thr Ser Glu Ser Gly Ala Gly Ser Glu Thr Ala Thr Ser Gly
   740 745 750
   Ser Glu Thr Ala Gly Ser Glu Thr Ala Thr Ser Gly Ser Glu Thr Ala
   755 760 765
   Gly Ser Glu Thr Ala Thr Ser Gly Ser Glu Thr Ala Gly Thr Ser Thr
   770 775 780
   Glu Ala Ser Glu Gly Ser Ala Ser Gly Thr Ser Glu Ser Ala Thr Ser
   785 790 795 800
   Glu Ser Gly Ala Gly Ser Glu Thr Ala Thr Ser Gly Ser Glu Thr Ala
   805 810 815
   Gly Ser Glu Thr Ala Thr Ser Gly Ser Glu Thr Ala Gly Thr Ser Glu
   820 825 830
   Ser Ala Thr Ser Glu Ser Gly Ala Gly Thr Ser Glu Ser Ala Thr Ser
   835 840 845
   Glu Ser Gly Ala Gly Ser Glu Thr Ala Thr Ser Gly Ser Glu Thr Ala
   850 855 860
<210> 52
   <211> 912
   <212> PRT
   <213> Artificial Sequence
<220>
   <221> source
   <223> /note="Description of Artificial Sequence: Synthetic polypeptide"
<400> 52
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
                   165                 170                 175
   Glu Ser Gly Pro Gly Thr Ser Thr Glu Pro Ser Glu Gly Ser Ala Pro
               180                 185                 190
   Gly Thr Ser Thr Glu Pro Ser Glu Gly Ser Ala Pro Gly Ser Pro Ala
           195                 200                 205
   Gly Ser Pro Thr Ser Thr Glu Glu Gly Thr Ser Thr Glu Pro Ser Glu
   210 215 220
   Gly Ser Ala Pro Gly Thr Ser Thr Glu Pro Ser Glu Gly Ser Ala Pro
   225 230 235 240
   Gly Thr Ser Glu Ser Ala Thr Pro Glu Ser Gly Pro Gly Thr Ser Thr
   245 250 255
   Glu Pro Ser Glu Gly Ser Ala Pro Gly Thr Ser Glu Ser Ala Thr Pro
   260 265 270
   Glu Ser Gly Pro Gly Ser Glu Pro Ala Thr Ser Gly Ser Glu Thr Pro
   275 280 285
   Gly Thr Ser Thr Glu Pro Ser Glu Gly Ser Ala Pro Gly Thr Ser Thr
   290 295 300
   Glu Pro Ser Glu Gly Ser Ala Pro Gly Thr Ser Glu Ser Ala Thr Pro
   305 310 315 320
   Glu Ser Gly Pro Gly Thr Ser Glu Ser Ala Thr Pro Glu Ser Gly Pro
   325 330 335
   Gly Ser Pro Ala Gly Ser Pro Thr Ser Thr Glu Glu Gly Thr Ser Glu
   340 345 350
   Ser Ala Thr Pro Glu Ser Gly Pro Gly Ser Glu Pro Ala Thr Ser Gly
   355 360 365
   Ser Glu Thr Pro Gly Thr Ser Glu Ser Ala Thr Pro Glu Ser Gly Pro
   370 375 380
   Gly Thr Ser Thr Glu Pro Ser Glu Gly Ser Ala Pro Gly Thr Ser Thr
   385 390 395 400
   Glu Pro Ser Glu Gly Ser Ala Pro Gly Thr Ser Thr Glu Pro Ser Glu
   405 410 415
   Gly Ser Ala Pro Gly Thr Ser Thr Glu Pro Ser Glu Gly Ser Ala Pro
   420 425 430
   Gly Thr Ser Thr Glu Pro Ser Glu Gly Ser Ala Pro Gly Thr Ser Thr
   435 440 445
   Glu Pro Ser Glu Gly Ser Ala Pro Gly Ser Pro Ala Gly Ser Pro Thr
   450 455 460
   Ser Thr Glu Glu Gly Thr Ser Thr Glu Pro Ser Glu Gly Ser Ala Pro
   465 470 475 480
   Gly Thr Ser Glu Ser Ala Thr Pro Glu Ser Gly Pro Gly Ser Glu Pro
   485 490 495
   Ala Thr Ser Gly Ser Glu Thr Pro Gly Thr Ser Glu Ser Ala Thr Pro
   500 505 510
   Glu Ser Gly Pro Gly Ser Glu Pro Ala Thr Ser Gly Ser Glu Thr Pro
   515 520 525
   Gly Thr Ser Glu Ser Ala Thr Pro Glu Ser Gly Pro Gly Thr Ser Thr
   530 535 540
   Glu Pro Ser Glu Gly Ser Ala Pro Gly Thr Ser Glu Ser Ala Thr Pro
   545 550 555 560
   Glu Ser Gly Pro Gly Ser Pro Ala Gly Ser Pro Thr Ser Thr Glu Glu
   565 570 575
   Gly Ser Pro Ala Gly Ser Pro Thr Ser Thr Glu Glu Gly Ser Pro Ala
   580 585 590
   Gly Ser Pro Thr Ser Thr Glu Glu Gly Thr Ser Glu Ser Ala Thr Pro
   595 600 605
   Glu Ser Gly Pro Gly Thr Ser Thr Glu Pro Ser Glu Gly Ser Ala Pro
   610 615 620
   Gly Thr Ser Glu Ser Ala Thr Pro Glu Ser Gly Pro Gly Ser Glu Pro
   625 630 635 640
   Ala Thr Ser Gly Ser Glu Thr Pro Gly Thr Ser Glu Ser Ala Thr Pro
   645 650 655
   Glu Ser Gly Pro Gly Ser Glu Pro Ala Thr Ser Gly Ser Glu Thr Pro
   660 665 670
   Gly Thr Ser Glu Ser Ala Thr Pro Glu Ser Gly Pro Gly Thr Ser Thr
   675 680 685
   Glu Pro Ser Glu Gly Ser Ala Pro Gly Ser Pro Ala Gly Ser Pro Thr
   690 695 700
   Ser Thr Glu Glu Gly Thr Ser Glu Ser Ala Thr Pro Glu Ser Gly Pro
   705 710 715 720
   Gly Ser Glu Pro Ala Thr Ser Gly Ser Glu Thr Pro Gly Thr Ser Glu
   725 730 735
   Ser Ala Thr Pro Glu Ser Gly Pro Gly Ser Pro Ala Gly Ser Pro Thr
   740 745 750
   Ser Thr Glu Glu Gly Ser Pro Ala Gly Ser Pro Thr Ser Thr Glu Glu
   755 760 765
   Gly Thr Ser Thr Glu Pro Ser Glu Gly Ser Ala Pro Gly Thr Ser Glu
   770 775 780
   Ser Ala Thr Pro Glu Ser Gly Pro Gly Thr Ser Glu Ser Ala Thr Pro
   785 790 795 800
   Glu Ser Gly Pro Gly Thr Ser Glu Ser Ala Thr Pro Glu Ser Gly Pro
   805 810 815
   Gly Ser Glu Pro Ala Thr Ser Gly Ser Glu Thr Pro Gly Ser Glu Pro
   820 825 830
   Ala Thr Ser Gly Ser Glu Thr Pro Gly Ser Pro Ala Gly Ser Pro Thr
   835 840 845
   Ser Thr Glu Glu Gly Thr Ser Thr Glu Pro Ser Glu Gly Ser Ala Pro
   850 855 860
   Gly Thr Ser Thr Glu Pro Ser Glu Gly Ser Ala Pro Gly Ser Glu Pro
   865 870 875 880
   Ala Thr Ser Gly Ser Glu Thr Pro Gly Thr Ser Glu Ser Ala Thr Pro
   885 890 895
   Glu Ser Gly Pro Gly Thr Ser Thr Glu Pro Ser Glu Gly Ser Ala Pro
   900 905 910
<210> 53
   <211> 146
   <212> PRT
   <213> Artificial Sequence
<220>
   <221> source
   <223> /note="Description of Artificial Sequence: Synthetic polypeptide"
<400> 53
   Gly Gly Thr Ser Glu Ser Ala Thr Pro Glu Ser Gly Pro Gly Thr Ser
   1 5 10 15
   Thr Glu Pro Ser Glu Gly Ser Ala Pro Gly Thr Ser Thr Glu Pro Ser
               20                  25                  30
   Glu Gly Ser Ala Pro Gly Thr Ser Glu Ser Ala Thr Pro Glu Ser Gly
           35                  40                  45
   Pro Gly Thr Ser Thr Glu Pro Ser Glu Gly Ser Ala Pro Gly Thr Ser
       50                  55                  60
   Thr Glu Pro Ser Glu Gly Ser Ala Pro Gly Thr Ser Glu Ser Ala Thr
   65                  70                  75                  80
   Pro Glu Ser Gly Pro Gly Thr Ser Thr Glu Pro Ser Glu Gly Ser Ala
                   85                  90                  95
   Pro Gly Thr Ser Thr Glu Pro Ser Glu Gly Ser Ala Pro Gly Thr Ser
               100                 105                 110
   Thr Glu Pro Ser Glu Gly Ser Ala Pro Gly Ser Pro Ala Gly Ser Pro
           115                 120                 125
   Thr Ser Thr Glu Glu Gly Thr Ser Thr Glu Pro Ser Glu Gly Ser Ala
       130                 135                 140
   Pro Gly
   145
<210> 54
   <211> 48
   <212> PRT
   <213> Artificial Sequence
<220>
   <221> source
   <223> /note="Description of Artificial Sequence: Synthetic polypeptide"
<400> 54
   Met Ala Glu Pro Ala Gly Ser Pro Thr Ser Thr Glu Glu Gly Thr Pro
   1               5                   10                  15
   Gly Ser Gly Thr Ala Ser Ser Ser Pro Gly Ser Ser Thr Pro Ser Gly
               20                  25                  30
   Ala Thr Gly Ser Pro Gly Ala Ser Pro Gly Thr Ser Ser Thr Gly Ser
           35                  40                  45
<210> 55
   <211> 48
   <212> PRT
   <213> Artificial Sequence
<220>
   <221> source
   <223> /note="Description of Artificial Sequence: Synthetic polypeptide"
<400> 55
   Met Ala Glu Pro Ala Gly Ser Pro Thr Ser Thr Glu Glu Gly Ala Ser
   1               5                   10                  15
   Pro Gly Thr Ser Ser Thr Gly Ser Pro Gly Ser Ser Thr Pro Ser Gly
               20                  25                  30
   Ala Thr Gly Ser Pro Gly Ser Ser Thr Pro Ser Gly Ala Thr Gly Ser
           35                  40                  45
<210> 56
   <211> 8
   <212> PRT
   <213> Artificial Sequence
<220>
   <221> source
   <223> /note="Description of Artificial Sequence: Synthetic peptide"
<400> 56
   Leu Thr Pro Arg Ser Leu Leu Val
   1               5
<210> 57
   <211> 8
   <212> PRT
   <213> Artificial Sequence
<220>
   <221> source
   <223> /note="Description of Artificial Sequence: Synthetic peptide"
<400> 57
   Leu Thr Pro Arg Ser Leu Leu Val
   1               5
<210> 58
   <211> 8
   <212> PRT
   <213> Artificial Sequence
<220>
   <221> source
   <223> /note="Description of Artificial Sequence: Synthetic peptide"
<400> 58
   Lys Leu Thr Arg Val Val Gly Gly
   1               5
<210> 59
   <211> 8
   <212> PRT
   <213> Artificial Sequence
<220>
   <221> source
   <223> /note="Description of Artificial Sequence: Synthetic peptide"
<400> 59
   Thr Met Thr Arg Ile Val Gly Gly
   1               5
<210> 60
   <211> 8
   <212> PRT
   <213> Artificial Sequence
<220>
   <221> source
   <223> /note="Description of Artificial Sequence: Synthetic peptide"
<400> 60
   Ser Pro Phe Arg Ser Thr Gly Gly
   1               5
<210> 61
   <211> 8
   <212> PRT
   <213> Artificial Sequence
<220>
   <221> source
   <223> /note="Description of Artificial Sequence: Synthetic peptide"
<400> 61
   Leu Gln Val Arg Ile Val Gly Gly
   1               5
<210> 62
   <211> 8
   <212> PRT
   <213> Artificial Sequence
<220>
   <221> source
   <223> /note="Description of Artificial Sequence: Synthetic peptide"
<400> 62
   Pro Leu Gly Arg Ile Val Gly Gly
   1               5
<210> 63
   <211> 8
   <212> PRT
   <213> Artificial Sequence
<220>
   <221> source
   <223> /note="Description of Artificial Sequence: Synthetic peptide"
<400> 63
   Ile Glu Gly Arg Thr Val Gly Gly
   1               5
<210> 64
   <211> 8
   <212> PRT
   <213> Artificial Sequence
<220>
   <221> source
   <223> /note="Description of Artificial Sequence: Synthetic peptide"
<400> 64
   Leu Thr Pro Arg Ser Leu Leu Val
   1               5
<210> 65
   <211> 8
   <212> PRT
   <213> Artificial Sequence
<220>
   <221> source
   <223> /note="Description of Artificial Sequence: Synthetic peptide"
<400> 65
   Leu Gly Pro Val Ser Gly Val Pro
   1               5
<210> 66
   <211> 8
   <212> PRT
   <213> Artificial Sequence
<220>
   <221> source
   <223> /note="Description of Artificial Sequence: Synthetic peptide"
<400> 66
   Val Ala Gly Asp Ser Leu Glu Glu
   1               5
<210> 67
   <211> 8
   <212> PRT
   <213> Artificial Sequence
<220>
   <221> source
   <223> /note="Description of Artificial Sequence: Synthetic peptide"
<400> 67
   Gly Pro Ala Gly Leu Gly Gly Ala
   1               5
<210> 68
   <211> 8
   <212> PRT
   <213> Artificial Sequence
<220>
   <221> source
   <223> /note="Description of Artificial Sequence: Synthetic peptide"
<220>
   <221> VARIANT
   <222> (2) .. (2)
   <223> /replace="Ala"
<220>
   <221> misc_feature
   <222> (2)..(2)
   <223> /note="Residue given in the sequence has no preference with respect to the residue in the annotation for said position"
<220>
   <221> MOD_RES
   <222> (3)..(3)
   <223> Any amino acid
<220>
   <221> MOD_RES
   <222> (6) .. (6)
   <223> Any amino acid
<220>
   <221> MOD_RES
   <222> (8)..(8)
   <223> Any amino acid
<400> 68
   Gly Pro Xaa Gly Leu Xaa Gly Xaa
   1               5
<210> 69
   <211> 8
   <212> PRT
   <213> Artificial Sequence
<220>
   <221> source
   <223> /note="Description of Artificial Sequence: Synthetic peptide"
<400> 69
   Gly Pro Ala Gly Leu Arg Gly Ala
   1               5
<210> 70
   <211> 8
   <212> PRT
   <213> Artificial Sequence
<220>
   <221> source
   <223> /note="Description of Artificial Sequence: Synthetic peptide"
<220>
   <221> MOD_RES
   <222> (3)..(3)
   <223> Any amino acid
<220>
   <221> MOD_RES
   <222> (6) .. (6)
   <223> Any amino acid
<220>
   <221> VARIANT
   <222> (7)..(7)
   <223> /replace="Ala"
<220>
   <221> misc_feature
   <222> (7)..(7)
   <223> /note="Residue given in the sequence has no preference with respect to the residue in the annotation for said position"
<220>
   <221> MOD_RES
   <222> (8)..(8)
   <223> Any amino acid
<400> 70
   Gly Pro Xaa Gly Leu Xaa Gly Xaa
   1               5
<210> 71
   <211> 8
   <212> PRT
   <213> Artificial Sequence
<220>
   <221> source
   <223> /note="Description of Artificial Sequence: Synthetic peptide"
<400> 71
   Ala Pro Leu Gly Leu Arg Leu Arg
   1 5
<210> 72
   <211> 8
   <212> PRT
   <213> Artificial Sequence
<220>
   <221> source
   <223> /note="Description of Artificial Sequence: Synthetic peptide"
<400> 72
   Pro Ala Leu Pro Leu Val Ala Gln
   1 5
<210> 73
   <211> 7
   <212> PRT
   <213> Artificial Sequence
<220>
   <221> source
   <223> /note="Description of Artificial Sequence: Synthetic peptide"
<400> 73
   Glu Asn Leu Tyr Phe Gln Gly
   1 5
<210> 74
   <211> 8
   <212> PRT
   <213> Artificial Sequence
<220>
   <221> source
   <223> /note="Description of Artificial Sequence: Synthetic peptide"
<400> 74
   Glu Asn Leu Tyr Phe Gln Gly Ser
   1 5
<210> 75
   <211> 8
   <212> PRT
   <213> Artificial Sequence
<220>
   <221> source
   <223> /note="Description of Artificial Sequence: Synthetic peptide"
<400> 75
   Asp Asp Asp Lys Ile Val Gly Gly
   1 5
<210> 76
   <211> 8
   <212> PRT
   <213> Artificial Sequence
<220>
   <221> source
   <223> /note="Description of Artificial Sequence: Synthetic peptide"
<400> 76
   Asp Asp Asp Lys Ile Val Gly Gly
   1 5
<210> 77
   <211> 8
   <212> PRT
   <213> Artificial Sequence
<220>
   <221> source
   <223> /note="Description of Artificial Sequence: Synthetic peptide"
<400> 77
   Leu Glu Val Leu Phe Gln Gly Pro
   1 5
<210> 78
   <211> 8
   <212> PRT
   <213> Artificial Sequence
<220>
   <221> source
   <223> /note="Description of Artificial Sequence: Synthetic peptide"
<400> 78
   Leu Glu Val Leu Phe Gln Gly Pro
   1 5
<210> 79
   <211> 8
   <212> PRT
   <213> Artificial Sequence
<220>
   <221> source
   <223> /note="Description of Artificial Sequence: Synthetic peptide"
<400> 79
   Leu Pro Lys Thr Gly Ser Glu Ser
   1 5
<210> 80
   <211> 8
   <212> PRT
   <213> Artificial Sequence
<220>
   <221> source
   <223> /note="Description of Artificial Sequence: Synthetic peptide"
<220>
   <221> VARIANT
   <222> (3)..(3)
   <223> /replace="Glu" or "Ala" or "Asp"
<220>
   <221> misc_feature
   <222> (3)..(3)
   <223> /note="Residue given in the sequence has no preference with respect to those in the annotation for said position"
<220>
   <221> MOD_RES
   <222> (6) .. (6)
   <223> Any amino acid
<220>
   <221> VARIANT
   <222> (7)..(7)
   <223> /replace="Lys" or "Ser"
<220>
   <221> misc_feature
   <222> (7)..(7)
   <223> /note="Residue given in the sequence has no preference with respect to those in the annotation for said position"
<400> 80
   Leu Pro Lys Thr Gly Xaa Glu Ser
   1               5
<210> 81
   <211> 47
   <212> PRT
   <213> Artificial Sequence
<220>
   <221> source
   <223> /note="Description of Artificial Sequence: Synthetic polypeptide"
<400> 81
   Ala Glu Pro Ala Gly Ser Pro Thr Ser Thr Glu Glu Gly Thr Pro Gly
   1 5 10 15
   Ser Gly Thr Ala Ser Ser Ser Pro Gly Ser Ser Thr Pro Ser Gly Ala
   20 25 30
   Thr Gly Ser Pro Gly Ala Ser Pro Gly Thr Ser Ser Thr Gly Ser
   35 40 45
<210> 82
   <211> 47
   <212> PRT
   <213> Artificial Sequence
<220>
   <221> source
   <223> /note="Description of Artificial Sequence: Synthetic polypeptide"
<400> 82
   Ala Glu Pro Ala Gly Ser Pro Thr Ser Thr Glu Glu Gly Ala Ser Pro
   1 5 10 15
   Gly Thr Ser Ser Thr Gly Ser Pro Gly Ser Ser Thr Pro Ser Gly Ala
   20 25 30
   Thr Gly Ser Pro Gly Ser Ser Thr Pro Ser Gly Ala Thr Gly Ser
   35 40 45
<210> 83
   <211> 912
   <212> PRT
   <213> Artificial Sequence
<220>
   <221> source
   <223> /note="Description of Artificial Sequence: Synthetic polypeptide"
<400> 83
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
                   165                 170                 175
   Glu Ser Gly Pro Gly Thr Ser Thr Glu Pro Ser Glu Gly Ser Ala Pro
               180                 185                 190
   Gly Thr Ser Thr Glu Pro Ser Glu Gly Ser Ala Pro Gly Ser Pro Ala
           195                 200                 205
   Gly Ser Pro Thr Ser Thr Glu Glu Gly Thr Ser Thr Glu Pro Ser Glu
       210                 215                 220
   Gly Ser Ala Pro Gly Thr Ser Thr Glu Pro Ser Glu Gly Ser Ala Pro
   225                 230                 235                 240
   Gly Thr Ser Glu Ser Ala Thr Pro Glu Ser Gly Pro Gly Thr Ser Thr
                   245                 250                 255
   Glu Pro Ser Glu Gly Ser Ala Pro Gly Thr Ser Glu Ser Ala Thr Pro
               260                 265                 270
   Glu Ser Gly Pro Gly Ser Glu Pro Ala Thr Ser Gly Ser Glu Thr Pro
           275                 280                 285
   Gly Thr Ser Thr Glu Pro Ser Glu Gly Ser Ala Pro Gly Thr Ser Thr
       290                 295                 300
   Glu Pro Ser Glu Gly Ser Ala Pro Gly Thr Ser Glu Ser Ala Thr Pro
   305                 310                 315                 320
   Glu Ser Gly Pro Gly Thr Ser Glu Ser Ala Thr Pro Glu Ser Gly Pro
                   325                 330                 335
   Gly Ser Pro Ala Gly Ser Pro Thr Ser Thr Glu Glu Gly Thr Ser Glu
               340                 345                 350
   Ser Ala Thr Pro Glu Ser Gly Pro Gly Ser Glu Pro Ala Thr Ser Gly
           355                 360                 365
   Ser Glu Thr Pro Gly Thr Ser Glu Ser Ala Thr Pro Glu Ser Gly Pro
       370                 375                 380
   Gly Thr Ser Thr Glu Pro Ser Glu Gly Ser Ala Pro Gly Thr Ser Thr
   385                 390                 395                 400
   Glu Pro Ser Glu Gly Ser Ala Pro Gly Thr Ser Thr Glu Pro Ser Glu
                   405                 410                 415
   Gly Ser Ala Pro Gly Thr Ser Thr Glu Pro Ser Glu Gly Ser Ala Pro
               420                 425                 430
   Gly Thr Ser Thr Glu Pro Ser Glu Gly Ser Ala Pro Gly Thr Ser Thr
           435                 440                 445
   Glu Pro Ser Glu Gly Ser Ala Pro Gly Ser Pro Ala Gly Ser Pro Thr
       450                 455                 460
   Ser Thr Glu Glu Gly Thr Ser Thr Glu Pro Ser Glu Gly Ser Ala Pro
   465                 470                 475                 480
   Gly Thr Ser Glu Ser Ala Thr Pro Glu Ser Gly Pro Gly Ser Glu Pro
                   485                 490                 495
   Ala Thr Ser Gly Ser Glu Thr Pro Gly Thr Ser Glu Ser Ala Thr Pro
               500                 505                 510
   Glu Ser Gly Pro Gly Ser Glu Pro Ala Thr Ser Gly Ser Glu Thr Pro
           515                 520                 525
   Gly Thr Ser Glu Ser Ala Thr Pro Glu Ser Gly Pro Gly Thr Ser Thr
       530                 535                 540
   Glu Pro Ser Glu Gly Ser Ala Pro Gly Thr Ser Glu Ser Ala Thr Pro
   545                 550                 555                 560
   Glu Ser Gly Pro Gly Ser Pro Ala Gly Ser Pro Thr Ser Thr Glu Glu
                   565                 570                 575
   Gly Ser Pro Ala Gly Ser Pro Thr Ser Thr Glu Glu Gly Ser Pro Ala
               580                 585                 590
   Gly Ser Pro Thr Ser Thr Glu Glu Gly Thr Ser Glu Ser Ala Thr Pro
           595                 600                 605
   Glu Ser Gly Pro Gly Thr Ser Thr Glu Pro Ser Glu Gly Ser Ala Pro
       610                 615                 620
   Gly Thr Ser Glu Ser Ala Thr Pro Glu Ser Gly Pro Gly Ser Glu Pro
   625                 630                 635                 640
   Ala Thr Ser Gly Ser Glu Thr Pro Gly Thr Ser Glu Ser Ala Thr Pro
                   645                 650                 655
   Glu Ser Gly Pro Gly Ser Glu Pro Ala Thr Ser Gly Ser Glu Thr Pro
               660                 665                 670
   Gly Thr Ser Glu Ser Ala Thr Pro Glu Ser Gly Pro Gly Thr Ser Thr
           675                 680                 685
   Glu Pro Ser Glu Gly Ser Ala Pro Gly Ser Pro Ala Gly Ser Pro Thr
       690                 695                 700
   Ser Thr Glu Glu Gly Thr Ser Glu Ser Ala Thr Pro Glu Ser Gly Pro
   705                 710                 715                 720
   Gly Ser Glu Pro Ala Thr Ser Gly Ser Glu Thr Pro Gly Thr Ser Glu
                   725                 730                 735
   Ser Ala Thr Pro Glu Ser Gly Pro Gly Ser Pro Ala Gly Ser Pro Thr
               740                 745                 750
   Ser Thr Glu Glu Gly Ser Pro Ala Gly Ser Pro Thr Ser Thr Glu Glu
           755                 760                 765
   Gly Thr Ser Thr Glu Pro Ser Glu Gly Ser Ala Pro Gly Thr Ser Glu
       770                 775                 780
   Ser Ala Thr Pro Glu Ser Gly Pro Gly Thr Ser Glu Ser Ala Thr Pro
   785                 790                 795                 800
   Glu Ser Gly Pro Gly Thr Ser Glu Ser Ala Thr Pro Glu Ser Gly Pro
                   805                 810                 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865 870 875 880
   Ala Thr Ser Gly Ser Glu Thr Pro Gly Thr Ser Glu Ser Ala Thr Pro
   885 890 895
   Glu Ser Gly Pro Gly Thr Ser Thr Glu Pro Ser Glu Gly Ser Ala Pro
   900 905 910
<210> 84
   <211> 913
   <212> PRT
   <213> Artificial Sequence
<220>
   <221> source
   <223> /note="Description of Artificial Sequence: Synthetic polypeptide"
<400> 84
   Ala Glu Pro Ala Gly Ser Pro Thr Ser Thr Glu Glu Gly Thr Pro Gly
   1               5                   10                  15
   Ser Gly Thr Ala Ser Ser Ser Pro Gly Ser Ser Thr Pro Ser Gly Ala
               20                  25                  30
   Thr Gly Ser Pro Gly Ala Ser Pro Gly Thr Ser Ser Thr Gly Ser Pro
           35                  40                  45
   Gly Ser Pro Ala Gly Ser Pro Thr Ser Thr Glu Glu Gly Thr Ser Glu
       50                  55                  60
   Ser Ala Thr Pro Glu Ser Gly Pro Gly Thr Ser Thr Glu Pro Ser Glu
   65                  70                  75                  80
   Gly Ser Ala Pro Gly Ser Pro Ala Gly Ser Pro Thr Ser Thr Glu Glu
                   85                  90                  95
   Gly Thr Ser Thr Glu Pro Ser Glu Gly Ser Ala Pro Gly Thr Ser Thr
               100                 105                 110
   Glu Pro Ser Glu Gly Ser Ala Pro Gly Thr Ser Glu Ser Ala Thr Pro
           115                 120                 125
   Glu Ser Gly Pro Gly Ser Glu Pro Ala Thr Ser Gly Ser Glu Thr Pro
       130                 135                 140
   Gly Ser Glu Pro Ala Thr Ser Gly Ser Glu Thr Pro Gly Ser Pro Ala
   145                 150                 155                 160
   Gly Ser Pro Thr Ser Thr Glu Glu Gly Thr Ser Glu Ser Ala Thr Pro
   165 170 175
   Glu Ser Gly Pro Gly Thr Ser Thr Glu Pro Ser Glu Gly Ser Ala Pro
   180 185 190
   Gly Thr Ser Thr Glu Pro Ser Glu Gly Ser Ala Pro Gly Ser Pro Ala
   195 200 205
   Gly Ser Pro Thr Ser Thr Glu Glu Gly Thr Ser Thr Glu Pro Ser Glu
   210 215 220
   Gly Ser Ala Pro Gly Thr Ser Thr Glu Pro Ser Glu Gly Ser Ala Pro
   225 230 235 240
   Gly Thr Ser Glu Ser Ala Thr Pro Glu Ser Gly Pro Gly Thr Ser Thr
   245 250 255
   Glu Pro Ser Glu Gly Ser Ala Pro Gly Thr Ser Glu Ser Ala Thr Pro
   260 265 270
   Glu Ser Gly Pro Gly Ser Glu Pro Ala Thr Ser Gly Ser Glu Thr Pro
   275 280 285
   Gly Thr Ser Thr Glu Pro Ser Glu Gly Ser Ala Pro Gly Thr Ser Thr
   290 295 300
   Glu Pro Ser Glu Gly Ser Ala Pro Gly Thr Ser Glu Ser Ala Thr Pro
   305 310 315 320
   Glu Ser Gly Pro Gly Thr Ser Glu Ser Ala Thr Pro Glu Ser Gly Pro
   325 330 335
   Gly Ser Pro Ala Gly Ser Pro Thr Ser Thr Glu Glu Gly Thr Ser Glu
   340 345 350
   Ser Ala Thr Pro Glu Ser Gly Pro Gly Ser Glu Pro Ala Thr Ser Gly
   355 360 365
   Ser Glu Thr Pro Gly Thr Ser Glu Ser Ala Thr Pro Glu Ser Gly Pro
   370 375 380
   Gly Thr Ser Thr Glu Pro Ser Glu Gly Ser Ala Pro Gly Thr Ser Thr
   385 390 395 400
   Glu Pro Ser Glu Gly Ser Ala Pro Gly Thr Ser Thr Glu Pro Ser Glu
   405 410 415
   Gly Ser Ala Pro Gly Thr Ser Thr Glu Pro Ser Glu Gly Ser Ala Pro
   420 425 430
   Gly Thr Ser Thr Glu Pro Ser Glu Gly Ser Ala Pro Gly Thr Ser Thr
   435 440 445
   Glu Pro Ser Glu Gly Ser Ala Pro Gly Ser Pro Ala Gly Ser Pro Thr
   450 455 460
   Ser Thr Glu Glu Gly Thr Ser Thr Glu Pro Ser Glu Gly Ser Ala Pro
   465 470 475 480
   Gly Thr Ser Glu Ser Ala Thr Pro Glu Ser Gly Pro Gly Ser Glu Pro
   485 490 495
   Ala Thr Ser Gly Ser Glu Thr Pro Gly Thr Ser Glu Ser Ala Thr Pro
   500 505 510
   Glu Ser Gly Pro Gly Ser Glu Pro Ala Thr Ser Gly Ser Glu Thr Pro
   515 520 525
   Gly Thr Ser Glu Ser Ala Thr Pro Glu Ser Gly Pro Gly Thr Ser Thr
   530 535 540
   Glu Pro Ser Glu Gly Ser Ala Pro Gly Thr Ser Glu Ser Ala Thr Pro
   545 550 555 560
   Glu Ser Gly Pro Gly Ser Pro Ala Gly Ser Pro Thr Ser Thr Glu Glu
   565 570 575
   Gly Ser Pro Ala Gly Ser Pro Thr Ser Thr Glu Glu Gly Ser Pro Ala
   580 585 590
   Gly Ser Pro Thr Ser Thr Glu Glu Gly Thr Ser Glu Ser Ala Thr Pro
   595 600 605
   Glu Ser Gly Pro Gly Thr Ser Thr Glu Pro Ser Glu Gly Ser Ala Pro
   610 615 620
   Gly Thr Ser Glu Ser Ala Thr Pro Glu Ser Gly Pro Gly Ser Glu Pro
   625 630 635 640
   Ala Thr Ser Gly Ser Glu Thr Pro Gly Thr Ser Glu Ser Ala Thr Pro
   645 650 655
   Glu Ser Gly Pro Gly Ser Glu Pro Ala Thr Ser Gly Ser Glu Thr Pro
   660 665 670
   Gly Thr Ser Glu Ser Ala Thr Pro Glu Ser Gly Pro Gly Thr Ser Thr
               675                 680                 685
   Glu Pro Ser Glu Gly Ser Ala Pro Gly Ser Pro Ala Gly Ser Pro Thr
       690                 695                 700
   Ser Thr Glu Glu Gly Thr Ser Glu Ser Ala Thr Pro Glu Ser Gly Pro
   705                 710                 715                 720
   Gly Ser Glu Pro Ala Thr Ser Gly Ser Glu Thr Pro Gly Thr Ser Glu
                   725                 730                 735
   Ser Ala Thr Pro Glu Ser Gly Pro Gly Ser Pro Ala Gly Ser Pro Thr
               740                 745                 750
   Ser Thr Glu Glu Gly Ser Pro Ala Gly Ser Pro Thr Ser Thr Glu Glu
           755                 760                 765
   Gly Thr Ser Thr Glu Pro Ser Glu Gly Ser Ala Pro Gly Thr Ser Glu
       770                 775                 780
   Ser Ala Thr Pro Glu Ser Gly Pro Gly Thr Ser Glu Ser Ala Thr Pro
   785                 790                 795                 800
   Glu Ser Gly Pro Gly Thr Ser Glu Ser Ala Thr Pro Glu Ser Gly Pro
                   805                 810                 815
   Gly Ser Glu Pro Ala Thr Ser Gly Ser Glu Thr Pro Gly Ser Glu Pro
               820                 825                 830
   Ala Thr Ser Gly Ser Glu Thr Pro Gly Ser Pro Ala Gly Ser Pro Thr
           835                 840                 845
   Ser Thr Glu Glu Gly Thr Ser Thr Glu Pro Ser Glu Gly Ser Ala Pro
       850                 855                 860
   Gly Thr Ser Thr Glu Pro Ser Glu Gly Ser Ala Pro Gly Ser Glu Pro
   865                 870                 875                 880
   Ala Thr Ser Gly Ser Glu Thr Pro Gly Thr Ser Glu Ser Ala Thr Pro
                   885                 890                 895
   Glu Ser Gly Pro Gly Thr Ser Thr Glu Pro Ser Glu Gly Ser Ala Pro
               900                 905                 910
   Gly
<210> 85
   <211> 144
   <212> PRT
   <213> Artificial Sequence
<220>
   <221> source
   <223> /note="Description of Artificial Sequence: Synthetic polypeptide"
<400> 85
   Thr Ser Glu Ser Ala Thr Pro Glu Ser Gly Pro Gly Thr Ser Thr Glu
   1               5                   10                  15
   Pro Ser Glu Gly Ser Ala Pro Gly Thr Ser Thr Glu Pro Ser Glu Gly
               20                  25                  30
   Ser Ala Pro Gly Thr Ser Glu Ser Ala Thr Pro Glu Ser Gly Pro Gly
           35                  40                  45
   Thr Ser Thr Glu Pro Ser Glu Gly Ser Ala Pro Gly Thr Ser Thr Glu
       50                  55                  60
   Pro Ser Glu Gly Ser Ala Pro Gly Thr Ser Glu Ser Ala Thr Pro Glu
   65                  70                  75                  80
   Ser Gly Pro Gly Thr Ser Thr Glu Pro Ser Glu Gly Ser Ala Pro Gly
                   85                  90                  95
   Thr Ser Thr Glu Pro Ser Glu Gly Ser Ala Pro Gly Thr Ser Thr Glu
               100                 105                 110
   Pro Ser Glu Gly Ser Ala Pro Gly Ser Pro Ala Gly Ser Pro Thr Ser
           115                 120                 125
   Thr Glu Glu Gly Thr Ser Thr Glu Pro Ser Glu Gly Ser Ala Pro Gly
       130                 135                 140